(19) Europäisches Patentamt
European Patent Office
Office européen des brevets

(11) EP 2 293 056 A2

(12) EUROPEAN PATENT APPLICATION

(43) Date of publication:
**09.03.2011 Bulletin 2011/10**

(51) Int Cl.:
***G01N 27/447*** *(2006.01)*

(21) Application number: **10180277.5**

(22) Date of filing: **19.04.2000**

(84) Designated Contracting States:
**AT BE CH CY DE DK ES FI FR GB GR IE IT LI LU MC NL PT SE**

(30) Priority: **20.04.1999 US 130238 P**
**25.02.2000 US 513395**
**25.02.2000 US 513486**
**25.02.2000 US 513907**

(62) Document number(s) of the earlier application(s) in accordance with Art. 76 EPC:
**00923511.0 / 1 194 768**

(71) Applicant: **Target Discovery, Inc.**
**San Carlos, CA 94070 (US)**

(72) Inventors:
- **Schneider, Luke**
**Half Moon Bay, CA 94019 (US)**
- **Hall, Michael**
**San Carlos, CA 94070 (US)**
- **Petesch, Robert**
**Newark, CA 94560 (US)**
- **Peterson, Jeffrey**
**Foster City, CA 94404 (US)**

(74) Representative: **Walker, Ross Thomson**
**Potts, Kerr & Co.**
**15 Hamilton Square**
**Birkenhead**
**Merseyside CH41 6BR (GB)**

Remarks:
This application was filed on 27-09-2010 as a divisional application to the application mentioned under INID code 62.

# (54) A method for analysing a metabolic pathway

(57) A method for analyzing a metabolic pathway, comprising (a) administering to a subject a known ratio of a labeled substrate and an unlabeled substrate, wherein said labeled substrate labeled with a stable isotope and said unlabeled substrate having a natural abundance of said stable isotope; (b) allowing the labeled substrate and the unlabeled substrate to be at least partially metabolized to form a labeled target metabolite and an unlabeled target metabolite; (c) generating mass spectral data from a plurality of samples from said subject at a plurality of time points; and (d) determining a plurality of relative abundances of said labeled target metabolite to said unlabeled target metabolite from the mass spectral data of at least one of said samples and comparing said plurality of relative abundances to said known ratio of said labeled substrate and said unlabeled substrate.

54
64
74
62
72
52
50
56
66
60
70
58
68
76
78
80
82

FIG. 1



EP 2 293 056 A2

## Description

CROSS-REFERENCES TO RELATED APPLICATIONS

This application claims the benefit of U.S. provisional application 60/130,238, filed April 20, 1999. This application is also related to U.S. provisional application 60/075,715 filed February 24, 1998; copending U.S. patent application number 09/513,486, filed February 25, 2000, entitled "Protein Separation Via Multidimensional Electrophoresis," and having attorney docket number 020444-000200US; copending U.S. patent application number 09/513,395, filed February 25, 2000, entitled "Methods for Protein Sequencing," and having attorney docket number 020444-000300US; copending U.S. application number 09/513,907, filed February, 25, 2000, entitled "Polypeptide Fingerprinting Methods and Bioinformatics Database System," and having attorney docket number 020444-000100US; copending U.S. patent application number __________________, filed April 19, 2000, entitled "Methods for Conducing Metabolic Analyses", and having attorney docket number 020444-000400US; and copending PCT application __________________, filed April 19, 2000, entitled "Labeling of Protein Samples", and having attorney docket number 020444-000500. All of these applications are incorporated by reference in their entirety for all purposes.

FIELD OF THE INTENTION

This invention relates to the fields of protein separation and proteomics, metabolite profiling, bioinformatics, medicine, and computer databases.

BACKGROUND OF THE INVENTION

A goal of genomics research and differential gene expression analysis is to develop correlations between gene expression and particular cellular states (*e.g.,* disease states, particular developmental stages, states resulting from exposure to certain environmental stimuli and states associated with therapeutic treatment). Such correlations have the potential to provide significant insight into the mechanism of disease, cellular development and differentiation, as well as in the identification of new therapeutics, drug targets, and disease markers. Correlations of patterns of gene expression can also be used to provide similar insights into disease and organism metabolism that can be used to speed the development of agricultural products, transgenic species, and for metabolic engineering of organisms to increase bioproduct yields or desirable metabolic activities.

Many functional genomic studies focus on changes in mRNA levels as being indicative of a cellular response to a particular condition or state. Recent research, however, has demonstrated that often there is a poor correlation between gene expression as measured by mRNA levels and actual active gene product formed (*i.e.*, protein encoded by the mRNA). [4] This finding is not surprising since many factors-including differences in translational efficiency, turnover rates, extracellular expression or compartmentalization, and post-translational modification-affect protein levels independently of transcriptional controls. Thus, the evidence indicates that functional genomics is best accomplished by measuring actual protein levels (*i.e.*, utilizing proteomic methods) rather than with nucleic acid based methods. The successful use of proteins for functional genomic analyses, however, requires reproducible quantification and identification of individual proteins expressed in cell or tissue samples.

It is at the protein level that metabolic control is exercised in cells and tissues. Comparison of the levels of protein expression between healthy and diseased tissues, or between pathogenic and nonpathogenic microbial strains, can speed the discovery and development of new drug compounds or agricultural products. Analysis of the protein expression pattern in diseased tissues or in tissues excised from organisms undergoing treatment can also serve as diagnostics of disease states or the efficacy of treatment strategies, as well as provide prognostic information regarding suitable treatment modalities and therapeutic options for individual patients.

Many proteins are expressed at varying levels in different cells. Proteins extracted from tissue or cell samples, using conventional techniques, must first be separated into individual proteins by gel or capillary electrophoresis or affinity techniques, before the individual proteins levels can be compared both within a sample and across samples obtained from different tissue sources. Because of the number of proteins expressed by a cell at any given time, multiple electrophoretic techniques (e.g., isoclectric focussing followed by electroporation through a polyacrylamide gel) are often applied to isolate all the individual proteins contained in a given sample.

Several techniques have been used to quantify the relative amounts of each protein present after the separation, including: staining proteins separated in a polyacrylamide gel with dyes (e.g., Brilliant Blue and Fast Green), with colloidial metals (e.g., gold or silver staining), or by prior labelling of the proteins during cellular synthesis by the addition of radioactive compounds (e.g., with 35S-methionine or 14C-amino acids, or 3H-leucine). Staining techniques yield poorly quantitative results because varying amounts of stain are incorporated into each protein and the stained protein must be resolved against the stained background of the gel or electroblotting substrate. Since radioactive labels are applied only to the proteins prior to separation, they overcome the background problem of staining techniques. However, feeding radioactive compounds to human subjects or handling radioactive materials in an uncontrolled field environment (e.g., crop plants) restricts the usefulness of this approach. Both staining and radiolabelling techniques also require inordinately long times to achieve detection. Staining and destaining of gels is a diffusion limited process requiring hours. Radiolabels must be quantified by exposing the labelled gel to photographic film or a phosphor screen for several hours to days while waiting for the radioactive decay process to produce a quantitative image. Direct infrared spectrophotometric interrogation of the proteins in a gel has also been used previously as a method for providing quantitative protein expression data. However, this quantitative resolution possible from this approach is adversely affected by variations in gel thickness and differential spreading of the protein spot between gels (changing the local concentration). Furthermore, the comparatively low absorption cross-section of proteins in the infrared limits the detection sensitivity. Analysis of the protein expression pattern does not provide sufficient information for many applications.

Several methods have also been proposed for the identification of proteins once they are resolved. The most common methods involve referencing the separation coordinates of individual proteins (e.g., isoelectric point and apparent molecular weight) to those obtained from archived separation coordinate data (e.g., annotated 2-D gel image databases) or control samples, performing a chemilytic or enzymatic digestion of a protein coupled with determination of the mass of the resulting peptide fragments and correlating this peptide mass fingerprint with that predicted to arise from the predicted genetic sequence of a set of known proteins (*see* James, P., M. Quandroni, E. Carafoli, and G. Gonnet, Biochem. Biophys. Res. Commun., 195:58-64 (1993); Yates, J.R., S. Speicher, P.R. Griffin, and T. Hunkapiller, Anal. Biochem., 214:397-408 (1993)), the generation of a partial protein sequence that is compared to the predicted sequences obtained from a genomic database (*see* Mann, M., paper presented at the IBC Proteomics conference, Boston, MA (Nov 10-11, 1997); Wilm, M., A. Shevchenko, T. Houthaeve, S. Breit, L. Schweiger, T. Fotsis and M. Mann, Nature, 379: 466-469 (1996); Chait, B.T, R. Wang, R.C. Beavis and S.B.H. Kent, Science, 262:89-92 (1993)), or combinations of these methods (*see* Mann, M., paper presented at the IBC Proteomics conference, Boston, MA (Nov 10-11, 1997); Wilm, M., A. Shevchenko, T. Houthaeve, S. Breit, L. Schweiger, T. Fotsis and M. Mann, Nature, 379:466-469 (1996); Chait, B.T, R. Wang, R.C. Beavis and S.B.H. Kent, Science, 262:89-92 (1993)). Recent work indicates that proteins can only be unambiguously identified through the determination of a partial sequence, called a protein sequence tag (PST), that allows reference to the theoretical sequences determined from genomic databases (*see* Clauser, K.R., S. C. Hall, D. M. Smith, J.W. Webb, L.E. Andrews, H. M. Tran, L.B. Epstein, and A.L. Burlingame, " Proc. Natl. Acad. Sci. (USA), 92: 5072-5076 (1995); Li, G., M. Walthan, N. L. Anderson, E. Unworth, A. Treston and J. N. Weinstein, Electrophoresis, 18: 391-402 (1997)). However, between 8 to 18 hours is currently required to generate a PST for a single protein sample by conventional techniques, with a substantial fraction of this time devoted to recovery of the protein sample from the separation method in a form suitable for subsequent sequencing (*see* Shevchenko, A., et al., Proc. Natl. Acad. Sci. (USA), 93:14440-14445 (1996); Mark, J., paper presented at the PE/Sciex Seminar Series, Protein Characterization and Proteomics: Automated high throughput technologies for drug discovery, Foster City, CA (March, 1998). This makes the identification of all separated proteins from a tissue a time and cost prohibitive endeavour. This has restricted more widespread use of proteomic methods, despite their advantages for functional genomics and inhabited the development of proteomic databases, analogous to the genome databases now available (e.g., Genbank and the Genome Sequence Database).

Thus, current methods for identifying and quantitating the protein expression patterns ("protein fingerprints") of cells, tissues, and organs are lacking sufficient resolution, precision, and/or sensitivity. The present invention addresses these features lacking in the methods known in the art.

Polypeptide Separation Methods: Capillary Electrophoresis

Two-dimensional (2-D) gel electrophoresis is currently the most widely adopted method for separating individual proteins isolated from cell or tissue samples [5, 6, 7]. Evidence for this is seen in the proliferation (more than 20) of protein gel image databases, such as the Protein-Disease Database maintained by the NIH [8]. These databases provide images of reference 2-D gels to assist in the identification of proteins in gels prepared from various tissues.

Capillary electrophoresis (CE) is a different type of electrophoresis, and involves resolving components in a mixture within a capillary to which an electric field is applied. The capillary used to conduct electrophoresis is filled with an electrolyte and a sample introduced into one end of the capillary using various methods such as hydrodynamic pressure, electroosmotically-induced flow, and electrokinetic transport. The ends of the capillary are then placed in contact with an anode solution and a cathode solution and a voltage applied across the capillary. Positively charged ions are attracted towards the cathode, whereas negatively charged ions are attracted to the anode. Species with the highest mobility travel the fastest through the capillary matrix. However, the order of elution of each species, and even from which end of the capillary a species elates, depends on its apparent mobility. Apparent mobility is the sum of a species electrophoretic mobility in the electrophoretic matrix and the mobility of the electrophoretic matrix itself relative to the capillary. The electrophoretic matrix may be mobilized by hydrodynamic pressure gradients across the capillary or by electroosmotically-induced flow (electrosomotic flow).

A number of different electrophoretic methods exist. Capillary isoelectric focusing (CIEF) involves separating analytes such as proteins within a pH gradient according to their isoelectric point (*i.e.*, the pH at which the analyte has no net charge) of the analytes. A second method, capillary zone Electrophoresis (CZE) fractionates analytes on the basis of their intrinsic charge-to-mass ratio. Capillary gel electrophoresis (CGE) is designed to separate proteins according to their molecular weight. (For reviews of electrophoresis generally, and CIEF and CZE specifically, *see*, *e.g.*, Palmieri, R. and Nolan, J.A., "Protein Capillary Electrophoresis: Theoretical and Experimental Considerations for Methods Development," in CRC Handbook of Capillary Electrophoresis: A Practical Approach, CRC Press, chapter 13, pp. 325-368 (1994) (electrophoresis generally); Kilar, F., "Isoelectric Focusing in Capillaries," in CRC Handbook of Capillary Electrophoresis: A Practical Approach, CRC Press, chapter 4, pp. 325-368 (1994); and McCormick, R.M., "Capillary Zone Electrophoresis of Peptides," in CRC Handbook of Capillary Electrophoresis: A Practical Approach, CRC Press, chapter 12, pp. 287-323 (1994). All of these references are incorporated by reference in their entirety for all purposes).

While 2-D gel electrophoresis is widely practiced, several limitations restrict its utility in functional genomics research. First, because 2-D gels are limited to spatial resolution, it is difficult to resolve the large number of proteins that are expressed in the average cell (1000 to 10,000 proteins). High abundance proteins can distort carrier ampholyte gradients in capillary isoelectric focusing electrophoresis and result in crowding in the gel matrix of size seiving electrophoretic methods (*e.g.*, the second dimension of 2-D gel electrophoresis and CGE), thus causing irreproducibility in the spatial pattern of resolved proteins [20, 21 and 22]. High abundance proteins can also precipitate in a gel and cause streaking of fractionated proteins [20]. Variations in the crosslinking density and electric field strength in cast gels can further distort the spatial pattern of resolved proteins [23, 24]. Another problem is the inability to resolve low abundance proteins neighboring high abundance proteins in a gel because of the high staining background and limited dynamic range of gel staining and imaging techniques [25, 22]. Limitations with staining also make it difficult to obtain reproducible and quantifiable protein concentration values. In some recent experiments, for example, investigators were only able to match 62% of test spots of the spots formed on 37 gels run under similar conditions [21; see also 28, 29]. Additionally, many proteins are not soluble in buffers compatible with acrylamide gels, or fail to enter the gel efficiently because of their high molecular weight [26,27].

Thus, currently used methods of capillary electrophoresis provide significant limitations with regard to their usefulness is providing a detailed protein expression fingerprint of a cell or tissue sample.

Protein Species Identification/ Protein Sequence Tags

In contrast to characterizing proteins on the basis of their electrophoretic mobility or isoelectric point, an approach to identifying the protein species that are expressed in a tissue or cell sample is to obtain partial or complete peptide sequence information from proteins purified from the sample. Needless to say, but this approach is laborious and is of limited sensitivity as it requires extensive and often problematic purification steps to isolate individual protein species to allow for unambiguous sequence determination, and in many cases is simply not feasible for proteins which are not highly abundant and/or are not readily purifiable free from contaminant protein Species.

It is also important that primary amino acid sequence or a partial sequence (i.e., a protein sequence tag, "PST") be determined so that the reason underlying changes in the protein expression pattern related to proteins that appearing at different separation coordinates, can be determined. Proteins may appear at more than one separation coordinate, depending on the degree of post-translational modification exercised on that protein by the cell or tissue. The separation coordinate for a protein may also change due to genetic mutations. Changes in the relative abundance of a protein at any given separation coordinate may also be due to changes in the regulation of gene expression. Only by unambiguously identifying each of the proteins resolved can the reason underlying any variations in protein expression across different samples be deduced.

Several methods have previously been proposed for determining the sequence or a protein sequence tag of separated proteins. These include: sequential rounds of N-terminal or C-terminal labeling followed by liberation and determination of the labeled amino acid, exoproteolytic digestion of the protein one amino acid at a time, endoproteolytic digestion of larger proteins into smaller peptides followed by N- and C-terminal labeling and amino acid determination, and mass spectrometric fragmentation pattern recognition. Sequential labeling and digestion techniques (e.g., Edman chemistry) are time consuming, even when automated, because the process must be repeated through many cycles before a sufficiently large protein sequence tag can be accumulated. Propagation of errors-i.e., either from incomplete labeling on each round, incomplete liberation of the labeled amino acid, or both-also limits the length of protein sequence that can be determined using these techniques. While a more complete protein sequence can be obtained by first using endoproteases to cleave the protein into smaller fragments prior to application of the sequential labeling and digestion chemistry, this also introduces the time and labor intensive step of reseparating and purifying the protein fragments, usually by reapplication of an electrophoretic separation technique. Determining the sequence order of these peptide fragments in the original protein can also present additional problems. Carboxy-terminal methoxy labeling of cyanogen bromide digests has been used to identify the C-terminal peptide fragment from other fragments formed by cyanogen bromide digestion of a larger protein.

Protein Sequence Determination by Mass Spectrometry

Mass spectrometric techniques are increasingly being applied to protein identification because of their speed advantage over the more traditional methods. Electrospray and matrix assisted laser desorption ionization (MALDI) are the most common mass spectrometric techniques applied to protein analysis because they are best able to ionize large, low volatility, molecular species. Two basic strategies have been proposed for the MS identification of proteins after separation: 1) mass profile fingerprinting ('MS fingerprinting')and 2) sequencing of one or more peptide domains by MS/MS ('MS/MS sequencing'). MS fingerprinting is achieved by accurately measuring the masses of several peptides generated by a proteolytic digest of the intact protein and searching a database for a known protein with that peptide mass fingerprint. MS/MS sequencing involves actual determination of one or more PSTs of peptides derived from the protein digest by generation of sequence-specific fragmentation ions in the quadrapole of an MS/MS instrument. Refinements in both of these techniques have also reduced the amount of individual proteins needed to achieve signature detection.

In one approach, a protein is chemilytically (e.g., cyanogen bromide) or enzymatically (e.g., trypsin) digested at sequence specific sites to form peptides. The specificity of the cleavage yields peptides of reproducible masses that can subsequently be determined by MS. The mass spectrometric peptide pattern detected from an individual protein is then compared to a database of similar patterns generated from purified proteins with known sequences or predicted from the theoretical protein sequence based on the expected digestion pattern. The identity of the unknown protein is then inferred to be that of the known protein that best matches its peptide mass fingerprint.

Historically, techniques such as Adman degradation have been extensively used for protein sequencing. However, sequencing by collision-induced dissociation MS methods (MS/MS sequencing) has rapidly evolved and has proved to be faster and require less protein than Edman techniques. MS sequencing is accomplished either by using higher voltages in the ionization zone of the MS to randomly fragment a single peptide isolated from a protein digest, or more typically by tandem MS using collision-induced dissociation in the ion trap (quadrapole). However, the application of CID methods to protein sequencing require that the protein first be chemilytically or enzymatically digested.

Several techniques can be used to select the peptide fragment used for MS/MS sequencing, including accumulation of the parent peptide fragment ion in the quadrapole MS unit, capillary electrophoretic separation coupled to ES-TOF MS detection, or other liquid chromatographic separations. The amino acid sequence of the peptide is deduced from the molecular weight differences observed in the resulting MS fragmentation pattern of the peptide using the published masses associated with individual amino acid residues in the MS, and has been codified into a semi-autonomous peptide sequencing algorithm. In this approach the peptide to be sequenced is typically accumulated in the quadrapole of a mass spectrometer. CID is then accomplished by injecting a neutral collision gas, typically Ar, into this ion trap to force high energy collisions with the peptide ion. Some of these collisions result in cleavage of the peptide backbone and the generation of smaller ions that, by virtue of their different mass to charge ratio, leave the quadrapole and are detected. The majority of the peptide cleavage reactions occur in a relatively few number of ways, resulting in a high abundance of certain types of cleavage ions. The peptide sequence is then deduced from the apparent masses of these high abundance peptide fragments detected.

Mass spectrometry has the additional advantage in that it can be efficiently coupled to electrophoretic separation techniques both with or without endoproteolytic (e.g., trypsin digestion) or chemilytic (e.g., cyanogen bromide) cleavage of the protein into smaller fragments. However, no mass spectrometric technique has previously been described that directly determines the protein sequence or a protein sequence tag of unknown proteins. Furthermore, no MS sequencing technique has previously been described that directly couples to electrophoretic methods used to separate large numbers of proteins from a mixed protein sample.

For example, in the mass spectrum of a 1425.7 Da peptide (HSDAVFTDNYTR) isolated in an MS/MS experiment acquired in positive ion mode, the difference between the full peptide 1425.7 Da and the next largest mass fragment ($y_{11}$, 1288.7 Da) is 137 Da. This corresponds to the expected mass of an N-terminal histidine residue that is cleaved at the amide bond. For this peptide, complete sequencing is possible as a result of the generation of high-abundance fragment ions that correspond to cleavage of the peptide at almost every residue along the peptide backbone. The generation of an essentially complete set of positively-charged fragment ions that include either end of the peptide is a result of the basicity of both the N- and C-terminal residues (H and R, respectively). If a basic residue is located at the N- or C-terminus, especially R, most of the ions produced in the CID spectrum will contain that residue since positive charge is essentially localized at that site. This greatly simplifies the resulting spectrum since these basic sites direct the fragmentation into a limited series of specific daughter ions. Peptides that lack basic residues tend to fragment into a more complex mixture of fragment ions that makes sequence determination more difficult.

Extending this idea, others demonstrated that attaching a hard positive charge to the N-terminus is an effective approach for directing the production of a complete series of N-terminal fragment ions from a parent peptide in CID experiments regardless of the presence of a basic residue at the N-terminus. Theoretically, all fragment ions are produced by charge-remote fragmentation directed by the fixed-charged group. Peptides have now been modified with several classes of fixed-charged groups, including dimethylalkylammonium, substituted pyridinium, quaternary phosphonium, and sulfonium derivatives. The characteristics of the most desirable labels are that they are easily synthesized, increase the ionization efficiency of the peptide, and (most importantly) direct the formation of a specific fragment ion series with minimal unfavorable label fragmentation. The most favorable derivatives that satisfy these criteria are those of the dimethylalkylammonium class with quarternary phosphonium derivatives being only less favorable due to their more difficult synthesis. Substituted pyridinium derivatives are better suited for high-energy CID as opposed to alkylammonium derivatives.

Despite some progress in peptide analysis, protein identification remains a major bottleneck in field of Proteomics, with up to 18 hours being required to generate a protein sequence tag of sufficient length to allow the identification of a single purified protein from its predicted genomic sequence. Unambiguous protein identification is attained by generating a protein sequence tag (PST), which is now preferentially accomplished by collision-induced dissociation in the quadrapole of an MS/MS instrument. Limitations on the ionization efficiency of larger peptides and proteins restrict the intrinsic detection sensitivity of MS techniques and inhibit the use of MS for the identification of low abundance proteins. Limitations on the mass accuracy of time of flight (TOF) detectors can also constrain the usefulness of MS/MS sequencing, requiring that proteins be digested by proteolytic and chemolytic means into more manageable peptides prior to sequencing. Clearly, rapid and cost effective protein sequencing techniques would improve the speed and lower the cost of proteomics research. Finally, the separation agents and buffers used in traditional protein separation techniques are often incompatible with MS identification methods.

<u>Labeling of Protein Samples</u>

The correlation of protein expression levels obtained from healthy and diseased tissue is the basis of proteomics research. Proteins extracted from tissue or cell samples typically must be separated into individual proteins by gel electrophoresis (O'Farrel, P.H., J Biol. Chem., 250:4007 (1975); Hochstrasser, D.F., et al., Anal Biochem., 173:424 (1988);Hühmer, A. F. R., et al., Anal. Chem., 69:29R-57R (1997); Garfin, D.E., Methods in Enzymology, 182:425 (1990)), capillary electrophoresis (Smith, R.D., et al., "Capillary electrophoresis-mass spectrometry," in: CRC Handbook of Capillary Electrophoresis: A Practical Approach, Chp. 8, pgs 185-206 (CRC Press, Boca Raton, FL, 1994); Kilár, F., "Isoelectric focusing in capillaries," in: CRC Handbook of Capillary Electrophoresis: A Practical Approach, Chp. 4, pgs. 95 -109 (CRC Press, Boca Ration, FL, 1994); McCormick, R. M., "Capillary zone electrophoresis of peptides," in CRC Handbook of Capillary Electrophoresis: A Practical Approach, Chp. 12, pgs 287-323 (CRC Press, Boca Raton, FL, 1994); Palmieri, R. and Nolan, J. A., "Protein capillary electrophoresis: theoretical and experimental considerations for methods development," in: CRC Handbook of Capillary Electrophoresis: A Practical Approach, Chp. 13, pgs 325-368 (CRC Press, Boca Raton, FL, 1994)), or affinity techniques (Nelson, R.W., "The use of affinity-interaction mass spectrometry in proteome analysis,"paper presented at the BC Proteomics conference, Coronado, CA (June 11-12, 1998); Young, J., "Ciphergen Biosystems," paper presented at the CHI Genomics Opportunities conference, San Francisco, CA (Feb. 14-15, 1998)), before quantification and comparison of their relative expression levels to those from comparative samples. The most commonly used Proteomics method is 2-D gel electrophoresis using staining and imagining techniques to quantify the protein levels present in the gel (Anderson, N.G. and N.L. Andersen, "Twenty years of two-dimensional electrophoresis: Past, present and future," Electrophoresis, 17:443 (1996)). However, the detection of low abundance proteins (Anderson, L., "Pharmaceutical Proteomics: Targets, mechanisms and function," paper presented at the BC Proteomics conference, Coronado, CA (June 11-12, 1998); McKee, A., "The Yeast Proteome," paper resented at the BC Proteomics conference, Coronado, CA (June 11-12, 1998)) and the reproducibility of protein staining and quantification techniques (Anderson, L., "Pharmaceutical Proteomics: Targets, mechanisms and function," paper presented at the BC Proteomics conference, Coronado, CA (June 11-12, 1998); McKee, A., "The Yeast Proteome," paper resented at the BC Proteomics conference, Coronado, CA (June 11-12, 1998); BioRad Molecular Imager FX and PDQuest 2-D analysis software seminar, presented at the IBC Proteomics conference, Coronado, CA (June 11-12,1998); Franzén, F., et al., Electrophoresis, 18:582 (1997)) have proved suspect.

The development of an automated, quantitative, and reproducible system, capable of analyzing protein expression levels directly from native human tissue samples, is expected to significantly impact the time and costs required to generate comparative protein expression data and potentially improve the quality of these data. The development of a labeled 2-D electrophoresis system would allow more rapid accumulation of protein-disease databases and would further speed the identification of new disease targets.

Despite the 2-D gel problems of gel reproducibility, the importance of proteomics research has already been well established. Steiner and coworkers reported the use of proteomics to understand the toxicology of two preclinical drug candidates in rat liver tissues (see, Steiner, "Proteome methods to profile mechanisms of toxicity" paper presented at the IBC Proteomics conference, Coronado, CA, June 11-12, 1998). Amott recently reported the identification of proteins whose expression appears to be related to hypertrophy in congestive heart failure, although, it has yet to be determined if any of these proteins are suitable drug targets (see, Arnott, "Protein differential display and mass spectrometry in the study of congestive heart failure" paper presented at the IBC Proteomics conference, Coronado, CA, June 11-12, 1998). Witzmann et al. report the use of proteomic studies of bovine testis to better understand the toxicology of 1,3,5-trinitrobenzene and 1,3-dinitrobenzene seen in *in vitro* tissue slices (see, Witzmann, et al., Electrophoresis, 18:642 (1997). Franzén et al. report the use of proteomic analysis to identify markers for malignant human breast tumors (see, Franzén et al., Electrophoresis, 18:582 (1997)).

Proteomics research also requires that proteins resolved in the separation process also be identified. Clauser *et al.*have suggested that proteins can only be unambiguously identified through the determination of PSTs that allow reference to the theoretical sequences determined from genomic databases (see, Clauser, et al., Proc. Natl. Acad. Sci. (USA), 92:5072-5076 (1995)). Li *et al.* appear to have proven this assertion by finding that the reliable identification of individual proteins by MS fingerprinting degenerated as the size of the comparative theoretical peptide mass database increased (see, Li, et al., Electrophoresis 18:391-402 (1997)). Li *et al.* also reported that they were only able to obtain peptide maps for the highest abundance proteins in the gel because of sensitivity limitations of the MS, even though their matrix assisted laser desorption MALDI methodology was demonstrated to improve the detection sensitivity over previously reported methods. Clearly, rapid and cost effective protein sequencing techniques will improve the speed and lower the cost of proteomics research.

Historically, techniques such as Edman degradation have been extensively used for protein sequencing. See, Stark, in: Methods in Enzymology, 25:103-120 (1972); Niall, in: Methods in Enzymology, 27:942-1011 (1973); Gray, in: Methods in Enzymology, 25:121-137 (1972); Schroeder, in: Methods in Enzymology, 25:138-143 (1972); Creighton, Proteins: Structures and Molecular Principles (W. H. Freeman, NY, 1984); Niederwieser, in: Methods in Enzymology, 25:60-99 (1972); and Thiede, et al. FEBS Lett., 357:65-69 (1995). However, sequencing by collision-induced dissociation mass spectrometry (MS) methods (MS/MS sequencing) has rapidly evolved and has proved to be faster and require less protein than Edman techniques. See, Shevchenko, A., et a/., Proc. Natl. Acad. Sci. (USA), 93:14440-14445 (1996); Wilm, et al., Nature, 379:466-469 (1996); Mark, J., "Protein structure and identification with MS/MS," paper presented at the PE/Sciex Seminar Series, Protein Characterization and Proteomics: Automated high throughput technologies for drug discovery, Foster City, CA (March, 1998); and Bieman, Methods in Enzymology, 193:455-479 (1990).

Two basic strategies have been proposed for the MS identification of proteins after their separation from a protein mixture: 1) mass profile fingerprinting ('MS fingerprinting', see James, et al., Biochen. Biophys. Res. Commun., 195:58-64 (1993) and Yates, et al., Anal. Biochem. 214:397-408 (1993)) and 2) sequencing of one or more peptide domains by MS/MS ('MS/MS sequencing', see Wilm, et al., Nature, 379;466-469 (1996); Chait, et al., Science, 262: 89-92 (1993); and Mann, M., paper presented at the IBC Proteomics conference, Boston, MA (November 10-11,1997)). MS fingerprinting is achieved by accurately measuring the masses of several peptides generated by a proteolytic digest of the intact protein and searching a database for a known protein with that peptide mass fingerprint, MS/MS sequencing involves actual determination of one or more PSTs of the protein by generation of sequence-specific fragmentation ions in the quadrapole of an MS/MS instrument.

In view of the increasing demand for proteomics data, methods are needed for the separation and sequencing of complex protein samples. Moreover, new protein labeling methods are needed to further facilitate separation and sequencing of proteins in a complex sample. Surprisingly, the present invention provides new methods for the labeling of protein mixtures. The techniques described herein can speed the process of protein identification and correlation of protein expression to disease conditions or development science.

<u>Metabolic Analysis</u>

One goal in biochemical research is to develop correlations between the presence, absence, concentration, conversion rates, or transport rates of certain molecules within cells, tissues,and particular cell or tissue states (*e.g.*, disease states, particular developmental stages, states resulting from exposure to certain environmental stimuli and states associated with therapeutic treatments). Such correlations have the potential to provide significant insight into the mechanism of disease, cellular development and differentiation, as well as in the identification of new therapeutics, drug targets and/or disease markers.

Genomics based studies are an example of one type of approach taken in such investigations. Typically, functional genomics focuses on the change in mRNA levels as being indicative of a cellular response to a particular condition or state. Recent research, however, has demonstrated that often there is a poor correlation between gene expression as measured by mRNA levels and active gene product formed (*i.e.,* protein encoded by the mRNA), This finding is not particularly surprising since many factors including differences in translational efficiency, turnover rates, extracellular expression or compartmentalization, and post-translational modification affect protein levels independently of transcriptional controls.

Another approach is proteomics which, as the term implies, focuses on the proteins present in various cellular states. The rationale for conducting proteomics investigations is based in part upon the view that certain aspects of cellular biology can be better understood by taking inventory of protein levels rather than nucleic acids levels, particularly given the findings just described that suggest that protein activity often hinges on factors other than the concentration of mRNA encoding the protein.

Instead of focusing exclusively on either nucleic acids or proteins, the current invention takes a different approach and examines the metabolites present in a cell formed through cellular metabolism. Such an approach is termed metomics. More specifically, metomics refers to the study of metabolic fluxes and changes in these fluxes as a function of the physiological state of an organism (or population of cells or tissue). Metomics studies can involve, for example, identifying specific metabolic patterns that cause or result from changes in the physiological state of an organism or cell population. Metomics studies can be correlated to changes in protein and mRNA expression patterns also resulting from changes in the physiological state of an organism or cell population.

Metabolism consists of a complex network of catabolic (energy and precursor producing) and anabolic (biosynthetic) enzymatic pathways that together support the maintenance and growth of the cell. The flow of chemicals through this network of enzymatic reactions varies with the cell cycle, (Ingraham, J. L., et al., Growth of the Bacterial Cell, Sinauer Associates, Sunderland, MA, (1983)) diet, availability of extracellular nutrients, and exposure to cellular stresses (*e.g.*, chemical and biochemical toxins or infectious agents). The major metabolic pathways and factors in their regulation are discussed in any general biochemical text book including, for example, Voet, D. and Voet, J.G., Biochemistry, John Wiley & Sons, New York (1990); Stryer, L., Biochemistry, 2nd ed., W.H. Freeman and Company, San Francisco (1981); and White, A., et al., Principles of Biochemistry, 6th ed., McGraw-Hill Book Company (1978), each of which is incorporated by reference in its entirety.

Because metabolism must be capable of adapting to varying conditions and stimuli, cells have a variety of mechanisms at their disposal to regulate metabolism. For example, certain regulatory mechanisms control the rate at which metabolites enter a cell. Since very few substances are capable of diffusing across a cellular membrane, such regulation typically occurs via one of the active or passive transport mechanisms of a cell.

0In addition to transport control, a number of different mechanisms can function to regulate the activity of an enzyme that is part of a metabolic pathway. For example, a product produced by the enzyme can act via feedback inhibition to regulate the activity of the enzyme. Enzymes can also be regulated by ligands that bind at allosteric sites (*i.e.*, sites other than the active site of the enzyme). It has been suggested that allosteric regulation is important in quick time responses (times less than that required for the induction and synthesis of new proteins, <10 min), as well as in the modulation of enzyme activity to changes in background requirements (feed-back control) (Chock, P. B., et al. Current Topics in Cellular Regulation., 27:3 (1985); Koshland, D. E., et al., Science, 217:220 (1982); Stadtman, E. R. and Chock, P. B., Current topics in Cellular Regulation, 13:53 (1978)). Allosteric regulation is the primary method used by bacteria to sense their environment, both by activity modulation of already synthesized proteins and by eliciting new protein synthesis via control of RNA polymerase promoter and repressor proteins (Monod, J., et al., J. Mol. Biol., 6:306 (1963)). Allosteric regulation can be associated with multimeric proteins (several subunits working in a concerted fashion) and/or within regulatory cascades in order to: (1) provide more sites for different regulatory ligands to affect activity, (2) amplify the rate of response, (3) amplify the magnitude of response, and/or (4) amplify the sensitivity of response (Chock, P. B., et al., Current Topics in Cellular Regulation., 27:3 (1985); Koshland, D. E., et al., Science, 217:220 (1982); Stadtman, E. R. and Chock, P. B., Current Topics in Cellular Regulation, 13:53 (1978)),

Expression regulation constitutes another metabolic regulatory mechanism. Concerted sets of genes, encoding small numbers of proteins, are often organized under the same transcriptional control sequence called an operon. However, where the necessary adaptive changes entail the induction of large numbers of proteins, many such operons can be linked in regulons. For example, in *E. coli* the following stimuli induce the number of proteins indicated in parentheses: (a) heat shock (17 proteins), (b) nitrogen starvation (≥ 5 proteins), (c) phosphate starvation (≥ 82 proteins), (d) osmotic stress (≥ 12 proteins), and (e) SOS response (17 proteins) (see, Neidhardt, F. C., in: Escherichia coli and Salmonella typhimurium: cellular and molecular biology, F. C. Neidhardt et al. (eds.), pg. 3, Amer Soc Microbiology, Washington, DC., (1987); Neidhardt, F. C. and Van Bogelen, R. A., in; Escherichia coli and Salmonella typhimurium Cellular and Molecular Biology., F. C. Neidhardt (ed.)., pg 1334, American Society of Microbiology, Washington, D. C., (1987); Magasanik, B. and Neidhardt, F. C., in Escherichia coli and Salmonella typhimurium Cellular and Molecular Biology., F. C. Neidhardt (ed.), pg 1318, American Society of Microbiology, Washington, D. C., (1987); (VanBogelen, R. A., et al., Electrophoresis, 11:1131 (1990)); Wanner, B. L., in: Escherichia coli and Salmonella typhimurium Cellular and Molecular Biology, F. C. Neidhardt (ed.), pg 1326, American Society of Microbiology, Washington, D. C., (1987)); (Christman, M. F. et. al, Cell, 14:753 (1985); and Walker, G. C., in Escherichia coli and Salmonella typhimurium Cellular and Molecular Biology, F. C. Neidhardt (ed.), pg 1346, American Society of Microbiology, Washington, D. C., (1987)). Thus, regulons enable cells to regulate genes that need to respond occasionally in a concerted fashion to a particular stimulus, but that at other times need to be independently responsive to individual controls (Neidhardt, F. C., in: Escherichia coli and Salmonella typhimurium: cellular and molecular biology, F. C. Neidhardt et al. (eds.), pg. 3, Amer Soc Microbiology, Washington, DC., (1987)).

Degradation is another regulatory mechanism for controlling metabolism. Most proteins are very stable, at least under conditions of balanced growth, probably because the cell pays such a high price to make them. However, several researchers have observed a limited class of cellular proteins (10 to 3 0 % of the total protein present during exponential growth in bacteria) that is unstable (exhibit half-lives of 60 min or less). Proteins within the class appear to be turned over quickly within 10 hours of any growth down shift, and during exponential growth (Nath, K. and Koch, A. L., J. Biol, Chem., 246:6956 (1971); St. John, A. C. and Goldberg, A. L., J. Bacteriol., 143:1223 (1980)). At least some of these labile proteins, during energy and nutrient down-shifts, are proteins of the protein synthesizing system (*e.g.,* ribosomal proteins) (Davis, B. D., et a/., J. Bacteriol., 166:439 (1986)); Ingraham, J. L., et al., Growth of the Bacterial Cell, Sinauer Associates, Sunderland, MA, (1983); Maruyama, H. B. and Okamura, S., J. Bacteriol., 110:442 (1972)). This conclusion is drawn from the observations that the apparent rate of protein synthesis per unit of protein synthesizing proteins decreases at low growth rates, but the time required for the initial synthesis of inducible enzymes remains constant at all growth rates (Ingraham, J. L., et al., Growth of the Bacterial Cell, Sinauer Associates, Sunderland, MA, (1983)).

Given the interrelatedness between different cell states and metabolism and the fact that the focus of metomics differs from genomics and proteomics, the present invention utilizes metomic studies to gain new insight into the correlation between cellular states and the biomolecules within the cell.

Computer Database and Bioinformatics

The methods described for identifying and/or quantitating the relative and/or absolute abundance of a variety of molecular and macromolecular species from a biological sample provide a superabundance of information which can be correlated with pathological conditions, predospositions to disease, drug testing, therapeutic monitoring, gene-disease causal linkages, identification of correlates of immunity and physiological status, among others. As the massive amounts of raw data generated by these methods are poorly suited for manual review and analysis without prior data processing using high-speed computers, several methods for indexing and retrieving biomolecular information have been proposed. U.S. Patents 6,023,659 and 5,966,712 disclose a relational database system for storing biomolecular sequence information in a manner that allows sequences to be catalogued and searched according to one or more protein function hierarchies. U.S. Patent 5,953,727 discloses a relational database having sequence records containing information in a format that allows a collection of partial-length DNA sequences to be catalogued and searched according to association with one or more sequencing projects for obtaining full-length sequences from the collection of partial length sequences. U.S. Patent 5,706,498 discloses a gene database retrieval system for making a retrieval of a gene sequence similar to a sequence data item in a gene database based on the degree of similarity between a key sequence and a target sequence. U.S. Patent 5,538,897 discloses a method using mass spectroscopy fragmentation patterns of peptides to identify amino acid sequences in computer databases by comparison of predicted mass spectra with experimentally-derived mass spectra using a closeness-of-fit measure. U.S. Patent 5,926,818 discloses a multi-dimensional database comprising a functionality for multi-dimensional data analysis described as on-line analytical processing (OLAP), which entials the consolidation of projected and actual data according to more than one consolidation path or dimension. U.S. Patent 5,295,261 reports a hybrid database structure in which the fields of each database record are divided into two classes, navigational and informational data, with navigational fields stored in a hierarchical topological map which can be viewed as a tree structure or as the merger of two or more such tree structures.

The aforementioned database art fails to provide a complete solution to the need to integrate, analyze, and catalogue in retrievable form, the wealth of potentially useful information obtained by the analytical methods disclosed herein and to provide correlations and pathognomonic linkages between such information and medical conditions and the like.

Applications of Protein Expression Datasets

Although the limited usefulness of existing protein expression profiling techniques have yielded fairly small and incomplete datasets of protein expression information, the art has been considering theoretical uses of higher resolution protein expression datasets, should they become available in view of new or improved techniques.

If high-resolution, high-sensitivity protein expression profiling methods and datasets were to become available to the art, significant progress in the areas of diagnostics, therapeutics, drug development, biosensor development, and other related areas would be possible. For example, multiple disease markers could be identified and utilized for better confirmation of a disease condition or stage (see US Patent numbers 5, 672,480; 5,599,677; 5,939,533; and 5,710,007). Subcellular toxicological information could be generated to better direct drug structure and activity correlations (see Anderson, L., "Pharmaceutical Proteomics: Targets, Mechanism, and Function," paper presented at the IBC Proteomics conference, Coronado, CA (June 11-12, 1998). Subcellular toxicological information can also be utilized in a biological sensor device to predict the likely toxicological effect of chemical exposures and likely tollerable exposure thresholds (see US Patent number 5,811,231).

The present invention provides compositions, methods, apparatus, and computer-based databasing systems for high-throughput, high-resolution, and sensitive protein expression profiling from samples containing a plurality of polypeptide species, such as for example cells, tissues, and organs of bacteria, plants, and animals, and related aspects and uses thereof.

The literature citations discussed herein are provided solely for their disclosure prior to the filing date of the present application. Nothing herein is to be construed as an admission that the inventors are not entitled to antedate such disclosure by virtue of prior invention.

## SUMMARY OF THE INVENTION

The present invention provides electrophoretic methods and devices for separating biological macromolecules (including polypeptides), methods for determining the partial or complete sequence of a polypeptide using mass spectroscopy, methods combining electrophoretic methods with polypeptide sequencing by mass spectroscopy, methods using the above to generate protein expression fingerprint datasets from a sample or a plurality of samples, and computer-based database query and retrieval systems for utilizing said protein expression fingerprint datasets for various uses, including but not limited to diagnostics, therapeutics, drug discovery, drug development, environmental monitoring by bioassay, toxin quantitation, biosensor development, gene therapy, pharmacological monitoring, illicit drug testing, transgenics, metabolic engineering, and related uses described herein or evident to the ordinarily-skilled artisan in view of the present teaching of the specification. The invention also provides the use of each of these methods, apparatuses, compositions, and computerized database query and retrieval systems.

In an aspect, the invention provides a method for separating a polypeptide species from a sample solution containing a plurality of polypeptide species and identifying said polypeptide species, the method comprising electrophoresing said sample solution containing a plurality of polypeptide species in a capillary electrophoresis device to separate and elute polypeptide species and thereby resolving said protein species based on at least one first biophysical parameter which discriminates protein species; and obtaining, by mass spectrographic fragmentation of eluted polypeptide species, a polypeptide sequence tag ("PST") identifying at least one resolved protein species. In a variation of the method, at least two capillary electrophoresis methods are used sequentially prior to mass spectrographic fragmentation of one or more eluted polypeptide specie, In a variation of the method, a suitable mass spectrometry label is covalently attached to polypeptide species prior to mass spectrographic fragmentation. In a variation of the method the PST comprises at least 2, and preferably 3 or 4 amino acid residues of the carboxy and/or amino terminal sequence of the eluted polypeptide species. In an embodiment of the method, at least 75 percent of polypeptide species present in the sample solution are separated and identified by PST determination. In an embodiment of the method, at least 5,000 unique polypeptide species present in the sample solution are separated and identified by PST determination; preferably at least 7,500 or more unique polypeptide species can be separated and identified in this method. In an embodiment of the method the polypeptide species in the sample solution are naturally-occurring polypeptide obtained from a sample of a tissue, organ, or cell population.

In an aspect, the invention provides a method of obtaining a protein expression profile from a sample containing a cell population or a protein containing extract thereof, the method comprising: electrophoresing in a first capillary electrophoresis apparatus a solution containing a plurality of protein species obtained from a cell population and thereby resolving said protein species based on at least one first biophysical parameter which discriminates protein species, eluting fractions from said first electrophoresis apparatus and electrophoresing said fractions, separately, in a second capillary electrophoresis apparatus and thereby resolving said protein species based on at least one second biophysical parameter which discriminates protein species, and eluting the protein species and identifying the PSTs of a plurality of protein species from the sample by mass spectroscopy fragmentation. In an embodiment, at least 1,000 resolved proteins from the sample are identified by PST determination; in an embodiment at least 5,000 to 7,500 or more resolved proteins from the sample are identified by PST determination. In a variation, two samples are employed, a first sample from a standard (control or normal) cell population and a second sample from a test cell population; test cell populations can be, for example and not limitation, cells of a different histological type than the standard cell population, pathological cells of the same histological type as the standard cells, treated cells that have been exposed to a toxicological or pharmacological agent but which are of the same histological type as the standard cells, cells of a different passage level or age or replicative potential than the standard cells, or any other variation apparent to those skilled in the art seeking to ascertain protein expression profile differences between a first cell sample and a second cell sample. In an embodiment the test cell population is a biopsy of a putative neoplastic lesion and the standard cell population is a biopsy of surrounding apparently non-neoplastic tissue of then same histological origin, both obtained from a human patient, animal, or plant (e.g., crown gall tumor).

The present invention provides a variety of electrophoretic methods and apparatus for separating mixtures of proteins. The methods involve conducting multiple capillary electrophoresis methods in series, wherein samples for each method other than the initial method contain only a subset of the proteins from the preceding step (e.g., from fractions containing resolved protein from the preceding method). By using a variety of techniques to control elution during electrophoresis, the methods are capable of resolving proteins in even complex mixtures such as obtained from tissues and native cells. Utilizing various labeling schemes and detection methods, certain methods can provide quantitative information on the amount of each of the separated proteins. Such information, can be used in the development of protein databases in which proteins expressed under certain conditions are characterized and catalogued. Comparative studies to identify proteins that are differentially expressed between different types of cells or tissues can also be conducted with the methods of the present invention. The methods can also be used in diagnostic, structure activity and metabolic engineering studies.

In general, the methods involve performing a plurality of electrophoretic methods in series. Each method in the series includes electrophoresing a sample containing multiple proteins to obtain a plurality of resolved proteins. The sample that is electrophoresed contains only a subset of the plurality of resolved proteins from the immediately preceding method in the series (except the first method of the series in which the sample is the initial sample that contains all the proteins). The resolved proteins from the final electrophoretic method are then detected using various techniques,

The electrophoretic methods typically are capillary electrophoresis methods, such as capillary isoelectric focusing electrophoresis (CIEF), capillary zone electrophoresis (CZE) and capillary gel electrophoresis (CGE), although the methods are amenable to other capillary electrophoresis methods as well. The particular order of the methods can vary. Typically, the methods utilize combinations of Electrophoretic methods which separate proteins on the basis of different characteristics (*e.g.*, size, charge, isoelectric point).

In certain methods, the proteins are labeled so that the resolved proteins are more easily detected and to increase the signal-to-noise ratio. Labeling also enables certain methods to be conducted such that the resolved proteins obtained from the final electrophoretic method are quantitated. Quantitation allows the relative abundance of proteins within a sample, or within different samples, to be determined, In certain methods, the time at which proteins are labeled is selected to precede electrophoresis by capillary zone electrophoresis. By selectively labeling certain residues, resolution of proteins during capillary zone electrophoresis can be increased.

Resolution, quantitation and reproducibility are enhanced by utilizing a variety of techniques to control elution of proteins during an electrophoretic method. The particular elution technique employed depends in part upon the particular electrophoretic method. However, in general, hydrodynamic, salt mobilization, pH mobilization and electroosmotic flow are utilized to controllably elute resolved proteins at the end of each electrophoretic separation.

Some methods provide for additional analysis after the electrophoretic separation. The type of analysis can vary and include, for example, infra-red spectroscopy, nuclear magnetic resonance spectroscopy, UV/VIS spectroscopy and complete or partial sequencing. In certain methods, proteins in the final fractions are further analyzed by mass spectroscopy to determine at least a partial sequence for each of the resolved proteins (*i.e.,* to determine a protein sequence tag).

Thus, certain other methods involve performing one or more capillary electrophoretic methods, each of the one or more methods involving: (i) electrophoresing a sample containing multiple proteins within an electrophoretic medium contained within a capillary, and withdrawing and collecting multiple fractions, each traction containing proteins resolved during the electrophoresing step. Each method in the series is conducted with a sample from a fraction collected in the preceding electrophoretic method, except the first electrophoretic method which is conducted with a sample containing the original mixture of proteins. Prior to conducting the last electrophoretic method, either the proteins in the initial sample are labeled (i.e., labeling precedes all the electrophoretic separations) or by labeling proteins contained in fraction collected prior to the last electrophoretic method. The final electrophoretic method is performed, and resolved protein within, or withdrawn from, the capillary utilized to conduct the final method is detected with a detector. Hence, the detector is adapted to detect resolved protein within the capillary used in the final method or is connected in line with the capillary to detect resolved proteins as they elute from the capillary. In some instances, the detected proteins are quantitated and further analyzed by mass spectroscopy to determine the relative abundance and to establish a protein sequence tag for each resolved protein.

In one aspect, the present invention provides a method for sequencing a portion of a protein, comprising:

(a) contacting a protein with a C-terminus or N-terminus labeling moiety to covalently attach a label to the C- or N-terminus of the protein and form a labeled protein; and
(b) analyzing the labeled protein using a mass spectrometric fragmentation method to determine the sequence of at least the two C-terminus or two N-terminus resides.

In one group of embodiments, the method further comprises:

(c) identifying the protein by using the sequence of the at least two C-terminus or two N-terminus residues to search predicted protein sequences from a database of gene sequence data.

In a variation, the method further comprises:

(d) further indentifying the protein by using one or more of the separation coordinates (i.e., approximate values of the biophysical parameters used to separate the protein prior to sequencing), for example, the apparent molecular weight, isoelectric point, or Electrophoretic mobility.

In another variation, the method further comprises:

(e) further identifying the protein by using other known biological or measurable biophysical parameters of the protein (e.g., cell or tissue type extracted from, subcellular localization, the total or partial amino acid composition, the masses of any peptides resulting from chemilytic or enzymatic digestion).

In a variation, the method further comprises assisted fragmentation of the labeled protein in the mass spectrometer through the use of reactive collision glasses. Illustrative reactive gases may include hydrazine, cyanogen bromide, hydrogen peroxide, ozone, and peracetic acid. Other similar reactive gases will be obvious to those skilled in the art.

In another variation, the method further comprises assisted fragmentation of the labeled protein in the mass spectrometer through the injection of high energy materials in the ionization zone. High energy materials may include transient compounds formed in a plasma or corona discharge, high energy electrons from a beta emitter or electron beam, high energy photons from a laser or high intensity light source of a minimum wavelenght of 560 nm. Other high energy materials will be obvious to those skilled in the art.

In another aspect, the present invention provides a method for sequencing a portion of a protein in a protein mixture, the method comprising:

(a) contacting the protein mixture with a C-terminus or N-terminus labeling moiety to covalently attach a label to the C- or N-terminus of the protein and form a labeled protein mixture;
(b) separating individual labeled proteins in the labeled protein mixture;
and
(c) analyzing the labeled proteins from step (b) by a mass spectrometric method to determine the sequence of at least two C-terminus or two N-terminus residues.

In one group of embodiments, the method further comprises:

(d) identifying the protein by using the sequence of at least two C-terminus or two N-terminus residues in combination with a separation coordinate of the labeled protein and the protein terminus location of the sequence to search predicted protein sequences from database of gene sequence data.

In each of the methods above, the use of nonproteolytic protein sequencing by in-source fragmentation provides advantages over conventional MS/MS sequencing approaches. One particular advantage is time savings due to elimination of protein, digestion steps and elimination of the need to accumulate low volatility peptide ions in the quadrapole. Another advantage is that fewer sequence ambiguities result due to the improved absolute mass accuracy gained by working at the low end of the mass spectrum. Yet another advantage is that better ionization efficiency and corresponding detection sensitivity result from using more energetic ionization conditions and adding one or more charged groups on the labelled fragments. A charged group consisting of a "hard" charge, that is a permanently ionized group such as tetraalkyl- or tetraaryl-ammonium, tetraalkyl-or tetraaryl-phoshonium, N-substituted pyridinium, or tetraalkyl- or tetraaryl-borate species. A charged group further consisting of a "soft" charge, that is an ionizable group which accepts or donates a proton to become ionized, such as carboxylate, phosphonate, sulfonate, alkyl ammonium, pyridinium species. This method provides a contiguous protein sequence tag (PST) that can be used both for unambiguous protein identification by query of a computer database containing genomic sequence information or mRNA sequence information to establish naturally-occurring encoding sequences corresponding to the PST or to generate an N- or C-terminal nucleic acid probe useful for isolating the corresponding cDNA from native cell or tissue samples by polymerase chain reaction amplification or nucleic acid hybridization techniques.

The invention further provides the identification and method of use of chemical labels suitable for enhanced quantitation of the proteins upon Electrophoretic separation and subsequent sequencing of said proteins. In one embodiment a single chemical label contains groups that: (i) react with primary amino or carboxylic acid functionalities on the protein, including the N-terminus and C-terminus, (ii) enhance detection sensitivity, and (iii) provide a unique mass signature for the N- or C-terminal labeled peptide fragments generated during fragmentation in a mass spectrometer. In a variation, the label may consist of a mixture of isotopically distinct labels, such that the unique mass signature consists of two or more peaks for each peptide fragment that are separated by more than one amu at a single charge state in the mass spectrum. In another variation, the unique mass signature component and the detection enhancement component may be one and the same. In another embodiment, the chemical label may be modified by partial cleavage and/or addition subsequent to its use for protein quantitation and prior to its use for protein sequencing. In one variation, label addition or cleavage is conducted in solution during withdrawl and transport between the last capillary separation step and injection into the mass spectrometer. In another variation, label addition or cleavage is conducted in the gas phase during ionization in the mass spectrometer.

The invention further provides a method incorporating volatile buffers and surfactants in the final capillary Electrophoretic method to facilitate direct coupling of the separation and mass spectrometric detection methods. A volatile buffer is a salt composed of an anion and cation that readily accept or give up a proton to for a volatile organic compound that negligably interfere with the ionization of proteins or peptides in the mass spectrometer. Illustrative examples include ammonium acetate, ammonium carbonate or bicarbonate, ammonium N-morpholinoethanesulfonate, triethylammonium acetate, pyridium acetate, and pryidium N-morpholinoethanesulfonate. Illustrative examples of volatile surfactants include ammonium, pyridinium, tetramethylammonium, and trimethyl ammonium salts of dodecylsulfate and partially fluorinated or perl$_1$uorinated carboxylic, sulfonic, or phosphonic acids of aliphatic hydrocarbons with at least 5 carbon atoms. Many other examples will be evident to those skilled in the art.

The present invention overcomes many of the difficulties associated with current MS-based protein sequencing technologies, including, for example, ionization inefficiency and inaccuracies in fragment mass. Because the methods of the invention preferably eliminate the need for proteolytic or chemolytic digestion of the protein, the present methods provide protein sequencing times that are significantly reduced from the times obtainable using prior methods. Moreover, because the proteins being sequenced are highly fragmented using the present methods, the ionization efficiency and the volatility of the resulting fragments are higher than those of the parent protein, thus leading to a detection sensitivity that is improved over prior methods.

The present invention provides a method of labeling a plurality of different proteins in a protein sample, the method comprising contacting the protein sample with a labeling agent comprising a unique ion mass signature component, a quantitative detection component and a reactive functional group to covalently attach a label to at least a portion of the plurality of different proteins. Preferably, the protein sample comprises at least five, more preferably at least 10, more preferably at least 50 and still more preferably at least 100 different proteins. The protein sample used herein is preferably from a biological sample (e.g., cells, tissues, fluids and organs of bacteria, plants, animals, and humans).

The labeling agents used herein have a unique ion mass signature component, a quantitative detection component and a reactive functional group. Preferred quantitative detection components are selected from radioisotopes, fluorescent residues and chromaphores. Other detection enhancement components are groups that impart a positively charged or negatively charged ionic species under fragmentation conditions in a mass spectrometer ionization chamber. Suitable groups include quaternary ammonium, quaternary phosphonium and quaternary aryl and alkyl borate groups. Preferred reactive functional groups are selected from functional groups reactive to primary amines and functional groups reactive to carboxylic acids. Suitable amine reactive groups include N-hydroxysuccinimide esters and isothiocyanates. Suitable carboxylic acid reactive groups include primary amines coupled through carbodiimide chemistries and anhydride chemistries. Preferred unique ion mass signature components are those groups that impart a mass to a protein fragment that does not match a residue mass for any of the 20 natural amino acids. Further preferred unique ion mass signature components are those that impart a mass to a protein fragment of from about 100 amu to about 700 amu. Still other preferred unique ion mass signature components are those that incorporate a ratio of stable isotopes into the labeling agent, preferably stable isotopes such as $^{2}H$, $^{13}C$, $^{15}N$ and $^{37}Cl$. More preferably, the number of stable isotopes incorporated into the label is sufficient to impart a 5 to 20 atomic mass unit difference between the isotopically-enriched and isotopically-depleted forms of the label. Most preferably, the ratio of isotopically-enriched and isotopically-depleted forms of the label are about equimolar.

The present invention provides apparatus and methods that have utility in purifying and detecting metabolites of interest. The purifying and detection methods enable one to determine how various parameters for metabolites of interest (e.g., metabolite concentration and/or flux) vary as a function of different cellular states or exposure to different stimuli. Thus, the methods can be used to screen for metabolites that are correlated with particular cellular states or stimuli. Such information can be used to develop metabolic "fingerprints" or "profiles" that are characteristic of different cellular states and/or responses to particular stimuli. The information can also be used to develop metomics databases. Once correlations, have been established, certain methods of the invention can be utilized to screen for particular states. For example, some methods screen individuals to identify those having, or at risk, for a particular disease based upon similarities between their metabolic profile and that of diseased and/or healthy individuals.

More specifically, the invention includes various separation methods. Certain methods involve performing a plurality of capillary electrophoresis methods in series. Each method in the series includes electrophoresing a sample containing multiple metabolites and potentially one or more target analytes of interest so that a plurality of resolved metabolites are obtained. The sample electrophoresed in each method contains only a subset of the plurality of resolved metabolites from the immediately preceding method in the series, except the first method of the series in which the sample is the initial sample. Fractions containing resolved metabolites from the final electrophoretic method are analyzed to detect the presence of the target analytes. The capillary electrophoresis methods within the series are selected from the group consisting of capillary isoelectric focusing electrophoresis, capillary zone electrophoresis and capillary gel electrophoresis.

In certain aspects, the invention provides various methods for analyzing metabolic pathways. Certain methods involve administering a substrate labeled with a stable isotope to a subject, the relative isotopic abundance of the isotope in the substrate being known prior to administering the substrate. The subject is then allowed sufficient time to at least partially metabolize the labeled substrate to form one or more target metabolites. The abundance of the isotope in a plurality of target analytes in a sample taken from the subject is then determined so that a value for the flux of each target analytes can be ascertained. The multiple target analytes for which a flux value is determined are either the substrate and/or one or more target metabolites. The abundance of the isotope in the target analytes is determined using an analyzer capable of determining the ratio of the isotopically enriched isotope to the more abundant isotope (*e.g.,* $^{12}C/^{13}C$, $^{14}N/^{15}N$, $^{16}O/^{18}O$ and $^{34}S/^{32}S$). Examples of such analyzers include mass spectrometers, infrared spectrometers and nuclear magnetic resonance spectrormeters.

Prior to determining the abundance of the isotope in the target analytes and corresponding flux values, typically the target analytes are at least partially separated from other components in the sample. Generality this is accomplished by performing a plurality of electrophoretic separation methods in series, such that samples from fractions obtained after one method are used in a subsequent electrophoretic method. The actual electrophoretic methods employed can vary, but typically include capillary isoelectric focusing electrophoresis, capillary zone electrophoresis and capillary gel electrophoresis. In some instances, separation and elution conditions of the electrophoretic methods are controlled so that separate fractions for one or more classes ofmetabolites (*e.g.*, proteins, polysaccharides, carbohydrates, nucleic acids, amino acids, nucleotides, nucleosides, fats, fatty acids, and organic acids) are obtained. This simplifies the analysis because one can simply analyze those fractions containing the class of components to which the target analyte belong.

The invention also provides analytic methods for analyzing metabolic pathways in which samples from a subject have been previously obtained. In such instances, certain methods involve separating at least partially a plurality of target analytes from other components contained in the sample obtained from the subject. The target analytes comprise a substrate labeled with a stable isotope and/or one or more target metabolites resulting from the metabolism of the substrate by the subject. A flux value for each target analyte is determined from knowledge of the isotopic abundance in the substrate prior to it being administered to the subject and by determining the abundance of the isotope in the target analytes.

Methods for screening metabolites to identify those correlated with various cellular states (e.g., certain diseases) are also included in the invention. Certain screening methods include administering a substrate labeled with a stable isotope to a test subject and a control subject, the relative isotopic abundance of the isotope in the substrate being known and the test subject having a disease under investigation. The labeled substrate is allowed to be at least partially metabolized by the test subject and control subject to form one or more target metabolites. The conditions under which the administering and allowing steps are performed are controlled so that they are the same for the test and control subject. A sample is obtained from the test and control subject and the relative abundance of the isotope in the target analyes determined to obtain a value for the flux of each target analyte. The flux values for the test and control subject are compared, a difference in the flux value for a target analyte in the test subject and corresponding flux value for the control subject indicating that such analyte is potentially correlated with the disease being studied.

When a sample has been previously acquired, certain screening methods involve analyzing a sample from a test subject having a disease, the sample comprising a substrate labeled with a stable isotope administered to the test subject and/or one or more target metabolites resulting from metabolism of the substrate by the test subject. The relative isotopic abundance of the isotope in the substrate is known at the time of administration, and the analyzing step includes determining the isotopic abundance of the isotope in a plurality of target analyte in the sample to determine a value for the flux of each target analytes. Flux values for the target analytes in the test subject are compared with flux values for a control subject, a difference in a flux value indicating that such analyte is correlated with the disease.

In another aspect, the invention includes methods for screening for the presence of a disease. Certain of these methods involve administering to a test subject a substrate labeled with a stable isotope, the relative abundance of the isotope in the substrate being known. Sufficient time is allowed for the labeled substrate to be at least partially metabolized by the test subject to form one or more target metabolites known to be correlated with the disease. A plurality of electrophoretic methods are performed in series to at least partially separate a plurality of target analytes from other biological components in a sample obtained from the test subject, the target analytes comprising the substrate and/or one or more of the target metabolites. Flux values for the target analytes are determined from the abundance of the isotope in that analyte.

The method is simplified when sample is provided. In such instances, certain method include analyzing a sample from a test subject, the sample comprising a substrate labeled with a stable isotope administered to the test subject and/or one or more target metabolites resulting from metabolism of the substrate by the test subject, the relative isotopic abundance of the isotope in the substrate knows at the time of administration. The analyzing step itself comprises determining the abundance of the isotope in a plurality of analytes in the sample to determine a value for the flux of each analyte, the plurality of analytes comprising the substrate and/or one or more of the target metabolites. For each target analyte, the determined flux value is compared with a corresponding reference flux value for the same target analytes to assess the test subject's risk of disease. The reference value can be representative of a healthy or diseased state.

The invention provides a method for identifying a high-resolution protein expression fingerprint for a cell type, tissue, or pathological sample, comprising obtaining a protein-containing extract of a cellular sample and electrophoresing said extract with a first capillary electrophoresis apparatus, eluting protein-containing fractions therefrom, electrophoresing said protein containing fractions on a second capillary electrophoresis apparatus, or plurality thereof in parallel, and identifying the species of proteins by fragmentation mass spectroscopy sequencing to obtain PSTs for a plurality of protein species, and compiling a dataset (or fingerprint record) containing the collection of PSTs obtained thereby. A variation of the method comprises quantitative detection of protein species and compiling a dataset wherein the relative abundance and/or absolute amount of a plurality of protein species eluted from said second capillary electrophoresis is/are cross-tabulated with the PST identification. A typical embodiment comprises attachment of a mass spectroscopy label to the proteins in the protein-containing prior to the last capillary electrophoresis step. In a variation, more than two capillary electrophoresis steps are used; in an embodiment, capillary isoelectric focusing (CIEF) is the first capillary electrophoresis, and the second capillary electrophoresis is either capillary zone electrophoresis (CZE) or capillary gel electrophoresis (CGE), The method can be modified so that substantially any metabolite or collection of metabolites (metabolite fingerprint) can be measured in addition to or in lieu of a protein expression fingerprint. In such modifications, the sample comprises the metabolites specie(s) to be measured and detection and separation apparatus to quantitate the metabolite specie(s) in the sample; suitable apparatus and methodology for performing metabolite fingerprinting is described herein.

A protein expression fingerprint comprises an array of at least 100 protein species each having a unique identifier (which may comprise PST and/or electrophoretic mobility data and/or pI and/or any other biophysical property ascertainable by capillary electrophoresis, and/or any other biophysical property known by virtue of the origin of the sample prior to electrophoresis, and/or any other measurable biophysical property), optionally including cross-tabulation with quantitative data indicating relative and/or absolute abundance of each species in the sample. A protein expression fingerprint record comprises a protein expression fingerprint cross-tabulated to data indicating sample source and optionally other bioinformational data (pathological condition, age, passage history, etc.). Similarly, a metabolite fingerprint comprises an array of at least 3 metabolite species, each less than 5,000 Daltons molecular weight and being other than polypeptides and polynucleotides, each metabolite having a unique identifier cross-tabulated to data indicating the relative or absolute abundance, ratio in comparison to one or more other measured metabolites, rates of appearance or disappearance, or the like.

In a variation, the invention provides a method for producing a computer database comprising a computer and software for storing in computer-retrievable form a collection of protein, expression fingerprint records cross-tabulated with data specifying the source of the protein-containing sample from which each protein expression fingerprint record was obtained. In a variation, at least one of the sources is from a tissue sample known to be free of pathological disorders. In a variation, at least one of the sources is known pathological tissue specimen, for example but not limitation a neoplastic lesion or a tissue specimen containing an infectious agent such as a virus, or the like. In a variation, the protein expression fingerprint records cross-tabulate at least the following parameters for each protein species in a sample: (1) a unique identification code, which can comprise a PST and/or characteristic electrophoretic separation coordinate; (2) sample source; optionally (3) absolute and/or relative quantity of the protein species present in the sample, optionally (4) presence or absence of amino or carboxyterminal post-translational modifications, and/or optionally (5) original electropherograms and/or mass spectra used to identify the proteins and PST. A database comprises a plurality of protein expression fingerprint records, each of which represents a protein expression fingerprint from one sample or a subfraction thereof.

The invention also provides for the storage and retrieval of a collection of such polypeptide fingerprints in a computer data storage apparatus, which can include magnetic disks, optical disks, magneto-optical disks, DRAM, SRAM, SGRAM, SDRAM, RDRAM, DDR RAM, magnetic bubble memory devices, and other data storage devices, including CPU registers and on-CPU data storage arrays. Typically, the polypeptide fingerprint records are stored as a bit pattern in an array of magnetic domains on a magnetizable medium or as an array of charge states or transistor gate states, such as an array of cells in a DRAM device (e.g., each cell comprised of a transistor and a charge storage area, which may be on said transistor). The invention provides such storage devices, and computer systems built therewith, comprising a bit pattern encoding a protein expression fingerprint record comprising unique identifiers for at least 100 protein species cross-tabulated with sample source. The invention provides a method for identifying related polynucleotide or polypeptide sequences, comprising performing a computerized comparison between a PST sequence stored in or retreived from a computer storage device or database and at least one other sequence; such comparison can comprise a sequence analysis or comparison algorithm or computer program embodiment thereof (e.g., PASTA, TFASTA, GAP, BESTFIT) and/or the comparison may be of the relative amount of a PST sequence in a pool of sequences determined from a polynucleotide sample of a specimen. The invention provides a computer system comprising a storage device having a bit pattern encoding a database having at least 100 protein expression fingerprint records obtained by the methods of the invention, and a program for sequence alignment and comparison to predetermined genetic or protein sequences. The invention also provides a magnetic disk, such as an IBM-compatible (DOS, Windows, Windows95/98/2000, Windows NT, OS/2) or other format (e.g., Linux, SunOS, Solaris, AIX, SCO Unix, VMS, MV, Mackintosh, etc.) floppy diskette or hard (fixed, Winchester) disk drive, comprising a bit pattern encoding a protein expression fingerprint record; often the disk will comprise at least one other bit pattern encoding a polynucleotide and/or polypeptide sequence other than a protein expression fingerprint record of the invention, typically in a file format suitable for retreival and processing in a computerized sequence analysis, comparison, or relative quantitation method. The invention also provides a network, comprising a plurality of computing devices linked via a data link, such as an Ethernet cable (coax or 10BaseT), telephone line, ISDN line, wireless network, optical fiber, or other suitable signal transmission medium, whereby at least one network device (e.g., computer, disk array, etc.) comprises a pattern of magnetic domains (e.g., magnetic disk) and/or charge domains (e.g., an array of DRAM cells) composing a bit pattern encoding a protein expression fingerprint record of the invention. The invention also provides a method for transmitting a protein expresison fingerprint record of the invention, which is uniquely determined by the methodology employed to generate it, comprising generating an electronic signal on an electronic communication device, such as a modem, ISDN terminal adapter, DSL, cable modem, ATM switch, or the like, whereby said signal comprises (in native or encrypted format) a bit pattern encoding a protein expression fingerprint record or a database comprising a plurality of protein expression fingerprint records obtained by the method of the invention, respectively.

The invention provides a computer system for comparing a query polypeptide sequence or query polynucleotide sequence to a database containing an array of PST sequences and other data structures of a protein expression fingerprint record obtained by the method of the invention, and ranking database sequences based on the degree of sequence identity and gap weight to query sequence. A central processor is initialized to load and execute computer program for alignment and/or comparison of amino acid sequences or nucleotide sequences. A query sequence comprising at least 4 amino acids or 12 nucleotides is entered into the central processor via I/O device. Execution of computer program results in central processor retreiving sequence data from data file, which comprises a binary description of a protein expression fingerprint record or portion thereof containing polypeptide sequence data for the record. Said sequence data or record, and said computer program can be transferred to secondary memory, which is typically random access memory (e.g., DRAM, SRAM, SGRAM, or SDRAM). Sequences are ranked according to the degree of sequence identity to the query sequence and results are output via an I/O device. For example and not to limit the invention, a central processor can be a conventional computer (e.g., Intel Pentium, PowerPC, Alpha, PA-8000, SPARC, MIPS 4400, MIPS 10000, VAX, etc.); a program can be a commercial or public domain molecular biology software package (e.g., UWGCG Sequence Analysis Software, Darwin, blastn); a data file can be an optical or magnetic disk, a data server, a memory device (e.g., DRAM, SRAM, SGRAM, SDRAM, EPROM, bubble memory, flash memory, etc.); an I/O device can be a terminal comprising a video display and a keyboard, a modem, an ISDN terminal adapter, an Ethernet port, a punched card reader, a magnetic strip reader, or other suitable I/O device.

The invention provides a computer program for comparing query polypeptide sequence(s) or query polynucleotide sequence(s) or a query protein expression fingerprint to a protein expression fingerprint database obtained by a method of the invention and ranking database sequences based on the degree of similarity of protein species expressed and rlative and/or absolute abundances in a sample. The initial step is input of a query polynucleotide or polypeptide sequence, or protein expression fingerprint record obtained bya method of the invention, input via n I/O device. A data file is accessed in to retreive a collection of protein expression fingerprint records for comparison to the query; said collection comprises protein expression fingerprint records obtained by a method of the invention. Individually or collectively sequences or other cross-tabulated information of the protein expression fingerprint collection are optimally matched to the query sequence(s) or query protein expression record such as by the algorithm of Needleman and Wunsch or the algorithm of Smith and Waterman or other suitable algorithm obtainable by those skilled in the art. Once aligned or matched, the percentage of sequence or fingerprint similarity is computed in for each aligned or matched sequence to generate a similarity value for each sequence or fingerprint in the protein expression fingerprint record collection as compared to the query sequence(s) or fingerprint(s). Sequences are ranked in order of greatest sequence identity or weighted match to the query sequence or query fingerprint, and the relative ranking of the sequence or fingerprint to the best matches in the collection of records is thus generated. A determination is made: if more sequences or fingerprint records exist in the data file, the additional sequences/fingerprints or a subset thereof are retreived and the process is iterated; if no additional sequences/fingerprints exist in the data file, the rank ordered sequences/fingerprints are via an I/O device, thereby displaying the relative ranking of sequences/fingerprints among the sequences/fingerprints of the data file optimally matched and compared to the query sequence(s) or fingerprint(s).

The invention also provides the use of a computer system described above, which comprises: (1) a computer, (2) a stored bit pattern encoding a collection of protein, expression fingerprint records obtained by the methods of the invention, which may be located in said computer, (3) a comparison sequence or fingerprint, such as a query sequence or a data file containing fingerprint information, and (4) a program for alignment and comparison, typically with rank-ordering of comparison results on the basis of computed similarity values. In an embodiment, neural network pattern matching/recognition software is trained to identify and match fingerprint records based on backpropagation using empirical data input by a user. The computer system and methods described permit the identification of the relative relationship of a query protein expression fingerprint to a collection of protein expression fingerprints; preferably all protein expression fingerprints (query and database) are obtained by the methods of the invention.

The invention also provides a computer system including a database containing a plurality of protein fingerprint records and/or metabolite fingerprint records in the form of tree-based or otherwise hierarchical navigational fields cross-tabulated to informational data such as one or more or the following: medical records, patient medical history, medical diagnostic test results of a patient, patient name, patient sex, patient age, patient genetic profile, patient diagnosis-related group code, patient therapy, time of day, vital signs of a patient, drug assay results of a patient, medical information of patient's blood relatives, and other similar medical, biological, and physiological information of a patient from which the sample(s) used to generate the protein fingerprint record and/or metabolite fingerprint record data was obtained. In an embodiment, a computer system comprising a database having a hybrid data structure with the navigational field(s) comprising a protein expression fingerprint obtained by a method of the invention and/or a metabolite expression fingerprint obtained by a method of the invention is employed to link to informational fields of the same or a related record which comprise medical information as described herein; the data structure can conform to the general description in U.S. Patent 5,295,261, which is incorporated herein by reference.

The invention also provides a computer system comprising a computer and a program employing a neural network trained to extract database records having a predicted or predetermined protein expression profile match that is pathognomonic for a predetermined disease or medical condition, predisposition to disease, or physiological state. In an illustrative embodiment, a blood or cellular sample from a patient is dialyzed according to a method of the invention to provide a predetermined protein expression record that is entered as a database query into a trained neural network that has been previously trained on a plurality of predetermined database records to establish correlative neural relationships between protein expression fingerprint data (navigation fields) and medical data (information field(s)), so that the query identifies the medical condition(s) most highly correlated in the trained neural network with the patient's protein expression fingerprint. The method can alternatively or in addition employ a predetermined metabolites profile fingerprint record obtained from the serum, blood, or other cellular sample to query a database of metabolite profile fingerprint records using a trained neutral network which links the query metabolite profile record to the database metabolites profile fingerprint records linked to the medical condition(s) most highly correlated in the trained neural network with the patient's metabolite profile fingerprint.

The invention also provides a computer system comprising a computer and a program employing a database comprising records having a field or plurality of fields comprising a protein expression fingerprint dataset obtained from a serum, blood, or other cellular samples of a patient and analyzed according to a method of the present invention, and further having one or a plurality of fields containing data obtained from a patient relating to symptoms, medical status, medical history, or other differential diagnosis information, which can be entered via a connection to the Internet or other TCP/IP or related networking system.

The invention provides for the use of the disclosed methods, compositions, apparatus, software, and information obtained thereby.

A further understanding of the nature and advantages of the invention will become apparent by reference to the remaining portions of the specification and drawings.

## BRIEF DESCRIPTION OF THE DRAWINGS

FIG. 1 its a schematic representation of one example of an electrophoretic system that can be utilized with certain methods of the invention.

FIG. 2A is a schematic representation of some of the major elements of an electrophoretic system utilized in conducting certain electrophoretic methods of then invention.

FIG. 2B is a cross-sectional view of a capillary showing the orientation of a porous plug inserted into the capillary to control electroosmotic flow in certain methods of the invention.

FIGS. 3A and 3B are top-views of certain elements of microfluidic devices that can be utilized to conduct certain electrophoretic methods of the invention.

FIG. 4 is an electropherogram for a sample containing five unlabeled proteins (hen white conalbumin, bovine serum albumin, bovine carbonic anhydrase II, carbonic anhydrase II, rabbit muscle GAPDH, and bovine ribonuclease A) as obtained following electrophoresis by capillary zone electrophoresis. Absorbance was monitored at 214 nm. Under the conditions of this particular experiment (see Example 1) in which the proteins were unlabled, the proteins were not resolved.

FIG. 5 is a plot of electrophoretic mobility for the five proteins listed in FIG. 4 under the same Electrophoresis conditions as described in FIG. 4.

FIG. 6 is a plot showing the correlations between Electrophoretic mobility and the predicted mass-to-charge ratio of the proteins at pH 4.0.

FIG. 7 is an electropherogram obtained during separation of a sample containing five sulfophenylisothiocyanate-labeled proteins (hen white conalbumin, bovine serum albumin, bovine carbonic anhydrase II, carbonic anhydrase II, rabbit muscle GAPDH, and bovine ribonuclease A) as obtained following electrophoresis by capillary zone electrophoresis. Absorbance was monitored at 214 nm. Under the conditions of this particular experiment (see Example 2) in which the proteins were labeled, the labeled proteins were partially resolved.

FIG. 8 is an electropherogram obtained during separation of a sample containing the proteins hen white conalbumin, bovine serum albumin, and bovine carbonic anhydrase II, by CIEF.

FIG. 9 is an electropherogram of a fraction (fraction F) obtained from the separation by CIEF shown in FIG. 7.

FIG. 10 is an electropherogram of a fraction (fraction G) obtained from the separation by CIEF shown in FIG. 7.

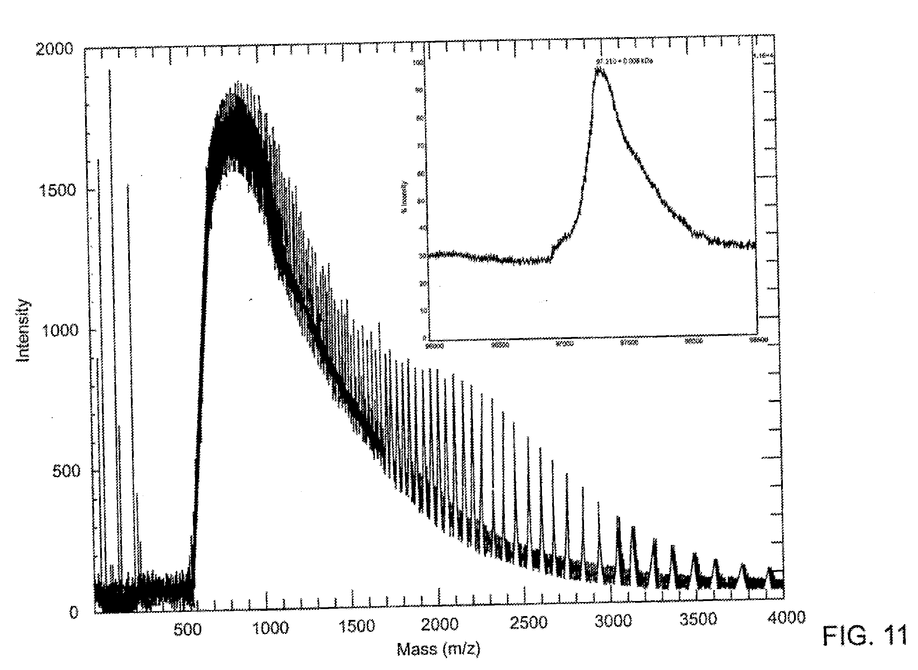

FIG. 11 provides the minimally fragmenting 12 V spectrum of glycogen phosphorylase A protein. A zero charge mass deconvolution of the multiply charged mass peaks observed between 700 and 4000 amu was prepared using the BioSpee Data Explorer™ software.

FIG. 12 illustrates the increase in relative abundance for peaks corresponding to the acetylated peptide masses, with increasing nozzle potential.

FIG. 13 provides an example of a substantially fragmented mass spectra, corresponding to 250 V nozzle potential for glycogen phosphorylase A.

FIG. 14 provides the minimally fragmenting 12 V spectrum of PITC-Bradykinin peptide. A zero charge mass deconvolution of the multiply charged mass peaks observed between 700 and 4000 amu was prepared using the BioSpec Data Explorer™ software,

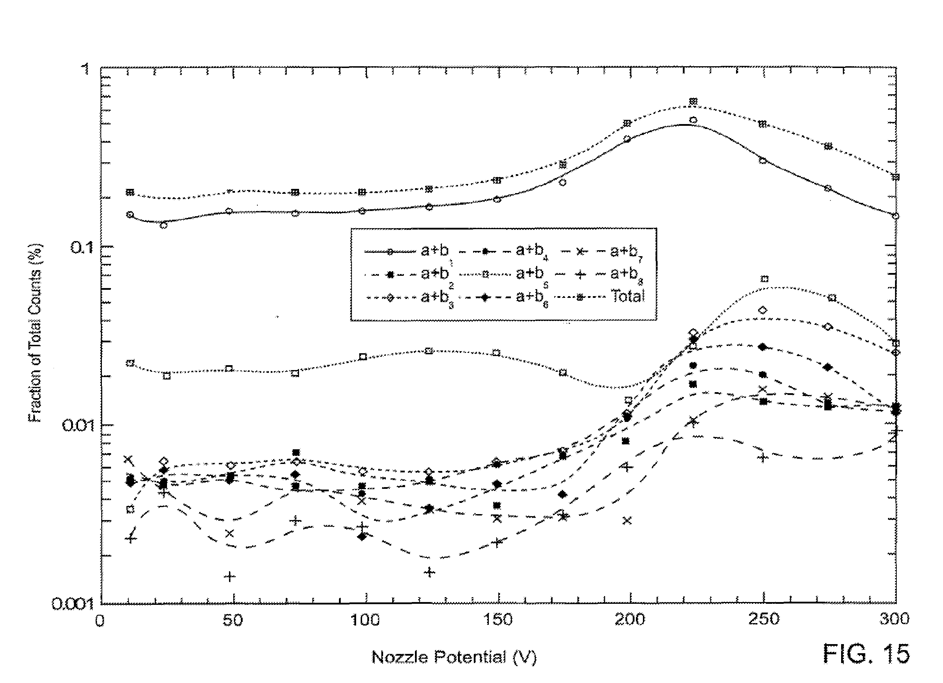

FIG. 15 illustrates the increase in relative abundance for peaks corresponding to the PITC-labeled peptide masses, with increasing nozzle potential.

FIG. 16 provides an example of substanitally fragmented mass spectra, corresponding to 250 V nozzle potential for PITC-labeled Bradykinin.

FIGS. 17 and 18 illustrate the peak counts corresponding to the a-ions (Figure 17) and b-ions (Figure 18) generated from the IMB-labeled peptide fragment masses were clearly observed to increase in relative abundance with increasing nozzle potential with a maximum fragmentation abundance noted at about 200V.

FIG. 19 shows the mass spectrum from SPITC-labeled apomyoglobin obtained in the negative ion made. The nozzle potential was increased from a minimum setting of 125 V to a maximum of 300V in 25-50 V increments with 1 minute of instrument equilibration time alotted before collecting spectra at each nozzle potential. A total of thirty 3-second spectra were accumulated for analysis at each nozzle potential.

FIG. 20 shows the increase in relative abundance for the $b_1$, $a_2$, $b_2$, $a_3$, and $b_3$ ions which occurs above nozzle potentials of 250V.

FIG. 21 shows the increase in relative abundance for the doubly charged $y_{1-7}$ ions generated from the C-terminal (2-aminoethyl)trimethylammonium-labeled Bradykinin peptide obtained in positive ion mode. The nozzle potential was increased from a minimum of 50 V to a maximum or 300 V in 50 V increments with 1 minute of instrument equilibration time alotted before collecting spectra at each nozzle potential. A total of sixty 3-second spectra were accumulated for analysis at each nozzle potential.

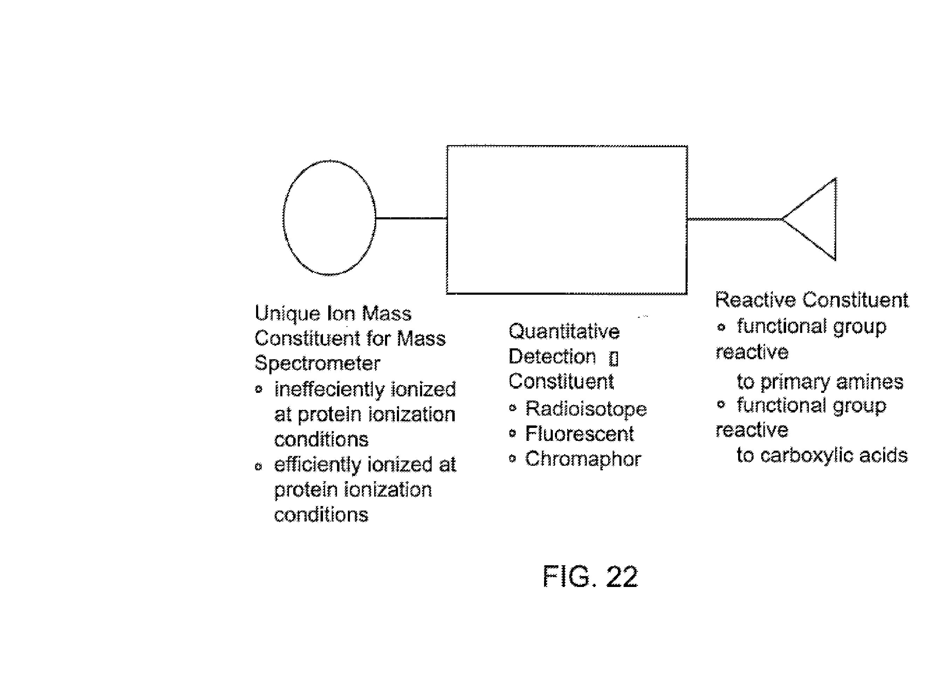

FIG. 22 is a schematic of the covalent chemical label described by the current invention.

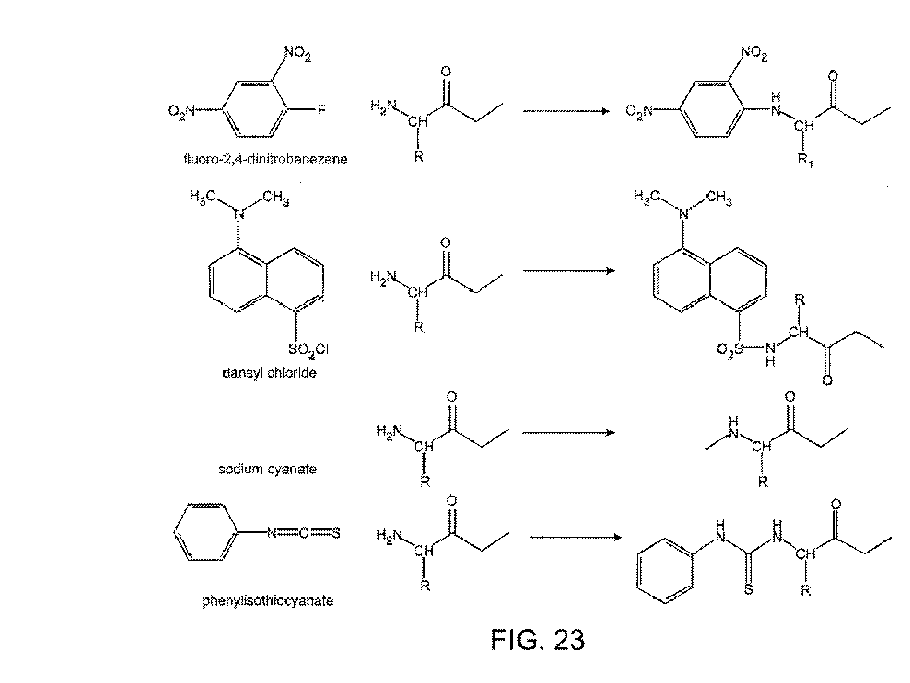

FIG. 23 depicts examples of covalent chemical labels encompassed by the current invention.

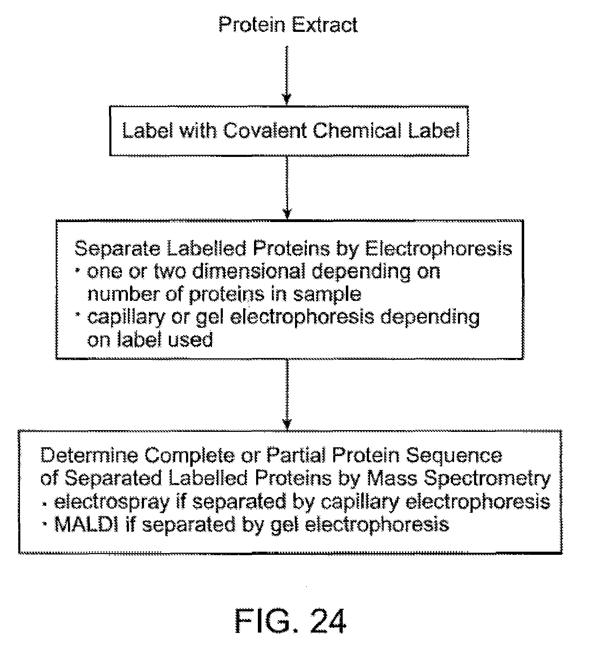

FIG. 24 is a schematic diagram depicting the process steps of an embodiment of the current invention.

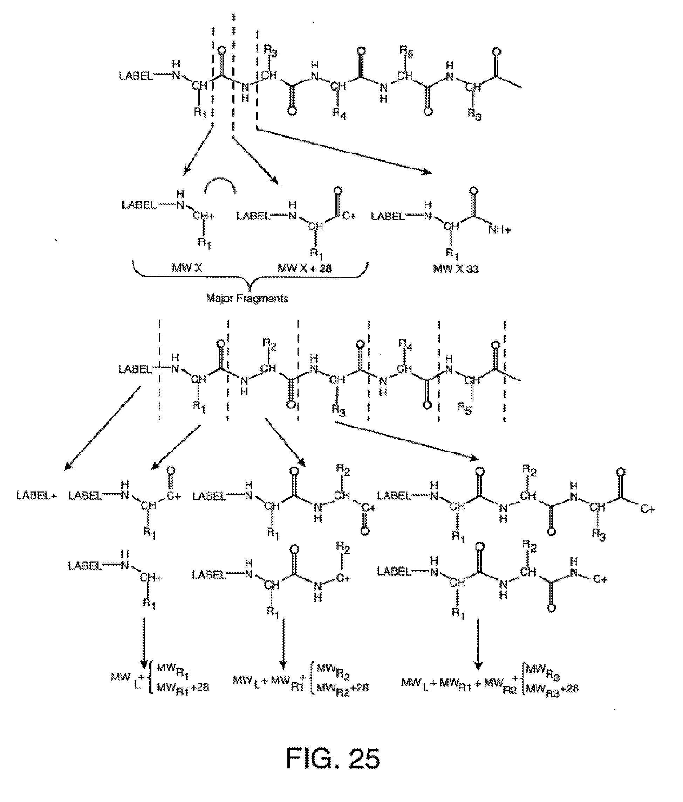

FIG. 25 is a schematic of the protein mass spectrometric fragmentation pattern expected and how to reconstitute the protein sequence from the mass spectrometric fragmentation pattern using the invention.

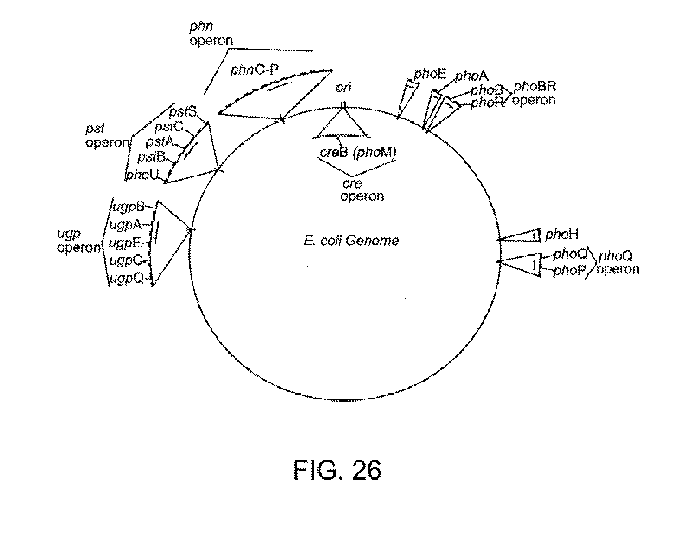

FIG. 26 is gene loci map of the pho regulon in Escherichia coli. Operons under pho regulatory control are denoted on the outside of the genome. The photo gene (part of the cre operon, which contains four genes) is denoted inside the genome.

FIG, 27 shows alkaline phosphatase (PhoA) and total protein synthesis rates shown by E. coli before and during phosphate starvation. The onset of phosphate starvation occurs just after three hours.

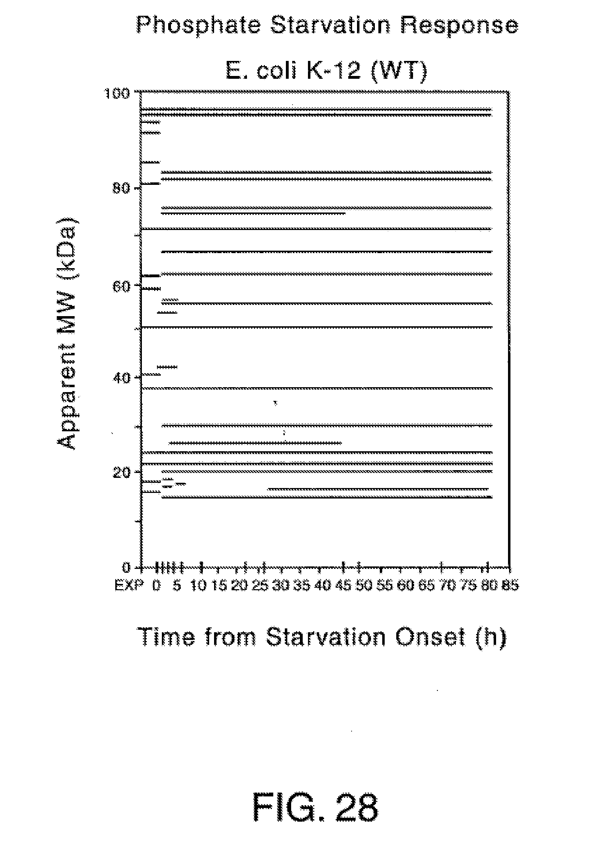

FIG. 28 shows the temporal expression of 53 proteins differentially expressed between exponential growth (EXP) and during phosphate starvation in E. coil,

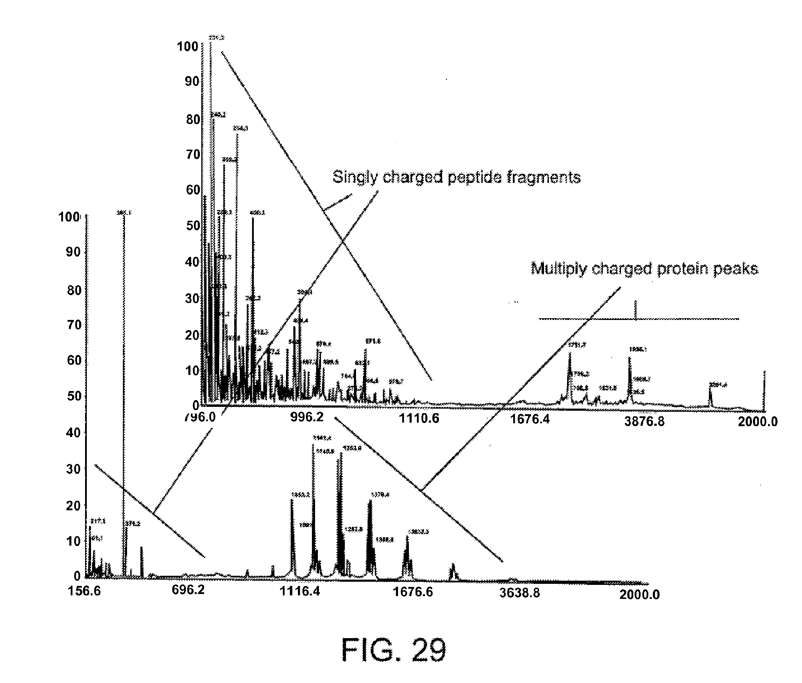

FIG. 29 shows variation in the efficiency of MS induced fragmentation of bovine ribonualease A in the ionization zone of an ESI-TOF instrument as a function of 20 the nozzle voltage (270 V in the top and 150 V in the bottom spectra, respectively).

FIG. 30 provides examples of "hard" and "soft" positively charged labels suitable for N-terminal sequencing and enhanced fluorescent detection of proteins by methods described herein and in U.S. Application Serial Nos. 09/513,395, 09/513,486 and 09/513,907.

FIG. 31 provides examples of "hard" and "soft" negatively charged labels suitable for N-termmal sequencing and enhanced fluorescent detection of proteins by methods described herein and in U.S. Application Serial Nos. 09/513,395, 09/513,486 and 09/513,907.

FIG. 32 provides examples of compounds suitable for C-terminal labeling through carbodiimide and anhydride attachment chemistries for C-terminal sequencing and enhanced fluorescent detection of proteins by methods described in described herein and in U.S. Application Serial Nos. 09/513,395, 09/513,486 and 09/513,907.

FIG. 33 shows (A) a section of the mass spectrum around 136.88 amu; (B) a similar section of the mass spectrum exactly 6.02 amu higher, corresponding to the position where the $[^{13}C]_6$-metabolite peak should exist; (C) the ratio of the counts at 142.90 to that at 136.88 amu positions in the mass spectrum, corresponding to the six carbon $^{13}C/^{12}C$ metabolite ratio; and (D) the metabolic flux, determined by curve fit to the change in the $^{13}C/^{12}C$ ratio over time at 136.88 amu.

FIG. 34 shows (A) a section of the mass spectrum around 150.87 amu; (B) a similar section of the mass spectrum exactly 6.02 amu higher, corresponding to the position where the $[^{13}C]_6$-metabolite peak should exist; (C) the ratio of the counts at 156.89 to that at 150.88 amu positions in the mass spectrum, corresponding to the six carbon $^{13}C/^{12}C$ metabolite ratio; and (D) the metabolic flux, determined by curve fit to the change in the $^{13}C/^{12}C$ ratio over time at 150.87 amu.

FIG. 35 shows (A) a section of the mass spectrum around 152.88 amu; (B) a similar section of the mass spectrum exactly 6.02 amu higher, corresponding to the position where the $[^{13}C]_6$-metabolite peak should exist; (C) the ratio of the counts at 152.90 to that at 152.88 amu positions in the mass spectrum, corresponding to the six carbon $^{13}C/^{12}C$ metabolite ratio; and (D) the metabolic flux, determined by curve fit to the change in the $^{13}C/^{12}C$ ratio over time at 152.88 amu.

FIG. 36 shows (A) a section of the mass spectrum around 281.77 amu; (B) a similar section of the mass spectrum exactly 6.02 amu higher, corresponding to the position where the $[^{13}C]_6$-metabolite peak should exist; (C) the ratio of the counts at 287.79 to that at 281.77 amu positions in the mass spectrum, corresponding to the six carbon $^{13}C/^{12}C$ metabolite ratio; and (D) the metabolic flux, determined by curve fit to the change in the $^{13}C/^{12}C$ ratio over time at 281.77 amu.

FIG. 37 shows (A) a section of the mass spectrum around 328.76 amu; (B) a similar section of the mass spectrum exactly 6.02 amu higher, corresponding to the position where the $[^{13}C]6$-metabolite peak should exist; (C) the ratio of the counts at 334.78 to that at 328.76 amu positions in the mass spectrum, corresponding to the six carbon $^{13}C/^{12}C$ metabolite ratio; and (D) the metabolic flux, determined by curve fit to the change in the $^{13}C/^{12}C$ ratio over time at 328,76 amu.

FIG. 38 shows (A) a section of the mass spectrum around 330.76 amu; (B) a similar section of the mass spectrum exactly 6.02 amu higher, corresponding to the position where the $[^{13}C]_6$-metabolite peak should exist; (C) the ratio of the counts at 336.78 to that at 330.76 amu positions in the mass spectrum, corresponding to the six carbon $^{13}C/^{12}C$ metabolite ratio; and (D) the metabolic flux, determined by curve fit to the change in the $^{13}C/^{12}C$ ratio over time at 330.76 amu.

FIG. 39 shows (A) a section of the mass spectrum around 494.84 amu; (B) a similar section of the mass spectrum exactly 6.02 amu higher, corresponding to the position where the $[^{13}C]_6$-metabolite peak should exist; (C) the ratio of the counts at 500.86 to that at 494.84 amu positions in the mass spectrum, corresponding to the six carbon $^{13}C/^{12}C$ metabolite ratio; and (D) the metabolic flux, determine by curve fit to the change in the $^{13}C/^{12}C$ ratio over time at 494.82 amu.

FIG. 40 shows (A) a section of the mass spectrum around 278.80 amu; (B) a similar section of the mass spectrum exactly 5.02 amu higher, corresponding to the position where the $[^{13}C]_5$-metabolite peak should exist; (C) the ratio of the counts at 283.82 to that at 278.80 amu positions in the mass spectrum, corresponding to the five carbon $^{13}C/^{12}C$ metabolite ratio; and (D) the metabolic flux, determined by curve fit to the change in the $^{13}C/^{12}C$ ratio over time at 278.80 amu.

FIG. 41 shows (A) a section of the mass spectrum around 280.80 amu; (B) a similar section of the mass spectrum exactly 5.02 amu higher, corresponding to the position where the $[^{13}C]_5$-metabolite peak should exist; (C) the ratio of the counts at 285.82 to that at 280.8 amu positions in the mass spectrum, corresponding to the five carbon $^{13}C/^{12}C$ metabolite ratio; and (D) the metabolic flux, determined by curve fit to the change in the $^{13}C/^{12}C$ ratio over time at 280.80 amu.

DETAILED DESCRIPTION

**Definitions**

Unless defined otherwise, all technical and scientific terms used herein generally have the same meaning as commonly understood by one of ordinary skill in the art to which this invention belongs. Generally, the nomenclature used herein and the laboratory procedures in molecular biology, organic chemistry described below are those well known and commonly employed in the art. Standard techniques are used for nucleic acid and peptide synthesis. Generally, enzymatic reactions and purification steps are performed according to the manufacturer's specifications. The techniques and procedures are generally performed according to conventional methods in the art and various general references *(see generally,* Sambrook et al MOLFCULAR CLONING: A LABORATORY MANUAL, 2d ed. (1989) Cold Spring Harbor Laboratory Press, Cold Spring Harbor, N.Y., which is incorporated herein by reference), which are provided throughout this document. The nomenclature used herein and the laboratory procedures in analytical chemistry, and organic synthetic described below are those known and employed in the art. Standard techniques, or modifications thereof, are used for chemical syntheses and chemical analyses.

Discussions of the various classes of metabolic compounds referenced herein can be found in any general biochemistry text book, including, for example, Voet, D. and Voet, J.G., Biochemistry, John Wiley & Sons, New York (1990); Stryer, L., Biochemistly, 2nd ed., W.H. Freeman and Company, San Francisco (1981); and White, A., et al., Principles of Biochemistry, 6th ed., McGraw-Hill Book Company (1978), each of which is incorporated by reference in its entirety.

A "nucleic acid" is a deoxyribonucleotide or ribonucleotide polymer in either single- or double-stranded form.

A "polynucleotide" refers to a single- or double-stranded polymer of deoxyribonucleotide or ribonucleotide bases.

As used herein, the terms "protein", "peptide" and "polypeptide" are used interchangeably and refer to a polymer of amino acid residues. For a general review, *see,* Spatola, A.F., in CHEMISTRY AND BIOCHEMISTRY OF AMINO ACIDS, PEPTIDES AND PROTEINS, B. Weinstem, eds., Marcel Dekker, New York, p. 267 (1983), which is incorporated by reference in its entirety. As used herein, the twenty conventional amino acids and their abbreviations follow conventional usage *(*Immunology - A Synthesis, 2nd ed., (E.S. Golub and D.R. Green, Eds.) Sinauer Associates, Sunderland, Massachusetts (1991)). In the polypeptides notation used herein, the lefthand direction is the amino terminal direction and the righthand direction is the carboxy-terminal direction, in accordance with standard usage and contention.

A "carbohydrate" refers to aldehyde or ketone derivatives of polyhydric alcohols. The term includes monosaccharides, oligosaccharides and polysaccharides. "Oligosaccharides" and "polysaccharides" are formed by condensation of monosaccharide residues. Oligosaccharides contain a relatively limited number of monosaccharide residues, and typically include di-, tri-, tetra- and pentasaccharides. Polysaccharides are polymers of high molecular weight formed from the condensation of many monosaccharides of the same type (homopolysaccharides) or two or more types (heteropolysaccharides). The molecular weight of polysaccharides can range into the millions of daltons. Specific examples of carbohydrates include glucose, galactose, xylose, fructose, sucrose, and glycogen. The tern "simple sugar" typically refers to monosaccharides.

The term "lipid" generally refers to substances that are extractable from animal or plant cells by nonpolar solvents. Materials falling within this category include the fatty acids, fats such as the mono-, di- and triacyl glycerides, phosphoglycerides, sphingolipids, waxes, terpenes and steroids. Lipids can also be combined with other classes of molecules to yield lipoproteins, lipoamino acids, lipopolysaccharides and proteolipids.

"Fatty acids" generally refer to long chain hydrocarbons (e,g., 6 to 28 carbon atoms) terminated at one end by a carboxylic acid group, although the hydrocarbon chain can be as short as a few carbons long (*e.g.*, acetic acid, propionic acid, n-butyric acid). Most typically, the hydrocarbon chain is acyclic, unbranched and contains an even number of carbon atoms, although some naturally occurring fatty acids have an odd number of carbon atoms. Specific examples of fatty acids include caprioic, lauric, myristic, palmitic, stearic and arachidic acids. The hydrocarbon chain can be either saturated or unsaturated.

"Fats" are a particular class of lipids and are esters of fatty acids and glycerol. Fats include mono-, di- and triacylglycerides.

A "nucleoside" is a compound of a sugar (typically a ribose or deoxyribose) attached to a purine or pyrimidine base via an N-glycosyl linkage.

A "nucleotide" refers to a phosphate ester of pentose sugars in which a nitrogenous base (typically a purine or pyrimidine base) is linked to the C(1') sugar residue. Most typically, a nucleotide is a nucleoside attached to a phosphoric group.

The term "steroid" refers to the large class of compounds that contain the tetracyclic cyclopenta[_]phenanthrene backbone that are part of the metabolism of an organism. A specific example is cholesterol.

The term "compound" or "component" refers to a molecule regardless of molecular weight found within an organism or cell. A compound or component can be from the same class of compounds as a substrate or metabolite.

An "organic acid" refers to any organic molecule having one or more carboxylic acid groups. The organic acid can be of varying length and can be saturated or unsaturated. Examples of organic acids include, but are not limited to, citric acid, pyruvic acid, succinic acid, malic acid, maleic acid, oxalacetic acid, and -ketoglutaric acid. Organic acids can include other function groups in addition to the carboxylic acid group including, for example, hydroxyl, carbonyl and phosphate.

The term "naturally-occurring" as used herein as applied to an object refers to the fact that an object can be found in nature. For example, a polypeptide or polynucleotide sequence that is present in an organism (including viruses) that can be isolated from a source in nature and which has not been intentionally modified by man in the laboratory is naturally-occurring. Generally, the term naturally-occurring refers to an object as present in a non-pathological (undiseased) individual, such as would be typical for the species.

The term "agent" is used herein to denote a chemical compound, a mixture of chemical compounds, an array of spatially localized compounds (*e.g.*, a VLSIPS peptide array, polynucleotide array, and/or combinatorial small molecule array), a biological macromolecule, a bacteriophage peptide display library, a bacteriophage antibody (e.g., scFv) display library, a polysome peptide display library, or an extract made from biological materials such as bacteria, plants, fungi, or animal (particularly mammalian) cells or tissues.

As used herein, "substantially pure" means an object species is the predominant species present *(i.e.,* on a molar basis it is more abundant than other species in the composition, with the exception of solvent species and metal ions), and preferably a substantially purified fraction is a composition wherein the object species comprises at least about 50 percent (on a molar basis) of all species present. Generally, a substantially pure composition will comprise more than about 80 to 90 percent of all species present in the composition. Most preferably, the object species is purified to essential homogeneity (contaminant species cannot, be detected in the composition by conventional detection methods) wherein the composition consists essentially of a single species.

As used herein "normal blood" or "normal human blood" refers to blood from a healthy human individual who does not have an active neoplastic disease or other disorder of lymphocytic proliferation, or an identified predisposition for developing a neoplastic disease. Similarly, "normal cells", "normal cellular sample", "normal tissue", and "normal lymph node" refers to the respective sample obtained, from a healthy human individual who does not have an active neoplastic disease or other lymphoproliferative disorder.

As used herein, the terms "pathognomonic concentration", "pathognomonic amount", and "pathognomonic staining pattern" refer to a concentration, amount, or localization pattern, respectively, of a protein or protein fingerprint in a sample, that indicates the presence of a pathological (e.g., neoplastic, senescent, immunodeficient, neurodegencrative, inflammatory, etc.) condition or a predisposition to developing a neoplastic disease, such as carcinoma, sarcoma, or leukemia. A pathognomonic amount is an amount of a protein or protein expression fingerprint feature in a cell or cellular sample that falls outside the range of normal clinical values that is established by prospective and/or retrospective statistical clinical studies. Generally, an individual having a neoplastic disease (e.g., carcinoma, sarcoma, or leukemia) will exhibit an amount of a predetermined protein or a matched protein expression fingerprint in a cell or tissue sample that is outside the range of concentrations that characterize normal, undiseased individuals; typically the pathognomonic concentration is at least about one standard deviation outside the mean normal value, more usually it is at least about two standard deviations or more above the mean normal value. However, essentially all clinical diagnostic tests produce some percentage of false positives and false negatives. The sensitivity and selectivity of the diagnostic assay must be sufficient to satisfy the diagnostic objective and any relevant regulatory requirements. In general, the diagnostic methods of the invention are used to identity individuals as disease candidates, providing an additional parameter in a differential diagnosis of disease made by a competent health professional.

The term "statistical correlation" refers to a statistical association between two variables or parameters as measured by any statistical test including, for example, chi-squared analysis, ANOVA or multivariate analysis. The correlation between one parameter (*e.g.*, value for the flux of a metabolite) and a second parameter (*e.g.*, disease state) is considered statistically significant if the probability of the result happening by chance (the P-value) is less than some predetermined level (*e.g.*, 0.05). The term "statistically significant difference" refers to a statistical confidence level, P, that is $< 0.25$, preferably $< 0.05$, and most preferably $< 0.01$. As used herein the term "physiological conditions" refers to temperature, pH, ionic strength, viscosity, and like biochemical parameters which are compatible with a viable organism, and/or which typically exist intracellularly in a viable cultured yeast cell or mammalian cell, For example, the intracellular conditions in a yeast cell grown under typical laboratory culture conditions are physiological conditions. Suitable in vitro reaction conditions for in vitro transcription cocktails are generally physiological conditions. In general, in vitro physiological conditions comprise 50-200 mM NaCl or KCl, pH 6.5-8.5, 20-45 C and 0.001-10 mM divalent cation (e.g., $Mg^{++}$, $Ca^{++}$); preferably about 150 mM NaCl or KCl, pH 7.2-7.6, 5 mM divalent cation, and often include 0.01-1.0 percent nonspecific protein (e.g., BSA). A non-ionic detergent (Tween, NP-40, Triton X-100) can often be present, usually at about 0.001 to 2%, typically 0.05-0.2% (v/v), Particular aqueous conditions may be selected by the practitioner according to conventional methods. For general guidance, the following buffered aqueous conditions may be applicable: 10-250 mM NaCl, 5-50 mM Tris HCl, pH 5-8, with optional addition of divalent cation(s) and/or metal chelators and/or nonionic detergents and/or membrane fractions and/or antifoam agents and/or scintillants.

As used herein, the term "multimer" comprises dimer and higher order complexes (trimer, tetramer, pentamer, hexamer, heptamer, octamer, etc.). "Homomultimer" refers to complexes comprised of the same subunit species, "Heteromultimer" refers to complexes comprised of more than one subunit species.

The tern "alkyl is used herein to refer to a branched or unbranched, saturated or unsaturated, monovalent hydrocarbon radical, generally having from about 1-30 carbons and preferably, from 4-20 carbons and more preferably from 6-18 carbons. When the alkyl group has from 1-6 carbon atoms, it is referred to as a "lower alkyl." Suitable alkyl radicals include, for example, structures containing one or more methylene, methme and/or methane groups. Branched structures have a branching motif similar to i-propyl, t-butyl, i-butyl, 2-ethylpropyl, *etc.* As used herein, the term encompasses "substituted alkyls," and "cyclic alkyl."

"Substituted alkyl" refers to alkyl as just described including one or more substituents such as lower alkyl, aryl, acyl, halogen *(i.e.,* alkylhalos, *e.g.*, $CF_3$), hydroxy, amino, alkoxy, alkylamino, acylamino, thioamido, acyloxy, aryloxy, aryloxyalkyl, mercapto, thia, aza, oxo, both saturated and unsaturated cyclic hydrocarbon, heterocycles and the like. These groups may be attached to any carbon or substituent of the alkyl moiety. Additionally, these groups may be pendent from, or integral to, the alkyl chain.

The term "aryl" is used herein to refer to an aromatic substituent, which may be a single aromatic ring or multiple aromatic rings which are fused together, linked covalently, or linked to a common group such as a methylene or ethylene moiety. The common linking group may also be a carbonyl as in benzophenone. The aromatic ring(s) may include phenyl, naphthyl, biphenyl, diphenylmethyl and benzophenone among others. The term "aryl" encompasses "arylalkyl" and "substituted aryl."

"Substituted aryl" refers to aryl as just described including one or more functional groups such as lower alkyl, acyl, halogen, alkylhalos (*e.g.* $CF_3$), hydroxy, amino, alkoxy, alkylamino, acylamino, acyloxy, phenoxy, mercapto and both saturated and unsaturated cyclic hydrocarbons which are fused to the aromatic ring(s), linked covalently or linked to a common group such as a methylene or ethylene moiety. The linking group may also be a carbonyl such as in cyclohexyl phenyl ketone. The term "substituted aryl" encompasses "substituted arylalkyl."

The term "arylalkyl" is used herein to refer to a subset of "aryl" in which the acryl group is attached to another group by an alkyl group as defined herein.

"Substituted arylalkyl" defines a subset of "substituted aryl" wherein the substituted aryl group is attached to another group by an alkyl group as defined herein.

The term "acyl" is used to describe a ketone substituent, -C(O)R, where R is alkyl or substituted alkyl, aryl or substituted aryl as defined herein.

The term "halogen" is used herein to refer to fluorine, bromine, chlorine and iodine atoms.

The term "hydroxy" is used herein to refer to the group -OH,

The term "amino" is used to -NRR', wherein R and R' are independently H, alkyl, aryl or substituted analogues thereof. "Amino" encompasses "alkylamino" denoting secondary and tertiary amines and "acylamino" describing the group RC(O)NR'.

The term "alkoxy" is used herein to refer to the -OR group, where R is alkyl, or a substituted analogue thereof. Suitable alkoxy radicals include, for example, methoxy, ethoxy, t-butoxy, etc.

As used herein, the term "aryloxy" denotes aromatic groups that are linked to another group directly through an oxygen atom. This term encompasses "substituted aryloxy" moieties in which the aromatic group is substituted as described above for "substituted aryl." Exemplary aryloxy moieties include phenoxy, substituted phenoxy, benzyloxy, phenethyloxy, etc.

As used herein "aryloxyalkyl" defines aromatic groups attached, through an oxygen atom to an alkyl group, as defined herein. The term "aryloxyalkyl" encompasses "substituted aryloxyalkyl" moieties in which the aromatic group is substituted as described for "substituted aryl."

As used herein, the term "mercapto" defines moieties of the general structure -S-R wherein R is H, alkyl, aryl or heterocyclic as described herein.

The term "saturated cyclic hydrocarbon" denotes groups such as the cyclopropyl, cyclobutyl, cyclopentyl, etc., and substituted analogues of these structures. These cyclic hydrocarbons can be single- or multi-ring structures.

The term "unsaturated cyclic hydrocarbon" is used to describe a monovalent non-aromatic group with at least one double bond, such as cyclopentene, cyclohexene, etc. and substituted analogues thereof. These cyclic hydrocarbons can be single- or multi-ring structures.

The term "heteroaryl" as used herein refers to aromatic rings in which one or more carbon atoms of the aromatic ring(s) are replaced by a heteroatom such as nitrogen, oxygen or sulfur. Heteroaryl refers to structures that may be a single aromatic ring, multiple aromatic ring(s), or one or more aromatic rings coupled to one or more non-aromatic ring (s). In structures having multiple rings, the rings can be fused together, linked covalently, or linked to a common group such as a methylene or ethylene moiety. The common linking group may also be a carbonyl as in phenyl pyridyl ketone. As used herein, rings such as thiophene, pyridine, isoxazole, phthalimide, pyrazole, indole, furan, etc. or benzo-fused analogues of these rings are defined by the term "heteroaryl."

"Heteroarylalkyl" defines a subset of "heteroaryl" wherein an alkyl group, as defined herein, links the heteroaryl group to another group.

"Substituted heteroaryl" refers to heteroaryl as just described wherein the heteroaryl nucleus is substituted with one or more functional groups such as lower alkyl, acyl, halogen, alkylhalos *(e.g.* $CF_3$), hydroxy, amino, alkoxy, alkylamino, acylamino, acyloxy, mercapto, etc. Thus, substituted analogues of heteroaromatic rings such as thiophene, pyridine, isoxazole, phthalimide, pytazole, indole, furan, etc. orbenzo-fused analogues of these rings are defined by the term "substituted heteroaryl."

"Substituted heteroarylalkyl" refers to a subset of "substituted heteroaryl" as described above in which an alkyl group, as defined herein, links the heteroaryl group to another group.

The term "heterocyclic" is used herein to describe a monovalent saturated or unsaturated non-aromatic group having a single ring or multiple condensed rings from 1-12 carbon atoms and from 1-4 heteroatoms selected from nitrogen, sulfur or oxygen within the ring. Such heterocycles are, for example, tetrahydrofuran, morpholine, pipendine, pyrrolidine, etc.

The term "substituted heterocyclic" as used herein describes a subset of "heterocyclic" wherein the heterocycle nucleus is substituted with one or more functional groups such as lower alkyl, acyl, halogen, alkylhalos (*e.g.* $CF_3$), hydroxy, amino, alkoxy, alkylamino, acylamino, acyloxy, mercapto, *etc*.

The term "heterocyclicalkyl" defines a subset of "heterocyclic" wherein an alkyl, group, as defined herein, links the heterocyclic group to another group.

I. Overview

The present invention provides methods and apparatus for achieving the separation of proteins, including significant resolution of proteins in complex mixtures from native cell and tissue samples. The invention is based in part upon the recognition that multidimensional electrophoretic methods involving multiple (typically different) electrophoretic methods performed in series utilizing controlled fractionation techniques to obtain defined fractions can be used to achieve high resolution of proteins. In a variation, labeling and detection steps can be included to increase sensitivity and to obtain accurate and reproducible quantitative information about the resolved proteins, in another variation, the buffer system can be altered in the last separation step, through the use of volatile salts, organic solvents, and ephemeral surfactants to make the eluent compatible with subsequent mass spectrometric analysis. Typically, the Electrophoretic methods are capillary electrophoresis methods, particularly combinations of capillary isoelectric focusing (CIEF), capillary zone Electrophoresis (CZE) and capillary gel electrophoresis (CGE).

Several features enable methods to be performed in a controlled and reproducible fashion. For example, once proteins have had an opportunity to fractionate within the electrophoretic medium contained within a capillary, elution conditions are tailored so that separated proteins are eluted in a controlled fashion to yield defined fractions in which the proteins contained within a fraction fall within a certain pH range, electrophoretic mobility range, or molecular weight range, for example. In certain methods, proteins are labeled at a selected stage of the separation process and the labeled proteins detected using a detector. Labeling enables proteins present at low concentration to more easily be detected and enhances reproducibility by increasing signal-to-noise ratios. The detector can be used to detect proteins as separated within an Electrophoretic cavity or after they are eluted from the cavity. The combination of labeling and detection also enables separated proteins to be quantified. In a variation, the labeling moiety consists of components that impart a covalent linkage to the N-terminus or C-terminus of the protein, at least one component that increases the detectability of the protein, and, optionally, a component that imparts a unique mass signature to the protein, or labelled peptide fragments of the protein in a mass spectrometer.

If additional information is desired, the methods can be expanded to include further analysis by techniques besides electrophoresis. For example, in certain method variations, fractions collected from the final electrophoretic method are individually analyzed by mass spectroscopy to obtain additional information, such as molecular weight and a partial sequence , the masses of chemilytic or enzymatically derived peptides, and total or partial amino acid compositions.. In a variation, the initial sample is fractionated by ammonium sulfate precipitation, subcellular fractionation, or chromatographic means (*e.g.*, reverse phase, size exclusion, affinity, and ion). In a further variation, the biological or biophysical parameters underlying each expansion to the method can be incorporated as separation parameters and utilized to further identify any protein species resolved by the method and/or annotate the description of the protein species resolved by the method in a database of such protein species,

Quantitative detection and the ability to automate the methods means that the methods are amenable to a variety of screening, comparative and diagnostic studies. For example, the methods can be utilized to develop comparative protein expression data. Such comparative studies can be utilized to identify markers of specific diseases, potential targets for pharmaceuticals and/or drug candidates. Once markers that are selectively expressed in certain disease states, for example, are identified, the methods of the invention have utility in diagnostic applications. In a variation, the methods can be incorporated into miniaturized separation and detection devices, in which a plurality of capillary Electrophoretic methods are used to resolve, detect, and quantify one or more protein markers for diagnostic purposes. The methods of the invention can also be utilized to develop a protein database that includes, for example, isoelectric points, apparent molecular weights and relative abundance information and partial or complete protein sequence information, for proteins obtained from different cells, tissues or physiological states. The methods also find utility in studies on structure/activity relationships and in metabolic engineering investigations in which one genetically modifies a certain gene and then determines what effects such a modification has on cellular protein expression.

II. Separation Methods

Summary

The present invention provides a variety of electrophoretic methods and apparatus for separating mixtures of proteins. The methods involve conducting multiple capillary electrophoresis methods in series, wherein samples for each method other than the initial method contain only a subset of the proteins from the preceding step (*e.g.*, from fractions containing resolved protein from the preceding method). By using a variety of techniques to control elution during electrophoresis, the methods are capable of resolving proteins in even complex mixtures such as obtained from tissues and native cells. Utilizing various labeling schemes and detection methods, certain methods can provide quantitative information on the amount of each of the separated proteins. Such information can be used in the development of protein databases in which proteins expressed under certain conditions are characterized and catalogued. Comparative studies to identify proteins that are differentially expressed between different types of cells or tissues can also be conducted with the methods of the present invention. The methods can also be used in diagnostic, structure activity and metabolic engineering studies.

In general, the methods involve performing a plurality of electrophoretic methods in series. Each method in the series includes electrophoresing a sample containing multiple proteins to obtain a plurality of resolved proteins, The sample that is electrophoresed contains only a subset of the plurality of resolved proteins from the immediately preceding method in the series (except the first method of the series in which the sample is the initial sample that contains all the proteins). The resolved proteins from the final electrophoretic method are then detected using various techniques.

The electrophoretic methods typically are capillary electrophoresis methods, such as capillary isoelectric focusing electrophoresis (CIEF), capillary zone electrophoresis (CZE) and capillary gel electrophoresis (CGE), although the methods are amenable to other capillary electrophoresis methods as well. The particular order of the methods can vary. Typically, the methods utilize combinations of electrophoretic methods which separate proteins on the basis of different characteristics (eg., size, charge, isoelectric point).

In certain methods, the proteins are labeled to more easily detect the resolved proteins, to alter the charge o f the proteins, to facilitate their separation, and/or to increase the signal-to-noise ratio. Labeling also enables certain methods to be conducted such that the resolved proteins obtained from the final electrophoretic method are quantitated. Quantitation allows the relative abundance of proteins within a sample, or within different samples, to be determined. In certain methods, the time at which proteins are labeled is selected to precede electrophoresis by capillary zone electrophoresis. By selectively labeling certain residues, resolution of proteins during capillary zone electrophoresis can be increased.

Resolution, quantitation and reproducibility are enhanced by utilizing a variety of techniques to control elution of proteins during an Electrophoretic method. The particular elution technique employed depends in part upon the particular electrophoretic method. However, in general, hydrodynamic, salt mobilization, pH mobilization and electroosmotic flow are utilized to controllably elute resolved proteins at the end of each electrophoretic separation.

Some methods provide for additional analysis after the electrophoretic separation. The type of analysis can vary and include, for example, infra-red spectroscopy, nuclear magnetic resonance spectroscopy, UV/VIS spectroscopy, fluorescence spectroscopy, and complete or partial sequencing. In certain methods, proteins in the final fractions are further analyzed by mass spectroscopy to determine at least a partial sequence for each of the resolved proteins (*i.e.*, to determine a protein sequence tag).

Thus, certain other methods involve performing one or more capillary electrophoretic methods, each of the one or more methods involving: (i) electrophoresing a sample containing multiple proteins within an electrophoretic medium contained within a capillary, and (ii) withdrawing and collecting multiple fractions, each fraction containing proteins resolved during the electrophoresing step. Each method in the series is conducted with a sample from a fraction collected in the preceding electrophoretic method, except the first electrophoretic method which is conducted with a sample containing the original mixture of proteins. The proteins are labeled prior to conducting the last electrophoretic method. Either the proteins in the initial sample are labeled (i.e., labeling precedes all the electrophoretic separations), or the proteins contained in fractions collected are labeled prior to the last electrophoretic method. The final electrophoretic method is performed, and resolved protein within, or withdrawn from, the capillary utilized to conduct the final method is detected with a detector. Hence, the detector is adapted to detect resolved protein within the capillary used in the final method or is connected in line with the capillary to detect resolved proteins as they elute from the capillary. In some instances, the detected proteins are quantitated and further analyzed by mass spectroscopy to determine their relative abundance and/or to establish a protein sequence tag for each resolved protein.

The present invention provides methods and apparatus for achieving the separation of proteins, including significant resolution of proteins in complex mixtures from native cell and tissue samples. The invention is based in part upon the recognition that multidimensional electrophoretic methods involving multiple (typically different) electrophoretic methods performed in series utilizing controlled fractionation techniques to obtain defined fractions can be used to achieve high resolution of proteins. Labeling and detection steps can be included to increase sensitivity, alter the separation coordinates of the proteins, and to obtain accurate and reproducible quantitative information about the resolved proteins. Typically, the electrophoretic methods are capillary electrophoresis methods, particularly combinations of capillary isoelectric focusing (CIEF), capillary zone electrophoresis (CZE) and capillary gel electrophoresis (CGE).

Several features enable methods to be performed in a controlled and reproducible fashion. For example, once proteins have had an opportunity to fractionate within the electrophoretic medium contained within a capillary, elution conditions are tailored so that separated proteins are eluted in a controlled fashion to yield defined fractions in which the proteins contained within a fraction fall within a certain pH range, electrophoretic mobility range, or molecular weight range, for example. In certain methods, proteins are labeled at a selected stage of the separation process and the labeled proteins detected using a detector. Labeling enables proteins present at low concentration to more easily be detected and enhances reproducibility by increasing signal-to-noise ratios. The detector can be used to detect proteins as separated within an electrophoretic cavity or after they are eluted from the cavity. The combination of labeling and detection also enables separated proteins to be quantified. The combination of labeling and separation can alter the net charge or solubility of the proteins causing a change in their separation coordinates, for example, their separation order, the fraction in which they are collected, and elution time.

If additional information is desired, the methods can be expanded to include further analysis by techniques besides electrophoresis. For example, in certain methods, fractions collected from the final electrophoretic method are individually analyzed by mass spectroscopy to obtain additional information, such as molecular weight and partial sequence.

Quantitative detection and the ability to automate the methods means that the methods are amenable to a variety of screening, comparative and diagnostic studies. For example, the methods can be utilized to develop comparative protein expression data. Such comparative studies can be utilized to identify markers of specific diseases, potential targets for pharmaceuticals and/or drug candidates. Once markers that are selectively expressed in certain disease states, for example, are identified, the methods of the invention have utility in diagnostic applications. The methods of the invention can also be utilized to develop a protein database that includes, for example, separation coordinates, isoelectric points, apparent molecular weights and relative abundance information for proteins in different cells, tissues or states. The methods also find utility in studies on structure/activity relationships and in metabolic engineering investigations in which one genetically modifies a certain gene and then determines what effects such a modification has on cellular protein expression.

## General Separation Methodology

The methods of the present invention utilize a combination of electrophoretic methods conducted in series to resolve mixtures of proteins. The methods are said to be conducted in series because the sample(s) electrophoresed in each method are from solutions or fractions containing proteins electrophoresed in the preceding methods, with the exception of the sample electrophoresed in the initial electrophoretic method. As used herein, the terms protein, peptide and polypeptide are used interchangeably and refer to a polymer of amino acid residues. The term also applies to amino acid polymers in which one or more amino acids are chemical analogues of corresponding naturally-occurring amino acids, including amino acids which are modified by post-translational processes (*e.g.*, glycosylation and phosphorylation).

The series of electrophoretic methods are typically conducted in such a way that proteins in an applied sample for each electrophoretic method of the series are isolated or resolved physically, temporally or specially to form a plurality of fractions each of which include only a subset of proteins of the applied sample. Thus, a fraction refers to a protein or mixture of proteins that are resolved physically, temporally or spacially from other proteins in a sample subjected to electrophoresis. Resolved proteins can refer to a single species or a mixture of proteins that are separated from other proteins during an electrophoretic method. As just noted, samples in the various electrophoretic methods are obtained from such fractions, with the exception of the first electrophoretic method in which the sample is the original sample containing all the proteins to be separated.

Typically, these multiple electrophoretic methods in the series separate proteins according to different characteristics. For example, one method can separate proteins on the basis of isoelectric points (*e.g.,* capillary isoelectric focusing electrophoresis), other methods can separate proteins on the basis of their intrinsic or induced (through the application of a label to certain ionizable amino acid residues) charge-to-mass ratio at any given pH (*e.g.*, capillary zone electrophoresis), whereas other methods separate according to the size of the proteins (*e.g.*, capillary gel electrophoresis). in which one genetically modifies a certain gene and then determines what effects such a modification has on cellular protein expression.

General Separation Methodology

The methods of the present invention utilize a combination of electrophoretic methods conducted in series to resolve mixtures of proteins. The methods are said to be conducted in series because the sample(s) electrophoresed in each method are from solutions or fractions containing proteins electrophoresed in the preceding method, with the exception of the sample electrophoresed in the initial electrophoretic method. As used herein, the terms protein, peptide and polypeptide are used interchangeably and refer to a polymer of amino acid residues. The term also applies to amino acid polymers in which one or more amino acids are chemical analogues of corresponding naturally-occurring amino acids, including amino acids which are modified by post-translational processes (*e.g.*, glycosylation and phosphorylation).

The series of electrophoretic methods are typically conducted in such a way that proteins in an applied sample for each electrophoretic method of the series are isolated or resolved physically, temporally or spacially to form a plurality of fractions each of which include only a subset of proteins of the applied sample. Thus, a fraction refers to a protein or mixture of proteins that are resolved physically, temporally or spacially from other proteins in a sample subjected to electrophoresis. Resolved proteins can refer to a single species or a mixture of proteins that are separated from other proteins during an electrophoretic method. As just noted, samples in the various electrophoretic methods are obtained from such fractions, with the exception of the first electrophoretic method in which the sample is the original sample containing all the proteins to be separated.

Typically, these multiple electrophoretic methods in the series separate proteins according to different characteristics. For example, one method can separate proteins on the basis of isoelectric points (*e.g.*, capillary isoelectric focusing electrophoresis), other methods can separate proteins on the basis of their intrinsic or induced (through the application of a label to certain ionizable amino acid residues) charge-to-mass ratio at any given pH (*e.g.*, capillary zone electrophoresis), whereas other methods separate according to the size of the proteins (*e.g.*, capillary gel electrophoresis). Such approaches that separate proteins through a series of electrophoretic methods are referred to herein as "multidimensional" electrophoretic methods, wherein each particular electrophoretic method constitutes a "dimension."

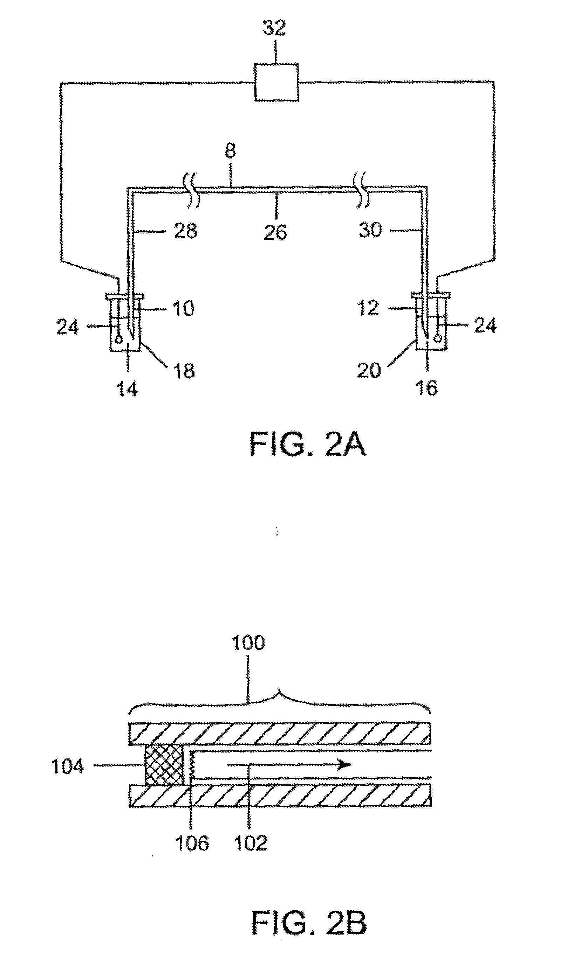

Apparatus used to conduct various electrophoretic methods are known in the art. In general, however, and as shown in FIG. 2A, the basic configuration of a typical capillary electrophoretic system utilized in certain methods of the invention includes a capillary 8 having two ends 10, 12. One end 10 is in contact with an anode solution or anolyte 14 contained in an anode reservoir 18 and the other end 12 is in contact with a cathode solution or catholyte 16 in a cathode reservoir 20. One electrode (the anode) 22 is positioned to be in electrical communication with the anode solution 14 and a second electrode 24 is positioned to be in electrical communication with the cathode solution 16. The cavity 26 of the capillary 8 is filled with an electrophoretic medium, which in some instances can include a polymer matrix. As used herein, the term anode refers to the positively charged electrode. Thus, negatively charged species move through the electrophoretic medium toward the anode. The term cathode refers to the negatively charged electrode; positively charged species migrate toward this electrode.

Sample is introduced into the capillary 8 via an inlet 28, and the protein components therein resolved as an electrical field is applied between the two electrodes 22, 24 by a power source 32 and the proteins separate within the electrophoretic medium contained within the separation cavity 26. Protein components can be controllably eluted from the capillary via outlet 30 by controlling various parameters such as electroosmotic flow (see *infra*) and/or by changing the composition of one or both of the reservoir solutions (*e.g.*, adjusting the pH or salt concentration). Typically, the inlet 28 and the outlet 30 are simply portions of the capillary formed to allow facile insertion into a container containing sample, anolyte or catholyte.

The term "capillary" as used in reference to the electrophoretic device in which electrophoresis is carried out in the methods of the invention is used for the sake of convenience. The term should not be construed to limit the particular shape of the cavity or device in which electrophoresis is conducted. In particular, the cavity need not be cylindrical in shape. The term "capillary" as used herein with regard to any electrophoretic method includes other shapes wherein the internal dimensions between at least one set of opposing faces are approximately 2 to 1000 microns, and more typically 25 to 250 microns. An example of a non-tubular arrangement that can be used in certain methods of the invention is the a Hele-Shaw flow cell. Further, the capillary need not be linear; in some instances, the capillary is wound into a spiral configuration, for example.

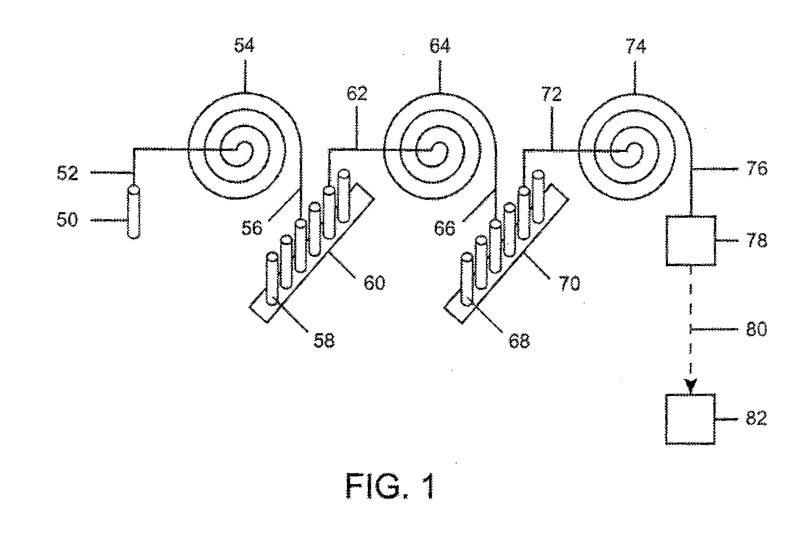

An example of a system utilized with certain methods of the invention is illustrated in FIG. 1. This particular example shows a system in which three electrophoresis methods (initial, intermediate and final methods) are linked. The particular number of electrophoretic methods conducted can vary, although the methods of the invention include at least two electrophoretic methods. Most typically, the methods utilize two or three electrophoretic separation methods.

As can be seen in FIG. 1, an initial sample containing a plurality of proteins is introduced from sample container 50 into a first separation cavity of a first capillary 54 via sample inlet 52 utilizing any of a number of methods known in the art. Examples of suitable methods include, pulling sample into the sample inlet 52 under vacuum (*e.g.,* by pulling a vacuum on the sample outlet) or pushing sample into the sample inlet 52 by pressurizing the sample container 50. Electromigration, often referred to as electrokinetic injection, is another option. Once the initial sample is introduced into sample inlet 52, the sample is then electrophoresed within the first separation cavity within the first capillary 54. The first separation cavity contains a desired electrophoretic medium in which proteins in the initial sample are at least partially resolved. Electrophoretic medium containing resolved proteins is withdrawn from the first cavity, typically out the end of the separation cavity opposite the end in which sample was introduced, although other withdrawal sites can be utilized (see *infra*). The withdrawn medium travels through outlet 56 and is collected in separate containers 58 as multiple fractions. As shown in FIG. 1B, the containers 58 into which fractions are collected are typically associated with a fraction collection device (a portion of which is shown 60) capable of automatically advancing a set of containers 58 to collect defined fractions (*e.g.*, fractions of a certain volume or covering a selected pH range).

A sample from a fraction collected from the first electrophoretic method is then withdrawn from one of the plurality of containers 58, again utilizing techniques such as those described *supra,* via a second sample inlet 62. Proteins in the sample from the fraction can then be further resolved by conducting an intermediate electrophoretic method (in the example shown in FIG. 1, the second electrophoretic method). The sample is introduced into a second capillary 64 via inlet 62 and the proteins within the sample further separated within the electrophoretic medium contained within the second separation cavity of the second capillary 64 and then eluted from the cavity via outlet 66. As with the first electrophoretic separation, the electrophoretic medium containing the resolved or partially resolved proteins is collected as separate fractions within containers 68 typically aligned and advanced by a second fraction collection device (a portion of which is shown 70).

A process similar to the second/intermediate method is conducted during the final electrophoretic method (the third electrophoretic separation method shown in FIG. 1). Sample is drawn via inlet 72 from a container 68 containing a fraction obtained during the preceding method and is introduced into a third or final electrophoretic cavity of a third capillary 74 containing a third electrophoretic medium in which proteins contained in the applied sample are separated still further yet by electrophoresis. The third electrophoretic medium containing the further isolated proteins is subsequently withdrawn through outlet 76.

As noted above, more than the three electrophoretic methods shown in FIG. 1 can be performed. Such methods essentially involve repeating the general steps described for the second/intermediate electrophoretic separation above one or more times.

Following the final electrophoretic separation, a variety of different options for analyzing the resolved proteins are available. As shown in FIG. 1, withdrawn electrophoretic medium can be passed through a detector 78 in fluid communication with the separation cavity of the last capillary 74 to detect the resolved proteins. The detector 78, or an optional quantifying device capable of receiving a signal from the detector (not shown), can be used to quantitate the amount of protein within a certain portion or fraction of the electrophoretic medium.

Alternatively, or in addition, fractions can be taken from the electrophoretic medium exiting the final capillary 74 or the detector 78 and analyzed by an analyzer 82 using some technique other than electrophoresis. Examples of such techniques include various spectroscopic methods (*e.g.*, IR, UV/VIS and NMR) and various mass spectroscopy methods (*e.g.*, electrospray ionization-time of flight [ESI-TOF] mass spectroscopy). Mass spectral data, for example, can be utilized to deduce a partial or full sequence of the protein(s) (*i.e.*, determine a protein sequence tag) within a particular fraction. FIG. 1 depicts a situation in which sample is withdrawn via line 80 (dashed to indicate optional nature of this step) to another analyzer 82 (*e.g.*, mass spectrometer).

A number of other configurations can be utilized. For example, the capillaries and detector(s) can be fabricated within a microfluidic chip (see *infra*).

The specific elution conditions utilized to withdraw resolved proteins from the separation cavity depends upon the type of electrophoretic method conducted and is described more fully below for each of the electrophoretic methods typically utilized in the present invention. In general, however, once proteins have been resolved, the conditions within the separation cavity are adjusted as necessary (or the initial conditions selected) to achieve selective or controlled elution of the proteins from the cavity. For example, elution can be achieved by adding salts to, or adjusting the pH of, the anode or cathode solution, by regulating electroosmotic flow, by applying hydrodynamic pressure or combinations of the foregoing.

Using the methods of the invention, resolved proteins can be isolated physically (*e.g.*, placement into different containers such as illustrated in FIG. 1), spatially (*e.g.*, spread throughout the electrophoretic medium contained in the separation cavity) and/or temporally (*e.g.*, controlling elution so different proteins within a sample elute from the capillary at different times). Thus, the methods of the invention can separate mixtures of proteins as a function of the composition of elution buffers and/or time, and are not limited to the spatial separation of proteins as are certain traditional two-dimensional (2-D) gel electrophoresis systems. Instead, with controlled elution, fractions can be collected so that proteins within a fraction fall within a range of isoelectric, electrophoretic mobility, or molecular weight values, for example. Controlled elution of proteins means that methods can be performed in a reproducible fashion. Such reproducibility is important in conducting comparative studies and in diagnostic applications, for example.

During the elution or withdrawing of resolved proteins, generally only a portion of the electrophoretic medium containing the resolved proteins is typically collected in any given fraction. This contrasts with certain 2-D methods in which a gel containing all the resolved proteins is exuded from the separation cavity and the exuded gel containing all the proteins is used to conduct another electrophoretic separation.

Spacially, physically or temporally resolved proteins obtained at the conclusion of one electrophoretic method are then used as the source of samples for further separation of proteins contained within the fraction during a subsequent electrophoretic method. As illustrated in FIG. 1, typically samples from different resolved fractions are sequentially electrophoresed on the same capillary. Normally another sample is not applied until the proteins in the preceding sample are sufficiently withdrawn from the separation cavity so that there is no overlap of proteins contained in different fractions. Sequential elution of fractions through the same column can significantly reduce or eliminate variations resulting from differences in cross-linking or electric field strength that can be problematic in certain slab gel electrophoretic methods. Hence, sequential separation can further enhance the reproducibility of the methods of the invention. Other methods, however, can be performed in a parallel format, wherein samples from different fractions are electrophoresed on separate capillaries. This approach allows for separations to be completed more quickly. However, the use of multiple capillaries can increase the variability in separation conditions, thereby reducing to some extent reproducibility between different samples.

In certain methods, proteins are labeled at a selected stage of the separation process and then detected using the detector. Labeling enables proteins present at low concentration to more easily be detected and enhances reproducibility by increasing signal-to-noise ratios. The detector can be used to detect proteins as separated within an electrophoretic cavity or after they are eluted from the cavity. The combination of labelling and detection also enables separated proteins to be quantified. The point in the overall method at which labeling is conducted depends in part on the particular electrophoretic methods being conducted as discussed more fully below. In general, however, labeling is typically conducted before a gel capillary electrophoretic separation is performed; whereas, labeling is normally conducted after capillary isoelectric focusing is performed rather than before. Labeling can also be used before a zone capillary electrophoresis spearation is performed as a means to modify the net charge on the proteins and their relative electrophoretic mobilities.

As noted above, some of the more commonly used electrophoretic methods utilized in the present invention are capillary isoelectric focusing electrophoresis, capillary zone electrophoresis and capillary gel electrophoresis. Specific issues regarding the performance of these methods are described in the following sections.

Capillary Isoelectric Focusing Electrophoresis (CIEF)

General

Isoelectric focusing is an electrophoretic method in which zwitterionic substances such as proteins are separated on the basis of their isoelectric points (pI). The pI is the pH at which a zwitterionic species such as a protein has no net charge and therefore does not move when subjected to an electric field. In the present invention, proteins can be separated within a pH gradient generated using ampholytes or other amphoteric substances within an electric field. A cathode is located at the high pH side of the gradient and an anode is located at the low pH side of the gradient.

Proteins introduced into the gradient focus within the pH gradient according to their isoelectric points and then remain there. General methods for conducting CIEF are described, for example, by Kilar, F., "Isoelectric Focusing in Capillaries," in CRC Handbook on Capillary Electrophoresis: A Practical Approach, CRC Press, Inc., chapter 4, pp. 95-109 (1994); and Schwartz, H., and T. Pritchett, "Separation of Proteins and Peptides by Capillary Electrophoresis: Application to Analytical Biotechnology," Part No. 266923 (Beckman-Coulter, Fullerton, CA, 1994); Wehr, T., Rodriquez-Diaz, R., and Zhu, M., "Capillary Electrophoresis of Proteins," (Marcel Dekker, NY, 1999), which are incorporated herein by reference in their entirety.

System and Solutions

Because CIEF is primarily an equilibrium technique with low current densities, capillary heating typically is not a problem. Therefore, fairly large bore capillaries can be utilized. Suitable sizes include, but are not limited to, capillaries having internal diameters of 2-600 μm, although more typically capillaries having internal diameters of 25-250 μm are utilized. The use of relatively large bore capillaries means the method can use relatively high protein loads, which facilitates detection in the following dimension(s). This feature of CIEF makes the method well-suited for the initial or one of the early electrophoretic separations in the series. However, smaller diameter capillaries enable temperature to be controlled more carefully and, in some methods, result in improved signal detection (*e.g.*, by laser induced fluorescence (LIF) detection of fluorescently labeled proteins).

The capillaries can have varying lengths. The length selected depends in part on factors such as the extent of separation required. Typically, the capillaries are about 10 to 100 cm in length, although somewhat shorter and longer capillaries can be used. While longer capillaries typically result in better separations and improved resolution of protein mixtures, longer capillaries also afford more opportunities for protein-wall interactions and lower field strength. Consequently, there tends to be an upper limit on capillary length beyond which resolution may be lost. Longer capillaries can be of particular use in resolving low abundance proteins. Further guidance on size and length of capillaries is set forth, for example, in Palmieri, R. and J. A. Nolan, "Protein capillary electrophoresis: Theoretical and experimental considerations for methods development," in: CRC Handbook of Capillary Electrophoresis: A Practical Approach, Chp. 13, pgs. 325-368 (CRC Press, Boca Raton, 1994).

Generally, the capillaries are composed of fused silica, although plastic capillaries and PYREX (*i.e.*, amorphous glass) can be utilized in certain methods. As noted above, the capillaries do not need to have a round or tubular shape. Other shapes wherein the internal dimension between opposing faces is within the general range set forth in this section can also be utilized.

A variety of different anode and cathode solutions can be used. Common solutions include sodium hydroxide as the catholyte and phosphoric acid as the anolyte. Similarly, a number of different ampholytes can be utilized to generate the pH gradient, including numerous commercially available ampholyte solutions (*e.g.*, BioLyte, Pharmalyte and Servalyte). The selection of ampholytes and the breadth of the ampholyte gradient can impact the resolution that is achieved by CIEF methods. Narrow ampholyte gradients increase the number of theoretical plates in the separation and can be beneficial for higher resolution separations over narrow pI ranges.

CIEF methods utilized in the separations of the invention can be conducted in capillaries containing polymeric matrices or in free solution (*i.e.*, no gel or other polymeric matrix). Polymer matricies are typically added to slow electroosmotic flow; however, in some instances, inclusion of polymeric matrices can restrict movement of larger proteins (*see*, *e.g.*, Patton, 26). The use of free solutions is preferable in such cases possibly in combination with other methods (*e.g.*, capillary coatings, gel plugs, or induced electric fields) to control the electroosmotic flow.

<u>Sample Preparation</u>

Typically protein samples to be electrophoresed by CIEF are denatured prior to loading the sample into the capillary. This ensures that the same proteins all have the same charge and thus identical proteins focus at the same location rather than potentially at multiple zones within the capillary. Denaturants (*e.g.*, urea), non- and zwitterionic-surfactants (e.g., IGEPAL CA-630 or 3-[{3-cholamidopropyl}dimethylammonio]-1-propane sulfonate) can also be used to suppress protein-wall and/or protein-protein interactions that can result in protein precipitation. Another advantage of denaturing the proteins prior to electrophoresis is that the results can be used in comparisons with archival data typically obtained under denaturing conditions.

A typical denaturing buffer includes urea and a nonionic or zwitterionic surfactant as denaturants; a reducing agent (*e.g.*, dithiothreitol (DTT) or mercaptoethanol) is typically included to reduce any disulfide bonds present in the proteins. Other denaturants besides urea that can be used include, but are not limited to, thiourea and dimethylformamide (DMF). Generally, guanidine hydrochloride is not utilized as a denaturant because of the very high ionic strength it imparts to a sample. Exemplary neutral detergents include polyoxyethylene ethers ("tritons"), such as nonaethylene glycol octylcyclohexyl ether ("TRITON" X-100), polyglycol ethers, particularly polyalkylene alkyl phenyl ethers, such as nonaethylene glycol octylphenyl ether ("NONIDET" P-40 or IGEPAL CA-630), polyoxyethylene sorbitan esters, such as polyoxyethylene sorbitan monolaurate ("TWEEN"-20), polyoxyethylene ethers, such as polyoxyethylene lauryl ether ($C_{12}E_{23}$) ("BRIJ"-35), polyoxyethylene esters, such as 21 stearyl ether ($C_{18}E_{23}$) ("BRIJ"721), N,N-bis[3-gluconamidopropyl]cholamide ("BIGCHAP"), decanoyl-N-methylglucamide, glucosides such as octylglucoside, 3-[{3-cholamidopropyl}dimethylammonio]-1-propane sulfonate and the like.

The optimal amount of denaturant and detergent depends on the particular detergent used. In general the denaturing sample buffers contain up to 10 M urea (more typically 4-8 M and most typically 6-8 M). Specific examples of suitable buffers (and denaturants and nonionic surfactants for inclusion therein) include those described by Hochstrasser *et al.*[5] and O'Farrell[6]. Denaturation is typically advanced by heating for 10 min at 95 °C prior to injection into the capillary. Adjustments in the denaturing sample buffers are made as necessary to account for any electroosmotic flow or heating effects that occur (*see, e.g.,* Kilar, F., "Isoelectric Focusing in Capillaries," in CRC Handbook on Capillary Electrophoresis: A Practical Approach, CRC Press, Inc., chapter 4, pp. 95-109 (1994)).

The amount of protein within a sample can vary and, as noted above, depends in part of the size of the capillary used. In general, the capillary is loaded with 0.1 to 5.0 mg of total protein. Samples can be spiked with one or more known pI standards to assess the performance of the method.

Elution

A variety of techniques can be utilized to elute or withdraw electrophoretic medium containing resolved proteins out from the capillary, but these methods fall into three general categories: hydrodynamic elution, electroelution and control of electroosmotic flow.

Hydrodynamic/Pressure Elution

Hydrodynamic or pressure elution involves applying pressure (or pulling a vacuum) via an appropriate pump connected with one end of the capillary (*see, e.g.* Kilar, F., "Isoelectric Focusing in Capillaries," in CRC Handbook on Capillary Electrophoresis: A Practical Approach, CRC Press, Inc., chapter 4, pp. 95-109 (1994)). However, hydrodynamic elution can cause band broadening and loss of resolution due to the parabolic flow profile that is formed in the capillary.

Electroelution

Electroelution, the other major approach, encompasses a variety of techniques and in general involves altering the solution at the anode and/or cathode to change some parameter (*e.g.*, pH, ionic strength, salt concentration) of the electrophoretic medium in the separation cavity sufficiently to effect elution.

Salt mobilization

One electroelution approach involves addition of a salt to the catholyte or anolyte, the salt having a non-acidic or non-basic counterion of the same charge as the acidic or basic species within the reservoir to which the salt is added so that the counterion migrates from the reservoir into the capillary. Since electrical neutrality must be maintained within the capillary, the movement of the counterion into the capillary results in a reduction of the concentration of protons or hydroxide within the capillary, and thus the pH is either raised or lowered. The theoretical basis for this type of mobilization is described by. S. Hjerten, J.-L. Liao, and K. Yao, J. Chromatogr., 387: 127 (1987). For example, if the catholyte is sodium hydroxide (*i.e.*, the basic species is hydroxide) then a salt having a negatively charged counterion other than hydroxide is added, for example sodium chloride. Movement of chloride ion into the capillary reduces the local concentration of hydroxide within the capillary, thereby decreasing the pH. As another example, if the anolyte is phosphoric acid, then a salt having a counterion other than a proton is added, for example sodium phosphate, In this instance, movement of sodium ion into the capillary reduces the local concentration of protons within the capillary thereby increasing the pH. As the pH is lowered or raised within regions of the capillary due to the presence of the added counterion, elution occurs since the ampholytes, and the focused proteins, migrate to the newly-defined pH regions corresponding to their isoelectric points. It has been shown that both the type and concentration of salt used for mobilization has impact on the resolution of eluted protein peaks [R. Rodriguez-Diaz, M. Zhu, and T. Wehr, J. Chromatogr. A, 772:145 (1997)]. In particular, the addition of sodium tetraborate instead of sodium chloride to the catholyte results in greatly increased resolution of separated proteins.

pH mobilization

Another technique, referred to herein as " pH mobilization" can also be utilized to elute proteins during CIEF. In this approach, an additive is added to either the anode or cathode solution to alter the pH of the solution. Unlike salt mobilization, however, the additive does not contribute a mobile counterion that moves into the capillary. Here, the elution occurs as a result of the pH gradient being redefined by the pH of one or both of the reservoirs; therefore, proteins with pI's that fall outside of this redefined pH gradient are eluted into either the anode or cathode reservoirs. Typically, the technique for cathodic mobilization would proceed as follows. Once the proteins are focused in an exemplary pI range of 3-10 using phosphoric acid as the anolyte and sodium hydroxide as the catholyte, the cathodic capillary end is immersed into a reservoir containing a solution that has a pH slightly less than 10, for example 50 mM imidazole (pKa 7) which has a pH of 9.85. The proteins are then allowed to refocus in the capillary, recognizable by a stabilization of the current through the capillary, the pI range now being defined by 3-9.85. Any proteins with an isoelectric point of 9.85 to 10 are eluted into the catholyte. The process can be repeated with catholyte containing a species that reduces the pH to slightly less than 9.85. In a stepwise fashion, the pH can be continued to be reduced to pH 7, thereby collecting separated proteins in fractions that span the range of 7~10. At this point, anodic mobilization can proceed by replacing the anolyte with acids of increasing pKa to selectively increase the pH from 3 to 7, thereby collecting fractions in the acidic range (pH 3-7). The number of fractions can vary depending on the desired fractionation resolution. Typically, these fractions are defined by differences of 0.05-0.5 pH units.

The technique of pH mobilization can be useful for protein samples containing a high concentraton of one or more proteins that may cause uneven spatial gradients inside the capillary. Using pH mobilization, only those proteins with isoelectric points below or above the pI range that is defined by the reservoir pH's are eluted. This elution would, therefore, be reproducible regardless of differences in the shape of the capillary pH gradient or the presence of uneven spatial gradients inside the capillary.

Electroosmotic Flow (EOF)

Regulating the magnitude of electroosmotic flow (EOF) significantly affects the preceding electroelution methods (see *supra*) and is another means by which resolved proteins can be selectively withdrawn upon conclusion of an isoelectric focusing separation. EOF is generated by the ionization of silanol functionalities on the surface of a silica capillary. Such ionization results in a layer of protons in the electrophoretic medium at the surface of the silica capillary. Once an electric field is applied, the layer of protons essentially constitutes a positively charged column of fluid which migrates toward the cathode, thereby causing bulk flow of the electrophoretic medium within the capillary. Apparent velocity of analytes is equal to the sum of the electroosmotic flow and their electrophoretic mobility. Thus, by controlling EOF, one can control or regulate the rate at which proteins move through the capillary. In CIEF methods, generally EOF should be controlled to allow proteins within an injected sample sufficient time to focus before the proteins begin eluting from the capillary.

A variety of techniques can be utilized to regulate EOF. One approach involves coating the walls of capillaries with various agents. For example, EOF along glass silicate surfaces can be substantially reduced by silanizing them with a neutral silane reagent that masks a substantial percentage of surface silanol groups (*e.g.*, polyacrylamide, polyethylene glycol and polyethylene oxide). The magnitude of EOF can be further controlled by using silanizing reagents that include positively or negatively charged groups. Positively charged coatings can be used to nullify surface negative charges to give a net surface charge of zero, so that EOF approaches zero. Coatings with higher positive charge densities can be used to reverse the direction of EOF for charged surface materials. This can be useful for slowing the net migration rates of positively charged sample species. Conversely, negatively charged coatings can be used to impart to or increase the magnitude of the negative charge on surfaces, so as to increase the net migration rates of negatively charged species. Representative positively charged coatings include trialkoxysilanes with polyethyleneimine, quaternized polyethyleneimine, poly(N-ethylaminoacrylamide) and chitosans, for example. Representative negatively charged coatings include trialkoxysilanes with carboxylate and sulfonate containing materials such as poly(methylglutamate) and 2-acrylamido-2-methylpropanesulfonate polymers, for example. It will be recognized that charged coatings can also effectively reduce sample adsorption, especially for samples having the same charge polarity as the coating.

The separation medium can also include soluble agents for dynamically coating the walls of the separation cavity, to help reduce EOF during electrophoresis. Such soluble coating agents include quaternary ammonium-containing polymers, methyl cellulose derivatives, cellulose acetate, polyethylene oxide, chitosan, polyvinyl alcohol, polyethylene glycol, polyethylenimine, and polyethylene oxide-polypropylene oxide-polyethylene oxide triblock copolymers, for example. Typically, soluble coating agents are included at concentrations of about 0.05% to about 4%, and more typically of about 1% to about 2%.

EOF and sample absorption can also be adjusted by including suitable reagents in the separation medium and running buffers. For example, negative surface charges can be masked by including a cationic additive in the medium, such as metal amine complexes, amines and polyamines such as propylamine, triethylamine, tripropylamine, triethanolamine, putrescine, spermine, 1,3-diaminopropane, morpholine, and the like. Zwitterionic species comprising both negatively and positively charged groups that are isoelectric at the pH of electrophoresis can also be used, such as trialkylammonium propyl sulfonates, where alkyl is methyl, ethyl, propyl, and longer alkyl chains.

Another approach involves the generation of a current that opposes EOF. Typically, this is accomplished by applying a thin film of metal (*e.g.*, iridium tin oxide or copper) to an external surface of the capillary. Application of current to the film generates a relatively small induced current within the capillary to reverse the EOF (*see*, *e.g.*, Schasfoort, R.B.M., Schlautmann, S., Hendrikse, J., and van den Berg, A., "Field-Effect Flow Control for Microfabricated Fluidic Networks," Science, 286:942-945

Placing a porous plug at a location upstream from where sample is introduced (upstream referring to a direction opposite the flow of proteins through the capillary) can also be utilized to control EOF. An example illustrating the location of the plug is illustrated in FIG. 2B where the capillary 100 extends from the anode reservoir (not shown) at one end and the cathode reservoir at the other end (not shown). Protein migration is in the direction of arrow 102 (*i.e.,* from the anode to cathode direction).

As can be seen, the porous plug 104 is positioned to be upstream of the trailing edge 106 of the sample once introduced into the capillary 100. The porous plug 104 is typically formed of a polymeric material and remains relatively stationary during electrophoretic runs. Examples of suitable materials from which the plug can be formed include polymerized acrylamide with diacrylamide crosslinkers and agarose. Although not intending to be bound by any particular theory, the porous plug 104 appears to function as a momentum transfer barrier by blocking replacement of bulk fluid that in the absence of the plug 104 would move toward the cathode reservoir.

In some methods, such as those containing large amounts of protein and/or a large number of different proteins, EOF should be reduced to very low levels to allow proteins the opportunity to focus before the electrophoretic medium begins eluting from the capillary due to EOF. In certain methods an EOF of = 0.5 x 10 $^{-6}$ cm$^2$/V-s (at pH 8.6, and 25 mM TRIS-phosphate) has been found to allow ample time for the necessary focusing of proteins before sample elutes from the capillary. Methods described above can reduce EOFs to these levels.

Thus, the foregoing approaches enable fractions to be collected according to different criteria. Electroelution techniques, for example, can be used to collect fractions having a defined pH range. EOF elution and pressure elution, in contrast, can be used to separate fractions according to time of elution. Other techniques can also be utilized to elute resolved proteins after CIEF (*see*, *e.g.* Kilar, F., "Isoelectric Focusing in Capillaries," in CRC Handbook or Capillary Electrophoresis: A Practical Approach, CRC Press, Inc., chapter 4, pp. 95-109 (1994)). The controlled elution techniques are useful for enhancing reproducibility, an important factor in comparative and diagnostic methods. Such techniques also provide improved tolerance of high abundance proteins as compared to methods relying on spatial separation.

Capillary Zone Electrophoresis (CZE)

General

Capillary zone electrophoresis is an electrophoretic method conducted in free solution without a gel matrix and results in the separation of molecules such as proteins based upon their intrinsic charge-to-mass ratio. One advantage to CZE methods is the ability to run with solvent systems that would normally be incompatible with typical water soluble gel matrices. Nonaqueous or water miscible solvent systems can be used to improve the solubility of hydrophobic and membrane bound proteins that would normally not be resolved by gel electrophoretic methods. General methods for conducting the method are described, for example, by McCormick, R.M. "Capillary Zone Electrophoresis of Peptides," in CRC Handbook of Capillary Electrophoresis: A Practical Approach, CRC Press Inc., chapter 12, pp. 287-323 (1994); Jorgenson, J.W. and Lukacs, K.D., J. High Resolut. Chromatogr. Commun., 4:230 (1981); and Jorgenson, J.W. and Lukacs, K.D., Anal. Chem. 53:1298 (1981)), each of which is incorporated by reference in its entirety.

System and Solutions

In general, the capillaries described above for CIEF are also suitable for conducting CZE methods. Often the capillaries have internal diameters of about 50 to 100 microns. Buffer composition and pH can significantly influence separations since separations in CZE are based upon charge-to-mass ratios and the charge of a protein is dependent upon the pH of the surrounding solution. At the extremes of pH (*i.e.*, below 2 and above 10) it is typically difficult to achieve resolution of proteins because all residues are either fully protonated or deprotonated and many proteins have a similar number of acidic and basic residues per unit mass. Selectivity is typically enhanced at intermediate pH. For proteins having a relatively high percentage of acidic residues, selectivity can often be enhanced near pH 4.5. For those proteins having a high concentration of basic residues, selectivity can be enhanced near pH 10.

In CZE, solutions at the anode and cathode are typically the same. The buffer utilized can be essentially any buffer, the choice of buffer being controlled in part by the pH range at which the electrophoretic method is conducted and its influence on the detector noise. Examples of useful buffers at low pH include, but are not limited to, phosphate and citrate; useful buffers at high pH include Tris/Tricine, borate and CAPS (3-(cyclohexylamino)-1-propane sulfonic acid). Further guidance regarding suitable buffers and buffer additives is described by McCormick, R.M. "Capillary Zone Electrophoresis of Peptides," in CRC Handbook of Capillary Electrophoresis: A Practical Approach, CRC Press Inc., chapter 12, pp. 287-323 (1994).

Elution

Elution can be accomplished utilizing some of the same methods described above for CIEF, namely pressure and EOF. As with CIEF, controlling EOF can be important in certain methods to prevent Electrophoretic medium containing protein from eluting from the capillary before the proteins within the loaded sample have had an opportunity to separate. EOF can be controlled using the same methods utilized for controlling EOF in CIEF methods (*e.g.*, coating the internal walls of the capillary, using a porous plug and generating an induced field to counteract EOF). Regulating and carefully selecting the pH and ionic strength of the electrophoretic medium is another technique that can be used. Because EOF results from ionization of the silanol groups on the interior capillary surface, by conducting CZE at relatively low pH (*e.g.*, pH 2-5, more typically about pH 3-4) the number of silanol groups that are ionized is reduced. Such a reduction reduces EOF. To prevent sample elution prior to complete separation, in certain analyses the EOF should be reduced to < 1 x 10$^{-4}$ cm$^2$/V-s (at pH 8.6 and 25 mM TRIS-phosphate buffer). EOFs of this level can be obtained using the methods just described.

Another approach that is described more fully below in the detection and labeling section is to label proteins in the sample prior to injecting the sample containing the protein into the capillary. By selecting labels that preferentially react with certain functional groups such as amino or carboxyl groups, the charge-to-mass ratio of certain proteins can be altered. Such alterations can improve the resolution of proteins during electrophoresis as well as improve their detectability. (See Examples 1 and 2 below).

Capillary Gel Electrophoresis (CGE)

General

Capillary gel electrophoresis refers to separations of proteins accomplished by sieving through a gel matrix, resulting in the separation of proteins by size. In one format, proteins are denatured with sodium dodecyl sulfate (SDS) so that the mass-to-charge ratio is determined by this anionic surfactant rather than the intrinsic mass-to-charge ratio of the protein [50, 2]. This means that proteins can be separated solely on the basis of size without charge factoring into the degree of separation. The application of general SDS PAGE electrophoresis methods to capillary electrophoresis (CGE) is described, for example, by Hjertén, S., "Free zone electrophoresis," Chromatogr. Rev., 9:122 (1967).

System and Solutions

The type of capillaries and their size are generally as described above for CZE. A variety of different buffers can be used, including commercially available buffers such as the "eCAP SDS" buffers manufactured by Beckman (see, also, 51, 30, 9 and 5). Various buffer additives can be utilized to increase resolution. Such additives, include, but are not limited to, small amounts of organic solvents, such as N,N-dimethylformamide, cyclohexyldiethylamine, dimethoxytetraethylene glycol and other polyols (*e.g.*, ethylene glycol and polyethylene glycol) (*see, e.g.,* [2] and [3]). The use of such solvents can improve the solubility of proteins in aqueous solution and enhance protein stability against thermal denaturation, [52] depress the electroosmotic flow in CZE and CGE [53], alter the electrical double-layer thickness at the capillary wall to inhibit protein binding interactions [47] and increase the viscosity of the running buffer which depresses the electroosmotic flow. Solvents utilized should be compatible with the polymer matrix inside the capillary.

Isotachophoresis (IPE) can be used in certain methods to increase resolution of proteins. For a general discussion of IPE, see, for example, B.J. Wanders and Everaerts, F.M., "Isotachophoresis in Capillary Electrophoresis," in CRC Handbook of Capillary Electrophoresis: A Practical Approach, chap. 5, pp. 111-127 (1994), which is incorporated by reference in its entirety. The velocity of a charged molecule moving through a capillary under a constant field strength depends on its relative mobility, which is a function of the mass/charge of the molecule, temperature, and viscosity of the medium through which it is moving. However, in the absence of an adequate concentration of highly mobile ions upstream of the sample ions, all the ions eventually have to migrate at the speed of the slowest ion once the electric field reaches a steady-state inside the capillary. This condition causes the anions to stack in order of their relative mobilities at the interface of the leading and terminating buffers.

Under SDS denaturing conditions, all the proteins present in the sample have nearly identical mass/charges. By using a higher mass/charge anion in the terminal buffer, one can force the proteins to move at a constant slow speed through the capillary. This has two effects. First, proteins "stack" at the terminal edge of the leading buffer increasing their effective concentration inside the capillary. Second, any separation between proteins is based on their size. Therefore, the use of a hybrid IPE-CGE method in which the IPE is used for sample "stacking" can improve the resolution possible in the subsequent CGE separation in some methods.

Various terminal buffer systems can be utilized in conjunction with IPE methods. In one system, ε-aminocaproic acid (EACA) is used as the terminal electrolyte because it has a high mass/charge at high pH (>6). Tris(hydroxyethyl) aminomethane (TRIS) citrate at 0.05M is used as the leading buffer at pH=4.8 and as an intermediate stacking buffer at pH=6.5. The sample proteins initially "stack" because EACA has a very low mobility in the pH 6.5 stacking buffer, but once the protein "stack" and EACA reach the lower pH leading buffer, the mobility of the EACA surpasses that of the proteins and separation commences (*see, e.g.,* [57]). This system can be used to create a hybrid single column IPE-CPAGE system.

A 2 buffer system for IPE for the separation of proteins involves dissolving sample in 0.01M acetic acid, which is also used as the terminal electrolyte. The leading and background buffer was 0.02M triethylamine-acetic acid solution at pH 4.4. The sample in terminal buffer is sandwiched between the leading and background buffer. IPE continues until the background buffer overtakes the leading edge of the terminal buffer, at which point IPE stops and separation begins (*see, e.g.*, [58]).

Another IPE approach that can be accomplished with any running buffer is to dissolve the sample in the running buffer but diluted to a lower ionic strength. This causes an increase in the electrical resistance in the capillary where the sample plug is loaded and correspondingly faster movement of the ions present in the sample matrix to running buffer boundary. The optimal ionic strength difference between the sample matrix and the running buffer is typically about 10-fold (*see, e.g.*, [43]).

Elution

In general, the discussion of elution for CZE applies to CGE. Elution can be accomplished utilizing pressure and EOF. As with CIEF and CZE, controlling EOF can be important in certain methods to prevent electrophoretic medium containing protein from eluting before the proteins within the applied sample have had an opportunity to separate. The methods described *supra* for CIEF and CZE can be used to control EOF at desired levels. To prevent sample elution prior to complete separation, in certain analyses the EOF should be reduced to < 1 x $10^{-4}$ $cm^2$/V-s (at pH 8.6 and 25 mM TRIS-phosphate buffer). EOF can be reduced to this range, for example, by controlling the pH of the buffer, by generation of a counteracting induced field, capillary coatings and a porous gel plug.

Combination with Detection Steps

In some instances, the proteins separated by the methods of the invention are subjected to further analysis by mass spectroscopy. In such instances, particular labels can be utilized to enhance separation of mass fragments into certain parts of the mass spectrum. Suitable labels in such methods are set forth more fully in copending application number 09/513,395, entitled "Methods for Protein Sequencing", having attorney docket number 020444-000300US, filed on February 25, 2000. This application is incorporated herein by reference in its entirety.

Quantitation of detected signals can be performed according to established methods. Peak height and peak area are typically used to quantify the amount of each resolved protein in the final electrophoretic dimension. In some methods, the peak height, peak width at the half height, peak area, and elution time for each peak are recorded. Peak shape (determined as the height to width ratio) can be used as a measure of the quality of the separation method. The resolution potential of the method can be determined by correlating the MW of the protein with the elution time (see, *e.g.*, [30] and [11]). By dividing the overall run time by the average peak width of each protein an estimate of the total number of proteins that can be resolved by the method (*e.g.*, proteins separated by at least one peak width can be considered a "resolved" protein) can be obtained. The reproducibility of the MW estimate can be determined by two methods. In one method, the apparent MW determined for each protein in three replicate runs by establishing the standard curve from one run and using that curve to determine the MW based on elution time from each subsequent run are compared (see, *e.g.,* [21]). In the second approach, the overall error of the method is determined from the standard deviation in the slope of the standard curve created using the data from all three replicate runs.

The labeling and direct detection approaches that can be used with certain methods of the invention can yield improved reproducibility in the quantification of relative protein expression levels compared to the staining and imaging methods utilized in conventional 2-D gels. Staining techniques frequently yield poorly quantitative results because varying amounts of stain are incorporated into each protein and the stained protein must be detected and resolved against the stained background of the gel or electroblotting substrate. Moreover, since the methods utilize combinations of Electrophoretic methods, an electropherogram that is directly comparable to archived 2-D gel image data is still obtained. This means that the methods remain comparable to 2-D gel information as compared to other non-electrophoretic based separations (*e.g.*, LC/MS/MS).

Exemplary Systems

The methods of the invention are amenable to a variety of different electrophoretic methods. The controlled elution techniques whereby defined fractions are separated spatially, physically or by time, and the labeling and detection methods can be utilized in a number of different electrophoretic techniques. As noted above, the number of electrophoretic methods linked in series is at least two, but can include multiple additional electrophoretic methods as well. In some instances, each electrophoretic method in the series is different; whereas, in other instances certain electrophoretic methods are repeated at different pH or separation matrix conditions.

Despite the general applicability of the methods, as noted above CIEF, CZE and CGE methods are specific examples of the type of electrophoretic methods that can be utilized according to the methods of the invention. In certain methods, only two methods are performed. Examples of such methods include a method in which CIEF is performed first followed by CGE. Labeling is typically performed after CIEF with detection subsequent to elution of protein from the CGE capillary. Protein eluting from the CIEF capillary can be detected using a UV/VIS spectrometer at 214 or 280 nm, for example. In another system, the first method is CZE and the final method is CGE. With this arrangement, labeling is typically performed prior to CZE to enhance resolution as described *supra.* Detection generally is not performed until the completion of the final electrophoretic separation. A third useful approach involves initially conducting CIEF followed by CZE and CGE. Labeling for such a system is typically done after CIEF and before CZE. Labeling at this point in the overall method avoids alteration of CIEF patterns (see *supra*) and allows for greater resolution during CZE. Detection is generally conducted at the conclusion of CGE (*i.e.*, with resolved protein within the capillary or after the proteins have eluted from the capillary). These are specific examples of systems that can be utilized; it should be understood that the invention is not limited to these particular systems. Other configurations and systems can be developed using the techniques and approaches described herein.

Samples

The methods of the invention can be used with a wide range of sample types. Essentially any protein-containing sample can be utilized with the methods described herein. The samples can contain a relatively small number of proteins or can contain a large number of proteins, such as all the proteins expressed within a cell or tissue sample, for example.

Samples can be obtained from any organism or can be mixtures of synthetically prepared proteins or combinations thereof. Thus, suitable samples can be obtained, for example, from microorganisms (*e.g.*, viruses, bacteria and fungi), animals (*e.g.*, cows, pigs, horses, sheep, dogs and cats), hominoids (*e.g.*, humans, chimpanzees, and monkeys) and plants. The term "subject" as used to define the source of a sample includes all of the foregoing sources, for example. The term "patient" refers to both human and veterinary subjects. The samples can come from tissues or tissue homogenates or fluids of an organism and cells or cell cultures. Thus, for example, samples can be obtained from whole blood, serum, semen, saliva, tears, urine, fecal material, sweat, buccal, skin, spinal fluid, tissue biopsy or necropsy and hair. Samples can also be derived from *ex vivo* cell cultures, including the growth medium, recombinant cells and cell components. In comparative studies to identify potential drug or drug targets (see *infra*), one sample can be obtained from diseased cells and another sample from non-diseased cells, for example.

Sample preparation for the different electrophoretic techniques is set forth above. If the sample contains cellular debris or other non-protein material that might interfere with separation during electrophoresis, such materials can be removed using any of a variety of known separation techniques including, for example, forcibly exuding the sample through sieve material, filtration and centrifugation. Samples whose ionic strength is particularly high can be desalted using established techniques such as dialysis and dilution and reconcentration.

In some instances in which the sample contains salts or other interfering components, buffer exchange can be performed to improve IPE "stacking" and improve reproducibility in elution times and peak shapes for electrophoretic methods. One useful way to implement dialysis to remove interfering compounds is to collect fractions directly in the dialysis chamber of a spin dialysis tube (Gilson/Amicon). The sample can then be spin dialyzed and resuspended in a 10-fold dilution of the running buffer to be utilized in the next electrophoretic separation of the series. This procedure has the advantages that: (1) in the case of CIEF, larger volumes of buffers can be used during electroelution of each fraction without diluting the proteins in each fraction, (2) the same sample volume can be used for each fraction injected into the second dimension and (3) smaller more concentrated sample volumes can be used in the second dimension because the dialyzed proteins can be resuspended in almost any buffer volume after dialysis.

Variations

The methods of the invention need not end with the last electrophoretic method of the series. As illustrated in FIG. 1, resolved proteins can be further analyzed by non-electrophoretic methods. Examples of such methods include infra-red spectroscopy, nuclear magnetic resonance spectroscopy, UV/VIS spectroscopy and complete or partial sequencing. Coupling the current electrophoretic-based method to various mass spectroscopy (MS) methods is one specific example of further analysis that can be conducted. A variety of mass spectral techniques can be utilized including several MS/MS methods and Electrospray-Time of Flight MS methods (see, *e.g.,* [61], [62], [63], and [64]). Such methods can be used to determine at least a partial sequence for proteins resolved by the electrophoretic methods such as a protein sequence tag (for a discussion or protein sequence tags, see, *e.g.,* [65] and [66]). Further discussion regarding combining the electrophoretic separations described herein with mass spectral analysis is set forth in U.S. provisional application 60/130,238 entitled "Rapid and Quantitative Protein Expression and Sequence Determination," filed April 20, 1999, and to which this application claims benefit and which is incorporated by reference in its entirety. Other mass spectral methods that can be combined with the methods of the present invention are described in copending U.S. application 09/513,395, entitled "Methods for Protein Sequencing," and having attorney docket number 020444-000300US, and copending U.S. application 09/513,486, entitled "Protein Separation via Multidimensional Electrophoresis" and, having attorney docket number 020444-000200US, both filed February 25, 2000 and both being incorporated by reference in their entirety.

Microfluidic Systems

In another variation, the capillaries are part of or formed within a substrate to form a part of microfluidic device that can be used to conduct the analyses of the invention on a very small scale and with the need for only minimal quantities of sample. In these methods, physical fractions of samples typically are not collected. Instead, resolved proteins are separated spatially or by time. Methods for fabricating and moving samples within microfluidic channels or capillaries and a variety of different designs have been discussed including, for example, U.S. Pat. Nos. 5,858,188; 5,935,401; 6,007.690; 5,876,675; 6,001,231; and. 5,976,336, all of which are incorporated by reference in their entirety.

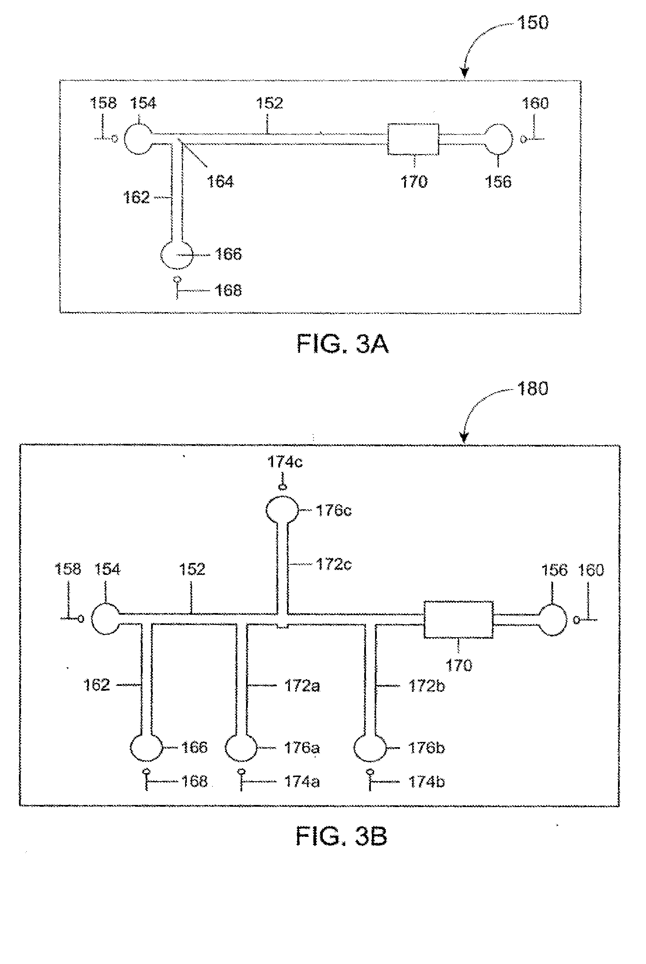

An example of a general system 150 that can be used with the methods of the present invention is depicted in FIG. 3A. The capillaries or channels are typically formed or etched into a planar support or substrate. A separation capillary 152 extends from an anode reservoir 154 containing anolyte to a cathode reservoir 156. The anode reservoir 154 and the cathode reservoir 156 are in electrical contact with an mode and cathode 158, 160, respectively. A sample injection channel 162 runs generally perpendicular to the separation capillary 152 and one end intersects at an injection site 164 slightly downstream of the anode reservoir 154. The other end of the sample injection capillary 162 terminates at a sample reservoir 166, which is in electrical communication with a sample reservoir electrode 168. A detector 170 is positioned to be in fluid communication with electrophoretic medium passing through the separation capillary 152 and is positioned downstream of the sample injection site 164 and typically somewhat upstream of the cathode reservoir 156. In this particular configuration, fractions are withdrawn into the cathode reservoir. Movement of electrophoretic medium through the various channels is controlled by selectively applying a field via one or more of the electrodes 158, 160 168. Application of a field to the electrodes controls the magnitude of the EOF within the various capillaries and hence flow through them.

An example of another configuration is illustrated in FIG, 3B. This system 180 includes the elements described in the system shown in FIG. 3A. However, in this arrangement, spacially or temporally resolved fractions can be withdrawn at multiple different locations along the separation capillary 152 via exit capillaries 172a, 172b and 172c. Each of these capillaries includes a buffer reservoir 176a, 176b, 176c, respectively, and is in electrical communication with electrodes 174a, 174b, 174c, respectively. Movement of electrophoretic medium along separation capillary 152 and withdrawal of fractions therefrom into the exit capillaries 172a, 172b and 172c can be controlled by controlling which electrodes along the separation capillary 152 and which of the exit capillary electrodes are activated. Alternatively, or in addition, various microfluidic valves can be positioned at the exit capillaries 172a, 172b and 172c to control flow. Typically, additional detectors are positioned at the various exit capillaries 172a, 172b and 172c to detect protein in fractions withdrawn into these capillaries.

The configuration illustrated in FIG. 3B can be used in a number of different applications. One example of an application for which this type of system is appropriate is a situation in which the type of samples being examined have been well characterized. If for example, certain fractions of proteins of interest have been previously established to fractionate at a particular location in the separation capillary 152, then the exit capillaries 172a, 172b and 172c can be positioned at those locations to allow for selective removal of the protein fraction(s) of interest.

In still another configuration, multiple exit capillaries branch from the end of the separation capillary 152 near the cathode reservoir 156, each exit capillary for withdrawing and transporting separate fractions. In this configuration also, withdrawal of fractionated protein from the separation capillary can be controlled by regulating EOF within the various capillaries and/or by microfluidic valves.

Other components necessary for conducting an electrophoretic analysis can be etched into the support, including for example the reservoirs, detectors and valves discussed *supra.*

Substrates

The substrate upon which the capillary or micro-channel network of the analytical devices of the present invention are formed can be fabricated from a wide variety of materials, including silicon, glass, fused silica, crystalline quartz, fused quartz and various plastics, and the like. Other components of the device (e,g., detectors and microfluidic valves) can be fabricated from the same or different materials, depending on the particular use of the device, economic concerns, solvent compatibility, optical clarity, mechanical strength and other structural concerns. Generally, the substrate is manufactured of a non-conductive material to allow relatively high electric fields to be applied to electrokinetically transport the samples through the various channels.

In the case of polymeric substrates such as plastics, the substrate materials can be rigid, semi-rigid, or non-rigid, opaque, semi-opaque or transparent, depending upon the use for which the material is intended. Plastics which have low surface charge when subjected to the electric fields of the present invention and thus which are of particular utility include, for example, polymethylmethacrylate, polycarbonate, polyethylene terepthalate, polystyrene or styrene copolymers, polydimethylsiloxanes, polyurethane, polyvinylchloride, polysulfone, and the like.

Devices which include an optical or visual detector are generally fabricated, at least in part, from transparent materials to facilitate detection of components within the separation channel by the detector.

Channel Structure/Formation

The size and shape of the channels or capillaries formed in the substrate of the present devices can have essentially any shape, including, but not limited to, semicircular, cylindrical, rectangular and trapezoidal. The depth of the channels can vary, but tends to be approximately 10 to 100 microns, and most typically is about 50 microns. The channels tend to be 20 to 200 microns wide.

Manufacturing of the channels and other elements formed in the surface of the substrate can be carried out by any number of microfabricating techniques that are known in the art. For example, lithographic techniques may be employed in fabricating glass or quartz substrates, for example, using established methods in the semiconductor manufacturing industries. Photolithographic masking, plasma or wet etching and other semiconductor processing technologies can be utilized to create microscale elements in and on substrate surfaces. Alternatively, micromachining methods, such as laser drilling, micromilling and the like, can be utilized. Manufacturing techniques for preparing channels and other elements in plastic have also been established. These techniques include injection molding techniques, stamp molding methods, using for example, rolling stamps to produce large sheets of microscale substrates, or polymer microcasting techniques, wherein the substrate is polymerized within a micromachined mold.

Further guidance regarding other designs and methods for using such microfluidic devices such as described above can be found, for example, in U.S. Pat. Nos. 5,858,188; 5,935,401; 6,007,690; 5,876,675; 6,001,231; and 5,976,336, all of which are incorporated by reference in their entirety.

Mass Spectrometric Detection and Sequencing

In a variation, the buffer system can be altered in the last separation step, through the use of volatile buffer salts, organic solvents, and ephemeral surfactants to make the eluent compatible with subsequent mass spectrometric analysis. A buffer salt consists of organic and inorganic species that may accept or reject a proton to create an ionic species. Volatile buffer salts consist of a subset of buffer salts that are substantially vaporized into the gas phase upon evaporation of water, where substantially vaporized is typically defined as greater than 50% mass volatility, more typically greater than 80% mass volatility, and most typically 90-100% mass volatility. Illustrative examples include salts selected from the groups of ammonium, alkyl- and aryl-ammonium, pyridinium, alkyl- and aryl-phosphonium, and alkyl- and aryl-sulfonium cations, and the groups of alkyl- and aryl-sulfonates, alkyl- and aryl-phosphonates, alkyl-or aryl-borates, alkyl- or aryl-carboxylates, halogenated carboxylates, carbonate, and bicarbonate anions. Illustrative non-typical examples include salts with at least one component selected from the group of sodium and potassium anions, or halide and sulfate anions.

An ephemeral surfactant consists of an anionic, cationic, neutral, or zwitterionic surfactant that are substantially vaporized into the gas phase or decompose to form species that substantially vaporize into the gas phase upon evaporation of water, where substantially vaporized is typically defined as greater than 50% mass volatility, more typically greater than 80% mass volatility, and most typically 90-100% mass volatility. Illustrative anionic examples include ammonium dodecyl sulfate, alkyl- or aryl-ammonium dodecylsulfate, and alkylammonium perfluoroalkylcarboxylates. Illustrative cationic examples include alkylammonium carboxylate or alkylphosphonium carboxylate species where at least one alkyl chain is typically 5-30 carbons long and more typically 6-15 carbons long and most typically 10-14 carbons long.

Preliminary Separation by Non-Electrophoretic Technique

The methods can also include an initial separation by a non-electrophoretic technique prior to commencing the electrophoretic separations. Essentially any type of technique capable of separating proteins can be utilized. Suitable methods include, but are not limited to, fractionation in a sulfate gradient, HPLC, ion exchange chromatography and affinity chromatography. (Please list other techniques that you consider important).

Exemplary Utilities

The methods and apparatus of the invention can be utilized to detect, characterize and/or identify many proteins (*e.g.*, hundreds or thousands of proteins in some methods) by controlling elution of fractionated proteins and utilizing various labeling and detection techniques. Consequently, the methods have multiple utilities including, but not limited to, various analytical applications (*e.g.*, monitoring certain protein levels as a function of external stimuli, or detecting specific proteins in complex compositions for identification purposes), clinical applications (*e.g.*, detecting and/or monitoring compositions of normal and diseased cells and tissues, diagnosing or monitoring disease, testing drug candidates for therapeutic efficacy and toxicity testing) and molecular biology and genetic research (*e.g.*, characterizing or monitoring molecular expression levels of gene products and determining the effects of the addition, mutation, deletion or truncation of a particular gene). In general, the methods and apparatus have utility in proteome research.

More specifically, the invention can be used in the development of protein databases in which, for example, proteins expressed under particular conditions are isolated, quantified, and identified. Using the controlled elution and detection methods described herein, certain methods can be utilized to determine and catalog a variety of chemical and physical characteristics of the resolved proteins, including, but not limited to, pI, and/or apparent molecular weight and/or relative abundance of proteins within a sample. This information can be further cross referenced with a variety of information regarding the source of the sample and the method by which it was collected. Examples of such information include genus, species, age, race, sex, environmental exposure conditions, subject's health, tissue type, method of sample collection and method of sample preparation prior to electrophoresis,

The methods also have value in a variety of comparative studies that can be utilized to identify potential drug targets and/or candidates. For example, the methods can be utilized to identify proteins that are differentially expressed in diseased cells as compared to normal cells. Such differentially expressed proteins can serve as targets for drugs or serve as a potential therapeutic. In a related fashion, the methods can be used in toxicology studies to identify proteins that are differentially expressed in response to particular toxicants. Such differentially expressed proteins can serve as potential targets or as potential antidotes for particular toxic compounds or challenges. The detection and labeling techniques of the invention can facilitate such investigations because these techniques enable even low abundance proteins to be detected and because enhanced reproducibility makes it easier to identify real differences in expression between different samples.

Proteomic studies using certain methods of the invention can detect mutations that result in premature termination of the gene transcript or in amino acid substitutions in the resulting gene product. The methods can also detect post translational modification events associated with disease that are not readily detectable or possible to detect using functional genomics. For example, proteomic methods can detect differences in protein folding, glycosylation patterns, phosphorylation events, and degradation rates.

The results of comparative studies are transferable to a variety of diagnostic applications. For example, the "marker" or "fingerprint" proteins identified during comparative studies as being characteristic of a particular disease can be used to diagnosis individuals to determine if they have the disease correlated with the marker. These markers can also be used in medical screening tests. Once such proteins have been identified, it is not necessary to examine all fractions. Instead, only those fractions potentially containing the marker proteins need be examined. The reproducibility of the methods facilitates such analyses. For systems integrated onto a chip or support (see *supra*), capillaries can be positioned at the appropriate locations along the separation cavity to withdraw only the relevant fractions potentially containing the marker protein(s) of interest.

As an example of a diagnostic application, proteomics analysis can be utilized in identifying diagnostic markers (*e.g.*, cell surface antigens or serum proteins) for immunodiagnostic assays. Purified samples of putative diagnostic proteins are recovered during proteomic analysis, and can be used to generate antibodies having specific binding affinity to the proteins. Such antibodies can be used to understand the link between the marker protein and the disease through immunological staining to localize the protein in diseased cells or to rapidly screen patients for the presence of the protein, showing its statistical link to the disease.

The methods of the invention have further utility in conducting structure activity studies. For instance, the methods can be used to determine the effect that certain chemical agents or combination of agents have on protein expression patterns. Alterations to the agent or combination can then be made and protein expression reassessed to determine what effect if any the alteration has on protein, expression. Such studies can be useful, for example, in making derivatives of a lead compound identified during initial drug screening trials.

III. Mass Spectroscopy Fragmentation

An aspect of the present invention resides in the development of a new nonproteolytic mass spectrometric method for protein sequencing. This method is conducted by labeling then or C-terminus of an intact protein with a unique mass tag, fragmenting the intact labeled protein in the ionization zone of a mass spectrometer (in-source fragmentation) and determining the sequence from the mass ladder of the resulting labeled peptide series. Labeled peptides are differentiated from unlabeled peptides by their unique mass signature in the resulting mass spectrum. In some embodiments, this process is accomplished in less than 1 min for a purified labeled protein, yielding a 500 to 1000-fold more rapid method than current MS/MS protein sequencing techniques.

The labeled proteins are highly fragmented in the ionization zone of the MS. This leads to increased ionization efficiency and volatility of the resulting labeled peptide fragment ions, relative to the parent protein, thus improving the overall detection sensitivity. The sequence is constructed from the low molecular weight end of the mass spectrum, providing greater absolute mass accuracy and more facile sequencing, including resolution of Q and K residues, from the resulting labeled peptide fragments.

The selection of an appropriate label for this technique requires consideration of several criteria. First, the label should be robust enough to survive the fragmentation conditions of the MS. Second, the label should also create a unique mass/charge (m/z) signature that is distinguishable from any unlabeled peptides generated from internal scissions of the protein backbone. Third, the label may also carry a hard charge to ensure that fragmentation produces high-abundance ions that include even uncharged N- and C-terminal residues. Example 6 using glycogen phosphorylase, carrying a natural N-terminal acetylation label, shows the generality of the technique.

In one aspect, the present invention provides a method for sequencing a portion of a protein, comprising:

(a) contacting a protein with a C-terminus or N-terminus labeling moiety to covalently attach a label to the C- or N-terminus of the protein and form a labelled protein; and
(b) analyzing the labeled protein using a mass spectrometric fragmentation method to determine the sequence of at least the two C-terminus or two N-terminus residues.

In this aspect of the invention the protein can be obtained from essentially any source. Preferably, the protein is isolated and purified to be free of interfering components. The isolated protein can be contacted with a C-terminus or N-terminus labeling moiety to covalently attach a label to the C- or N-terminus of the protein to form a labeled protein, suitable for analysis by mass spectrometric fragmentation methods.

<u>Labeled Proteins</u>

In one aspect, the present invention provides a method of labeling a plurality of different proteins in a protein sample, the method comprising contacting the protein sample with a labeling agent having a unique ion mass signature component, a quantitative detection component and a reactive functional group to covalently attach the label to at least a portion of the plurality of different proteins.

In this aspect of the invention the proteins can be obtained from essentially any source. Preferably, the proteins are at least partially isolated or purified to be free of interfering components. The isolated proteins can be contacted with a labeling moiety, preferably a C-terminus or N-terminus labeling moiety to covalently attach a label to the C- or N-terminus of at least a portion of the proteins to form a mixture of labeled proteins, suitable for further purification and analysis by mass spectrometric fragmentation methods.

The chemical modification of proteins to facilitate their direct detection is not new, particularly for protein separations conducted in capillary electrophoresis. See, Palmieri, R. and Nolan, J. A., "Protein capillary electrophoresis: theoretical and experimental considerations for methods development," in: CRC Handbook of Capillary Electropho resis: A Practical Approach, Chp. 13, pgs 3 25-368 (CRC Press, Boca Raton, FL, 1994); Pritchett, T., et al., "Quantitation of bioactive peptides in serum by capillary electrophoresis with laser-induced fluroescence immunodetection (CE-LIF-ID), Application Information, A-1791A (Beckman Instruments, Fullerton, CA 1995); Jorgenson, J.W. and K.D. Lukacs, J. High Resolut. Chromatogr. Chromatogr. Commun., 4:230 (1981); Bodhe, A.M., et al., Anal. Biochem., 164:39-43 (1987); and Guzman, N.A., et al.,J. Chromatogr., 608:197-204 (1992). Fluorescamine has been used for the detection of proteins in gels after electrophoresis and in electroblots. See, Vandekerckhoye, J., Eur. J. Biochem., 152:9-19 (1985). However, the direct fluorescent labeling of a plurality of proteins to facilitate their detection after 2-D electrophoretic separation techniques does not appear to have been previously reported,

Rose and Jorgensen (J. Chromatogr., 447:117 (1988)) used o-phthaldialdehyde to derivatize the effluent from a capillary zone electrophoresis separation to enhance post-capillary fluorescence detection. Pritchett et al. ("Quantitation of bioactive peptides in serum by capillary electrophoresis with laser-induced fluroescence immunodetection (CE-LIF-ID), Application Information, A-1791A (Beckman Instruments, Fullerton, CA 1995)) demonstrated a competitive fluorescent immunoassay using an antigen (An) labeled with the Cy5™ cyanine dye (at a 1:1 molar ratio) and laser induced fluorescence (LIF) detection after CE separation of the immune reaction product from human serum. They report a detection sensitivity of $10^{-9}$ M for the angiotensin II antigen (An) with the competitive CE immunoassay. Absolute detection sensitivity of the CE-LIF system was not reported, but can be estimated from the reported dilution factor (10) and probable sample loading (10-20$\mu$L) to be about $10^6$ molecules, almost 3 orders-of-magnitude better than the comparable sensitivity of silver staining (assuming a 40 kDa average protein).

The use of chemical derivatization is also the basis of many protein identification techniques (Stark, G. R., Methods in Enzymology, 25:103-120 (1972); Niall, H. D., "Automated Edman degradation: the protein sequenator," in: Methods in Enzymology, 27:942-1011 (1973), Gray, W. R., Methods in Enzymology, 25:121-137 (1972); Schroeder, W. A., Methods in Enzymology, 25:138-143 (1972); Creighton, T. E., Proteins: Structures and Molecular Principles (W. H. Freeman, NY, 1984); Niederwieser, A., Methods in Enzymology, 25:60-99 (1972)) where the N- or C-terminal amino acid is covalently labeled with a molecule that facilitates its detection in chromatographic analyses conducted after it is enzymatically or chemically cleaved from the protein. Wu and coworkers (Wu, et al., Anal. Biochem., 235:161-174 (1996); Watson, J.T. and J. Wu, Polym. Prep., 37:3 18 (1996)), Denslow and Nguyen (Denslow, N.D. and H.P. Nguyen, in: Techniques in Protein Chemistry VII, Marshak, D.R., ed., pgs. 241-248 (Academic Press, San Diego, CA, 1996)) and Ming *et al.* (Ming, D., et al., BioTechniques, 18:808-810 (1995)) have reported methods for the chemical derivatizatian of cysteine and cystine groups to facilitate identification of the number of such groups and their positions in proteins by MALDI-TOF mass spectromehy (MS). Murphy and Fenselau (Murphy, C.M. and C. Fenselau, Anal. Chem., 67:1644-1645 (1995)) demonstrated the use of methanolysis of the homoserine lactone residues created during cyanogen bromide digestions to add 32 mass units to all internally generated carboxyl terminal peptides. The peptide fragment containing the original C-terminus is converted to a methyl ester, adding only 14 Da to its mass, and thus is distinguishable in the MS. Rose *et al.* (Rose, K., et al., Biochem. J., 250:253-259 (1988)) demonstrated a similar approach in which typtic digestion of the protein is conducted in a 1:1 molar ratio of $H_2{}^{18}O$: $H_2{}^{16}O$. Thus, half of the carboxyl termini of the resulting internally generated tryptic fragments would be labeled with $^{18}O$ and exhibit a unique 50:50 isotopic split in the resulting mass spectrum. The original carboxyl terminus would remain unlabeled and be easily distinguished.

The present invention resides in a labeling procedure for a protein or mixture of proteins that simultaneously prepares the protein(s) for high precision post-separation detection and subsequent mass spectrometric sequencing and identification, which is preferably nonproteolytic and nonchemolytic. The present method is practiced by labeling the N- or C-terminus of an intact protein or mixture of proteins with a label having a unique ion mass component, a quantitative detection component and a reactive functional group for attaching to the protein or proteins. The resulting labeled protein mixture can then be separated and detected according to methods described herein.

Typically, these separation methods involve a conducting a plurality of capillary electrophoretic methods (dimensions), wherein samples containing a plurality of proteins are labeled in a dimension prior to the last electrophoretic separation dimension and labeled protein detection and quantification is conducted at the end of the last electrophoretic dimension. In some embodiments, protein detection and quantitation is accomplished by laser induced fluorescence. In some embodiments, this process results in the detection of 0.01 to 0.001 ng of labeled protein, yielding a 10 to 100-fold more sensitive detection method than current gel staining techniques. In some embodiments, this process results in a 1 to 5% standard deviation in the relative abundance of proteins contained in a sample, yielding a 10-fold more reproducible measure of protein abundance than current gel staining techniques.

The protein identification methods will typically involve fragmenting the intact labeled protein in the ionization zone of a mass spectrometer (*e.g.*, in-source fragmentation) and determining the sequence from the mass ladder of the resulting labeled peptide series. Labeled peptides are differentiated from unlabeled peptides by their unique mass signature in the resulting mass spectrum. In some embodiments, this process is accomplished in less than 1 min for a purified labeled protein, yielding a 500 to 1000-fold more rapid method than current MS/MS protein sequencing techniques.

The labeled proteins are highly fragmented in the ionization zone of the MS, in a manner that is preferably influenced by the presence of the label. Preferred labels lead to increased ionization efficiency and enhanced volatility of the resulting labeled peptide fragment ions, relative to the parent protein, thus improving the overall detection sensitivity. The sequence of the protein or protein sequence tag is preferably constructed from the low molecular weight end of the mass spectrum, providing advantages over prior methods, such as greater absolute mass accuracy and more facile sequencing, including resolution of Q and K residues, from the resulting labeled peptide fragments.

The selection of an appropriate label for this technique requires consideration of several criteria, First, the label is preferably robust enough to survive the fragmentation conditions of the MS. For example, sulfur-containing labels are generally less robust their non-sulfur containing analogs. Second, the label preferably also creates a unique mass/charge (m/z) signature that is distinguishable from any unlabeled peptides generated from internal scissions of the protein backbone. Third, the label will also carry a quantitative detection enhancement component such as an ionizable or permanently ionized group to ensure that fragmentation produces high-abundance ions that include even uncharged N- and C-terminal residues, and/or a chromaphoric or fluorophoric group that can be optically detected with high sensitivity.

The labeling of proteins with various agents in an aqueous or mixed aqueous/organic solvent milieu is known in the art and a wide range of labeling reagents and techniques useful in practicing the present invention are readily available to those of skill in the art. *See*, for example, Means et al., CHEMICAL MODIFICATION OF PROTEINS, Holden-Day, San Francisco, 1971; Feeney et al., MODIFICATION OF PROTEINS: FOOD, NUTRITIONAL AND PHARMACOLOGICAL ASPECTS, Advances in Chemistry Series, Vol. 198, American Chemical Society, Washington, D.C., 1982; Feeney et al., FOOD PROTEINS: IMPROVEMENT THROUGH CHEMICAL AND ENZYMATIC MODIFICATION, Advances in Chemistry Series, Vol. 160, American Chemical Society, Washington, D.C., 1977; and Hermanson, BIOCONJUGATE TECHNIQUES, Academic Press, San Diego, 1996.

Labeling can be conducted and PSTs determined from either the N- or C-terminal end of the protein. About 59-90% of eukaryotic proteins are N-terminal acetylated and are thus refractory to N-terminus labeling. However, the natural N-acetyl group of such proteins can sometimes be used as a label for purposes of this invention, but only where one or more of the amino acids within 4 residues of the N-terminus is ionizable (*e.g.*, is a lysine, arginine, histidine, aspartic acid, or glutamic acid residue) or can be derivatized to be ionizable (e.g., tyrosine, serine, and cysteine residues). Accordingly, strategies to label either the N- or C-termini are provided to afford the greatest degree of sequencing ability for any given protein. Once a label is selected, a deconvolution algorithm can be modified to search for masses that correspond to any modified residues.

As noted above, the following considerations are relevant to the selection of a labeling agent:

i) the mass of the label is preferably unique and preferably shifts the fragment masses to regions of the spectrum with low background;
ii) the label preferably contains fixed positive or negative charges to direct remote charge fragmentation at the N- or C-terminus;
iii) the label is preferably robust under the fragmentation conditions and does not undergo unfavorable fragmentation;
iv) the labeling chemistry is preferably efficient under a range of conditions, particularly denaturing conditions, thereby reproducibly and uniformly labeling the N- or C-terminus;
v) the labeled protein preferably remains soluble in the MS buffer system of choice; and
vi) the label preferably increases the ionization efficiency of the protein, or at least does not suppress it;
vii) the label may contain a mixture of two or more isotopically distinct species to generate a unique mass spectrometric pattern at each labeled fragment position.

In view of the label selection criteria, preferred labeling moieties are those that have a detection enhancement component, an ion mass signature component and a C-terminus or N-terminus reactive functional group. The reactive group can be directly attached to either or both of the other two label components.
In another embodiment, the reactive functional group is separated from one or both of the detection enhancement component and the ion mass signature component by a linker. The linker is preferably designed such that it is chemically stable and inert, and such that it allows efficient separation of the reactive group and at least one of the other two components of the tag Within a preferred embodiment of the invention, the linker is composed of a hydrocarbon chain or, most preferably, of a hydrocarbon chain linked to an aryl or heteroaryl ring and preferably provides additional separation between the ionizable group and the isothiocyanate group.
As will be understood by one of ordinary skill in the art, a virtually limitless array of hydrocarbon chains and modified hydrocarbon chains may be utilized within the present invention. Preferred hydrocarbon chains which are attached to the phenyl ring may be found in the family of alkanes, with particularly preferred linkers ranging from 2 carbon atoms to about 20 carbon atoms in length. Within a preferred embodiment of the invention, the linker is a phenethyl group.

IV. Label Composition and Linkage Chemistry

Protein Mixtures

Suitable protein samples can be obtained from essentially any biological sample, such as a cell, or a tissue sample derived from a patient. In a preferred embodiment, a sample is obtained from human cells in fluids (e.g., blood, cerebral spinal fluid, and the like) or other tissues such as cells derived from, for example, biopsy or necropsy samples of tumors. Although the sample is typically taken from a human patient, the samples can also be prepared from cells from eukaryotes in general, including plants, vertebrates and invertebrates, and in mammals in particular, such as dogs, cats, sheep, cattle and pigs, and most particularly primates such as humans, chimpanzees, gorillas, macaques, and baboons, and rodents such as mice, rats, and guinea pigs. Microbial cultures can also be used as a source of protein samples.
The cell or tissue simple from which the protein sample is prepared is typically taken from a patient suspected of having, for example, cancer or another disease. Methods of isolating cell and tissue samples are well known to those of skill in the art and include, but are not limited to, aspirations, tissue sections, needle biopsies, and the like. Frequently the sample will be a "clinical sample" which is a sample derived from a using any standard technique known to the skilled artisan. For example, the host cells can be lysed to release the contents of the cytoplasm by French press, homogenization, and/or sonication. The homogenate can then be centrifuged.
For protein samples wherein the polypeptide has formed inclusion bodies in the periplasm, the inclusion bodies can often bind to the inner and/or outer cellular membranes and thus will be found primarily in the pellet material after centrifugation. The pellet material can then be treated with a chaotropic agent such as guanidine or urea to release, break apart, and solubilize the inclusion bodies. Transmembrane and lipophilic proteins can be solubilized from the pellet material after centrifugation through the use of chaotropic agents, surfactants and organic solvents. The polypeptides in their soluble form can then be isolated by immunoprecipitation, acid precipitation (e.g., 5-10% trichloroacetic acid), ammonium sulfate precipitation, solvent precipitation, or other methods known to those of skill in the art. Other methods of protein isolation are described in, for example, Marston et al. Meth. Enz., 182:264-275 (1990).
For protein and polypeptide mixtures wherein inclusion bodies are not formed to a significant degree in the periplasm of the host cell, the polypeptides will be found primarily in the supernatant after centrifugation of the cell homogenate, and the polypeptides can be isolated from the supernatant using methods such as various types of chromatography (immunoaffinity, molecular sieve, and/or ion exchange), and/or high pressure liquid chromatography. In some cases, it may be preferable to use more than one of these methods for complete purification.

Following isolation of protein or polypeptide mixtures as noted, the protein samples will typically be diluted in an appropriate buffer solution or, in some instances, be concentrated. Any of a number of standard aqueous buffer solutions, employing one of a variety of buffers, such as phosphate, tris(hydroxymethyl)aminomethane, or the like, at physiological pH can be used. The protein sample from sources stated above can contain at least five, at least ten, at least 50 or at least 100 or more proteins.

Labeling Agents

A variety of labeling agents are useful in the present invention. Selection of an appropriate labeling agent requires consideration of several criteria, selected from:

i) the mass of the label is preferably unique and preferably shifts the fragment masses to regions of the mass spectrum with low background;
ii) the label preferably contains fixed positive or negative charges to direct remote charge fragmentation at the N- or C-terminus;
iii) the label is preferably robust under the fragmentation conditions and does not undergo unfavorable fragmentation;
iv) the labeling chemistry is preferably efficient under a range of conditions, particularly denaturing conditions, thereby reproducibly and uniformly labeling the N- or C-terminus;
v) the labeled protein preferably remains soluble in the CE and MS buffer systems of choice; and
vi) the label preferably increases the ionization efficiency of the protein, or at least does not suppress it;
vii) the label may contain a mixture of two or more isotopically distinct species to generate a unique mass spectrometric pattern at each labeled fragment position.

In view of the label selection criteria, preferred labeling moieties are those that have a detection enhancement component, an ion mass signature component and a C-terminus or N-terminus reactive functional group. The reactive group can be directly attached to either or both of the other two label components.

In another embodiment, the reactive functional group is separated from one or both of the detection enhancement component and the ion mass signature component by a linker. The linker is preferably designed such that it is chemically stable and inert, and such that it allows efficient separation of the reactive group and at least one of the other two components of the tag. Within a preferred embodiment of the invention, the linker is composed of a hydrocarbon or polyethylene oxide chain or, most preferably, of a hydrocarbon or polyethylene oxide chain linked to an aryl or heteroaryl ring and preferably provides additional separation between the ionizable group and the reactive functional group.

As will be understood by one of ordinary skill in the art, a variety of hydrocarbon chains and modified hydrocarbon chains can be utilized within the present invention. Preferred hydrocarbon chains which are attached to a phenyl ring and are alkylene groups. Particularly preferred linkers range from 2 carbon atoms to about 20 carbon atoms in length. Within a preferred embodiment of the invention, the linker is a phenethyl group.

The present invention more generally embodies a chemical labeling moiety, comprising:

(i) a detection enhancement component.
(ii) a component that exhibits a unique ion mass signature in a mass spectrometer and imparts that signature to peptide fragments attached to the labeling moiety, and
(iii) a component that binds the chemical agent covalently to the protein at specific positions, most preferably to the N-terminal amine or C-terminal carboxyl terminus of a protein.

In a variation of the method, the labeling moiety is

(iv) attached to all the proteins in a mixture containing a plurality of proteins prior to at least one electrophoretic separation step.

In a variation of the method, the labeling moiety

(v) alters the intrinsic (or native) charge on a protein, altering its separation coordinate in an electrophoretic mode.

In a variation of the method, the mass and/or charge of the labeling moiety

(vi) is altered through the addition or cleavage of one or more components after detection and quantification in the final electrophoresis step and before its use in the mass spectrometer for partial sequencing of a protein.

In one embodiment the labeling moiety is used to quantitate the relative or absolute amount of a majority of proteins present in a mixed sample after separation of the labeled proteins during electrophoresis. Fluorescent, UV or visible dyes, and radioactive detection enhancement components are typical in this embodiment because of their intrinsically higher limits of detection. Fluorescent constituents are most typical for capillary electrophoresis separations because of the ready availability for fluorescent detectors for these electrophoresis units. Radioactive constituents are most typical for other modes electrophoresis separations because of the ready availability of phosphor screens and photographic film detection techniques for Electrophoretic gels. The most typical detection enhancement component for MS detection is a charged or readily ionizable component. In a variation of the embodiment, more than one detection enhancement component may be employed on a labeling moiety.

In another embodiment, the labeling moeity imparts a unique mass signature to the protein or fragmented peptides derived from the protein in a mass spectrometer, such that the unique mass signature can be used to determine a partial protein sequence extending from the label. In a variation of the method, the label is attached to the N-terminus or C-terminus of the protein, allowing the determination of an N- or C-terminal protein sequence. In a variation of the method, the unique mass signature of the label is created as a function of the sum of the masses of the detection enhancement component and the reactive component after reaction with the protein. In a variation of the method, the unique mass signature is imparted by the use of mixtures of one or more isotopically enriched variants of the chemical moiety. In a variation of the method, the unique mass signature is imparted by mixtures of substantially identical chemical moieties that differ from each other by a chemical group substitution. In another embodiment, the same component may be used for both quantitative detection and to exhibit a unique ion mass signature. An example of such a constituent would be a napthalenic constituent (such as in dansyl chloride), which is both fluorescent and ionized in the mass spectrometer, but is not limited to a napthalenic constituent.

Ion Mass Signature Component

The ion mass signature component is the portion of the labeling moiety which preferably imparts a unique ion mass signature in mass spectrometric analyses. The sum of the masses of all the constituent atoms of the label is preferably uniquely different than the fragments of all the possible amino acids. As a result, the labeled amino acids and peptides are readily distinguished from unlabeled amino acids and peptides by their ion/mass pattern in the resulting mass spectrum. In a preferred embodiment, the ion mass signature component imparts a mass to a protein fragment produced during mass spectrometric fragmentation that does not match the residue or a- and b-ion mass for N-terminal sequencing, or y-ion mass for C-terminal sequencing for any of the 20 natural amino acids.

As will be understood by one of skill in the art, spurious mass spectral peaks can arise not only from the fragmentation of unlabeled amino acids and peptides but also from impurities in the sample and/or matrix. In order to further increase the uniqueness of the ion mass signature of the label and to be able to identify desired labeled fragment peaks amongst this "noise," it is preferable to shift the labeled fragments to regions of less spectral noise by optimizing the mass of the label. For example, it is preferred that the label mass generate an ion greater than 100 amu and less than 700 amu. This may be done by increasing the molecular weight of a low molecular weight label or by increasing the number of charges on a high molecular weight label.

An alternative method for providing a more unique mass signature to a labeling moiety is to incorporate stable isotopes in the label (*see,* for example, Gygi et al., Nature Biotechnol. 17: 994-999 (1999)). For example, by incorporating eight deuterium atoms into a labeling moiety and labeling the protein with a 50:50 mixture of the deuterated and nondeuterated label, the resulting singly-charged fragments that include the label are easily identified as equally intense doublets; one at the mass corresponding to the species with the nondeuterated label and the other at the mass corresponding to the species with the deuterated label with a spacing of 8 amu. In a preferred embodiment, the mass difference is from about 1 to about 20 amu at the single charge state. In the most preferred embodiment the mass difference is from about 4 to about 10 amu at the single charge state.

Another method for providing a more unique mass signature to a labeling moiety is to incorporate a mixture of alkyl and/or aryl substitutions onto the label, such that the corresponding set of fragment peaks is easily recognizable in the mass spectrum. For example, the protein can be labeled with a mixture of a label that contains a trimethyl ammonium group and the same label that contains a dimethylethylammonium group in place of the trimethyl ammonium group. This labeling moiety produces two fragment ion peaks for each amino acid in the sequence that differ by 14 amu from each other. It will be apparent to those skilled in the art that many such combinations can be derived.

Detection Enhancement Components

A detection enhancement component, as used herein, refers to a portion of the labeling moiety that facilitates detection and quantitation of the protein fragments by mass spectroscopy, other spectroscopic methods (e.g., UV/Vis, ESR, NMR and the like), or scintillation counting. Accordingly, in one group of embodiments, the detection enhancement component can provide charged (positively or negatively) ionic species under ionization conditions in a mass spectrometer ionization chamber, such that ionization efficiency of the protein is improved. For many of the detection enhancement components, the amount of ionized species present will depend on the medium used to solubilize the protein. Preferred detection enhancement components (*i.e.*, species that can generate a positive or negative charge) can be classified into two categories: 1) components that carry "hard" charge, and 2) components that carry "soft" charge.

Components that carry "hard" charge are arrangements of atoms that are ionized under all conditions, regardless of medium pH. "Hard" positively-charged detection enhancement components include, but are not limited to, tetraalkyl or tetraaryl ammonium groups, tetraalkyl or tetraaryl phosphonium groups, and N-alkylated or N-acylated heterocyclyl and heteroaryl (*e.g.*, pyridinium) groups. "Hard" negatively-charged detection components include, but are not limited to, tetraalkyl or tetraacyl borate groups.

Components that carry "soft" charge are arrangements of atoms that are ionized at a specific pH, respectively (*i.e.*, bases and acids). Within the context of the current invention, "soft" positive charges include those bases with a pKa of greater than 8, preferably greater than 10, and most preferably greater than 12. Within the context of the current invention, "soft" negative charges include those acids with a pKa of less than 4.5, and preferably less than 2, and most preferably less than 1. At the extremes of pKa, the "soft" charges approach classification as "hard" charges. "Soft" positively-charged detection enhancement components include, but are not limited to, 1°, 2°, and 3° alkyl or aryl ammonium groups, substituted and unsubstituted heterocyclyl and heteroaryl (*e.g.*, pyridinium) groups, alkyl or aryl Schiff base or imine groups, and guanidino groups. "Soft" negatively-charged detection enhancement components include, but are not limited to, alkyl or aryl carboxylate groups, alkyl or aryl sulfonate groups, and alkyl or aryl phosphonate or phosphate groups.

For both "hard" and "soft" charged groups, as will be understood by one of ordinary skill in the art, the groups will be accompanied by counterions of opposite charge. For example, within various embodiments, the counterions for positively-charged groups include oxyanions of lower alkyl organic acids (*e.g.*, acetate), halogenated organic acids (*e.g.*, trifluoroacetate), and organosulfonates (*e.g.*, N-morpholinoethane sulfonate). The counterions for negatively-charged groups include, for example, ammonium cations, alkyl or aryl ammonium cations, and alkyl or aryl sulfonium cations.

The detection enhancement component of the label can also be multiply charged or capable of becoming multiply charged. For example, a label with multiple negative charges can incorporate one or more singly charged species (*e.g.*, carboxylate) or it can incorporate one or more multiply charged species (*e.g.*, phosphate). In a representative example of this embodiment of the invention a species bearing multiple carboxylates, such as, for example a polyaminocarboxylate chelating agent (*e.g.*, EDTP, DTPA) is attached to the protein. Methods of attaching polyammocarboxylates to proteins and other species are well known in the art. *See*, for example, Meares et al,, "Properties of In Vivo Chelate-Tagged Proteins and Polypeptides." In, MODIFICATION OF PROTEINS: FOOD, NUTRITIONAL, AND PHARMACOLOGICAL ASPECTS;" Feeney, et al., Eds., American Chemical Society, Washington, D.C., 1982, pp. 370-387, Kasina et al., Bioconjugate Chem., 9: 108-117 (1998); Song et al., Bioconjugate Chem., 8: 249-255 (1997).

In a similar manner, labels having multiple positive charges can be purchased or prepared using methods accessible to those of skill in the art. For example, a labeling moiety bearing two positive charges can be rapidly and easily prepared from a diamine (*e.g.*, ethylenediamine). In a representative synthetic route, the diamine is monoprotected using methods known in the art and the non-protected amine moiety is subsequently dialkylated with a species bearing one or more positive charges (*e.g.*, (2-bromoethyl)trimethylammonium bromide) (Aldrich)). Deprotection using art-recognized methods provides a reactive labeling species bearing at least two positive charges. Many such simple synthetic routes to multiply charged labeling species will be apparent to one of skill in the art. In another embodiment, a mass spectrometer detection enhancement component may consist of a component that enhances the solubility of the protein in volatile nonaqueous solvents.

While charged labels are preferred, components that are neutral but are in close proximity to protein residues that carry "soft" charge (*e.g*, lysine, histidine, arginine, glutamic acid, or aspartic acid) can be used as detection enhancement components. In this case, the label carries no ionized or ionizable groups, and the detection enhancement is provided the increased volatility of the protein caused by neutralizing ionizable residues and allowing the amount of volatile organic cosolvent to be increased. When such a component carries a unique ion mass it can also serve for generating a protein sequence tag when a nearby protein residue carries charge. Within the context of the present invention, close proximity is defined as within about 4 residues from the labeled terminus of the protein, and more preferably within about 2 resides of the labeled terminus of the protein. Examples include phenylisothiocyanate and N-acetyl groups.

In another group of embodiment, the detection enhancement component is a detectable moiety that can be detected by, for example, spectroscopy (*e.g.*, UV/Vis, fluorescence, electron spin resonance (ESR), nuclear magnetic resonance (NMR) and the like), or scintillation counting (detection of radioactive isotopes), *etc.* When the protein is detected by UV/Vis, it is generally desirable to attach a chromophoric label to the protein (*e.g.*, phenyl, napthyl, *etc.*). Similarly, for detection by fluorescence spectroscopy, a fluorophore is preferably attached to the protein. For ESR, the detectable moiety can be a free radical, such as a moiety including a nitroxide group. When the protein is detected by an NMR method, the detectable moiety can be enriched with an NMR accessible nuclei, such as fluorine, $^{13}C$, and the like.

In a presently preferred embodiment, the detectable moiety is a fluorophore. Many reactive fluorescent labels are commercially available from, for example, the SIGMA chemical company (Saint Louis, MO), Molecular Probes (Eugene, OR), R&D systems (Minneapolis, MN), Pharmacia LKB Biotechnology (Piscataway, NJ), CLONTECH Laboratories, Inc. (Palo Alto, CA), Chem Genes Corp., Aldrich Chemical Company (Milwaukee, WI), Glen Research, Inc., Research Organic (Cleveland, OH), GIBCO BRL Life Technologies, Inc. (Gaithersburg, MD), Fluka Chemica- Biochemika Analytika (Fluka Chemie AG, Buchs, Switzerland), and PE-Applied Biosystems (Foster City, CA), as well as many other commercial sources known to one of skill. Furthermore, those of skill in the art will recognize how to select an appropriate fluorophore for a particular application and, if it not readily available commercially, will be able to synthesize the necessary fluorophore *de novo* or synthetically modify commercially available Fluorescent compounds to arrive at the desired fluorescent label.

There is a great deal of practical guidance available in the literature for selecting an appropriate fluorophore for a particular detectable tag, as exemplified by the following references: Pesce et al., Eds., FLUORESCENCE SPECTROSCOPY (Marcel Dekker, New York, 1971); White et al., FLUORESCENCE ANALYSIS: A PRACTICAL APPROACH (Marcel Dekker, New York, 1970); and the like. The literature also includes references providing exhaustive lists of fluorescent and chromogenic molecules and their relevant optical properties *(see,* for example, Berlman, HANDBOOK OF FLUORESCENCE SPECTRA OF AROMATIC MOLECULES, 2nd Edition (Academic Press, New York, 1971); Griffiths, COLOUR AND CONSTITUTION OF ORGANIC MOLECULES (Academic Press, New York, 1976); Bishop, Ed., INDICATORS (Pergamon Press, Oxford, 1972); Haugland, HANDBOOK OF FLUORESCENT PROBES AND RESEARCH CHEMICALS (Molecular Probes, Eugene, 1992) Pringsheim, FLUORESCENCE AND PHOSPHORESCENCE (Interscience Publishers, New York, 1949); and the like. Further, there is extensive guidance in the literature for derivatizing such molecules for covalent attachment via readily available reactive groups that can be added to a molecule.

The diversity and utility of chemistries available for conjugating fluorophores to other molecules is exemplified by the extensive body of literature on preparing nucleic acids derivatized with fluorophores. *See*, for example, Haugland (*supra*)*;* Ullman et al., U.S. Pat, No. 3,996,345; Khanna et al., U.S. Pat. No. 4,351,760. Thus, it is well within the abilities of those of skill in the art to choose a suitable fluorophore and to conjugate the fluorophore to a protein or polypeptide.

In addition to fluorophores that are attached directly to a protein, the fluorophores can also be attached by indirect means. In an exemplary embodiment, a ligand molecule (*e.g.*, biotin) is preferably covalently bound to the protein. The ligand then binds to another molecule (*e.g.*, streptavidin), which is either inherently detectable or covalently bound to a signal system, such as a fluorophore described above. In a variation for proteins denatured with binding surfactants (e.g., sodium dodecyl sulfate), such as those separated in the CGE stage of a multidimensional capillary electrophoretic separation, detection can be facilitated with a detection enhancement component that is non-covalently attached to the protein through a detergent-binding fluorophor such as NanoOrange™ and Sypro™ dyes (Molecular Probes, Inc.). These compounds have several desirable qualities, including the following: 1) excellent reproducibility in binding and therefore in protein quantitation, 2) generality of binding independent of protein type, and 3) fluorescent behavior only when the dye is bound to detergent-coated proteins. In this variation, the unique mass signature component may be separately bound to the protein at the N-terminus or C-terminus through covalent means.

Suitable fluorescent compounds (or fluorophores) include fluorescein and its derivatives, rhodamine and its derivatives, dansyl, umbelliferone, etc. Presently preferred fluorophores of use in conjunction with the methods of the invention are the fluoresceins and rhodamine dyes. Many suitable forms of these compounds are widely available commercially with substituents on their phenyl moieties, which can be used as the bonding functionality for attachment of the fluorophore to a protein. Another group of preferred fluorescent compounds are the naphthylamines, having an amino group in the alpha or beta position. Included among such naphthylamino compounds are 1-dimethylaminonaphthyl-5-sulfonate, 1-anilino-8-naphthalene sulfonate and 2-p-toluidinyl-6-naphthalene sulfonate. Other suitable fluorophores include 3-phenyl-7-isocyanatocoumarin, acridines, such as 9-isothiocyanatoacridine and acridine orange; N-(p-(2-benzoxazolyl)phenyl)maleimide; benzoxadiazoles, stilbenes, pyrenes, and the like.

Useful fluorescent detectable moieties can be made to fluoresce by exciting them in any manner known in the art, including, for example, with light or electrochemical energy (*see,* for example, Kulmala et al, Analytica Chimica Acta 386:1 (1999)). Means of detecting fluorescent labels are well known to those of skill in the art. Thus, for example, fluorescent labels can be detected by exciting the fluorophore with the appropriate wavelength of light and detecting the resulting fluorescence. The fluorescence can be detected visually, by means of photographic film, by the use of electronic detectors such as charge coupled devices (CCDs) or photomultipliers and the like.

The fewer the processing steps between any separation technique and MS sequencing method, the faster that proteins can be identified, and the lower the cost of proteomic research. Typical electrophoresis buffers (eg., Hochstrasser et al. Anal Biochern., 173:424 (1988). and O'Farrel, J Biol. Chem., 250:4007 (1975)) contain components (e.g., tris (hydroxymethyl)aminomethane buffers and sodium dodecyl sulfate, that supress the ionization of proteins in the mass spectrometer. These components may be replaced with other more volatile components (e.g., morpholinoalkylsulfonate buffers and ephemeral surfactants) that do not suppress ionization in the MS. In another embodiment, the samples are diluted with ammonium bicarbonate or ammonium acetate buffer to provide a volatile proton source for the mass spectrometer. Wilm, M. et al., Anal. Chem., 68:1-8 (1996). In another embodiment, a buffer exchange is conducted by chromatographic or tangential flow dialysis as the sample is transported from the outlet of the separation process to the inlet of the MS.

Reactive Groups

A third component of the labeling moiety is a functional group which is reactive with the N-terminus amino group, the C-terminus amino group or another constituent of the N- or C-terminus amino acid.

The reactive functional group can be located at any position on the labeling agent or tag. For example, the reactive group can be located on an aryl nucleus or on a chain, such as an alkyl chain, attached to an aryl nucleus. When the reactive group is attached to an alkyl, or substituted alkyl chain tethered to an aryl nucleus, the reactive group is preferably located at a terminal position of an alkyl chain. Reactive groups and classes of reactions useful in practicing the present invention are generally those that are well known in the art of bioconjugate chemistry. Currently favored classes of reactions are those which proceed under relatively mild conditions in an aqueous or mixed aqueous/organic solvent milieu.

Particularly preferred chemistries that target the primary amino groups in proteins (including the N-terminus) include, for example: aryl fluorides (see, ) Sanger, F., Biochem. J., 39:507 (1945); Creighton, T. E., Proteins: Structures and Molecular Principles (W. H. Freeman, NY, 1984); Niederwieser, A., in : Methods in Enzymology, 25:60-99 (1972); and Hirs, C.H.W., et al., Arch. Biochem. Biophys., 111:209-222 (1965), sulfonyl chlorides (Gray, W. R., in: Methods in Enzymology, 25:121-137 (1972)), cyanates (Stark, G. R., in: Methods in Enzymology, 25:103-120 (1972)), isothiocyanates (Niall, H. D., in: Methods in Enzymology, 27:942-1011 (1973)), imidoesters (Galella, G., et al., Can. J. Biochem., 60-71-80 (1982)), N-hydroxysuccinimidyl esters (Lomant, A.J., et al., J. Mol. Biol., 104:243-261 (1976)), O-acylisoureas (Lomant, A.J., et al., J. Mo/. Biol., 104:243-261 (1976)), chlorocarbonates and carbonylazides (Solomons, T.W.G, Organic Chemistry (John Wiley & Sons, NY, 1976), aldehydes (Novotny et al., Anal. Chem., 63:408 (1991) and Novotny et al., J. Chromatography, 499:579 (1990)), and alkylhalides and activated alkenes (Wagner, D.S., et al., Biol Mass Spectrometry, 20:419-425 (1991)).

Preferred examples of chemical constituents that react with the carboxyl groups of proteins are benzyl halides (Solomons, T.W.G, Organic Chemistry (John Wiley & Sons, NY, 1976); Merrifield, B., Science, 232:341-347 (1986); and Horton, H. R., et al., Methods in Enzymology, 25:468 (1972)), carbodiimide (Yamada, H., et al., Biochem., 20:4836-4842)), particularly if stabilized using N-hydroxysuccinimide (see, Grabarek, Z., et al., Anal. Biochem. 185:131-135 (1990), and anhydrides (see, Werner, et al., "A New Simple Preparation Device for Protein/Peptide Sequencing," poster presentation at the Ninth Symposium of the Protein Society). The carbodiimide/N-hydroxysuccinimide approach is expected to label carboxyl-containing amino acid residues (*e.g.*, aspartate and glutamate) along with that of the C-terminus. The anhydride approach, however, can uniquely be used to discriminate between the carboxyl residues and C-terminal carboxyl group of the protein. These and other useful reactions are discussed in, for example, March, ADVANCED ORGANIC CHEMISTRY, 3rd Ed., John Wiley & Sons, New York, 1985; Hermanson, BIOCONJUGATE TECHNIQUES, Academic Press, San Diego, 1996; and Feeney et al., MODIFICATION OF PROTEINS; Advances in Chemistry Series, Vol. 198, American Chemical Society, Washington, D.C., 1982.

The reactive functional groups can be chosen such that they do not participate in, or interfere with, the reactions necessary to assemble the tag. Alternatively, a reactive functional group can be protected from participating in the reaction by the presence of a protecting group. Those of skill in the art understand how to protect a particular functional group such that it does not interfere with a chosen set of reaction conditions. For examples of useful protecting groups, *see,* for example, Greene et al., PROTECTIVE GROUPS IN ORGANIC SYNTHESIS, John Wiley & Sons, New York, 1991.

A variety of functional groups are described below with references to conditions for appropriate attachment. One of skill in the art will understand that each of the groups noted will be further modified to incorporate a unique ion mass signature component and a quantitative detection component according to established chemical protocols,

In slightly alkaline (pH 8-9) solutions, Sanger's reagent (1-fluoro-2,4-dinitrobenzene) will undergo nucleophilic attack by primary amines to form a stable secondary aryl amine. (Solomons, T.W.G, Organic Chemistry (John Wiley & Sons, NY, 1976)) Sanger's reagent also reacts with the ε-amino group of lysine residues, as well as histidine and tyrosine residues at pH 10 and 40°C (albeit after a well defined lag phase). (Hirs, C.H.W., Arch, Biochem. Biophys., 111:209-222 (1965)) Reductive amination (Solomons, T.W.G, Organic Chemistry (John Wiley & Sons, NY, 1976)) can also be used to increase the degree of substitution of an amine using aldehydes and ketones, (Novotny et al., Anal. Chem., 63:408 (1991); Novotny et al., J. Chromatography, 499:579 (1990)) but is of reduced efficiency in aqueous solutions.

Dansyl chloride undergoes a similar nucleophilic attack by the amines in proteins at alkaline pH, producing an aromatic sulfonamide (Gray, W. R., Methods in Enzymology, 25:121-137 (1972)) However, sulfonyl chlorides, depending on the pH, can also react with secondary amines.(Solomons, T.W.G, Organic Chemistry (John Wiley & Sons, NY, 1976)) The aromatic constituent enables fluorescence detection of the reaction product. Dansyl chloride also reacts with the ε-amino group of lysine. (Gray, W. R., Methods in Enzymology, 25:121-137 (1972))

Potassium cyanate can also be used for labeling the amino groups of proteins at alkaline pH. See, Stark, et al., (Stark, G. R.., Methods in Enzymology, 25: 103-120 (1972)). Similarly, phenylisothiocyanate (Niall, H. D., Methods in Enzymology, 27:942-1011(1973)) has been used to label the amino groups of proteins at alkaline pH. Cyanate forms an N-terminal amide and isothiocyanate a thiamide. Isothiocyanates are also commonly used for the attachment of fluorescent labels to proteins. (Hermanson, G., Bioconjugate Techniques (Academic Press, 1995); Haugland, R.P., Handbook of Fluorescent Probes and Research Chemicals (Molecular Probes, Eugene, OR, 1996))

Imidoesters are widely used for protein crosslinking (Hermanson, G., Bioconjugate Techniques (Academic Press, 1995); Hartman, F.C. and F. Wold, Biochem., 6:2439-2448 (1967)) and the conjugation of fluorophors or other moieties to proteins. (Haugland, R.P., Handbook of Fluorescent Probes and Research Chemicals (Molecular Probes, Eugene, OR, 1996)) Nucleophillic attack of the imidoester by primary amines on the protein at alkaline pH (8-9) result in the formation of an amidine bond. (Browne, D.T. and S.B.H. Kent, Biochem. Biophys. Res. Commun., 67: 126-132 (1975)) N-Hydroxylsuccinimidyl (NHS) esters are also commonly used for protein crosslinking and the attachment of labels to proteins through an amide bond. (Hermanson, G., Bioconjugate Techniques (Academic Press, 1995); Haugland, R.P., Handbook of Fluorescent Probes and Research Chemicals (Molecular Probes, Eugene, OR, 1996)) NHS esters react with excellent specificity for the primary ε-amino groups of lysine (Cuatrecasas, P. and I. Parikh, Biochem., 11: 2291-2299 (1972)) and the α-amino group of the N-terminus of proteins, (Hermanson, G., Bioconjugate techniques (Academic Press, 1995)) leaving other amine-containing residues intact. The rate of imidylester hydrolysis is controlled by the pH. (Hermanson, G., Bioconjugate Techniques (Academic Press, 1995))

Benzylchorocarbonate, benzyl chloroformate, and t-butoxycarbonylazide are commonly used to block reactive amine groups in polyp eptide synthesis reactions. (Solomons, T.W.G, Organic Chemistry (John Wiley & Sons, NY, 1976); Merrifield, B., Science, 232:341-347 (1986).) Both are highly efficient derivatizing agents under alkaline conditions at room temperature. Both reagents yield similar acid labile amidoesters.

The ε-amino group of lysine is preferentially labeled in all the above examples because proximity to the neighboring carbonyl group causes the α-amino N-terminal group to be less nucleophilic. (Solomons, T.W.G, Organic Chemistry (John Wiley & Sons, NY, 1976)) The added resonance stabilization of the amines in arginine and histidine make these residues less reactive than the α-amino group of the N-terminus and these residues are not expected to be significantly labeled under the right reaction conditions.

Merrifield (Merrifield, B., Science, 232:341-347 (1986)) capitalized on the nucleophilic substitution of benzyl chloride by carboxylate ions (Horton, H. R. and D. E. Koshland, Jr., Methods in Enzymology, 25:468 (1972)) in his classic solid phase peptide synthesis method. The benzyl halide reaction forms a benzyl ester.

Carbodiimides react with carboxyl groups to form an O-acylisourea intermediate that is highly unstable in aqueous solution but can be stabilized through the addition of N-hydroxysuccinimide resulting in the formation of an acid stable intermediate that can be made to react with primary amines at alkaline conditions, producing an amide. (Grabarek, Z. and J. Gergely, Anal. Biochem, 185:131-135 (1990)) The carboxyl terminus, glutamate and aspartate residues are all targets for carbodiimides in proteins at acidic pH (4.5-5). (Hermanson, G., Bioconjugate Techniques (Academic Press, 1995)) Carbodiimide chemistry could be useful for labeling the C-terminus of protein if an excess of primary amines is added to the protein solution to inhibit crosslinking reactions, or in a two-step process involving the use of an amine containing fluorescent molecule through the N-hydroxysuccinimide intermediate.

In the presence of bases (e.g., 2,6-lutidine), acetic anhydride [Dupont, et al., PE Biosystems, Inc. Application Note, http://www.pbio.com] reacts with carboxy groups on proteins either in free solution or immobilized to a solid support (eg., polyvinylidene fluoride) to form mixed anhydrides. The resulting C-terminal α-amino anhydride is able to cyclize to form an oxazolone intermediate. The mixed anhydrides formed from glutamate and aspartate residues fail to cyclize. The C-terminal oxazolone is resistant to subsequent nucleophilic addition under basic conditions , while the other anhydrides are not. Therefore, the carboxyl residues can be selectively protected before the C-terminus. The C-terminus can then be labeled by lowering the pH with the addition of acid (e.g., TFA) and subsequently adding primary amines or other nucleophiles carrying suitable detection enhancement and unique mass signature components. However, this approach fails when the C-terminal residue is a proline.

Table 1 provides a non-limiting list of a number of labeling moieties useful in the labels of the present invention.

Table 1

| Label | Source | Linkage Formed |
|---|---|---|
| **Amine Labeling** | | |
| 2,4,6-trinitrobenzenesulfonic acid | Aldrich | Aryl amine |
| Lissamine™rhodamine B sulfonyl chloride | Molecular Probes | Sulfonamide |

(continued)

| Label | Source | Linkage Formed |
|---|---|---|
| **Amine Labeling** | | |
| 2',7'-dichlorofluoroscein-5-isothiocyanate | Molecular Probes | Thiourea |
| 4,4-difluoro-5,7-dimethyl-4-bora-3a,4a-diaza-s-indacene-3-propionic acid, sulfosuccinimidyl ester | Molecular Probes | Amide |
| Nahthalene-2,3-dicarboxylaldehyde | Molecular Probes | Isoindole |
| **Carboxyl Labeling** | | |
| 5-(bromomethyl)fluorescein | Molecular Probes | Ester |
| N-cyclohexyl-N'-(4-(dimethylamino) naphthyl)carbodimide | Molecular Probes | N-Acylurea |
| 1-ethyl-3-(3-dimethylaminopropyl)-carbodiimide hydrochloride with N-hydroxysuccinimide and 5-aminofluorescein | Pierce Aldrich Molecular Probes | Amide |

One of skill in the art will understand that labeling techniques are readily available for a number of the labeling moieties. An example of an N-terminus labeling group (dansyl chloride) and a C-terminus labeling group (carbodiimide) are provided as illustrative of the invention, with references to a more complete description of their use. The focus on these two labeling moieties is for clarity of illustration and does not limit the scope of the invention.

Dansyl chloride undergoes a nucleophilic attack by the amines in proteins at alkaline pH, producing an aromatic sulfonamide. Sulfonyl chlorides, however, depending on the pH, can also react with secondary amines. The aromatic constituent enables spectroscopic (*e.g.*, fluorescence) detection of the reaction product. Dansyl chloride also reacts with the ε-amino group of lysine. The pK differences between α- and ε-amines can be exploited to modify one of these groups preferentially to the other.

Carbodiimides react with carboxyl groups to form an O-acylisourea intermediate that is highly unstable in aqueous solution but can be stabilized through the addition of N-hydroxysuccinimide resulting in the formation of an acid stable intermediate that can be made to react with primary amines, producing an amide. The carboxyl terminus, glutamate and aspartate residues are all targets for carbodiimides in proteins at acidic pH (4.5.-5). Carbodiimide chemistry is useful for labeling the C-terminus of protein. When carbodiimide chemistry is utilized, it is generally preferred that an excess of amine is added to the protein solution to inhibit crosslinking reactions. In another exemplary embodiment, a protein amine is labeled in a two-step process; an amine-containing fluorescent molecule is tethered to the protein through an N-hydroxysuccinimide intermediate of the protein or of a spacer arm attached to the protein.

Synthesis

Once the reactive group, linker, and ionizable groups have been selected, the final compound is synthesized by one of ordinary skill in the art utilizing standard organic chemistry reactions. A preferred compound for use within the present invention is PETMA-PITC, or an analogous agent. This compound retains the excellent characteristics of phenylisothiocyanate in the coupling. Furthermore, the compound performs well as a label in analytical methods because the electron structure of the phenyl ring is sufficiently separated from the quaternary ammonium group by the ethyl linker, thus allowing the isothiocyanate to react undisturbed by the quaternary ammonium group. Preparation of PETMA-PITC, C5 PETMA-PITC and PITC-311 are described in Aebersold et al., U.S. Patent No. 5,534,440, issued July 9, 1996.

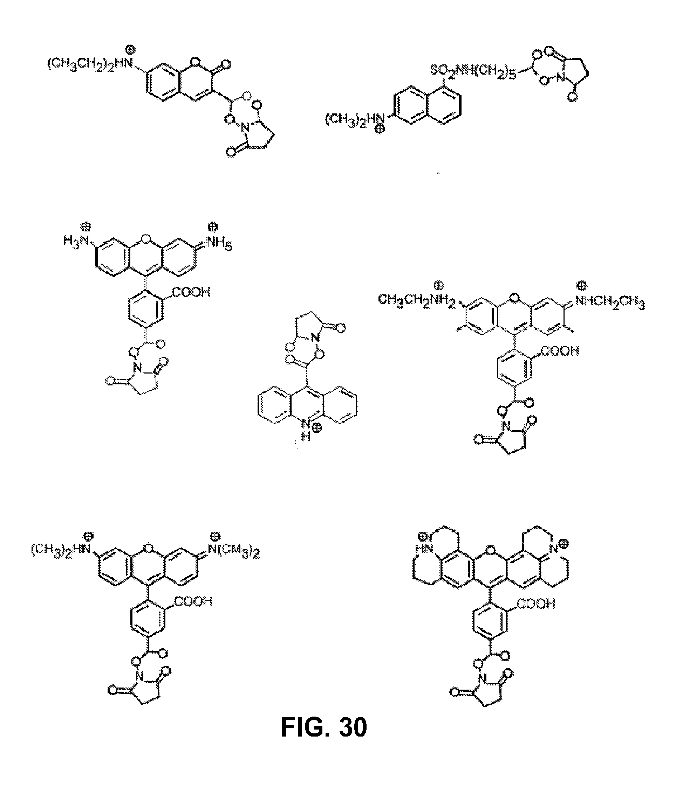

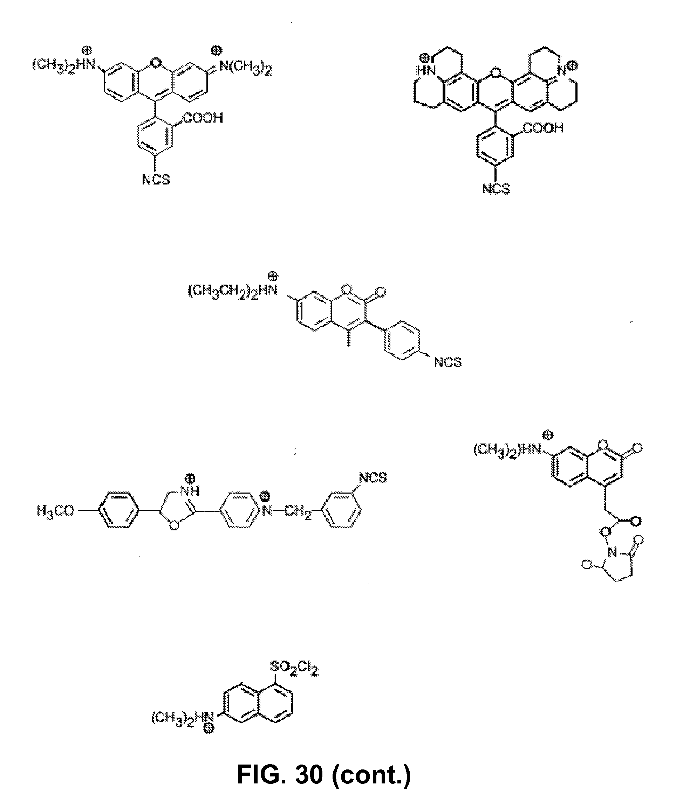

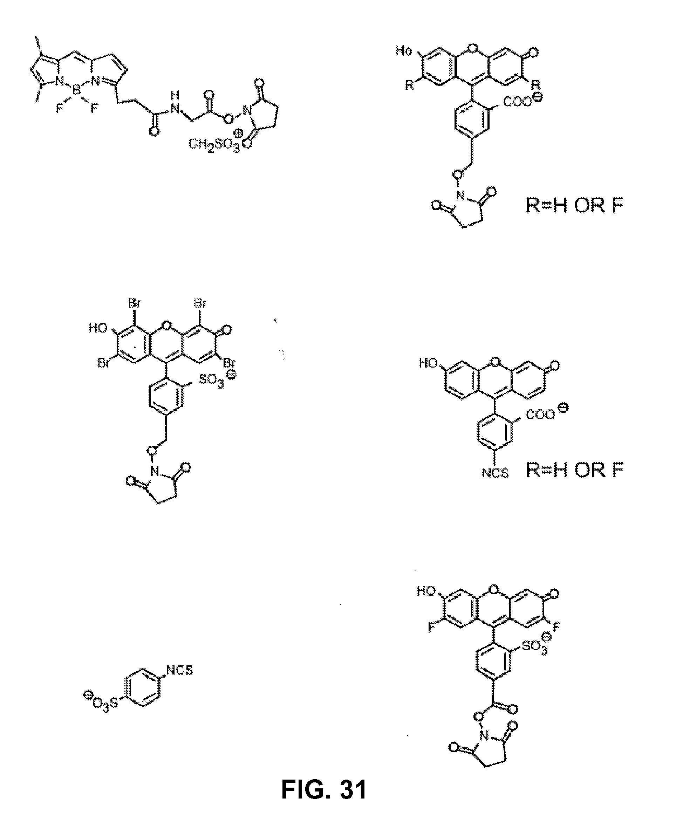

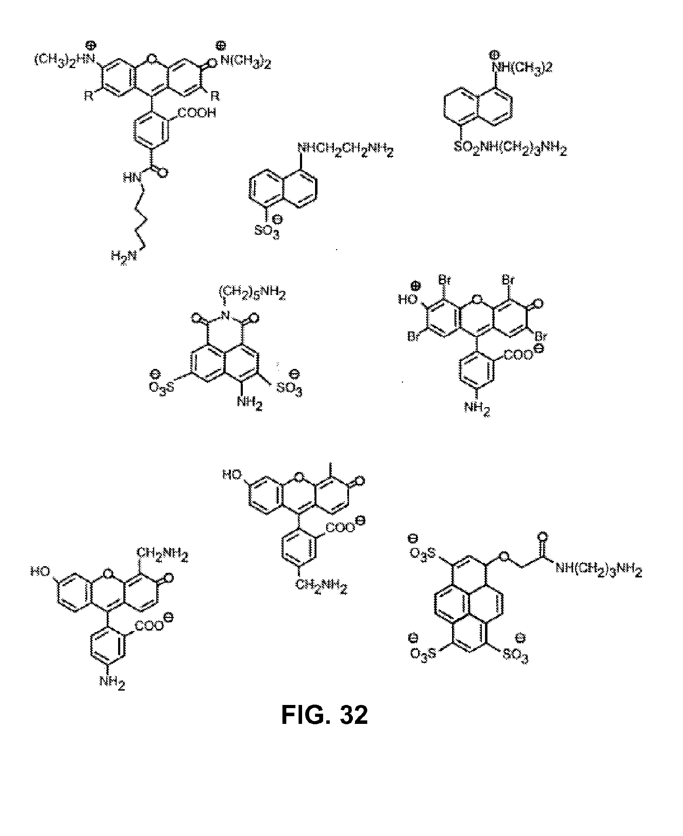

Other suitable commercially available labels that satisfy the labeling agent criteria set forth above, include sulfophenyl isothiocyanate, N-aminopropyl pyridine (attached to the C-terminus through carbodiimide chemistry), and the species shown in Figures 1-3. Figures 1 and 2 show examples of cationic and anionic fluorescent N-terminal labels bearing NHS-ester, isothiocyanate and sulfonyl chloride reactive groups. Examples include both "hard" and "soft" positive charges (e.g., "hard" charge represented by dialkyl immonium and "soft" charge represented by anilinium) (Figure 30) and "soft" negative charges (i.e., sulfonate and carboxylate) (Figure 31). Figure 32 shows examples of both cationic and anionic fluorescent labels bearing primary amino groups that can be used for C-terminal labeling via the carbodiimide or anhydride approaches. All of the labels in Figures 1-3 have a MW ≥ 200; thus, the smallest singly-charged fragment mass of an amino acid would be ~229 which is the sum of the mass of the a-ion of glycine (29) and the label weight (200). Since the largest fragment of an amino acid (i.e., the y-ion of tryptophan) has a mass of ~205, all the labels shown would produce initial fragments of interest with masses that are higher, and thus uniquely different, than those of the naturally-occurring amino acids. In a preferred embodiment, the labels shown with higher molecular weights (e.g., the fluorescein derivatives MW>500) would shift the fragment masses to at least 500 or more, which avoids the low m/z range of the MS which tends to be populated with spurious contaminant and matrix peaks.

Labeling Procedure

With the selection of a suitable labeling moiety, conditions for attaching the label to the protein should ensure that the N- or C-terminus of the protein is uniformly labeled and that the labeled protein remains soluble in appropriate CE and MS buffer systems. Typically, labeling will be carried out under denaturing conditions (*e.g.*, surfactants or 8M urea). Surfactant and urea both suppress MS ionization. In a variation of the method, techniques that provide rapid clean up and transfer of the labeled protein sample to a suitable MS buffer should also be employed. In another variation of the method, these denaturants will naturally be resolved from the labeled proteins during electrophoretic separation steps conducted post-labeling.

As noted, some salts (*e.g.*, TRIS and SDS) and urea present in electrophoresis buffers can suppress ionization of the labeled proteins and can generate small mass/charge ions that potentially confuse sequence analysis. Accordingly, spin dialysis procedures can be employed to rapidly exchange buffer systems prior to MS analysis. Alternatively, desalting columns (e.g., the ZipTip™ tip sold by Millipore) can be used for sample clean up and buffer exchange. Desalted samples can be resuspended in 0.1M ammonium bicarbonate as described by Wilm and Mann with minimal addition of methanol, or in 0.01M ammonium acetate buffer (with 0.1% formic acid) with minimal addition of acetonitrile as described by Mark (see "Protein structure and identification with MS/MS," paper presented at the PE/Sciex Seminar Series, Protein Characterization and Proteomics: Automated high throughput technologies for drug discovery, Foster City, CA (March, 1998)).

The coupling rates of the compound may be tested to ensure that the compound is suitable for sequencing polypeptides. In general, the faster the coupling rate the more preferred the compound. Coupling rates of between 2 and 10 minutes at 50 oC to 70 oC are particularly preferred. Similarly, fast reaction rates are also preferred, because exposure to the reaction mixture over an extended period of time might hydrolyze the peptide bonds, or lead to inefficient and irreproducible side reactions with the polypeptide residues, which could complicate mass spectral deconvolution.

In another preferred embodiment, one or more of the components of a protein mixture is reversibly attached to a solid support prior to the label being attached to a polypeptide. Various materials may be used as solid supports, including, for example, numerous resins, membranes or papers. These supports may additionally be derivatized to incorporate a cleavable functionality. A number of cleavable groups that may be used for this purpose include disulfides (-S-S-), glycol (-CH[OH]-CH[OH]-), azo (-N=N-), sulfone (-S[=O]-), and ester (-COO-) linkages (*see,* Tae, Methods in Enzymology, 91:580 (1983)). Supports which are particularly preferred include membranes such as Sequelon TM (Milligen/Biosearch, Burlington, Mass.). Representative materials for the construction of these supports include, among others, polystyrene, porous glass, polyvinylidinefluoride and polyacrylamide. In particular, polystyrene supports include, among others: (1) a (2-aminoethyl) aminomethyl polystyrene (*see,* Laursen, J. Am. Chem. Soc. 88: 5344 (1966)); (2) a polystyrene similar to number (1) with an aryl amino group (*see*, Laursen, Eur. J. Biochem. 20: 89 (1971)); (3) amino polystyrene (*see,* Laursen et al., FEBS Lett. 21: 67 (1972)); and (4)triethylenetetramine polystyrene (*see,* Horn et al., FEBS Lett. 36:285 (1973)). Porous glass supports include: (1) 3-aminopropyl glass (*see,* Wachter et al., FEBS Lett. 35: 97 (1973)); and (2)N-(2-aminoethyl)-3-aminopropyl glass (*see,* Bridgen, FEBS Lett. 50: 159 (1975)). Reaction of these derivatized porous glass supports with p-phenylene diisothiocyanate leads to activated isothiocyanato glasses (*see*, Wachter *et al.*, *supra*). Polyacrylamide-based supports are also useful, including a cross-linked β - alanylhexamethylenediamine polydimethylacrylamide (*see,* Atherton et al., FEBS Lett. 64: 173 (1976)), and an N-aminoethyl polyacrylamide (*see*, Cavadore et al., FEBS Lett. 66: 155 (1976)).

One of ordinary skill in the art will readily utilize appropriate chemistry to couple the polypeptide to the solid supports described above (*see*, generally Machleidt and Wachter, Methods in Enzymology: [29] New Supports in Solid-Phase Sequencing 263-277 (1974). Preferred supports and coupling methods include the use of aminophenyl glass fiber paper with EDC coupling (*see,* Aebersold et al., Anal. Biochem. 187: 56-65 (1990)); DITC glass filters (*see,* Aebersold et al., Biochem. 27: 6860-6867 (1988) and the membrane polyvinylidinefluoride (PVDF) (Immobilon P TM, Milligen/Biosearch, Burlington, Mass.), along with SequeNet TM chemistry (*see,* Pappin et al., CURRENT RESEARCH IN PROTEIN CHEMISTRY, Villafranca J. (ed.), pp. 191-202, Academic Press, San Diego, 1990)).

In the practice of the present invention, attachment of the polypeptide to the solid support may occur by either covalent or non-covalent interaction between the polypeptide and solid support. For non-covalent attachment of the polypeptide to the solid support, the solid support is chosen such that the polypeptide attaches to the solid support by non-covalent interactions. For example, a glass fiber solid support may be coated with polybrene, a polymeric quaternary ammonium salt (*see*, Tarr et al., Anal. Biochem., 84:622 (1978)), to provide a solid support surface which will non-covalently attach the polypeptide. Other suitable adsorptive solid phases are commercially available. For example, polypeptides in solution may be immobilized on synthetic polymers such as polyvinylidine difluoride (PVDF, Immobilon, Millipore Corp., Bedford, Mass.) or PVDF coated with a cationic surface (Immobilon CD, Millipore Corp., Bedford, Mass.). These supports may be used with or without polybrene. Alternatively, polypeptide samples can be prepared for sequencing by extraction of the polypeptide directly from polyacrylamide by a process called electroblotting. The electroblotting process eliminates the isolation of polypeptide from other peptides which may be present in solution. Suitable electroblotting membranes include Immobilon and Immobilon CD (Millipore Corp., Bedford, Mass.).

More recently, automated methods have been developed that allow chemistries to be performed on polypeptides immobilized on solid supports by non-covalent, hydrophobic interaction. In this approach, the samples in aqueous buffers, which may contain salts and denaturants, are pressure-loaded onto columns containing a solid support. The bound polypeptide is then pressure-rinsed to remove interfering components, leaving the bound polypeptide ready for labeling (*see*, Hewlett-Packard Product Brochure 23-5091-5168E (Nov., 1992) and Horn, U.S. Patent No. 5,918,273 (June 29,1999).

The bound polypeptide is reacted under conditions and for a time sufficient for coupling to occur between the terminal amino acids of the polypeptide and the labeling moiety. The physical properties of the support may be selected to optimize the reaction conditions for a specific labeling moiety. For example, the strongly polar nature of the PETMA-PITC dictates covalent attachment of the polypeptide. Preferably, coupling with the amino groups of the polypeptide occurs under basic conditions, for example, in the presence of an organic base such as trimethylamine, or N-ethylmorpholine. In a preferred embodiment, the label is allowed to react with the bound peptide in the presence of 5% N-ethylmorpholine in methanol:water (75:25 v/v). Because of the mode of attachment, excess of reagent, coupling base and reaction by-products can be removed by very polar washing solvents prior to removal and sequencing of the labeled polypeptide by mass spectrometry. Various reagents are suitable as washing solvents, including, for example, methanol, water, mixtures of methanol and water, or acetone.

Less polar reagents, such as PITC-311, may be reacted with polypeptides attached to a sold support preferably by hydrophobic, non-covalent interactions. In this case, less polar washes are preferred, such as heptane, ethylacetate, and chloroform. Following the washing cycle, the labeled polypeptide is dissociated from the solid support by elution with solvent containing 50% to 80% of aqueous methanol or acetonitrile.

When the labeling reaction is conducted entirely in solution phase, the reaction mixture is preferably submitted to a purification cycle, such as dialysis, gel permeation chromatography, and the like.

Still other conditions for labeling proteins can be found in, for example, Means et al., CHEMICAL MODIFICATION OF PROTEINS, Holden-Day, San Francisco, 1971; Feeney et al., MODIFICATION OF PROTEINS: FOOD, NUTRITIONAL AND PHARMACOLOGICAL ASPECTS, Advances in Chemistry Series, Vol. 198, American Chemical Society, Washington, D.C., 1982; Feeney et al., FOOD PROTEINS: IMPROVEMENT THROUGH CHEMICAL AND ENZYMATIC MODIFICATION, Advances in Chemistry Series, Vol. 160, American Chemical Society, Washington, D.C., 1977; and Hermanson, BIOCONJUGATE TECHNIQUES, Academic Press, San Diego, 1996.

Labeling can be conducted and PSTs determined from either the N- or C-terminal end of the protein. About 59-90% of eukaryotic proteins are N-terminal acetylated (see, Creighton, T. E., Proteins: Structures and Molecular Principles (W. H. Freeman, NY, 1984) and are thus refractory to N-terminus labeling. However, the natural N-acetyl group of such proteins can sometimes be used as a label for purposes of this invention, but only where one or more of the amino acids within 4 residues of the N-terminus is ionizable (*e.g.*, is a lysine, arginine, histidine, aspartic acid, or glutamic acid residue) or can be derivatized to be ionizable (e.g., tyrosine, serine, and cysteine residues). Accordingly, strategies to label either the N- or C-termini are provided to afford the greatest degree of sequencing ability for any given proteins.

Sequencing Labeled Proteins

In another aspect, the present invention provides a method for sequencing a portion of a protein in a protein mixture, the method comprising:

(a) contacting the protein mixture with a C-terminus or N-terminus labeling moiety to covalently attach a label to the C- or N-terminus of the protein and form a labeled protein mixture;
(b) separating individual labeled proteins in the protein mixture; and
(c) analyzing the labeled proteins from step (b) by a mass spectrometric method to determine the sequence of at least two C-terminus or two N-terminus residues.

In one group of embodiments, the method further comprises:

(d) identifying the protein by using the sequence of at least two C-terminus or two N-terminus residues in combination with a separation coordinate of the labeled protein and the protein terminus location of the sequence to search predicted protein sequences from a database of gene sequence data.

Separation

In a preferred embodiment, the tagging procedure is performed on a mixture of proteins. Following the tagging procedure the mixture of proteins is submitted to a separation process, which preferably, allows the separation of the protein mixture into discrete fractions. Each fraction is preferably substantially enriched in only one labeled protein of the protein mixture.

The methods of the present invention are utilized in order to determine the sequence of a polypeptide. Within preferred embodiments of the invention, the polypeptide is "substantially pure," which means that the polypeptide is about 80% homogeneous, and preferably about 99% or greater homogeneous. Many methods well known to those of ordinary skill in the art may be utilized to purify the polypeptide prior to determining its amino acid sequence. Representative examples include HPLC, Reverse Phase-High Pressure Liquid Chromatography (RP-HPLC), gel electrophoresis, chromatography, or any of a number of peptide purification methods (*see,* generally the series of volumes entitled METHODS IN PROTEIN SEQUENCE ANALYSIS).

Even more preferred is the use of capillary electrophoresis and particularly, multi-dimensional capillary electrophoresis, such as that described in the commonly assigned co-pending U.S. Patent Application Serial No.09/513,486, titled "Protein Separation via Multidimensional Electrophoresis," bearing Attorney Docket No. 020444-000200US and filed on February 25, 2000.

Although substantially pure polypeptides are preferably utilized within the methods described herein, it is also possible to determine the sequence of polypeptide mixtures. Briefly, in one embodiment, an algorithm is utilized in order to determine all of the hypothetical sequences with a calculated mass equal to the observed mass of one of the peptides in the mixture. *See*, Johnson et al., Protein Science 1:1083-1091 (1992). These sequences are then assigned figures of merit according to how well each of them accounts for the fragment ions in the tandem mass spectrum of the peptide utilizing such algorithms, the sequence of polypeptides within the mixture may be readily determined. As described above, the methods herein are particularly useful for identifying proteins from a healthy or diseased tissue sample. In one group of embodiments, the methods are applied to both a mixture of proteins from a healthy tissue sample and a mixture of proteins from a diseased tissue sample. Accordingly, the protein mixtures used in this aspect of the invention can be obtained from essentially any source. Methods of isolating proteins from tissue samples are well known.

Within the present invention, the polypeptide with a derivatized terminal amino acid is sequenced by a mass spectrometer. Various mass spectrometers may be used within the present invention. Representative examples include, triple quadrupole mass spectrometers, magnetic sector instruments (magnetic tandem mass spectrometer, JEOL, Peabody, Mass.); ion-spray mass spectrometers, Bruins et al., Anal. Chem. 59: 2642-2647 (1987); electrospray mass spectrometers, Fenn et al., Science 246: 64-71 (1989); laser desorption time-of-flight mass spectrometers, Karas et al., Anal. Chem. 60: 2299-2301 (1988), and a Fourier Transform Ion Cyclotron Resonance Mass Spectrometer (Extrel Corp., Pittsburgh, Mass.). Within a preferred embodiment, an electrospray mass spectrometer (Mariner™ model, PE Biosystems, Foster City, California) is utilized to fragment the derivatized terminal polypeptide, and a time-of-flight detector with better than 50 ppm mass accuracy is used to determine the sequence from the masses of the labeled fragments.

One of skill in the art will appreciate that the sequence information obtained using the methods of the invention can be combined with other characteristics of the protein under analysis to even further reduce the number possible identities of the protein. Thus, in a preferred embodiment, the method of the invention combines information from a protein sequence tag with one or more other protein characteristics to identify the protein. Data that is useful to supplement the sequence data includes, but is not limited to, amino acid composition, the number and identity of specific residues (*e.g.* cysteine), cleavage information, proteolytic (*e.g.*, tryptic) and or chemolytic peptide mass, subcellular location, and separation coordinates (*e.g.*, retention time, pI, 2-D electrophoresis coordinates, *etc.*). Other forms of data characteristic of a particular protein or class of proteins that can be combined with information from the PSTs of the invention to identify a protein will be apparent to those of skill in the art. As the body of data characteristic of a particular protein becomes more comprehensive, proteins under analysis can be identified using shorter protein sequence tags.

Thus, in a further preferred embodiment, information regarding one or more characteristics of a protein is combined with information from a PST of about 4 amino acids in length, more preferably about 3 amino acids in length, more preferably still, about 2 amino acids in length is used to identify the protein. The materials, methods and devices of the present invention are further illustrated by the examples which follow. These examples are offered to illustrate, but not to limit the claimed invention.

Thus, in a further preferred embodiment, information regarding one or more characteristics of a protein is combined with information from a PST of about 4 amino acids in length, more preferably about 3 amino acids in length, more preferably still, about 2 amino acids in length is used to identify the protein.

V. Metabolite Profiling (Metomics)

Overview

The present invention provides methods and apparatus for conducting metabolic analyses, including methods for purifying metabolites of interest, screens to identify metabolites that are correlated with certain diseases and diagnostic screens for identifying individuals having, or being susceptible to, a disease.

Certain methods of the invention provide Electrophoretic methods for separating various metabolites using a plurality of electrophoretic methods performed in series. Such separation methods can be utilized to conduct various metabolic analyses. For example, certain analytical methods of the invention involve administering a substrate labeled with a stable isotope to a subject. The isotopic composition or enrichment of the substrate prior to administration is known, After waiting a period of time to permit the substrate to be utilized, a sample is withdrawn from the subject and used to determine the isotopic composition of multiple target analytes, the target analytes comprising the substrate and/or one or more target metabolites formed from the substrate. Typically, samples are obtained from the subject at different time points and the abundance of the isotope determined for the target analytes in each sample. In this way, the isotopic composition of the substrates can be measured as a function of time to allow a flux value for each of the target analytes to be determined. Various methods can be utilized to determine relative isotopic abundance of the isotope in the target analytes, including nuclear magnetic resonance spectroscopy, infrared spectroscopy and mass spectroscopy.

Unlike certain other methods that focus on the concentration of a particular metabolite, certain methods of the invention are designed to determine flux rather than a single concentration value. This simplifies the methods because flux values can be determined from the relative abundance of the isotope label in the target analytes rather than having to determine absolute concentration values. Furthermore, flux determinations provide insight into certain biological processes that are not observable from simple concentration determinations. For example, while concentration values may appear constant, flux can actually be changing. The concentration of any metabolite is determined by the rates of all reactions involving the formation, conversion, and transport of that metabolite. Therefore, increases in any two specific reactions (fluxes) involving both the formation and removal (conversion or transport) of the metabolite can yield the same apparent concentration of the metabolite. Flux can be altered in response to a number of different stimuli, and thus can serve as sensitive indicator of certain cellular states. For example, flux can be altered in response to factors such as physiological state, exposure to toxins and environmental insults, as well as various disease states such as infection, cancer, inflammation and genetic based defects in metabolism. Thus, flux can be used to detect diverse cellular conditions or states that are not necessarily detectable by other methods.

In some methods of the invention, the samples obtained from the subject are purified prior to determining the isotopic abundance of the isotope in the analytes. The purification procedure is used to at least partially remove other components in the cell from the target analytes of interest. Typically, this is accomplished by separating components within the sample by multiple electrophoretic methods (*i.e.*, multiple dimensions) performed in series.

Certain methods combine the electrophoretic separation aspects of the invention with certain mass spectroscopy techniques of the invention. Such arrangements enable relatively complex samples to be sufficiently reduced in complexity so that samples containing a relatively limited number of target analytes can be directly injected into the mass spectrometer to determine the isotopic abundance in the various target analytes of interest. Such systems can be automated to permit high throughput analysis of metabolic samples.

The flux values determined for the various target analytes can be used in a variety of different applications. For example, flux values for various subjects or various physiological conditions (*e.g.*, diseased or normal) can be used directly as inputs into a database. The flux values can also be employed in various screening applications. For example, the flux values from a test subject can be compared with corresponding flux values for a diseased subject to identify potential markers for the disease (*i.e.*, metabolites that appear to be correlated with the disease. Groups of flux values can be used to develop a "fingerprint" for different cellular states. Once a correlation between a disease state and one or more metabolites have been made, flux values for test subjects can be compared with flux values for individuals having different diseases. Lack of a statistically significant difference between the test and diseased subjects indicates that the test subject has the disease or is susceptible to the disease. Changes in metabolic flux can be manifested as a change in the relative amounts of alternative analytes produced from a single substrate at metabolic branch points, and as the rates at which analyses resulting from serial conversions of a single substrate are produced.

Methods

A. General

By feeding a tissue, population of cells or an organism an isotopically-enriched substrate and following the ratio of isotopic to nonisotopic metabolites in the cell over time, one can generate a quantitative picture of cellular metabolism. The relative metabolic flux can be ascertained by determining the ratio of the amount of isotopically enriched analytes to normal analytes at any given time using a variety of different detectors capable of detecting the relative abundance of different isotopes (*e.g.*, mass spectrometry). At each metabolic branch point, the relative ratio of isotopic to nonisotopic products on each side of the branch point provides an indication of the flux of metabolite diverted into each branch of the metabolic pathway. Following the rate of change of the isotopic ratio in identifiable metabolites along a linear metabolic pathway in pulse labeled cultures provides an estimate of the metabolic flux through each step of the pathway. Metabolites become isotopically enriched in front of slow kinetic steps and remain isotopically poor immediately after these steps. Once specific changes in cellular metabolism, such as induced by toxic challenge or infection, are identified using the techniques described herein, one can synthesize isotopically enriched compounds that can be used as specific diagnostic markers of these metabolic changes, wherein the substrate is only metabolized or fails to be metabolized in response to a specific disease state (see *e.g.*, U.S. Patent Nos. 4,830,010; 5,542,419; 6,010,846 and 5,924,995).

B. Administering Labeled Substrate to Subject

1. Types of Subject

A "subject" as used herein generally refers to any living organism from which a sample is taken to conduct a metabolic analysis. Subjects include, but are not limited to, microorganisms (*e.g.*, viruses, bacteria, yeast, molds and fungi), animals (*e.g.,* cows, pigs, horses, sheep, dogs and cats), hominoids (*e.g.*, humans, chimpanzees, and monkeys) and plants. The term includes transgenic and cloned species. The term also includes cell or tissue cultures that can be cultured to carry on the metabolic process under investigation, The term "patient" refers to both human and veterinary subjects.

If the subject is a population of cells or a cell culture, any of the standard cell culture systems known in the art can be used. Examples of suitable cell types include, but are not limited to, mammalian cells (*e.g.*, CHO, COS, MDCK, HeLa, HepG2 and BaF3 cells), bacterial cells (*e.g.*, E. coli), and insect cells (*e.g.*, Sf9). Further guidance regarding cell cultures is provided in Sambrook et al. MOLECULAR CLONING: A LABORATORY MANUAL, 2d ed. (1989) Cold Spring Harbor Laboratory Press, Cold Spring Harbor, N.Y.

2. Types of Substrate

As used herein, the term "substrate" when used within the context of the chemical species being administered to a subject refers to any species capable of being metabolized by the subject of interest. Exemplary substrates include, but are not limited to, proteins, carbohydrates, amino acids, nucleotides, nucleosides, nucleic acids, fats, fatty acids, and steroids. A "metabolite" refers to a product derived from enzymatic conversion ofa substrate administered to a subject, the conversion occurring as part of a metabolic process of the subject. A "target metabolite" is a metabolite under study in an analysis (*e.g.*, a metabolite for which a flux value is to be determined).

A substrate labeled with a stable isotope refers to a substrate having a distribution of stable isotopes significantly different from that found in the corresponding naturally occurring substrate. The term stable isotope refers to isotopes of an element that are not radioactive. The isotopes typically used in the methods of the invention include $^{13}C$, 2H, $^{15}N$, $^{18}O$, and $^{34}S$. Hence, when substrates are labeled with $^{13}C$, the substrate includes a mixture of carbon isotopes where the $^{13}C$ isotope is incorporated in the substrate at an abundance level detectably greater than its natural abundance. For $^{13}C$ detection by mass spectrometry, this level is 2-100%, and preferably 10-100% of all the C atoms present in the substrate (*i.e.*, the atoms in the substrate collectively have the indicated percentage enrichment). For substrates labeled with $^{15}N$, a detectable level is 0.75-100% $^{15}N$, and preferably 4-100% $^{15}N$. For substrates labeled with $^{18}O$, a detectable level is 0.4-100% $^{18}O$, and preferably 2-100% $^{18}O$. For compounds labeled with $^{34}S$, a detectable level is 8.4-100% $^{34}S$ and preferably 42-100% $^{35}S$. In some instances, the desired abundance level is obtained by mixing substrates that are isotopically enriched with non-enriched substrate.

2. Amount of Substrate

The amount of substrate added varies depending upon several factors. In general, however, the substrate is added at a safe and effective amount. As used herein, the term "safe and effective amount" means that the amount of substrate is sufficient so that the isotopic abundance, or at least a ratio of isotopic abundances, can be determined with the detector of choice, but not so high so that the substrate causes undue adverse side effects. Thus, the amount administered should be commensurate with a reasonable risk/benefit ratio. For example, if the substrate is labeled with $^{13}C$, then the substrate is administered to the patient in sufficient quantity such that the $^{13}C/^{12}C$ ratio in the target analyte(s) can be determined. However, the amount of substrate administered is below the amount that causes undesired side effects (*e.g.*, toxicity, irritation or allergic response). The safe and effective amount depends upon factors such as the nature and amount of the sample acquired (*e.g.*, gas or liquid and/or acquisition site), the weight of the test subject, and the relative concentration of the isotope in the substrate.

3. Mode of Administration

Typically a mixture of known amounts of both labeled and unlabeled substrate is administered to a subject. The mixture may contain between 5-95% relative abundance of labeled substrate. More preferably, the mixture contains between 25-75% labeled substrate. Most preferably, the mixture contains about equimolar ratios of labeled and unlabeled substrate.

In certain methods, the substrate is administered to the subject as a pulse. Pulsed additions or pulsed labeling refers to the timed addition of an isotopically labeled substrate, wherein the relative isotopic abundance of the isotopes is known. Long pulses can be used to estimate net synthesis rates of particular biomolecules starting from the time of the pulse. In this instance, previous biomass contains no label, but new biomass begins to accumulate the isotope in proportion to the isotopic abundance of the label in the substrate. If the pulse duration is long compared to the turnover of the substrate and target analytes of interest, the net synthesis rate is measured. Short pulses (significantly shorter than the turnover rate) do not account for degradation and recycle, so provide an estimate of the unidirectional synthesis rate.

The mode by which the substrate is administered to the subject can vary but should be administered in such a way that the substrate can be metabolized within a reasonable time frame. The substrate can be administered in substantially pure form or as part of a composition. Compositions can include pharmaceutically acceptable components including, but not limited to, diluents, emulsifiers, binders, lubricants, colorants, flavors and sweetners, so long as these components do not interfere with the metabolism of the substrate being administered. Guidance on the incorporation of such optional components is discussed, for example, in The Theory and Practice of Industrial Pharmacy (L. Lachman, et al., Ed.) 1976; and Remington's Pharmaceutical Sciences, Mace Publishing Company, Philadelphia, PA, 17th ed., (1985); and Langer, Science 249:1527-1533 (1990), each of which is incorporated by reference in its entirety.

In some instances, the substrate is administered orally in solid form (*e.g.,* solid tablet, capsule, powder, pill granule) or as part of a liquid solution (*e.g.*, emulsion, suspension). When shaping into the form of tablets, as the carrier for the substrate, there can be used excipients such as lactose, sucrose, sodium chloride, glucose, urea, starch, calcium carbonate, kaolin, crystalline cellulose, silicic acid, and potassium phosphate; binders such as water, ethanol, propanol, simple syrup, glucose solution, starch solution, gelatin solution, carboxymethyl cellulose, hydroxypropyl cellulose, methyl cellulose, and polyvinyl pyrrolidone; disintegrators such as carboxymethyl cellulose sodium, carboxymethyl cellulose calcium, low-substitution degree hydroxypropyl cellulose, dried starch, sodium alginate, agar powder, laminaran powder, sodium bicarbonate, and calcium carbonate; surfactants such as polyoxyethylene sorbitan fatty acid ester, sodium lauryl sulfate, and monoglyceride stearate; disintegration inhibitors such as sucrose, stearin, cacao butter, and hydrogenated oil; absorption accelerators such as quaternary ammonium base, and sodium lauryl sulfate; humectants such as glycerin and starch; absorbents such as starch, lactose, kaolin, bentonite, and colloidal silicic acid; and lubricants such as purified talc, stearate, borax and polyethylene glycol. Furthermore, tablets can be optionally formed into tablets subjected to normal tablet coating, such as sugar coated tablets, gelatin coated tablets, enteric coated tablets, film coated tablets, or double tablets and multilayer tablets.

When shaping into the form of pills, as the carrier for the substrate, there can be used excipients such as glucose, lactose, starch, cacao butter, hardened vegetable oil, kaolin, and talc; binders such as gum arabic, tragacanth powder, gelatin, and ethanol; and disintegrators such as laminarane and agar.

The substrate, alone or in combination with other suitable components, can also be made into aerosol formulations (*i.e.*, they can be "nebulized") to be administered via inhalation. Aerosol formulations can be placed into pressurized acceptable propellants, such as dichlorodifluoromethane, propane, nitrogen.

Suitable formulations for rectal administration include, for example, suppositories, which consist of the packaged active ingredient with a suppository base. Suitable suppository bases include natural or synthetic triglycerides, paraffin hydrocarbons, polyethylene glycol, cacao butter, higher alcohols, esters of higher alcohols, gelatin, and semisynthetic glycerides. In addition, it is also possible to use gelatin rectal capsules that consist of a combination of the substrate with a base, including, for example, liquid triglycerides, polyethylene glycols, and paraffin hydrocarbons.

Formulations of the substrate suitable for parenteral administration, such as, for example, by intraarticular (in the joints), intravenous, intramuscular, intradermal, intraperitoneal, and subcutaneous routes, include aqueous and non-aqueous, isotopic sterile injection solutions, which can contain antioxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. In the practice of this invention, compositions can be administered, for example, by intravenous infusion, orally, topically, intraperitoneally, intravesically or intrathecally. The compositions are formulated as sterile, substantially isotonic and in full compliance with all Good Manufacturing Practice (GMP) regulations of the U.S. Food and Drug Administration.

When the substrate is administered to a population of cells, the cells are typically suspended in a matrix containing the isotopically-enriched substrate. The matrix is typically an aqueous solution and can also contain other nutrients. Depending upon the number of cells, the cells can be suspended in standard culture flasks or within the wells of a microtiter plate, for example.

B. Sample Collection

1. Sample Sources and Types

As noted above, the methods of the invention can be used to analyze metabolism in essentially any living organism. The samples can come from tissues or tissue homogenates, fluids of an organism or cells or cell cultures. Generally, samples are obtained from the body fluid of an organism. Such fluids include, but are not limited to, whole blood, plasma, serum, semen, saliva, urine, sweat, spinal fluid, saliva, gastrointestinal fluids, sweat, cerebral fluid, and lacrimal fluids. In some instances, samples are obtained from fecal material, buccal, skin, tissue biopsy or necropsy and hair. Samples can also be derived from *ex vivo* cell cultures, including the growth medium, recombinant cells and cell components. In comparative studies to identify potential drug or drug targets (see *infra*), one sample can be obtained from a diseased subject or cells and another sample a non-diseased subject or from non-diseased cells, for example.

2. Collection Options

Certain methods involve withdrawing a sample of blood from the subject. If whole blood is used, the sample typically is lysed by any of the methods known to those of skill in the art including, for example, freezing/thawing the sample. Urine can be collected by collecting the urine of the subject in a clean container. In some instances, a sample is obtained from the breath of an individual (*e.g.*, when the target metabolite is carbon dioxide). A variety of different devices and methods have been developed to collect breath samples. For example, the breath of a subject can be captured by having the subject inflate an expandable collection bag (*e.g.,* a balloon). The sample can then be transferred to a commercially available storage container for subsequent storage and/or transport (*e.g.*, the VACUTAINER manufactured by Becton-Dickenson Company). Other breath collection devices are described in U.S. Pat. Nos. 5,924,995 and 5,140,993, which are incorporated by reference in their entirety. Tissue samples may be obtained by biopsy.

In the case of cell or tissue cultures, cells are collected by centrifugation or filtration and then lysed according to standard protocols (*e.g.*, sonication, blending, pressurization, freeze thawing and denaturation). Alternatively, cells can be collected and lysed by the addition of trichloroacetic acid (to a final concentration of 5-10% weight to volume), or similar use of other membrane lytic solvents (*e.g.*, chloroform, diethyl ether, toluene, acetone, and ethanol). Such membrane lyric solvents can be used to precipitate macromolecular components and selectively solubilize small molecule metabolites as a precursor to subsequent electrophoretic separation techniques.

C. Target Analyte Separation

1. Preliminary Purification

Depending on the complexity of the sample (*i.e.*, the number and different types of components within the sample), the target analytes (*i.e.*, substrate and/or target metabolites) are first at least partially purified from other components within the sample. If the sample contains cellular debris or other material that might interfere with separation, such materials can be removed using any of a variety of known separation techniques including, for example, forcibly extruding the sample through sieve material, filtration, centrifugation (*e.g.*, density gradient centrifugation), and various chromatographic methods (*e.g.*, gel filtration, ion exchange or affinity chromatography).

Many macromolecules (*e.g.*, proteins and nucleic acids) can be separated from small molecules (*e.g.*, nucleotides, acetyl CoA, mono- and disaccharides, amino acids) by lysing the cells and quantitatively precipitating the macromolecules by treating the lysed cells with cold trichloroacetic acid (*e.g.*, 5-10 % TCA weight to volume for 30 min on ice), while most of the small molecules in the cell remain soluble. Additional separation methods are discussed, for example, by Hanson and Phillips (Hanson, R. S. and Phillips, J. A., In: Manual of methods for general bacteriology, Gerhardt et al. (eds.)., Am. Soc. Microbiol., Washington, D.C., p. 328 (1981)).

2. Multidimensional Electrophoresis

Once such initial purification steps have been completed (if necessary), the target analytes are typically further purified by conducting a plurality of electrophoretic methods conducted in series. For optimal performance, samples whose ionic strength is particularly high can be desalted using established techniques such as dialysis and dilution and reconcentration prior to conducting the electrophoretic methods. The methods are said to be conducted in series because the sample(s) electrophoresed in each method are from solutions or fractions containing components electrophoresed in the preceding method, with the exception of the sample electrophoresed in the initial electrophoretic methods. Each of the different electrophoretic methods is considered a "dimension", hence the series constitutes an "multidimensional" separation.

The series of electrophoretic methods are typically conducted in such a way that components in an injected sample for each electrophoretic method of the series are isolated or resolved physically, temporally or spacially to form a plurality of fractions, each of which include only a subset of components contained in the sample. Thus, a fraction refers to a solution containing a component or mixture of components that are resolved physically, temporally or spacially from other components in a sample subjected to electrophoresis. Hence, resolved components can refer to a single component or a mixture of components that are separated from other components during an electrophoretic method. As just noted, samples in the various Electrophoretic methods are obtained from such fractions, with the exception of the first electrophoretic method in which the sample is the original sample containing all the components to be separated.

Typically, these multiple electrophoretic methods in the series separate components according to different characteristics. For example, one method can separate components on the basis of isoelectric points (*e.g.*, capillary isoelectric focusing electrophoresis), other methods can separate components on the basis of their intrinsic or induced (through the application of a label to certain ionizable groups) charge-to-mass ratio at any given pH (*e.g.*, capillary zone electrophoresis), whereas other methods separate according to the size of the components (*e.g.*, capillary gel electrophoresis).

Apparatus used to conduct various electrophoretic methods are known in the art. In general, however, and as shown in FIG. 2A, the basic configuration of a typical capillary electrophoretic system utilized in certain methods of the invention includes a capillary 8 having two ends 10, 12. One end 10 is in contact with an anode solution or anolyte 14 contained in an anode reservoir 18 and the other end 12 is in contact with a cathode solution or catholyte 16 in a cathode reservoir 20. One electrode (the anode) 22 is positioned to be in electrical communication with the anode solution 14 and a second electrode 24 is positioned to be in electrical communication with the cathode solution 16. The cavity 26 of the capillary 8 is filled with an electrophoretic medium, which in some instances can include a polymer matrix. As used herein, the term anode refers to the positively charged electrode. Thus, negatively charged species move through the electrophoretic medium toward the anode. The term cathode refers to the negatively charged electrode; positively charged species migrate toward this electrode. The anolyte is the solution in which the anode is immersed and the catholyte is the solution in which the cathode is immersed.

Sample is introduced into the capillary 8 via an inlet 28, and the components therein resolved as an electrical field is applied between the two electrodes 22, 24 by a power source 32 and the components separate within the electrophoretic medium contained within the separation cavity 26. Components can be controllably eluted from the capillary via outlet 30 by controlling various parameters such as electroosmotic flow (see *infra*) and/or by changing the composition of one or both of the reservoir solutions (*e.g.*, adjusting the pH or salt concentration). Typically, the inlet 28 and the outlet 30 are simply portions of the capillary formed to allow facile insertion into a container containing sample, anolyte or catholyte.

The term "capillary" as used in reference to the electrophoretic device in which electrophoresis is carried out in the methods of the invention is used for the sake of convenience. The term should not be construed to limit the particular shape of the cavity or device in which electrophoresis is conducted. In particular, the cavity need not be cylindrical in shape. The term "capillary" as used herein with regard to any electrophoretic method includes other shapes wherein the internal dimensions between at least one set of opposing faces are approximately 2 to 1000 microns, and more typically 25 to 250 microns. An example of a non-tubular arrangement that can be used in certain methods of the invention is the a Hele-Shaw flow cell (see, *e.g.*, U.S. Pat. No. 5,133,844; and Gupta, N.R. et al., J. Colloid Interface Sci. 222:107-116 (2000). Further, the capillary need not be linear; in some instances, the capillary is wound into a spiral configuration, for example.

An example of a system utilized with certain methods of the invention is illustrated in FIG. 1. This particular example shows a system in which three electrophoresis methods (initial, intermediate and final methods) are linked. The particular number of electrophoretic methods conducted can vary, although the methods of the invention generally include at least two electrophoretic methods, Most typically, the methods utilize two or three electrophoretic separation methods.

As can be seen in FIG. 1, an initial sample containing a plurality of components is introduced from sample container 50 into a first separation cavity of a first capillary 54 via sample inlet 52 utilizing any of a number of methods known in the art. Examples of suitable methods include, pulling sample into the sample inlet 52 under vacuum (*e.g.*, by pulling a vacuum on the sample outlet) or pushing sample into the sample inlet 52 by pressurizing the sample container 50. Electromigration, often referred to as electrokinetic injection, is another options. Once the initial sample is introduced into sample inlet 52, the sample is then electrophoresed within the first separation cavity within the first capillary 54. The first separation cavity contains a desired electrophoretic medium in which components in the initial sample are at least partially resolved. Electrophoretic medium containing resolved components is withdrawn from the first cavity, typically out the end of the separation cavity opposite the end in which sample was introduced, although other withdrawal sites can be utilized (see *infra*). The withdrawn medium travels through outlet 56 and is collected in separate containers 58 as multiple fractions. As shown in FIG. 1B, the containers 58 into which fractions are collected are typically associated with a fraction collection device (a portion of which is shown 60) capable of automatically advancing a set of containers 58 to collect defined fractions (*e.g.*, fractions of a certain volume or covering a selected pH range).

A sample from a fraction collected from the first electrophoretic method is then withdrawn from one of the plurality of containers 58, again utilizing techniques such as those described *supra,* via a second sample inlet 62. Components in the sample from the fraction can then be further resolved by conducting an intermediate electrophoretic method (in the example shown in FIG. 1, the second electrophoretic method). The sample is introduced into a second capillary 64 via inlet 62 and the components within the sample further separated within the electrophoretic medium contained within the second separation cavity of the second capillary 64 and then eluted from the cavity via outlet 66. As with the first electrophoretic separation, the electrophoretic medium containing the resolved or partially resolved components is collected as separate fractions within containers 68 typically aligned and advanced by a second fraction collection device (a portion of which is shown 70).

A process similar to the second/intermediate method is conducted during the final electrophoretic method (the third Electrophoretic separation method shown in FIG. 1). Sample is drawn via inlet 72 from a container 68 containing a fraction obtained during the preceding method and is introduced into a third or final electrophoretic cavity of a third capillary 74 containing a third electrophoretic medium in which components contained in the applied sample are further separated by electrophoresis. The third electrophoretic medium containing the further isolated proteins is subsequently withdrawn through outlet 76.

As noted above, more than the three electrophoretic methods shown in FIG. 1 can be performed. Such methods essentially involve repeating the general steps described for the second/intermediate electrophoretic separation above one or more times.

Following the final electrophoretic separation, a variety of different options for analyzing the resolved components are available. As shown in FIG. 1, withdrawn electrophoretic medium can be passed through an optional detector 78 in fluid communication with the separation cavity of the last capillary 74 to detect the resolved components (*e.g.*, labeled proteins). The detector 78, or an optional quantifying device capable of receiving a signal from the detector (not shown), can be used to quantitate the amount of components within a certain portion or fraction of the electrophoretic medium. Detectors can also be utilized to monitor the progress of separation after other columns as will.

Fractions are taken from the electrophoretic medium exiting the final capillary 74 or the detector 78 and analyzed by an analyzer 82 to determine the molecular weight of the components within a fraction. In particular, the analyzer is used to determine the abundance of the enriched isotope in the target analytes. As described *infra*, a variety of analyzers and techniques can be utilized to make this determination. For example, the analyzer 82 can be a mass spectrometer or an infrared spectrometer. Mass spectral data, for example, can be utilized to determine the mass of the various components within a fraction. The ratio of labeled and unlabeled target analytes can be determined from the relative signal intensities for the labeled and unlabeled target analytes in the mass spectrum.

The specific elution conditions utilized to withdraw resolved components from the separation cavity depends upon the type of electrophoretic method conducted and is described more fully below for each of the electrophoretic methods typically utilized in the present invention. In general, however, once components have been resolved within the separation cavity, the conditions within the cavity are adjusted as necessary (or the initial conditions selected) to achieve selective or controlled elution of the components from the cavity. For example, elution can be achieved by adding salts to, or adjusting the pH of, the anode or cathode solution, by regulating electroosmotic flow, by applying hydrodynamic pressure or combinations of the foregoing.

Using the methods of the invention, resolved components can be isolated physically (*e.g.*, placement into different containers such as illustrated in FIG. 1), spatially (*e.g.*, spread throughout the Electrophoretic medium contained in the separation cavity) and/or temporally (*e.g.*, controlling elution so different components within a sample elute from the capillary at different times). Thus, the methods of the invention can separate mixtures of components as a function of the composition of elution buffers and/or time. The methods are not limited to the spatial separation of components as are certain traditional gel electrophoresis systems (*e.g.*, 2-D gel electrophoresis systems for protein separation or pulsed-field and sequencing gel systems for nucleic acid separations), or two-dimensional thin layer chromatography (2-D TLC) methods (for small molecule metabolite separations). Instead, with controlled elution, fractions can be collected so components within a fraction fall within a range of isoelectric points and electrophoretic mobilities, for example. Controlled elution of components means that methods can be performed in a reproducible fashion. Such reproducibility is important in conducting comparative studies and in diagnostic applications, for example.

During the elution or withdrawing of resolved components, generally only a portion of the electrophoretic medium containing the resolved component is typically collected in any given fraction. This contrasts with certain 2-D methods in which a gel containing all the resolved components (*e.g.*, proteins) is extruded from the separation cavity and the extruded gel containing all the components is used to conduct another electrophoretic separation. This also contrasts with certain 2-D thin layer chromatography methods in which all the metabolites are separated by their relative affinities for the matrix in a line using one solvent system and are reseparated based on altered affinities by a second solvent system applied perpendicularly to the direction of flow of the first solvent system.

Spacially, physically or temporally resolved components obtained at the conclusion of one electrophoretic method are then used as the source of samples for further separation of components contained within the fraction during a subsequent electrophoretic method. As illustrated in FIG. 1, typically samples from different resolved fractions are sequentially electrophoresed on the same capillary. Normally another sample is not applied until the components in the preceding sample are sufficiently withdrawn from the separation cavity so that there is no overlap of components contained in different fractions. Sequential elution of fractions through the same column can significantly reduce or eliminate variations resulting from difference in cross-linking or electric field strength that can be problematic in certain slab gel electrophoretic methods. Hence, sequential separation can further enhance the reproducibility of the methods of the invention. Other methods, however, can be performed in a parallel format, wherein samples from different fractions are electrophoresed on separate capillaries. This approach allows for separations to be completed more quickly, However, the use of multiple capillaries can increase the variability in separation conditions, thereby reducing to some extent reproducibility between different samples.

In certain methods, the electrophoretic methods are conducted so that pools containing similar components are obtained. For example, the electrophoretic conditions can be controlled so that after the first or first few electrophoretic methods at least one pool containing primarily related components is obtained (*e.g.*, a pool containing primarily proteins, polysaccharides, nucleic acids, amino acids, nucleotides, nucleosides, oligosaccharides, phosphorylated mono- or oligosaccharides, fats, fatty acids or organic acids). Pools of related components can be obtained by capitalizing on the distinctive feature of the different classes of components within a cell. For example, some classes of components are primarily singly charged (*e.g.,* phosphorylated mono- or oligosaccharides), whereas others are primarily zwitterionic (*e.g.*, amino acids, proteins, nucleotides and some fats). CIEF can be used to resolve different zwitterionic components and can also be used to separate zwitterionic species from non-zwitterionic species. Large components (*e.g.*, proteins) can be separated from smaller components (*e.g.*, amino acids, mono- and disaccharides, nucleotides and nucleosides) using CGE. Through judicious selection of pH and buffer conditions, one can control the charge on various components and effect separation of components having different charge-to-mass ratios by CZE. For example, certain buffers can be utilized that selectively complex with certain components to introduce a desired charge to the selected components. An example of such a buffer is a borate buffer that can be used to complex to carbohydrates, thereby imparting a negative charge to the carbohydrates present in the sample. Additional details regarding the Electrophoretic methods are set forth *infra*.

By controlling the electrophoretic conditions to initially separate a complex mixture into pools of different classes of components, one can simplify an analysis considerably. For example, if the metabolite of interest is a carbohydrate, by controlling conditions appropriately so that a pool of carbohydrates is obtained (*e.g.*, using borate buffers), one can ignore fractions containing other classes of compounds. Thus, subsequent electrophoretic separations can simply be conducted with a sample from the pool(s) of interest. Alternatively, if the pool of similar compounds is sufficiently small, individual components of the pool can be completely resolved by mass spectrometric means after the electrophoretic separations. Similarly, once conditions have been established for a particular metabolite, it is not necessary to analyze all fractions obtained from the various electrophoretic methods. The reproducibility of the method enables a sample to be taken only from the few fractions obtained adjacent the fraction(s) previously established to contain the target analytes of interest. Nonetheless, because certain methods can be automated, even during initial screening tests, for example, one can quickly analyze all the final fractions. Even scanning the mass spectrum to identify signals for mass fragments of interest can be automated through the use of computer programs to speed analysis.

D. Detection

Once the target analytes have been at least partially purified from other molecules in the sample, the relative abundance of the isotope in the unmetabolized substrate and/or target analyses is determined. Typically, this involves determining the ratio of the enriched isotope to the more abundant isotope (*e.g.*, $^{12}C/^{13}C$, $^{14}N/^{15}N$, $^{16}O/^{18}O$ and $^{34}S/^{32}S$), although other measures of abundance can also be determined.

The measurement of the concentration of the enriched stable isotope can be made according to a variety of options. One approach is to determine the relative abundance of the isotopic label by mass spectrometry. The target analytes generate distinct signals in the mass spectrum according to the mass to charge ratio of the substrate. The relative signal intensities for the different isotopic forms present enables the relative abundance of the different isotopic forms of each target analyte to be calculated, regardless of the absolute concentration of the analyte in the sample.

Methods for analyzing various biological molecules by mass spectrometry have been established. Mass spectrometry can be used according to known methods to determine the masses of relatively small molecules (*e.g.*, nucleosides, nucleotides, mono and di-saccharides) as well as relatively large molecules. For example, mass spectrometry has increasingly been applied to protein identification. Electrospray and matrix assisted laser desorption ionization (MALDI) are the most commonly used mass spectrometric techniques applied to protein analysis because they are best able to ionize large, low volatility molecular species,

In the case of DNA, the DNA can by hydrolyzed to deoxyribonucleosides using standard methods of hydrolysis. For example, the DNA can be hydrolyzed enzymatically, such as for example with nucleases or phosphatases, or non-enzymatically with acids, bases or other methods of chemical hydrolysis. Alternatively, intact DNA polymers can be analyzed. Deoxyribonucleosides can then be prepared for mass spectroscopic analysis using standard techniques (e.g., synthesis of trimethylsilyl, methyl, acetyl and related derivatives or direct probe injection).

For the following major classes of metabolites, the following sources provide additional guidance on mass spectral analysis of such molecules and are incorporated by reference in their entirety: (1) lipids (*see*, *e.g.*, Fenselau, C., "Mass Spectrometry for Characterization of Microorganisms", ACS Symp. Ser., 541:1-7 (1994); (2) volatile metabolite (*see, e.g.,* Lauritsen, F.R. and Lloyd, D., "Direct Detection of Volatile Metabolites Produced by Microorganisms," ACS Sympl Ser., 541:91-106 (1994)); (3) carbohydrates (*see*, *e.g.*, Fox, A. and Black, G.E., "Identification and Detection of Carbohydrate Markers for Bacteria", ACS Symp. Ser. 541: 107-131 (1994); (4) nucleic acids (*see*, *e.g.*, Edmonds, C.G., et al., "Ribonucleic acid modifications in microorganisms", ACS Symp. Ser., 541:147-158 (1994); and (5) proteins (*see, e.g.,* Vorm, O. et al., "Improved Resolution and Very High Sensitivity in MALDI TOF of Matrix Surfaces made by Fast Evaporation," Anal. Chem. 66:3281-3287 (1994); and Vorm, O. and Mann, M., "Improved Mass Accuracy in Matrix-Assisted Laser Desorption/Ionization Time-of-Flight Mass Spectrometry of Peptides", J. Am. Soc. Mass. Spectrom. 5: 955-958 (1994)). Further details regarding mass spectral analysis is set forth *infra.*

Labeled carbon dioxide ($[^{13}C]CO_2$) can also be detected using mass spectrometry. Such approaches are described, for example, by Ewing, G.W., Instrumental Methods of Chemical Analysis, 4th ed., (1975); and Klein, P., et al., "Stable Isotopes and Mass Spectrometry in Nutrition Science", Analytical Chemistry Symposium Series 21:155-166 (1984), both of which are incorporated by reference in their entirety.

An alternative to detection by mass spectrometry is to detect the isotope label using infrared (IR) spectroscopy or nuclear magnetic resonance spectroscopy (NMR). Various target analytes can be detected using this approach, including carbon dioxide, for example. IR and NMR methods for conducting isotopic analyses are discussed, for example, in U.S. Pat. No. 5,317,156; Klein, P. et al., J. Pediatric Gastroenterology and Nutrition 4:9-19 (1985); Klein, P., et al,., Analytical Chemistry Symposium Series 11:347-352 (1982); and Japanese Patent Publications No. 61-42219 and 5-142146, all of which are incorporated by reference in their entirety.

In certain methods, target analytes partially or completely purified by the Electrophoretic methods are subsequently transported directly to an appropriate detector for analyzing the isotopic composition of the target analytes. In some methods, samples are withdrawn from the individual fractions collected during the final electrophoretic separation and injected directly onto a mass spectrometer to determine relative abundances.

E. Flux Determination

In general, the flux of metabolites through each reaction step in any given pathway depends on the relative rates of the forward reaction and reverse reactions. As used herein, flux refers to the rate of change in concentration of a target analyte as a function of time and sample size. The metabolic flux through any single metabolic conversions can be determined from the change in the relative abundance ($RA_t$) of isotopically labeled analyte over time (t) according to the equation:

$$\text{Flux}_{\text{analyte}} = \frac{\ln\left\{1 - {RA_t}/{RA_{ss}}\right\}}{(t)(\text{unit of sample})}$$

where $RA_{ss}$ is the relative abundance of the labeled metabolite at long times. Relative abundance (RA) is the relative concentrations of isotopically labeled substrate and/or target metabolites (i.e., the target analytes) determined from the ratio of the abundances of isotopic label in the target analytes. In some embodiments, the steady-state relative abundance of the isotope can be considered equal to the known ratio in the initial substrate administered to the subject, such that a only a single sample is needed to determine the metabolic flux. In another embodiment, the steady-state relative abundance of the isotope can be predicted from simultaneous solution of the above equation for two or more relative abundance measurements taken from samples taken at different time points. In another embodiment, the steady-state relative abundance of the isotope can be measured directly from samples taken at long times.

It is apparent to those skilled in the art that an alternative form of the above equation can be used to determine the flux of an analyte from the depletion of isotopically labeled analyte or substrate following a reduction in the relative abundance or isotopically labeled substrate. This alternative form is:

$$\mathrm{Flux}_{\mathrm{analyte}} = \frac{\ln\left\{\frac{(RA_t - RA_{ss})}{(RA_o - RA_{ss})}\right\}}{(t)(\text{unit of sample})}$$

where $RA_o$ is the initial relative abundance of the isotopically labeled analyte prior to the administration of substrate to change the relative abundance. In one embodiment, $RA_o$ is measured directly prior to administration of the new substrate. In another embodiment, $RA_o$ is assumed to be the same as the relative isotope abundance in the substrate administered prior to the change.

The relative metabolic flux of substrate into any metabolic branch (i) in a network of n branched metabolic pathways is determined from the ratio of relative abundances of iotopically labeled analyte appearing in analytes downstream in each branch (j) of the metabolic pathway at any time (t), but preferably at long times (i.e., at the steady-state condition), according to the equation:

$$\mathrm{Flux}^{i}_{\mathrm{branch}} = \frac{RA^{i}_{t}}{\sum_{j=1}^{n} RA^{j}_{t}}\left[\mathrm{Flux}_{\mathrm{substrate}}\right]$$

To determine flux, typically one or more samples are withdrawn from the subject at different predetermined time points. The samples are then treated, optionally purified, and then analyzed as described above to determine one or more values for the relative concentration of the isotopic label in the target analytes at a sampling time(s) (t). These values can then be utilized in the formula set forth above to determine a flux rate for each of a plurality of target analytes. In some instances, the target analytes used to determine flux are all organic compounds (*i.e.*, the analytes do not include carbon dioxide, for example).

It is apparent to those skilled in the art that more accurate flux determinations and standard errors of the estimated fluxes can also be made using statistical curve fitting or parameter fitting methods generally known in the art [e.g., Zar, J.H. Biostatistical Analysis, (Prentice-Hall, Englewood Cliffs, NJ, 1974)] and isotopic ratio data obtained from a plurality of samples taken at different times.

The metabolic flux through a pathway depends on the rate determining step(s) within the pathway. Because these steps are slower than subsequent steps in the pathway, a product of a rate determining step is removed before it can equilibrate with reactant. Further guidance on flux and methods for its determination is provided, for example, by Newsholme, E.A.. et al., Biochem. Soc. Symp. 43:183-205 (1978); Newsholme, E.A,, et al., Biochem. Soc. Symp. 41: 61-110 (1976); and Newsholme, E.A., and Sart., C., Regulation in Metabolism, Chaps. 1 and 3, Wiley-Interscience Press (1973).

V. Synthesis of Labeled Metabolic Substrates

The synthesis of isotopically labeled biological compounds has been well established for a variety of different types of compounds including, for example, nucleic acids, proteins, carbohydrates, as well as glycolysis and other metabolic pathways intermediates. Methods for isotopically labeling nucleic acids are discussed, for example, in U.S. 6,010,846; the labeling of carbohydrates is discussed in U.S. Pat. No. 4,656,133; and methods for labeling glycolysis intermediates is discussed in U.S. Pat. No. 5,439,803, all of which are incorporated by reference in their entirety.

In some instances, isotopically labeled biological compounds are obtained by feeding live organisms a diet enriched in one or more stable isotopes, harvesting and purifying the desired isotopically enriched compounds resulting from natural metabolism of the isotopically-enriched diet. Alternatively, isotopically-enriched substrates can be synthesized chemically from isotopically enriched precursors. Many suitable substrates for metomics studies (e.g., [$^{13}$C]-fatty acids, [$^{2}$H,$^{13}$C and $^{15}$N]-amino acids, [$^{13}$C and $^{2}$H]-peptides, and [$^{13}$C and $^{15}$N]-nucleotides) are available from commercial sources such as Isotec (Miamisburg, OH), ICN Pharmaceuticals (Costa Mesa, CA), and Sigma-Aldrich (St. Louis, MO).

VI. Capillary Electrophoresis Methods for Metabolite Profiling

A. Capillary Isoelectric Focusing Electrophoresis (CIEF) for metabolites

1. General

Isoelectric focusing is an electrophoretic method in which zwitterionic substances such as proteins, nucleotides, amino acids and some fats are separated on the basis of their isoelectric points (pI). The pI is the pH at which a zwitterionic species such as a protein has no net charge and therefore does not move when subjected to an electric field. In the present invention, zwitterionic species can be separated within a pH gradient generated using ampholytes or other amphoteric substances within an electric field. A cathode is located at the high pH side of the gradient and an anode is located at the low pH side of the gradient.

Zwitterionic species introduced into the gradient focus within the pH gradient according to their isoelectric points and then remain there. The focused components can then be selectively eluted as described below. General methods for conducting CIEF are described, for example, by Kilar, F., "Isoelectric Focusing in Capillaries," in CRC Handbook on Capillary Electrophoresis: A Practical Approach, CRC Press, Inc., chapter 4, pp. 95-109 (1994); and Schwartz, H., and T. Pritchett, "Separation of Proteins and Peptides by Capillary Electrophoresis: Application to Analytical Biotechnology," Part No. 266923 (Beckman-Coulter, Fullerton, CA, 1994); Wehr, T., Rodriquez-Diaz, R., and Zhu, M., "Capillary Electrophoresis of Proteins," (Marcel Dekker, NY, 1999), which are incorporated herein by reference in their entirety.

2. System and Solutions

Because CIEF is primarily an equilibrium technique with low current densities, capillary heating typically is not a problem. Therefore, fairly large bore capillaries can be utilized. Suitable sizes include, but are not limited to, capillaries having internal diameters of 2-600 μm, although more typically capillaries having internal diameters of 25-250 μm are utilized. The use of relatively large bore capillaries means the method can use relatively high sample loads, which facilitates detection in subsequent dimensions. This feature of CIEF makes the method well suited for the initial or one of the early electrophoretic separations in the series. However, smaller diameter capillaries enable temperature to be controlled more carefully and, in some methods, result in improved signal detection (*e.g.*, by laser induced fluorescence (LIF) detection of fluorescently labeled proteins).

The capillaries can have varying lengths. The length selected depends in part on factors such as the extent of separation required. Typically, the capillaries are about 10 to 100 cm in length, although somewhat shorter and longer capillaries can be used. While longer capillaries typically result in better separations and improved resolution of complex mixtures, longer capillaries also afford more opportunities for interactions between species in the sample and the capillary wall and lower field strength. Consequently, there tends to be an upper limit on capillary length beyond which resolution may be lost. Longer capillaries can be of particular use in resolving low abundance compounds. Further guidance on size and length of capillaries is set forth, for example, in Palmieri, R. and J. A. Nolan, "Protein capillary electrophoresis: Theoretical and experimental considerations for methods development," in CRC Handbook of Capillary Electrophoresis: A Practical Approach, Chp. 13, pgs. 325-368 (CRC Press, Boca Raton, 1994).

Generally, the capillaries are composed of fused silica, although plastic capillaries and PYREX (*i.e.*, amorphous glass) can be utilized in certain methods. As noted above, the capillaries do not need to have a round or tubular shape. Other shapes wherein the internal dimension between opposing faces is within the general range set forth in this section can also be utilized.

A variety of different anode and cathode solutions can be used. Common solutions include sodium hydroxide as the catholyte and phosphoric acid as the anolyte. Similarly, a number of different ampholytes can be utilized to generate the pH gradient, including numerous commercially available ampholyte solutions (*e.g.*, BioLyte, Pharmalyte and Servalyte). The selection of ampholytes and the breadth of the ampholyte gradient can impact the resolution that is achieved by CIEF methods. Narrow ampholytes gradients increase the number of theoretical plates in the separation and can be beneficial for higher resolution separations over narrow pI range.

CIEF methods utilized in the separations of the invention can be conducted in capillaries containing polymeric matrices or in free solution (*i.e.*, no gel or other polymeric matrix). Polymer matrices are typically added to slow electroosmotic flow; however, in some instances, inclusion of polymeric matrices can restrict movement of larger proteins (Patton, W.F., "Defining protein targets for drug discovery using Proteomics," paper presented at the IBC Proteomics conference, Coronado, CA (June 11-12, 1998)). The use of free solutions is preferable in such cases possibly in combination with other methods (*e.g.*, capillary coatings, gel plugs, or induced electric fields) to control the electroosmotic flow.

3. Sample Preparation for Metabolite Profiling

In some instances, samples to be electrophoresed by CIEF are subjected to denaturants to denature certain macromolecules, particularly proteins. This ensures that the same components all have the same charge and thus identical components focus at the same location rather than potentially at multiple zones within the capillary. Denaturants (*e.g.*, urea), non- and zwitterionic-surfactants (*e.g.*, IGEPAL CA-630 or 3-[{3-cholamidopropyl}dimethylammonio]-1-propane sulfonate) can also be used to suppress protein-wall and/or protein-protein interactions that can result in protein precipitation. An advantage of denaturing the proteins within a sample prior to electrophoresis is that the results can be used in comparisons with archival data typically obtained under denaturing conditions.

A typical denaturing buffer includes urea and a nonionic or zwitterionic surfactant as denaturants; a reducing agent (*e.g.*, dithiothreitol (DTT) or mercaptoethanol) is typically included to reduce any disulfide bonds present in the proteins. Other denaturants besides urea that can be used include, but are not limited to, thiourea and dimethylformamide (DMF). Generally, guanidine hydrochloride is not utilized as a denaturant because of the very high ionic strength it imparts to a sample. Exemplary neutral detergents include polyoxyethylene ethers ("tritons"), such as nonaethylene glycol octylcyclohexyl ether ("TRITON" X-100), polyglycol ethers, particularly polyalkylene alkyl phenyl ethers, such as nonaethylene glycol octylphenyl ether ("NONIDET" P-40 or IGEPAL CA-630), polyoxyethylene sorbitan esters, such as polyoxyethylene sorbitan monolaurate ("TWEEN"-20), polyoxyethylene ethers, such as polyoxyethylene lauryl ether ($C_{12}E_{23}$) ("BRIJ"-35), polyoxyethylene esters, such as 21 stearyl ether ($C_{18}E_{23}$) ("BRIJ"721), N,N-bis[3-gluconamidopropyl]cholamide ("BIGCHAP"), decanoyl-N-methylglucamide, glucosides such as octylglucoside, 3-[{3-cholamidopropyl}dimethylammonio]-1-propane sulfonate and the like.

The optimal amount of denaturant and detergent depends on the particular detergent used. In general the denaturing sample buffers contain up to 10 M urea (more typically 4-8 M and most typically 6-8 M). Specific examples of suitable buffers (and denaturants and nonionic surfactants for inclusion therein) include those described by Hochstrasser et al. (Anal. Biochem. 173:424 (1988)) and O'Farrell (J. Biol. Chem. 250:4007 (1975)). Denaturation is typically advanced by heating for 10 min at 95 °C prior to injection into the capillary. Adjustments in the denaturing sample buffers are made as necessary to account for any electroosmotic flow or heating effects that occur (*see, e.g.,* Kilar, F., "Isoelectric Focusing in Capillaries," in CRC Handbook on Capillary Electrophoresis: A. Practical Approach, CRC Press, Inc., chapter 4, pp. 95-109 (1994)).

The amount of sample injected can vary and, as noted above, depends in part of the size of the capillary used. In general, the capillary is loaded with 0.1 to 5.0 mg of sample. Samples can be spiked with one or more known pl standards to assess the performance of the method.

4. Elution

A variety of techniques can be utilized to elute or withdraw electrophoretic medium containing resolved compounds out from the capillary, but these methods fall into three general categories: hydrodynamic elution, electroelution and control of electroosmotic flow.

a. Hydrodynamic/Pressure Elution

Hydrodynamic or pressure elution involves applying pressure (or pulling a vacuum) via an appropriate pump connected with one end of the capillary (*see, e.g.* Kilar, F., "Isoelectric Focusing in Capillaries," in CRC Handdbook on Capillary Electrophoresis: A Practical Approach, CRC Press, Inc., chapter 4, pp. 95-109 (1994)). However, hydrodynamic elution can cause band broadening and loss of resolution due to the parabolic flow profile that is formed in the capillary.

b. Electroelution

Electroelution, the other major approach, encompasses a variety of techniques and in general involves altering the solution at the anode and/or cathode to change some parameter (*e.g.*, pH, ionic strength, salt concentration) of the electrophoretic medium in the separation cavity sufficiently to effect elution.

i. Salt mobilization

One electroelution approach involves addition of a salt to the catholyte or anolyte, the salt having a non-acidic or non-basic counterion of the same charge as the acidic or basic species within the reservoir to which the salt is added so that the counterion migrates from the reservoir into the capillary. Since electrical neutrality must be maintained within the capillary, the movement of the counterion into the capillary results in a reduction of the concentration of protons or hydroxide within the capillary, and thus the pH is either raised or lowered. The theoretical basis for this type of mobilization is described by S. Hjerten, J.-L. Liao, and K. Yao, J. Chromatogr., 387:127 (1987). For example, if the catholyte is sodium hydroxide (*i.e.*, the basic species is hydroxide) then a salt having a negatively charged counterion other than hydroxide is added, for example sodium chloride. Movement of chloride ion into the capillary reduces the local concentration of hydroxide within the capillary, thereby decreasing the pH. As another example, if the anolyte is phosphoric acid, then a salt having a counterion other than a proton is added, for example sodium phosphate. In this instance, movement of sodium ion into the capillary reduces the local concentration of protons within the capillary thereby increasing the pH. As the pH is lowered or raised within regions of the capillary due to the presence of the added counterion, elution occurs since the ampholytes, and the focused components, migrate to the newly-defined pH regions corresponding to their isoelectric points. It has been shown that both the type and concentration of salt used for mobilization has impact on the resolution of eluted compound peaks (*see, e.g.,* R. Rodriguez-Diaz, M. Zhu, and T. Wehr, J. Chromatogr. A, 772:145 (1997)). For example, the addition of sodium tetraborate instead of sodium chloride to the catholyte results in greatly increased resolution of separated proteins.

ii. pH mobilization

Another technique, referred to herein as "pH mobilization" can also be utilized to elute compounds during CIEF. In this approach, an additive is added to either the anode or cathode solution to alter the pH of the solution. Unlike salt mobilization, however, the additive does not contribute a mobile counterions that moves into the capillary. Here, the elution occurs as a result of the pH gradient being redefined by the pH of one or both of the reservoirs; therefore, components with pI's that fall outside of this redefined pH gradient are eluted into either the anode or cathode reservoirs. Typically, the technique for cathodic mobilization proceeds as follows. Once the components are focused (*e.g.*, in a pI range of 3-10 using phosphoric acid as the anolyte and sodium hydroxide as the catholyte) the cathodic capillary end is immersed into a reservoir containing a solution that has a pH slightly less than 10, for example 50 mM imidazole (pKa 7) which has a pH of 9.85. The components are then allowed to refocus in the capillary, recognizable by a stabilization of the current through the capillary, the pI range now being defined by 3-9.85. Any components with an isoelectric point of 9.85 to 10 are eluted into the catholyte. The process can be repeated with catholyte containing a species that reduces the pH to slightly less than 9.85. In a stepwise fashion, the pH can continued to be reduced to pH 7, thereby collecting separated components in fractions that span the range of 7-10. At this point, anodic mobilization can proceed by replacing the anolyte with acids of increasing pKa to selectively increase the pH from 3 to 7, thereby collecting fractions in the acidic range (pH 3-7). The number of fractions can vary depending on the desired fractionation resolution. Typically, these fractions are defined by differences of 0.05-0.5 pH units.

The technique of pH mobilization can be useful for samples containing a high concentration of one or more components (*e.g.*, proteins) that may cause uneven spatial gradients inside the capillary. Using pH mobilization, only those components with isoelectric points below or above the pI range that is defined by the reservoir pH's are eluted. This elution is, therefore, reproducible regardless of differences in the shape of the capillary pH gradient or the presence of uneven spatial gradients inside the capillary.

c. Electroosmotic Flow (EOF)

Regulating the magnitude of electroosmotic flow (EOF) significantly affects the preceding electroelution methods (see *supra*) and is another means by which resolved components can be selectively withdrawn upon conclusion of an isoelectric focusing separation. EOF is generated by the ionization of silanol functionalities on the surface of a silica capillary. Such ionization results in a layer of protons in the electrophoretic medium at the surface of the silica capillary. Once an electric field is applied, the layer of protons essentially constitutes a positively charged column of fluid that migrates toward the cathode, thereby causing bulk flow of the electrophoretic medium within the capillary. Apparent velocity of components is equal to the sum of the electroosmotic flow and their electrophoretic mobility. Thus, by controlling EOF, one can control or regulate the rate at which components move through the capillary. In CIEF methods, generally EOF should be controlled to allow components within an injected sample sufficient time to focus before the proteins begin eluting from the capillary.

A variety of techniques can be utilized to regulate EOF. One approach involves coating the walls of capillaries with various agents. For example, EOF along glass silicate surfaces can be substantially reduced by silanizing them with a neutral silane reagent that masks a substantial percentage of surface silanol groups (*e.g.,* polyacrylamide, polyethylene glycol and polyethylene oxide). The magnitude of EOF can be further controlled using silanizing reagents that include positively or negatively charged groups. Positively charged coatings can be used to nullify surface negative charges to give a net surface charge of zero, so that EOF approaches zero. Coatings with higher positive charge densities can be used to reverse the direction of EOF for charged surface materials. This can be useful for slowing the net migration rates of positively charged sample species. Conversely, negatively charged coatings can be used to impart to or increase the magnitude of the negative charge on surfaces, so as to increase the net migration rates of negatively charged species. Representative positively charged coatings include trialkoxysilanes with polyethyleneimine, quaternized polyethyleneimine, poly(-N-ethylaminoacrylamide) and chitosans, for example. Representative negatively charged coatings include trialkoxysilanes with carboxylate and sulfonate containing materials such as poly(methylglutamate) and 2-acrylamido-2-methylpropanesulfonate polymers, for example. Charged coatings can also effectively reduce sample adsorption, especially for samples having the same charge polarity as the coating.

The separation medium can also include soluble agents for dynamically coating the walls of the separation cavity, to help reduce EOF during electrophoresis. Such soluble coating agents include quaternary ammonium-containing polymers, methyl cellulose derivatives, cellulose acetate, polyethylene oxide, chitosan, polyvinyl alcohol, polyethylene glycol, polyethylenimine, and polyethylene oxide-polypropylene oxide-polyethylene oxide triblock copolymers, for example. Typically, soluble coating agents are included at concentrations of about 0.05% to about 4%, and more typically of about 1% to about 2%.

EOF and sample absorption can also be adjusted by including suitable reagents in the separation medium and running buffers. For example, negative surface charges can be masked by including a cationic additive in the medium, such as metal amine complexes, amines and polyamines such as propylamine, triethylamine, tripropylamine, triethanolamine, putrescine, spermine, 1,3-diaminopropane, morpholine, and the like. Zwitterionic species comprising both negatively and positively charged groups that are isoelectric at the pH of electrophoresis can also be used, such as trialkylammonium propyl sulfonates, where alkyl is methyl, ethyl, propyl, and longer alkyl chains.

Another approach involves the generation of a current that opposes EOF. Typically, this is accomplished by applying a thin film of metal (*e.g.*, iridium tin oxide or copper) to an external surface of the capillary. Application of current to the film generates a relatively small induced current within the capillary to reverse the EOF (*see*, *e.g.*, Schasfoort, R.B.M., Schlautmann, S., Hendrikse, J., and van den Berg, A., "Field-Effect Flow Control for Microfabricated Fluidic Networks," Science, 286:942-945 (1999)).

Placing a porous plug at a location upstream from where sample is introduced (upstream referring to a direction opposite the flow of components through the capillary) can also be utilized to control EOF. An example illustrating the location of the plug is illustrated in FIG. 2B where the capillary 100 extends from the anode reservoir (not shown) at one end and the cathode reservoir at the other end (not shown). Component migration is in the direction of arrow 102 (*i.e.*, from the anode to cathode direction).

As can be seen, the porous plug 104 is positioned to be upstream of the trailing edge 106 of the sample once introduced into the capillary 100. The porous plug 104 is typically formed of a polymeric material and remains relatively stationary during electrophoretic runs. Examples of suitable materials from which the plug can be formed include polymerized acrylamide with diacrylamide crosslinkers and agarose. Although not intending to be bound by any particular theory, the porous plug 104 appears to function as a momentum transfer barrier by blocking replacement of bulk fluid that in the absence of the plug 104 would move toward the cathode reservoir.

In some methods, such as those containing large amounts of a particular component (*e.g.*, a protein) and/or a large number of different components, EOF should be reduced to very low levels to allow components the opportunity to focus before the electrophoretic medium begins eluting from the capillary due to EOF. In certain methods an EOF of $= 0.5 \times 10^{-6}$ $cm^2/V$-s (at pH 8.6, and 25 mM TRIS-phosphate) has been found to allow ample time for the necessary focusing of proteins before sample elutes from the capillary. Methods described above can reduce EOFs to these levels.

Thus, the foregoing approaches enable fractions to be collected according to different criteria. Electroelution techniques, for example, can be used to collect fractions having a defined pH range. EOF elution and pressure elution, in contrast, can be used to separate fractions according to time of elution. Other techniques can also be utilized to elute resolved proteins after CIEF (*see*, *e.g.* Kilar, F., "Isoelectric Focusing in Capillaries," in CRC Handbook or Capillary Electrophoresis.: A Practical Approach, CRC Press, Inc., chapter 4, pp. 95-109 (1994)). The controlled elution techniques are useful for enhancing reproducibility, an important factor in comparative and diagnostic methods. Such techniques also provide improved tolerance of high abundance components such as proteins as compared to methods relying on spatial separation.

B. Capillary Zone Electrophoresis (CZE) for Metabolite Profiling

1. General

Capillary zone electrophoresis is an electrophoretic method conducted in free solution without a gel matrix and results in the separation of charged components (e.g., proteins, amino acids, fatty acids, fats, sugar phosphates, nucleic acids, nucleotides and nucleosides) based upon their intrinsic charge-to-mass ratios. One advantage to CZE methods is the ability to run with solvent systems that would normally be incompatible with typical water soluble gel matrices. Nonaqueous or water miscible solvent systems can be used to improve the solubility of hydrophobic and membrane bound components that would normally not be resolved by aqueous Electrophoretic methods. General methods for conducting the method are described, for example, by McCormick, R.M. "Capillary Zone Electrophoresis of Peptides," in CRC Handbook of Capillary Electrophoresis: A Practical Approach, CRC Press Inc., chapter 12, pp. 287-323 (1994); Jorgenson, J.W. and Lukacs, K.D., J. High Resolut. Chromatogr. Commun., 4:230 (1981); and Jorgenson, J.W. and Lukacs, K.D., Anal. Chem. 53:1298 (1981)), each of which is incorporated by reference in its entirety.

2. System and solutions

In general, the capillaries described above for CIEF are also suitable for conducting CZE methods. Often the capillaries have internal diameters of about 50 to 100 microns. Buffer composition and pH can significantly influence separations since separations in CZE are based upon charge-to-mass ratios and the charge of a component is dependent upon the pH of the surrounding solution. At the extremes of pH (*i.e.,* below 2 and above 10) it is typically difficult to achieve resolution of many components because most charged groups on the components are either fully protonated or deprotonated and many components have a similar numbers of acidic and basic residues per unit mass. Selectivity is typically enhanced at intermediate pH. For components having a relatively high percentage of acidic groups, selectivity can often be enhanced near pH 4.5. For those components having a high concentration of amine residues, selectivity can be enhanced near pH 10.

In CZE, solutions at the anode and cathode are typically the same. The buffer utilized can be essentially any buffer, the choice of buffer being controlled in part by the pH range at which the electrophoretic method is conducted and its influence on the detector noise. Examples of useful buffers at low pH include, but are not limited to, phosphate and citrate; useful buffers at high pH include Tris/Tricine, borate and CAPS (3-(cyclohexylamino)-1-propane sulfonic acid). Further guidance regarding suitable buffers and buffer additives is described by McCormick, R.M. "Capillary Zone Electrophoresis of Peptides," in CRC Handbook of Capillary Electrophoresis; A Practical Approach, CRC Press Inc., chapter 12 , pp. 287-323 (1994).

In some instances, multiple CZE separations can be conducted with different pH buffers to affect the fractionation of components. For example, a buffer of pH 3 can be used to resolve net positively charged amine functional components (*e.g.,* amino acids, and nucleosides), from neutral (*e.g.*, oligosaccharides, polysaccharides, simple sugars, and fatty acids), and from net negatively charged components (e.g., phosphosugars and nucleotides). Fractions collected from this first CZE dimension can subsequently be further resolved in a second CZE dimension at another pH. For example, the amino acids can be resolved from nuceosides by a second CZE dimension conducted at pH 7 and fatty acids can be resolved from the amino acids at pH of 5.5. The phosphosugars can be further resolved from carboxylic acids by subsequent CZE separation at pH 11.

3. Elution

Elution can be accomplished utilizing some of the same methods described above for CIEF, namely pressure and EOF. As with CIEF, controlling EOF can be important in certain methods to prevent electrophoretic medium containing components from eluting from the capillary before the components within the loaded sample have had an opportunity to separate. EOF can be controlled using the same methods utilized for controlling EOF in CIEF methods (*e.g.*, coating the internal walls of the capillary, using a porous plug and generating an induced field to counteract EOF). Regulating and carefully selecting the pH and ionic strength of the electrophoretic medium is another technique that can be used. Because EOF results from ionization of the silanol groups on the interior capillary surface, by conducting CZE at relatively low pH (*e.g.*, pH 2-5, more typically about pH 3-4) the number of silanol groups that are ionized is reduced. Such a reduction reduces EOF, To prevent sample elution prior to complete separation, in certain analyses the EOF should be reduced to $< 1 \times 10^{-4}$ $cm^2$/V-s (at pH 8.6 and 25 mM TRIS-phosphate buffer). EOF of this level can be obtained using the methods just described.

Covalent modification of one or more analytes can also be used strategically as a means to control component elution. This technique involves adding a chemical moiety to certain components in the sample or a fraction collected from a CE step prior to injecting the sample into the next capillary. By selecting modifying agents that preferentially react with certain functional groups such as amino or carboxyl groups, the charge-to-mass ratio of certain components can be altered. Such alterations can improve the resolution of components during electrophoresis as well as improve their detectability.

D. Capillary Gel Electrophoresis (CGE) for Metabolite Profiling

1. General

Capillary gel electrophoresis refers to separations of proteins, nucleic acids, or other macromolecules accomplished by sieving through a gel matrix, resulting in separation according to size. In one format, proteins are denatured with sodium dodecyl sulfate (SDS) so that the mass-to-charge ratio is determined by this anionic surfactant rather than the intrinsic mass-to-charge ratio of the protein (Cantor, C.R. and Schimmel, P. R., Biophysical Chemistry, W.H. Freeman & Co., NY, (1980)). This means that proteins can be separated solely on the basis of size without charge factoring into the degree of separation. The application of general SDS PAGE electrophoresis methods to capillary electrophoresis (CGE) is described, for example, by Hjertén, S., Chromatogr. Rev., 9:122 (1967).

2. System and Solutions

The type of capillaries and their size are generally as described above for CZE. A variety of different buffers can be used, including commercially available buffers such as the "eCAP SDS" buffers manufactured by Beckman (Hjertén, S., Chromatogr. Rev., 9:122 (1967); Beckman Instruments, "eCAP SDS 200: Fast, reproducible, quantitative protein analysis," BR2511B, Beckman Instruments, Fullerton, CA, (1993); Gottlieb, M. and Chavko, M., Anal. Biochem., 165:33 (1987); Hochstrasser, D.F., et al., Anal Biochem., 173:424 (1988)). Various buffer additives can be utilized to increases resolution. Such additives, include, but are not limited to, small amounts of organic solvents, such as N,N-dimethylformamide, cyclohexyldiethylamine, dimethoxytetraethylene glycol and other polyols (*e.g.*, ethylene glycol and polyethylene glycol) (*see, e.g.,* Palmieri, R. and Nolan, J. A., "Protein capillary electrophoresis: Theoretical and experimental considerations for methods development, in: CRC Handbook of Capillary Electrophoresis: A Practical Approach, Chp. 13, pgs. 325-368, CRC Press, Boca Raton, (1994); Wanders, B.J. and Everaerts, F. M., "Isotachophoresis in capillary electrophoresis," in: CRC Handbook of Capillary Electrophoresis : A Practical Approach, Chp. 5, pgs. 111-127, CRC Press, Boca Ration, FL, (1994)). The use of such solvents can improve the solubility of certain compounds such as lipophyllic components in aqueous solution and enhance their stability against thermal denaturation, (Martinek, K., et al., FEBS Lett., 51:152-155 (1975)) depress the electroosmotic flow in CZE and CGE (Altria, K.D. and Simpson, C.F., Anal. Proc., 23:453 (1986)), alter the electrical double-layer thickness at the capillary wall to inhibit protein binding interactions (Me Cormick, R.M., "Capillary zone electrophoresis of peptides," in: CRC Handbook of Capillary Electrophoresis: A Practical Approach, Chp. 12, pgs. 287-323, CRC Press, Boca Raton, FL, (1994)) and increase the viscosity of the running buffer which depresses the electroosmotic flow. Solvents utilized should be compatible with the polymer matrix inside the capillary.

Isotachophoresis (IPE) can be used in certain methods to increase resolution of charged components. For a general discussion of IPE, see, for example, B.J. Wanders and Everaerts, F.M., "Isotachophoresis in Capillary Electrophoresis," in CRC Handhook of Capillary Electrophoresis: A Practical Approach, chap. 5, pp. 111-127 (1994), which is incorporated by reference in its entirety. The velocity of a charged molecule moving through a capillary under a constant field strength depends on its relative mobility, which is a function of the mass/charge of the molecule, temperature, and viscosity of the medium through which it is moving. However, in the absence of an adequate concentration of highly mobile ions upstream of the sample ions, all the ions eventually have to migrate at the speed of the slowest ion once the electric field reaches a steady-state inside the capillary. This condition causes the anions to stack in order of their relative mobilities at the interface of the leading and terminating buffers.

Under SDS denaturing conditions, all the components present in the sample have nearly identical mass/charges. By using a higher mass/charge anion in the terminal buffer, one can force the components to move at a constant slow speed through the capillary. This has two effects. First, components "stack" at the terminal edge of the leading buffer increasing their effective concentration inside the capillary. Second, any separation between components is based on their size. Therefore, the use of a hybrid IPE-CGE method in which the IPE is used for sample "stacking" can improve the resolution possible in the subsequent CGE separation in some methods.

Various terminal buffer systems can be utilized in conjunction with IPE methods. In one system, ε-aminocaproic acid (EACA) is used as the terminal electrolyte because it has a high mass/charge at high pH (>6). Tris(hydroxyethyl) aminomethane (TRIS) citrate at 0.05M is used as the leading buffer at pH=4.8 and as an intermediate stacking buffer at pH=6.5. The sample components (*e.g.*, proteins) initially "stack" because EACA has a very low mobility in the pH 6.5 stacking buffer, but once the protein "stack" and EACA reach the lower pH leading buffer, the mobility of the EACA surpasses that of the components in the sample and separation commences (see, *e.g.*, Schwer, C, and Lottspeich, F., J. Chromatogr., 623:345 (1992)). This system can be used to create a hybrid single column IPE-CPAGE system.

A 2 buffer system for IPE for the separation of proteins involves dissolving sample in 0.01M acetic acid, which is also used as the terminal electrolyte. The leading and background buffer was 0.02M triethylamine-acetic acid solution at pH 4.4. The sample in terminal buffer is sandwiched between the leading and background buffer. IPE continues until the background buffer overtakes the leading edge of the terminal buffer, at which point IPE stops and separation begins (see, *e.g.*, Foret, F. et al., J. Chromatogr., 608:3 (1992)).

Another IPE approach that can be accomplished with any running buffer is to dissolve the sample in the running buffer but diluted to a lower ionic strength. This causes an increase in the electrical resistance in the capillary where the sample plug is loaded and correspondingly faster movement of the ions present in the sample matrix to running buffer boundary. The optimal ionic strength difference between the sample matrix and the running buffer is typically about 10-fold (*see, e.g.,* Shihabi, Z.K. and Garcia, L. L., "Effects of sample matrix on separation by capillary electrophoresis," in:

CRC Handbook of Capillary Electrophoresis: A Practical Approach, Chp. 20, pgs. 537-548, CRC Press, Boca Raton, FL, (1994)).

3. Elution

In general, the discussion of elution for CZE applies to CGE. Elution can be accomplished utilizing pressure and EOF. As with CIEF and CZE, controlling EOF can be important in certain methods to prevent electrophoretic medium containing components from eluting before the components within the applied sample have had an opportunity to separate. The methods described *supra* for CIEF and CZE can be used to control EOF at desired levels. To prevent sample elution prior to complete separation, in certain analyses the EOF should be reduced to < 1 x $10^{-4}$ $cm^2$/V-s (at pH 8.6 and 25 mM TRIS-phosphate buffer). EOF can be reduced to this range, for example, by controlling the pH of the buffer, by generation of a counteracting induced field, capillary coatings and a porous gel plug.

E. Detection subsequent to Separation

As indicated in FIG. 1, electrophoretic solution withdrawn during the final electrophoretic separation can be directed toward an analyzer 82 for the determination of the relative abundance of the labeled and unlabeled analytes. This arrangement provides considerable flexibility with regard to the nature of detection and does not limit the methods to the standard absorbance and fluorescence techniques. The analyzer need not be positioned to detect eluted components as shown in FIG. 1, however. In other arrangements, the analyzer is adapted so that it can scan resolved components within the separation cavity of the capillary tube itself. An example of such an arrangement involves the use of a near IR analyzer to detect $^{13}$C abundance in the resolved components. Since the metabolic flux is only determined from the relative abundance of the isotopic ratios of the analytes of interest, any detector able to resolve the relative abundance of labeled and unlabeled analytes can be used with the method, The quantitative sensitivity and accuracy of the detector are relatively unimportant, since each analyte is present simultaneously in both the labeled and unlabeled forms. The detector should only be capable of precisely quantifying the relative abundance of the isotope.

In some instances, a detector can be placed at the end of the final capillary column (and/or between other columns) to monitor flow and/or to determine when fractions should be collected.

F. Exemplary Systems: Metabolite Profiling

The methods of the invention are amenable to a variety of different electrophoretic methods. The controlled elution techniques whereby defined fractions are separated spatially, physically or by time, and the labeling and detection methods can be utilized in a number of different electrophoretic techniques. As noted above, the number of electrophoretic methods linked in series is typically at least two, but can include multiple additional electrophoretic methods as well. In some instances, each electrophoretic method in the series is different; whereas, in other instances certain electrophoretic methods are repeated at different pH or separation matrix conditions.

Despite the general applicability of the methods, as noted above CIEF, CZE and CGE methods are specific examples of the type of electrophoretic methods that can be utilized according to the methods of the invention. In certain methods, only two methods are performed. Examples of such methods include a method in which CIEF is performed first followed by CGE. Labeling, if performed, is typically performed alter CIEF with detection subsequent to elution of components from the CGE capillary. In another system, the first method is CGE and the final method is CZE. Isotope detection generally is not performed until the completion of the final electrophoretic separation. However, as indicated above, UV/VIS or LIF detection may be used during any or all separation dimensions to monitor the progress of the separations, particularly to determine when fractions are to be collected. A third useful approach involves conducting multiple CZE dimensions. These are specific examples of systems that can be utilized; it should be understood that the invention is not limited to these particular systems. Other configurations and systems can be developed using the techniques and approaches described herein.

VII. Mass Spectroscopy Methods for Metabolite Profiling

Charged or ionizable analytes can be detected by a variety of mass spectrometric methods. Certain method include electrospray (ESI) and matrix assisted laser desorption ionization (MALDI) methods coupled with time-of-flight (TOF) ion detection. ESI and MALDI are preferred because they are low energy ionization methods, generally resulting in low fragmentation of most analytes, and are suitable for the ionization of the broadest possible array of target analytes. TOF detection is useful because the accuracy of this technique in determining mass generally allows isotopic resolution to the single atomic mass unit level, even for multiply charged species. However, other mass spectrometric ionization and detection techniques can be usefully employed where the analytes are particularly robust to fragmentation, the isotopic differences between labeled and unlabeled analytes is sufficiently large, and/or the number of charge states sufficiently low, to achieve resolution of the labeled and unlabeled analytes.

## VIII. Microfluidic Systems for Metabolite Profiling

### A. Examples of configurations

In a variation of the electrophoresis systems described *supra*, the capillaries are part of or formed within a substrate to form a part of a microfluidic device that can be used to conduct the analyses of the invention on a very small scale and with the need for only minimal quantities of sample. In these methods, physical fractions of samples typically are not collected. Instead, resolved components are separated spatially or by time. Methods for fabricating and moving samples within microfluidic channels or capillaries and a variety of different designs have been discussed including, for example, U.S. Pat. Nos. 5,858,188; 5,935,401; 6,007,690; 5,876,675; 6,001,231; and 5,976,336, all of which are incorporated by reference in their entirety.

An example of a general system 150 that can be used with the methods of the present invention is depicted in FIG. 3A. The capillaries or channels are typically formed or etched into a planar support or substrate. A separation capillary 152 extends from an anode reservoir 154 containing anolyte to a cathode reservoir 156. The anode reservoir 154 and the cathode reservoir 156 are in electrical contact with an anode and cathode 158, 160, respectively. A sample injection channel 162 runs generally perpendicular to the separation capillary 152 and one end intersects at an injection site 164 slightly downstream of the anode reservoir 154. The other end of the sample injection capillary 162 terminates at a sample reservoir 166, which is in electrical communication with a sample reservoir electrode 168. A detector 170 is positioned to be in fluid communication with electrophoretic medium passing through the separation capillary 152 and is positioned downstream of the sample injection site 164 and typically somewhat upstream of the cathode reservoir 156. In this particular configuration, fractions are withdrawn into the cathode reservoir 156. Movement of electrophoretic medium through the various channels is controlled by selectively applying a field via one or more of the electrodes 158, 160 168. Application of a field to the electrodes controls the magnitude of the EOF within the various capillaries and hence flow through them.

An example of another configuration is illustrated in FIG. 3B. This system 180 includes the elements described in the system shown in FIG. 3A. However, in this arrangement, spacially or temporally resolved fractions can be withdrawn at multiple different locations along the separation capillary 152 via exit capillaries 172a, 172b and 172c. Each of these capillaries includes a buffer reservoir 176a, 176b, 176c, respectively, and is in electrical communication with electrodes 174a, 174b, 174c, respectively. Movement of electrophoretic medium along separation capillary 152 and withdrawal of fractions therefrom into the exit capillaries 172a, 172b and 172c can be controlled by controlling which electrodes along the separation capillary 152 and which of the exit capillary electrodes are activated. Alternatively, or in addition, various microfluidic valves can be positioned at the exit capillaries 172a, 172b and 172c to control flow, Typically, additional detectors are positioned at the various exit capillaries 172a, 172b and 172c to detect components in fractions withdrawn into these capillaries.

The configuration illustrated in FIG. 3B can be used in a number of different applications. One example of an application for which this type of system is appropriate is a situation in which the type of samples being examined has been well characterized. If for example, certain fractions of components of interest have been previously established to fractionate at a particular location in the separation capillary 152, then the exit capillaries 172a, 172b and 172c can be positioned at those locations to allow for selective removal of the component(s) of interest.

In still another configuration, multiple exit capillaries branch from the end of the separation capillary 152 near the cathode reservoir 156, each exit capillary for withdrawing and transporting separate fractions. In this configuration also, withdrawal of fractionated components from the separation capillary can be controlled by regulating EOF within the various capillaries and/or by microfluidic valves.

Other components necessary for conducting an electrophoretic analysis can be etched into the support, including for example the reservoirs, detectors and valves discussed *supra.*

### 2. Substrates

The substrate upon which the capillary or micro-channel network of the analytical devices of the present invention are formed can be fabricated from a wide variety of materials, including silicon, glass, fused silica, crystalline quartz, fused quartz and various plastics, and the like. Other components of the device (*e.g.*, detectors and microfluidic valves) can be fabricated from the same or different materials, depending on the particular use of the device, economic concerns, solvent compatibility, optical clarity, mechanical strength and other structural concerns. Generally, the substrate is manufactured of a non-conductive material to allow relatively high electric fields to be applied to electrokinetically transport the samples through the various channels.

In the case of polymeric substrates such as plastics, the substrate materials can be rigid, semi-rigid, or non-rigid, opaque, semi-opaque or transparent, depending upon the use for which the material is intended. Plastics which have low surface charge when subjected to the electric fields of the present invention and thus which are of particular utility include, for example, polymethylmethacrylate, polycarbonate, polyethylene terepthalate, polystyrene or styrene copolymers, polydimethylsiloxanes, polyurethane, polyvinylchloride, polysulfone, and the like.

Devices which include an optical or visual detector are generally fabricated, at least in part, from transparent materials to facilitate detection of components within the separation channel by the detector.

2. Channel Structure/Formation

The size and shape of the channels or capillaries formed in the substrate of the present devices can have essentially any shape, including, but not limited to, semicircular, cylindrical, rectangular and trapezoidal. The depth of the channels can vary, but tends to be approximately 10 to 100 microns, and most typically is about 50 microns. The channels tend to be 20 to 200 microns wide.

Manufacturing of the channels and other elements formed in the surface of the substrate can be carried out by any number of microfabricating techniques that are known in the art. For example, lithographic techniques may be employed in fabricating glass or quartz substrates, for example, using established methods in the semiconductor manufacturing industries. Photolithographic masking, plasma or wet etching and other semiconductor processing technologies can be utilized to create microscale elements in and on substrate surfaces. Alternatively, micromachining methods, such as laser drilling, micromilling and the like, can be utilized. Manufacturing techniques for preparing channels and other elements in plastic have also been established. These techniques include injection molding techniques, stamp molding methods, using for example, rolling stamps to produce large sheets of microscale substrates, or polymer microcasting techniques, wherein the substrate is polymerized within a micromachined mold.

Further guidance regarding other designs and methods for using such microfluidic devices such as described above can be found, for example, in U.S. Pat. Nos. 5,858,188; 5,935,401; 6,007,690; 5,876,675; 6,001,231; and 5,976,336, all of which are incorporated by reference in their entirety.

IX. Exemplary Utilities

The methods and apparatus of the invention can be used to separate and detect a variety of different types of metabolic compounds, including, but not limited to, proteins, nucleic acids, polysaccharides, lipids, fatty acids, amino acids, nucleotides, nucleosides, monosaccharides and disaccharides. Consequently, the methods and apparatus can be used in a variety of metabolic applications. For example, the methods can be used to determine the flux of various metabolites. This capability can be used in biochemical, and especially metabolic, research in determining how the flux of metabolites varies as a function of different cellular states or in response to various external stimuli. The methods have value in clinical research by determining how the flux rates of various metabolites can vary between healthy and diseased states.

More specifically, the invention can be used to develop metomic databases. Such databases can include, for example, a register of various metabolites detected for a particular state or physiological condition of a subject. The database can be cross-referenced with additional information regarding the subject and/or the metabolite. For example, concerning the subject, the database can include information on the genus, species, age, race, sex, environmental exposure conditions, health status, sample collection methodology and type of sample. Flux values can be included for each of the metabolites stored in the database and can be cross indexed with metabolite concentration values, enzyme or transport protein concentration values responsible for the metabolic flux, or gene expression values corresponding to the proteins responsible for the metabolic flux.

Where the fluxes of a plurality of analytes are determined that represent separable components of overall cellular metabolism, a metabolic fingerprint of the subject can be obtained. Analytes from separable components of the overall metabolism are functionally defined as compounds sufficiently separated by a series of enzymatic conversion steps that the isotopic enrichment introduced by any single substrate can not be detected above the natural abundance of the isotope in that analyte, such that a second substrate must be introduced to measure the flux. In general, this functional criteria is satisfied if the target analyte is more than 5 conversion steps removed from the added substrate. For example, the administration of labeled glucose as a substrate is suitable for the determining the flux of several phosphosugars in the glycolysis pathway. However, such administration is generally not sufficient to raise the relative abundance of $^{13}C$ in amino acids, fatty acids, and proteins because of the large number of conversion steps separating the substrate from these target analytes. In such instances, the administration of an isotopically labeled amino acid can be used to determine the flux of the amino acids and proteins; the administration of an isotopically labeled fatty acid or acetate can be used to determine the metabolic flux of fatty acids.

In certain methods, a plurality of metabolically separable substrates can be administered simultaneously to a subject and a plurality of metabolically separable target analytes detected from a single sample obtained after a predetermined time from the subject. In a variation of such methods, each of the metabolically separable substrates can be labeled with a different stable isotope. For example [$^{18}$O]-glucose, [$^{15}$N]-phenylalanine, and [$^{13}$C]-acetate can be administered simultaneously to a subject to determine target analyte fluxes in the glycolysis, amino acid, and fatty acid metabolic pathways.

The invention can be employed in various screening applications. For example, the apparatus and methods of the invention can be used to identify metabolites that are correlated with certain cellular states (*e.g.*, certain diseases). For example, the methods can be utilized to identify metabolites whose concentration or flux varies between healthy and diseased individuals or cells. Enzymes responsible for controlling the concentration *and flux of such metabolites are thus identified as potential targets for drug therapy, for instance. In like manner, certain methods can be used to undertake toxicology studies to identify which metabolites, and thus the enzyme(s) controlling their formation, are affected by a toxic challenge.

Screening methods to correlate metabolites and certain cellular states are similar to the general analytical methods set forth *supra.* For instance, a substrate labeled with a stable isotope is administered to a test subject having a disease and at least partially metabolized by the test subject. Generally, one then partially or fully separates the target analytes of interest from other components in the sample under evaluation utilizing the various separation techniques described above. The relative abundance of the isotope in the target analytes is determined using a method capable of detecting the different isotopes to determine a flux value for each of the target analytes in the test subject. These determined values are then compared with the corresponding flux values for a control that serves as a reference for flux values in a non-diseased state.

The control can be a value (*e.g.*, an average or mean value) for a control subject(s) (*i.e.*, someone without the disease) determined under similar conditions. Alternatively, the control can be a range of values previously established to be representative of a non-diseased state. A difference (*e.g.*, a statistically significant difference) between flux values for test and control indicates that the particular metabolite is correlated with the disease. Such a metabolite is a "marker" or potential marker for the disease. The flux values for the control subject can be data obtained previously under like conditions to the test, or the flux values can be determined for a control subject undergoing simultaneous treatment with the test subject under identical conditions.

Of course, similar screening methods can be conducted to develop correlations between certain metabolites and cellular states other than disease states. For example, methods can be conducted to identify metabolites that are correlated with particular developmental stages, states resulting from exposure to certain environmental stimuli and states associated with particular therapeutic treatments.

Multiple metabolites found to have a statistically significant difference in flux values between diseased and control subjects (*i.e.*, markers) can be used to develop a "metabolic flux fingerprint or simply a "fingerprint" for the disease. Such a fingerprint can subsequently be used to diagnosis the disease (see *infra*). Typically, such a fingerprint includes at least 2, 3, 4, or 5 metabolites found to be correlated with a disease. In other instances, the fingerprint includes at least 6, 7, 8, 9 or 10 such metabolites, and in still other instances 10, 15, or 20 or more such metabolites.

The results from comparative studies are transferable to a variety of diagnostic applications. For example, the "marker" or "fingerprints" can be used to screen or diagnose subjects to determine if they have, or are susceptible to, a particular disease. The methods track those described *supra,* except that the substrate labeled with the isotope is administered to a subject suspected to have the disease or susceptible to it (or simply an interested individual seeking to determine if they have, or are susceptible to, the disease). Flux values for the test analyte(s) (*i.e.*, a "metabolic profile" for the test subject) are than compared with reference flux values for individual test analytes (markers) or collections of markers (fingerprints).

The reference values to which the determined values are compared can be representative of either a healthy or diseased state. Furthermore, the reference value can be a particular value or a range of values correlated with either a healthy or diseased state. For example, the reference can be a value (*e.g.*, an average or mean value) for a control subject or subjects either having or not having the disease, the reference value determined under conditions similar to those under which the test subject was tested. Alternatively, the reference can be a range of values drawn from a population of control subjects either having or not having the disease.

If the reference is for a normal or healthy state, a difference (*e.g.*, a statistically significant difference) between flux values for test subject and reference indicates that the test subject has, or is at risk of acquiring, the disease. Alternatively, lack of a difference indicates that the test subject does not have the disease and/or is at not at risk for acquiring the disease. If, however, the reference is representative of a diseased state, then a difference (*e.g.*,a statistically significant difference) between test and reference values indicates that the test subject does not have and/or is not at risk of acquiring the disease. Conversely, lack of a indicates that the test subject either has or is susceptible to acquiring the disease.

Diagnostic screens are not limited to simply detecting disease states. The screens can also be used to detect other types of cellular states such as certain developmental states or toxic states, for example.

When conducting such screening tests, typically the analysis can be simplified. For example, once markers for a disease have been identified, one can establish separation conditions such that the fraction(s) containing the markers or interest is (are) known. Thus, during the screening tests, only the components in those particular fractions need to be evaluated. The reproducibility of the separation and detection aspects of the invention facilitate such analyses.

Such screening methods can be conducted for a variety of different diseases. Diseases that can be evaluated with the methods of the invention include, but are not limited to, various types of cancer, autism, microbial and viral infections, and various digestive disorders.

The methods of the invention have further utility in conducting structure activity studies. For example, the methods can be used to determine the effect that certain chemical agents or combination of agents generally have on metabolism and, more specifically, the effect on the flux of certain metabolites of interest. Such tests can identify agents that are disruptive to metabolism and pinpoint the particular metabolites effected. In other applications, once an agent has been tested initially, the agent or combination of agents can be modified and the analysis repeated to determine what effect, if any, the modifications had on metabolism. Such studies can be useful, for example, in making derivatives of a lead compound identified during initial drug screening trials.

Metabolic engineering studies can also be conducted using the methods of the invention. In such studies, a gene involved in metabolism can be genetically engineered to include certain desired mutations, or the promoter of a gene can be genetically engineered to increase or decrease the relative expression level of the gene. Using the methods described herein, one can determine what effect, if any, the genetically engineered changes have on the metabolism of the test subject.

## Bioinformatics Database Comprising Protein Expression Fingerprint and/or Metabolite Profile Fingerprint Data

### Overview

The present invention provides powerful analytic methods that yield information on the molecular species present in a sample, their unique identifier characteristics (e.g., amino acid sequence tag, PST; separation coordinate, etc.) and further can provide a quantitative measurment of multiple of said molecular species. This powerful analytic tool provides for the potential to correlate the relative or absolute abundance of a plurality of molecular species in a sample with additional data, such as medical information of a patient from whom the sample was obtained or biological and biochemical information in the case of the use of the present invention in a research setting to identify novel drugs and toxins.

In an aspect, the invention provides a method of obtaining a protein expression profile from a sample containing a cell population or a protein containing extract thereof, the method comprising: electrophoresing in a first capillary electrophoresis apparatus a solution containing a plurality of protein species obtained from a cell population and thereby resolving said protein species based on at least one first biophysical parameter which discriminates protein species, eluting fractions from said first electrophoresis apparatus and electrophoresing said fractions, separately, in a second capillary electrophoresis apparatus and thereby resolving said protein species based on at least one second biophysical parameter which discriminates protein species, and eluting the protein species and identifying the PSTs of a plurality of protein species from the sample by mass spectroscopy fragmentation. In an embodiment, at least 1,000 resolved proteins from the sample are identified by PST determination; in an embodiment at least 5,000 to 7,500 or more resolved proteins from the sample are identified by PST determination. In a variation, two samples are employed, a first sample from a standard (control or normal) cell population and a second sample from a test cell population; test cell populations can be, for example and not limitation, cells of a different histological type than the standard cell population, pathological cells of the same histological type as the standard cells, treated cells that have been exposed to a toxicological or pharmacological agent but which are of the same histological type as the standard cells, cells of a different passage level or age or replicative potential than the standard cells, or any other variation apparent to those skilled in the art seeking to ascertain protein expression profile differences between a first cell sample and a second cell sample. Alternatively, a plurality of non-protein metabolites can be profiled according to the methods of the invention, either in conjunction with ascertaining a protein expression fingerprint or separately.

In an aspect the invention provides a method of obtaining a metabolite profile from a sample containing a cell population or a metabolite-containing extract thereof, the method comprising: separating in a first apparatus a solution containing a plurality of metabolite species obtained from a cell population or non-cellular sample and thereby resolving said metabolite species based on at least one first biophysical parameter, and identifying a unique mass signature and/or separation coordinate based on the separation in siad first apparatus of a plurality of metabolite species from the sample by mass spectroscopy fragmentation. In an embodiment, at least 5 resolved metabolites from the sample are identified by mass signature and/or separation coordinate determination; in an embodiment at least 7 to 500 or more resolved metabolites from the sample are identified by mass spectrometry determination. In a variation, two samples are employed, a first sample from a standard (control or normal) cell population and a second sample from a test cell population; test cell populations can be, for example and not limitation, cells of a different histological type than the standard cell population, pathological cells of the same histological type as the standard cells, treated cells that have been exposed to a toxicological or pharmacological agent but which are of the same histological type as the standard cells, cells of a different passage level or age or replicative potential than the standard cells, or any other variation apparent to those skilled in the art seeking to ascertain metabolite profile differences between a first cell sample and a second cell sample. A plurality of non-protein metabolites can be profiled according to the methods of the invention, either in conjunction with ascertaining a protein expression fingerprint or separately.

Database Systems and Computerized Data Analysis and Retreival Systems

The present invention relates generally to relational databases for storing and retrieving biological information. More particularly the invention relates to systems and methods for providing unique identifier data (e.g., sequences) of biological molecules (e.g., proteins), quantitative information on the relative or absolute abundance of uniquely identifiable biological macromolecules (including encoding mRNA species), and optionally many other types of data (e.g., medical record information, differential diagnosis and/or prognosis information) in a relational format allowing retrieval in a client-server environment. Informatics is the study and application of computer and statistical techniques to the management of information. In genome projects, bioinformatics includes the development of methods to search databases quickly, to analyze nucleic acid sequence information, and to predict protein sequence, structure and function from DNA sequence data. In proteomic projects, this can involve the correlation of unique identifier information with structural information to provide a protein expression profile, and, when cross-tabulated with other data (e.g., medical, etc.) can provide insight into the function of certain proteins and their encoding genes in a cell or organism.

The present invention provides relational database systems for storing and analyzing biomolecular unique identifier information together with biological annotations detailing the source and interpretation of the sequence data. The present invention provides a powerful database tool for drug development and other research and development purposes, as well as profound and powerful tools for diagnosising and treating human disease.

The invention also provides a computer system including a database including protein expression fingerprint record libraries for one or more types of organisms, cell types, or medical sample, which libraries have records having multiple unique protein identifier fields crosslinked to quantitative information about the uniquely identified species and/or quantitative information (e.g., a change in separation coordinate under a condition). The system also includes a user interface capable of receiving a selection of one or more probe sequences for use in determining homologous matches between one or more protein tags (PST) sequences and the deduced amino acid sequences from encoding polynucleotide sequences in the genomic libraries, and displaying the results of the determination.

Also provided is a computer program product including a computer-usable medium having computer-readable program code embodied thereon relating to a database including protein expression fingerprint libraries for one or more types of organisms, cell, or pathological condition. The libraries have multiple protein expression fingerprint records which serve as navigational fields and are linked to informational fields that can be used for identifying data correlations and other relationships. The computer program product includes computer-readable program code for providing, within a computing system, an interface for receiving a selection of two or more protein expression fingerprint records for comparison, determining post-translational modifications and/or changes in reltive or absolute abundance of protein species uniquely identified in each fingerprint record and common or unique to the set of records being compared, and displaying the results of the determination.

Additionally provided is a computer program product including a computer-usable medium having computer-readable program code embodied thereon relating to a database including protein expression fingerprint record libraries for one or more types of organisms, tissue, cell type, or pathological condition. The computer program product includes computer-readable program code for providing, within a computing system, an interface for inputting a query to the database and obtaining output of the query results.

Database Structure

Although described initially with respect to a protein expression fingerprint obtained by a method of the present invention, the relational database structure can be readily modified to include, or to be separately, a database containing metabolite profile fingerprint records.

A protein expression fingerprint record comprises a navigational field and an information field, preferably comprising a plurality of informational fields and preferably comprising a plurality of navigational fields, each linked to one or more informational fields in the record. For example and not limitation, a navigational field can comprise a unique amino acid sequence tag (PST), a separation coordinate, or a combination of both which can be represented in a variety of manners, one of which is a concatenated binary string appending a unique PST signature sequence to a binary bit-string representation of a separation coordinate, among many other alternatives that can be selected at the discretion of those skilled in the art. For each protein species detected in a sample, at least one unique identifier attribute will comprise a navigational field. For example and not limitation, an informational field can comprise any of a variety of other information that relates to its respective navigational field element (i.e., uniquely identified protein species), such as information about the patient form which the sample was obtained, information about the sample, information about the relative or absolute amount of the uniquely identified protein species present in the sample, including ratios of various isoforms, post-translational modifications, and the like. A typical protein expression fingerprint record represents data from a sample and can contain 25-100 or more uniquely identified navigational fields, each cross tabulated to one or more informational fields, often including the relative abundance of the particular protein species in a sample. In a library of protein expression fingerprint records, the library members will share a plurality of common navigational fields (e.g., unique protein #100 and unique protein #101), so that software for comparing the library members across common fields and/or for identifying and weighting the existence of common navigational fields can be performed and calculations and correlative measures of statistically significant relationships between library members can be performed, either via user-inputted query to the database or automatically via a neural network program trained to identify and weight intrinsic relationships between data fields, or between combinations and subcombinations of data fields, among the library members. It is often preferable to use the unique identifiers of protein species each as a navigational field, so that the combination of all unique identifiers in a protein expression fingerprint record will comprise a set of navigational fields that are compared between records to search for correlations between informational fields linked to each unique protein identifier navigational field and to rank-order records in the set based on a similarity score, which is calculated using any suitable comparison algorithm the user desires to program, or, if the user wishes, to train a neural network using a standard set of data (e.g. by backpropagation or other training strategy using protein expression fingerprint records from predetermined controls and predetermined variants having the same or similar phenotype) to reflect those field comparison weightings which provide the output that provides the predictive accuracy that the user desires.

In an exemplary embodiment, the protein expression fingerprint record database in a computer system comprises a navigational field comprising the unique PSTs represented as a binary string relationally linked to an information field comprising an integer or floating point value representing the absolute or relative abundance, in the sample, represented by the relationally linked PST identifier. Each protein expression fingerprint record can comprise multiple informational fields which relate to abundance or separation coordinate information for each unique PST, and further the protein expression fingerprint record itself can be linked to additional informational fields that relate to data regarding the sample, patient, condition, or treatment.

Other database structures and query methodologies will be apparent to those skilled in the art having read the present specification.

It is to be understood that the above description is intended to be illustrative and not restrictive. Many embodiments will be apparent to those of skill in the art upon reading the above description. The scope of the invention should, therefore, be determined not with reference to the above description, but should instead be determined with reference to the appended claims, along with the full scope of equivalents to which such claims are entitled. The disclosures of all articles and references, including patent applications and publications are incorporated herein by reference.

The following examples are offered to illustrate, but no to limit the claimed invention.

## EXPERIMENTAL EXAMPLES

### EXAMPLE 1

cZE Separation of Unlabeled Proteins

Each of five proteins (see Table 2) were obtained from Sigma-Aldrich and were suspended at 5 mg/ml in an aqueous denaturing sample buffer consisting of 25 mM tris(hydroxymethyl)aminomethane phosphate (pH 4.0), 0.5% by weight IGEPAL CA-630 (obtained from Sigma-Aldrich, Cat # I3021), and 1% by weight tris(2-carboxyethylphosphine) hydrochloride (obtained from Pierce, Cat # 20490ZZ). The protein samples were denatured in this sample buffer by heating at 95 °C for 15 min. Each of the five denatured protein samples were diluted into a cZE sample buffer to create a final solution consisting of 25 mM tris(hydroxymethyl)aminomethane phosphate buffer (pH 4.0), 8 M Urea, and a final concentration of 0.2 mg/ml of each of the five proteins. Control samples were also prepared of each denatured protein separately at 0.5 mg/ml final concentration in the same sample buffer.

Table 2: Protein Standards

| Protein | Cat # | pI | MW (kDa) |
|---|---|---|---|
| Hen egg white conalbumin | C 0755 | 6.0, 6.3, 6.6 | 76.0 |

(continued)

| Protein | Cat # | pI | MW (kDa) |
|---|---|---|---|
| Bovine serum albumin | B 4287 | 5.4, 5.5, 5.6 | 66.2 |
| Carbonic Anhydrase II | T 6522 | 4.5 | 21.5 |
| GAPDH Rabbit muscle | G 2267 | 8.3, 8.5 | 36.0 |
| Bovine ribonuclease A | R 5503 | 9.6 | 13.7 |

The mixed protein sample and each of the control samples were run by cZE in a 60 cm x 75 μm fused silica capillary (Beckman Coulter). An 800μm detection window was located 50 cm from the cathodic end of the capillary. A 160 nl sample volume was pressure injected at the cathodic end and the separations conducted at 500 V/cm in a 25 mM TRIS-phosphate and 8 M urea running buffer at pH 4.0. Protein detection was accomplished by UV adsorption at 214 nm.

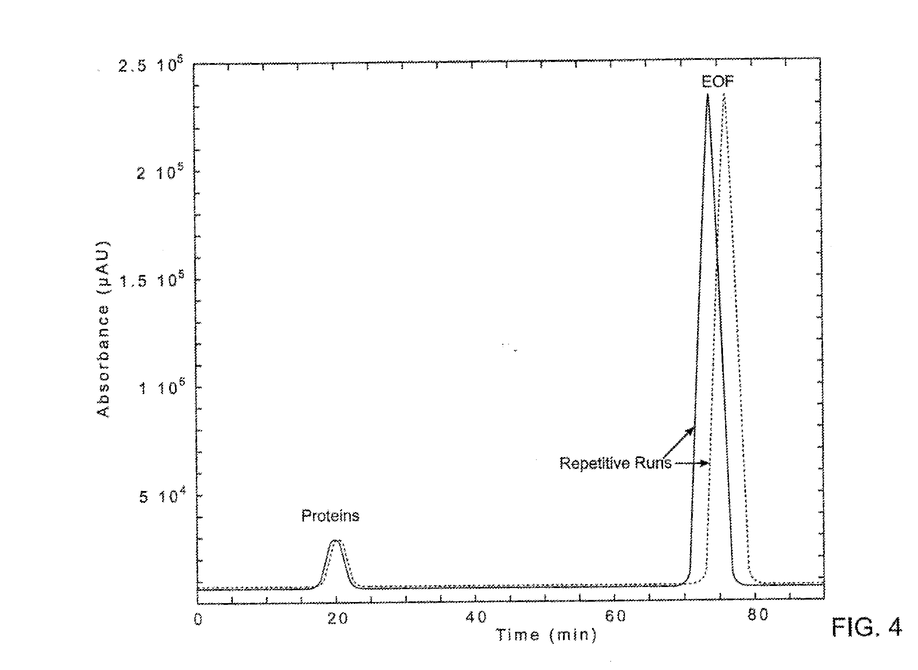

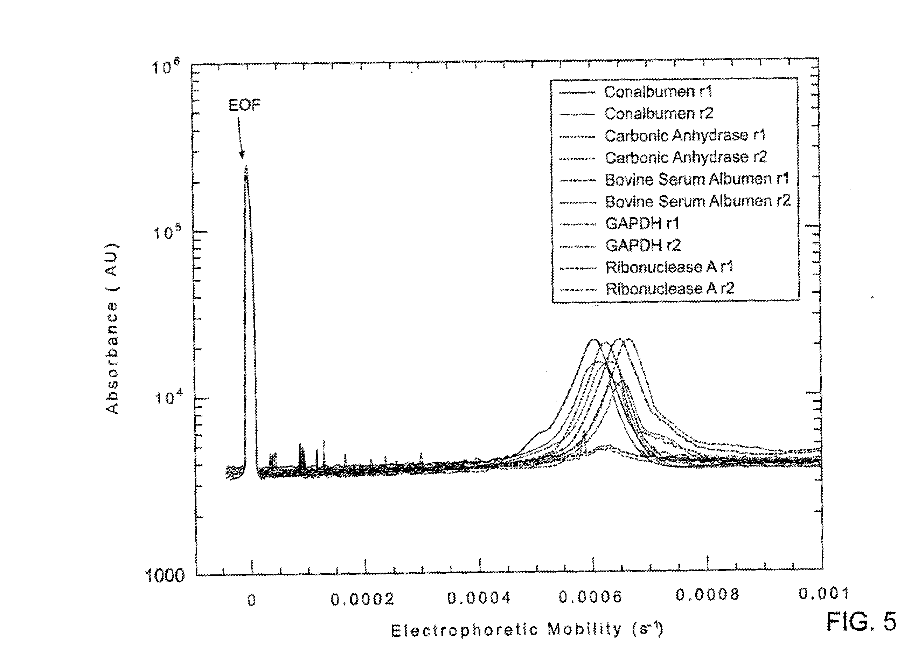

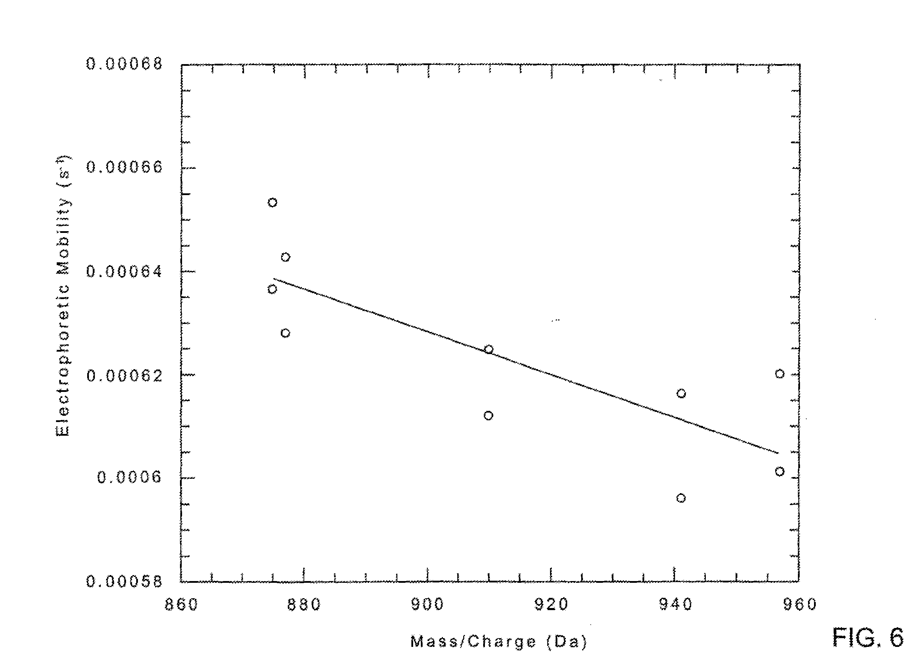

The individual unlabeled proteins were not resolved under these conditions (see FIG. 4). The electrophoretic mobility of each protein was determined from replicate runs of the individual protein controls (FIG. 5) and correlated with the predicted mass to charge ratio of the proteins at pH 4.0 (FIG. 6). The mass to charge ratio for each of the unlabeled proteins was determined from the published protein sequences obtained through Genbank in the manner described by Canter, C.R. and Schimmel, P.R., Biophysical Chemistry, W.H. Freeman and Co., New York, (1980), which is incorporated by reference in its entirety.

EXAMPLE 2

cZE Separation of Labeled Proteins

Each of the five proteins described in Example 1 was suspended at 10 mg/ml in the same denaturing buffer described in Example 1 with the exception that an equal mass of sodium dodecyl sulfate was used in place of IGEPAL CA-630. The denatured protein samples were labeled with 4-sulfophenylisothiocyanate (SPITC) obtained from Sigma-Aldrich (Cat # 85,782-3) and used as supplied. Labeling was accomplished by adding 10 μl of triethylamine, 10 μl of 2 M acetic acid and 20 μl of a 10% by weight solution of SPITC in water to 100 μl of each denatured protein sample. The reaction mixture was heated at 50 °C for 24 h.

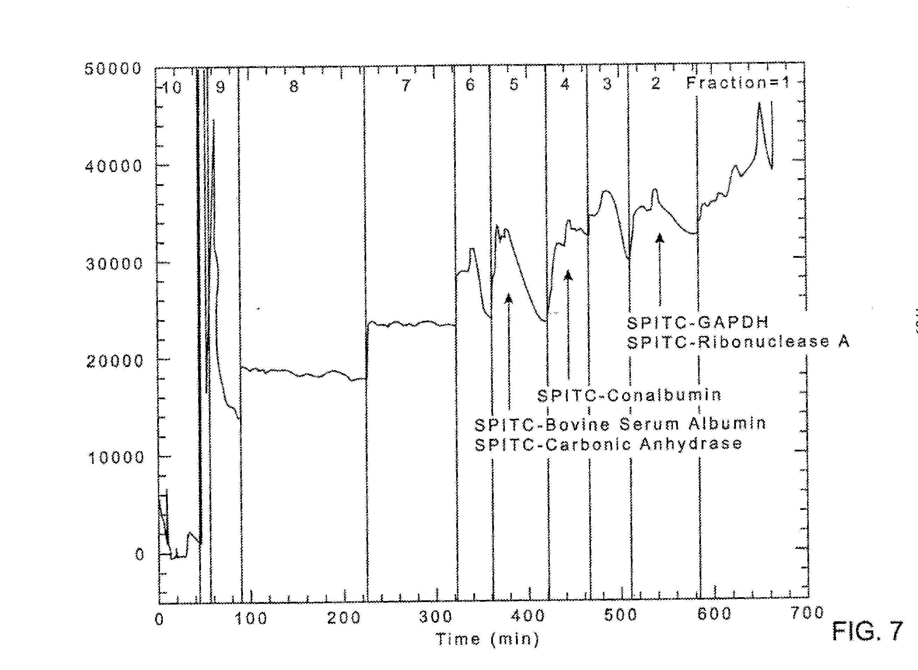

A quantity of 50 μl of each of the SPITC-labeled protein standards was mixed together and separated by cZE as described in Example 1, with the exception that the pH of the separation buffer was adjusted to 3.0. The individual SPITC-labeled proteins were resolved (FIG. 7). Thus, this example taken in view of the results for Example 1 in which unlabeled proteins were poorly resolved demonstrates the positive effect that labeling can have when done prior to a cZE separation. Fractions were collected by electroelution into separate vials containing the separation buffer at the times indicated. The identies of the SPITC-labeled proteins were determined by subsequent cGE analysis of the fractions.

EXAMPLE 3

CIEF First Dimension Separation with Fraction Collection

Bovine Serum Albumin, Carbonic Anhydrase, and Conalbumin were used as supplied from Sigma-Aldrich (Table 2). Each protein was denatured as described in Example 1. A 10 μl aliquot, of each denatured protein sample was added to 200 μl of the cIEF focusing buffer. The cIEF focusing buffer consisted of 0.4% by weight hydroxymethyl cellulose solution (Backman-Coulter eCAP cIEF Gel Buffer, Cat # 477497) containing 1% by volume pH 3-10 Ampholytes (Fluka, Cat # 10043) and 1% by weight 3-[(3-cholamidopropyl)dimethlammonio]-1-propane sulfonate.

A poly(ethylene glycol)-coated 60 cm long 100 μm internal diameter fused silica capillary (Supelcowax 10, Supelco, Cat # 25025-U) was filled with the protein sample in the focusing buffer. The capillary contents were focused between 10 mM phosphoric acid and 20 mM NaOH reservoirs for 7.5 min at 500 V/cm and 25 °C. A 0.5 psi pressure gradient was then applied between the anolyte and catholyte reservoirs to facilitate the elution of the focused proteins in the direction of the electroosmotic flow.

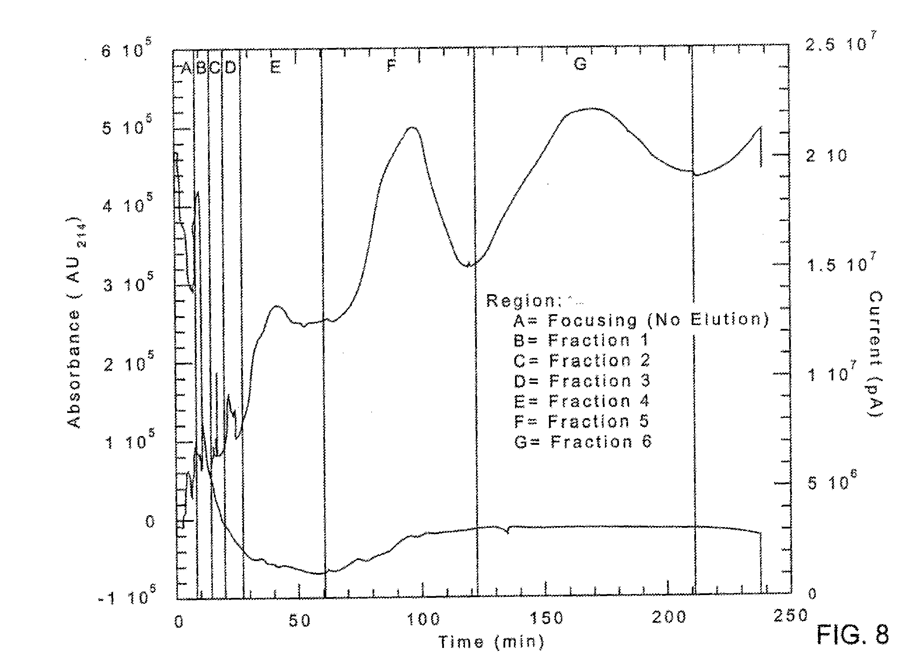

The protein peaks were detected by monitoring the ultraviolet absorption at 214 nm through an optical window in the capillary positioned 50 cm from the low pH end. The current through the capillary was also monitored (FIG. 8). Fractions (B-G) were collected into 50 μl of 20 mM NaOH contained in separate reservoir vials for the times depicted (FIG. 8). Only fractions F and G were found to contain protein (see Example 4). Fraction G was found to contain carbonic anhydrase and no conalbumin or bovine serum albumin. Conalbumin and bovine serum albumin were found to coelute in the peak observed in fraction F. This experiment illustrates the partial separation of a mixture of proteins in a single dimension. Further resolution was achieved in the second dimension (see Example 4).

EXAMPLE 4

CGE Second Dimension Separation of CIEF Fractions

Each of the CIEF fractions (B-G) collected during the CIEF separation described in Example 3 were evaporated in a Savant Model SC210A Spin-Vap to a final volume of 5 μl to concentrate any protein present in the fraction. A quantity of 10 μl of SDS sample buffer was added to each protein concentrate. The SDS sample buffer consisted of 100 μl of eCAP SDS sample buffer (Beckman Coulter, Cat # 241525), 10 μl of eCAP Orange G Reference Marker (Beckman Coulter, Cat # 241524), and 90 μl of anhydrous glycerol.

Each sample was then run in cGE mode using a linear poly(acrylamide)-coated fused silica capillary 60 cm long with a 100 μm internal diameter. The eCAP SDS 14-200 Gel buffer (Beckman-Coulter Cat # 477416) was used for the separation and in both reservoirs. The separation was conducted at 20 °C and 500 V/cm for 50 min. Ultraviolet detection of the proteins was accomplished at 214 nm through an optical window positioned 50 cm from the sample injection end of the capillary. Molecular weight calibration was conducted in a separate run using eCAP MW Standards (Beckman-Coulter Cat # 477418) as described by the manufacturer. A 100 sec sample injection at 0.5 psi was used to load each sample into the capillary.

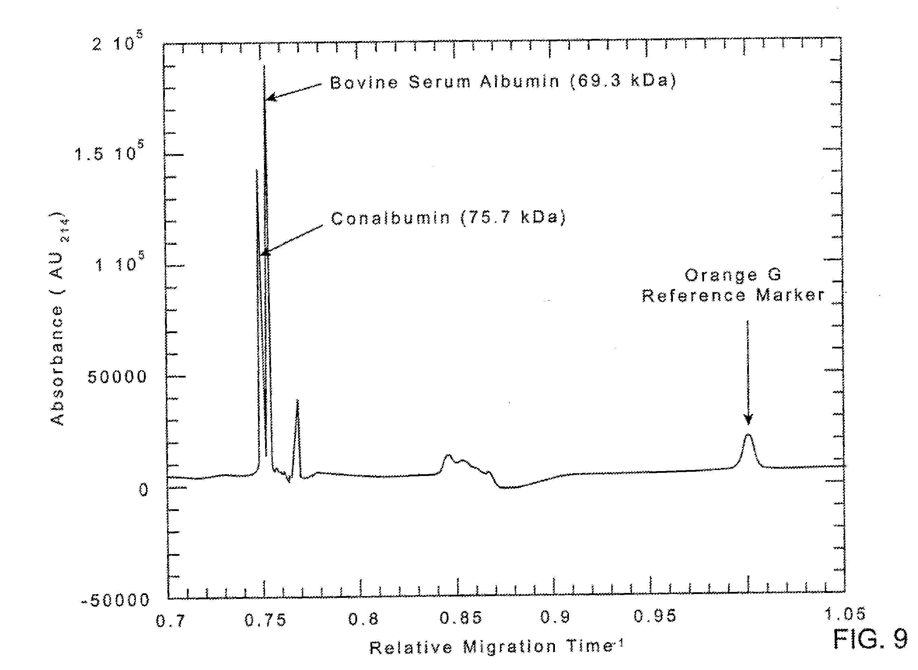

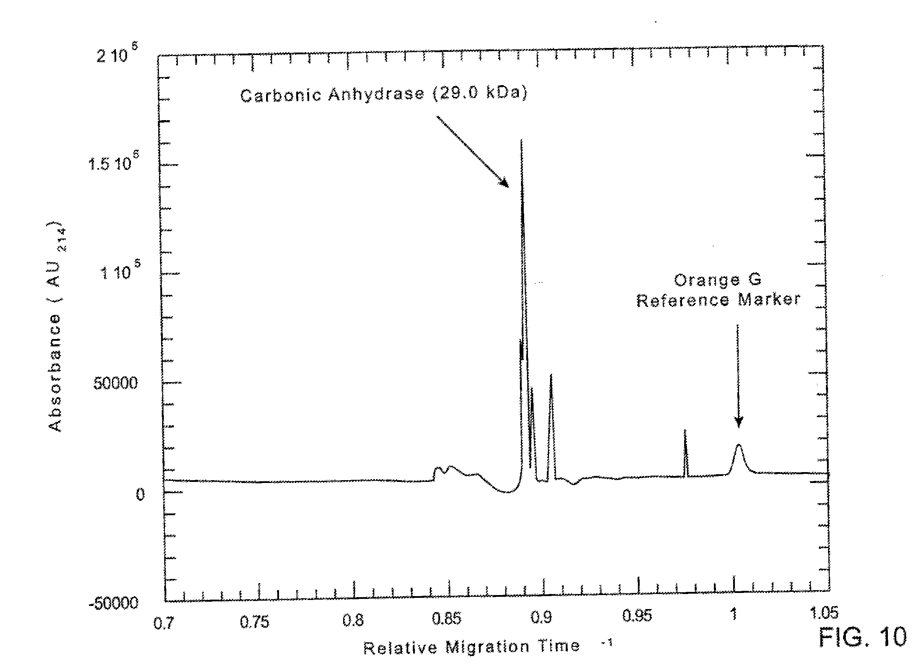

The resulting electropherograms showed no detectable protein in any cIEF fraction except fractions F (FIG. 9) and G (FIG. 10). The molecular weight of the two proteins seen in fraction F (FIG. 9) correspond to that of bovine serum albumin and conalbumin (Table 2). The molecular weight of the protein seen in fraction G (FIG. 10) corresponded to that of carbonic anhydrase (Table 1). It is observed that the second cGE dimension was necessary to fully resolve bovine serum albumin from conalbumin, which were not resolved by a single cIEF mode (Example 3).

EXAMPLE 5

Use of Methods in Proteomics Analysis for Distinguishing Between Healthy and Cancerous Tissue

This example illustrates the use of the present invention for distinguishing between healthy and cancerous tissue. The present invention can be used to directly analyze the protein expression pattern of healthy and cancerous and metastasized tissues to elucidate patterns of gene expression and translate such relations to the various aspects of onset, staging and metastases in cancers, such as prostrate, breast, colon and skin.

The methods of the invention can significantly decrease the time necessary to conduct functional genomics analysis of the mechanism of disease and can lead to the identification of new therapeutic targets, diagnostic markers, and drug products (*i.e.*, where a specific cellular protein may itself act as a therapeutic agent). By using proteomic analysis the number of genes that must be investigated is reduced 10-fold (from the 50,000 to 150,000 human genes to the 2,000-10,000 genes actually being expressed to form proteins in the target tissue). Through quantitative comparison of the protein expression pattern of healthy and diseased tissue, the number of candidate genes that may play roles in the progression of the disease is further reduced about 100-fold. Finally, through the subsequent generation of protein sequence tags (PTSs; *i.e.,* a partial amino acid sequence) each of the proteins that show differential expression can be uniquely identified in a manner that allows them to be tracked back to the genome for complete sequencing (*e.g.*, mutation detection).

Initially, tissue samples are obtained from diseased subjects and control subjects (*e.g.*, individuals not known to have the particular cancer being studied). The tissue samples from each individual are homogenized according to known methods. Depending upon the sample, the resulting homogenate is filtered or centrifuge to remove cellular debris. Samples are taken from the homogenate and the proteins therein denatured by adjusting the samples to contain urea (6-8 M), detergent (*e.g.*, 1% by weight sodium dodecyl sulfate) and 1% by weight dithiothreitol. Samples are heated at 95 °C for 15 minutes to speed denaturation.

Samples (5 μl) are then electrophoresed by CIEF on a column (75 micron insidediameter by 60 cm long). Acolyte is initially 10 mM phosphoric acid and the catholyte is initially 20 mM sodium hydroxide. Separations are conducted at 500 V/cm. Fractions of resolved proteins are eluted by increasing the sodium chloride concentration of the catholyte solution from 10 mM to 100 mM in 96 incremental units. Fractions are collected by sequentially inserting the high pH end of the capillary into 200 μl of each salt concentration in catholyte solution contained in the wells of a 96 well plate. The separation current is allowed to reequilibrate before the capillary end is moved to the next fraction.

Prior to labeling, fractions are concentrated using a rotary evaporator. Protein in the collected fractions is labeled by reacting the proteins with fluoroscein isothiocyanate as described in Example 2 for sulfophenylisothiocyanate.

Fractions containing the labeled proteins are separately electrophoresed by CZE. The labeled proteins are diluted into a CZE sample buffer to form a final solution consisting of 25 mM tris(hydroxymethyl)aminomethane phosphate buffer (pH 4.0), 8 M urea, and a final concentration of about 1 mg/ml of protein. The mixed protein sample and each of the control samples are run in CZE mode in a 60 cm x 75 μm fused silica capillary (Beckman Coulter). An 800 μm window is located 50 cm from the cathodic end of the capillary. A 160 nl sample volume is pressure injected at the cathodic end and the separations conducted at 500 V/cm in a 25 mM TRIS-phosphate and 8 M urea running buffer at pH 4.0. Proteins are eluted by the residual EOF in the capillary. Fractions are again collected on the basis of elution time in the wells of a 96 well microtiter plate as the capillary is progressively advanced from one well to the next. Each well contains 200 μl of the cZE separation buffer. This process is repeated with samples from the other fractions collected during CIEF.

Samples from CZE fractions are further resolved by CGE. Fractions from CZE are separately concentrated by rotary evaporation to a final liquid volume of about 5 μl. The protein sample is isolated from crystalized urea by refrigerated (4 °C) centrifugation. Ten microliters of SDS sample buffer is added to each vial of protein concentrate. The SDS sample buffer consists of 100 μl of eCAP SDS sample buffer (Beckman Coulter, Cat # 241525), 10 μl of eCAP Orange G Reference Marker (Beckman Coulter, Cat # 241524), and 90 μl of anhydrous glycerol.

Each sample is run in cGE mode using a linear poly(acrylamide)-coated fused silica capillary 60 cm long with a 100 μm internal diameter. Commercially available eCAP SDS 14-200 Gel buffer (Beckman-Coulter Cat # 477416) is used for the separation and included in both reservoirs. The separation is conducted at 20 °C and 500 V/cm for 50 min. Molecular weight calibration is conducted in a separate run using eCAP MW Standards (Beckman-Coulter Cat # 477418) as described by the manufactured. A 100 see sample injection at 0.5 psi is used to load each sample into the capillary. Resolved proteins are detected by fluoroscein fluorescence with a 466 nm laser induced fluorescence detector.

EXAMPLE 6

This example illustrates the use of inverted mass ladder sequencing to determine the sequence of glycogen phosphorylase.

Glycogen phosphorylase A (EC 2.4.1.1) is a member of a group of proteins that are acetylated at the amino-terminus (see, Persson, et al., Eur. J. Biochem. 152:523-527 (1985). This acetyl group can be attached to the N-terminus via natural biochemical means, as is the case in glycogen phosphorylase. N-terminal acetylation can also be accomplished through published protocols (see, Lomant, et al., J. Mol. Biol., 104:243-261 (1976)) using N-hydroxysuccimidyl- or sulfo-N-hydroxysuccimidyl-acetate, which are commercially available from Pierce Chemical Co., Rockford, IL. This acetyl group provides a unique mass signature for inverted mass ladder sequence determination.

Acetylated glycogen phosphorylase A was purchased from Sigma-Aldrich Chemical Co. (Catalog # P1261). The protein was dissolved in 4 mM ammonium acetate buffer ((pH=5.0) at 0.72 mg/mL. This sample (500 μL) was purified of residual nonvolatile ions and low molecular weight protein and peptide impurities by dialysis using a Microcon (Millipore Corporation) spin dialysis tube with a 50,000 MW cutoff membrane. The sample was dialyzed 10 times against the 4 mM ammonium acetate buffer following Microcon product instructions. The retentate was recovered in 460 μL of the ammonium acetate buffer, yielding a final protein concentration of about 0.8 mg/mL.

The recovered retentate was subjected to in-source fragmentation in an electrospray-time-of-flight mass spectrometer-a Mariner™ (PE Biosystems, Inc.) equipped with the commercial Microspray ion source. The mass spectrometer settings were optimized and the instrument was calibrated immediately prior to injecting the glycogen phosphorylase sample according to the published instrument protocols. The sample was fed continuously into the microspray source at a rate of 0.4μL/min. The nozzle potential was increased from the minimum setting of 12 V to a maximum of 350 V in 25 V increments with 5 minutes instrument equilibration time alotted before collecting spectra at each nozzle potential. A total of thirty 3-second spectra were accumulated for analysis at each nozzle potential.

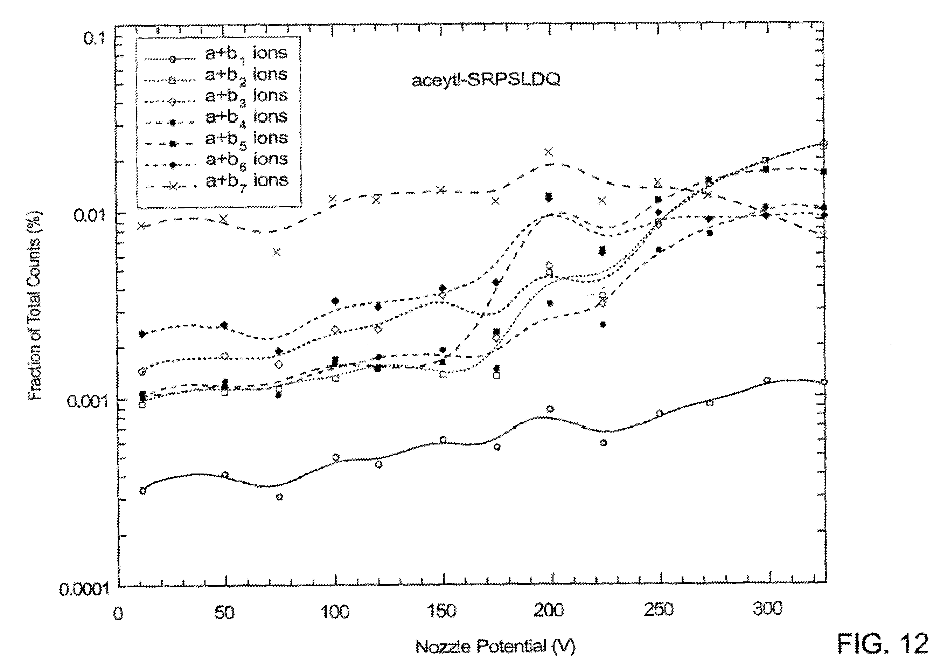

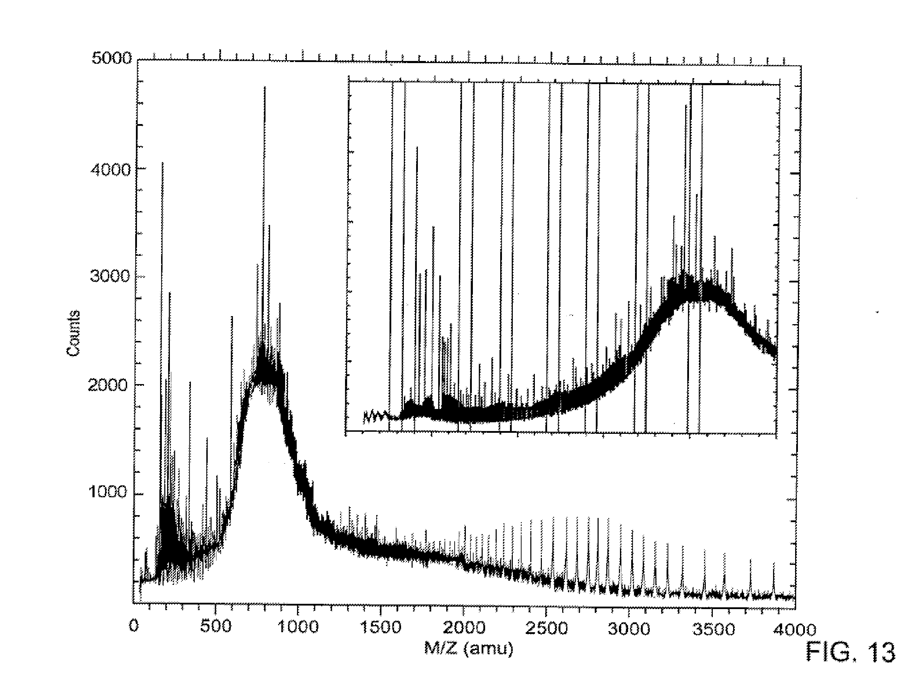

The identity and purity of the parent glycogen phosphorylase A protein was determined at the minimally fragmenting 12 V spectrum (Figure 1) by conducting a zero charge mass deconvolution of the multiply charged mass peaks observed between 700 and 4000 amu using the BioSpec Data Explorer™ software (Version 3.0) supplied by the vendor. The N-terminal sequence of glycogen phosphorylase was determined by inspecting the resulting mass spectra to determine the relative abundance of the possible acetylated peptides at each nozzle potential. Peaks corresponding to the acetylated peptide masses were clearly observed to increase in relative abundance with increasing nozzle potential (Figure 12). Figure 12 shows the cumulative relative abundance of both the a- and b-ions for each peptide mass in the sequence. An example of a substantially-fragmented mass spectra, corresponding to 250V nozzle potential of 250 V is shown in Figure 13. Those mass fragments showing increased abundance at nozzle potentials above 200V correspond to the published amino-terminal sequence for glycogen phosphorylase, acetyl-SRPLSD (see Persson, et al., *ibid.*).

The lack of a ionizable residue on either the amino-terminal serine or the acetate label prevented direct detection of the first amino acid in the sequence. However, the identity of this amino acid is readily deduced from the cumulative mass of the second peptide fragment (corresponding to acetyl-SR), which creates the first detectable positively charged ion from the R-residue. The sequence of the peptide mass ladder became ambiguous beyond the sixth amino acid residue at all nozzle potentials tested.

EXAMPLE 7

This example illustrates the use of inverted mass ladder sequencing to determine the sequence of bradykinin labeled with phenylisothiocyanate.

Bradykinin, a 9 amino acid peptide, was purchased from Sigma-Aldrich (Cat # B3259) and used as supplied. Bradykinin (5 mmoles) was solubilized in 100 μL of coupling buffer consisting of 10 μL of triethylamine (neat), 10 μL of 2 M acetic acid, 5 μL of sequencing grade phenylisothiocyanate (PITC) purchased from Pierce (Cat # 26422), and 2 mL of 50% aqueous methanol. The coupling mixture was incubated for 10 min at 55°C. The reaction mixture was cooled to room temperature and extracted twice with 150 μL of 2:1 (v/v) heptane/ethyl acetate solution. The extracted sample was lyophilized and resuspended to 2 μM PITC-bradykinin in a 50% aqueous acetonitrile solution containing 1% by volume acetic acid.

The PITC-labeled Bradykinin was subjected to in-source fragmentation in an electrospray-time-of-flight mass spectrometer-a Mariner™ (PE Biosystems, Inc.) equipped with the standard commercial pneumatic electrospray ion source. The mass spectrometer settings were optimized and the instrument was calibrated immediately prior to injecting the PITC-.Bradykinin sample according to the published instrument protocols. The sample was fed continuously into the electrospray source at a rate of 5 μL/min. The nozzle potential was increased from the minimum setting of 12 V to a maximum of 350 V in 25 V increments with 1 minute of instrument equilibration time alotted before collecting spectra at each nozzle potential. A total of thirty 3-second spectra were accumulated for analysis at each nozzle potential.

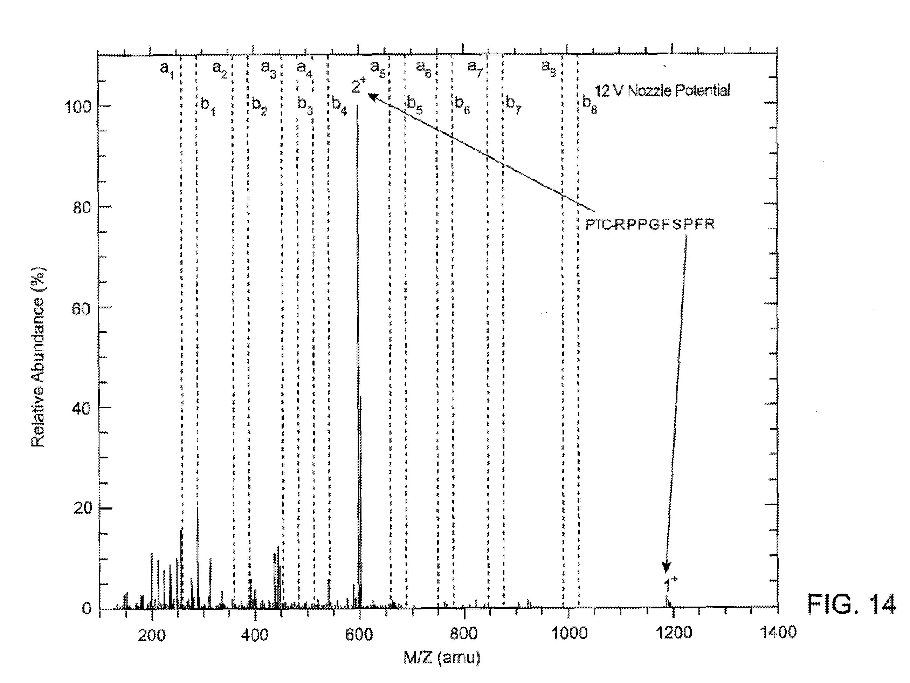

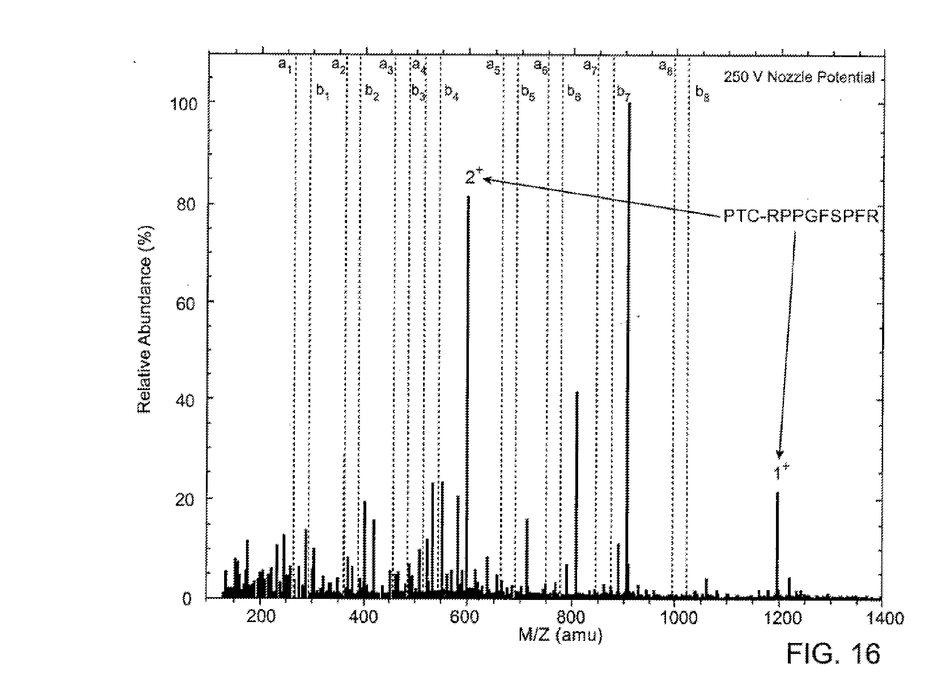

The identity and purity of the parent PITC-Bradykinin peptide was determined at the minimally fragmenting 12 V spectrum (Figure 14) based on the calculated mass for the expected reaction product. The concentration of residual unlabeled Bradykinin was determined by standard addition to be less than 5%. The N-terminal sequence of Bradykinin was determined by inspecting the resulting mass spectra to determine the relative abundance of the possible PITC-labeled peptide fragments at each nozzle potential. Peaks corresponding to the PITC-labeled peptide masses were clearly observed to increase in relative abundance with increasing nozzle potential (Figure 5). Figure 5 shows the cumulative relative abundance of the sum of the a- and b-ions for each peptide mass in the sequence. An example of a substantially-fragmented mass spectra, corresponding to a nozzle potential of 250 V is shown in Figure 16. Those mass fragments showing increased abundance at nozzle potentials above 200V correspond to the published amino-terminal sequence for Bradykinin (see Sigma Product Catalog, Biochemicals and Reagents for Life Science Research, 1999).,

Some of the PITC-Bradykinin fragments are seen to overlap the peaks of other ions produced by the sample matrix. The $b_1$-ion (PITC-R) overlapped the first monoisotopic peak of an ion identified as being produced from the sample matrix (in the absence of labeled Bradykinin). The abundance of this matrix ion was found to remain invariant with nozzle potential. Similarly, the $a_2$-ion peak (PITC-RP) was found to overlap the second isotope peak of another ion produced by the matrix. In this case the matrix ion was found to disappear with increasing nozzle potential. The expected relative abundance of the first through third isotope species and inspection of both the a- and b-ion positions were used to determine and deconvolute these overlap in the mass spectra as previously described (see, Hines, et al., Am. Soc. Mass. Spec. 3:326-336 (1992)).

EXAMPLE 8

This example illustrates the use of inverted mass ladder sequencing to determine the sequence of bradykinin labeled with iminobiotin.

Bradykinin was purchased from Sigma-Aldrich (Cat # B3259) and used as supplied. The N-hydroxysuccimidyl (NHS) ester of iminobiotin was purchased from Pierce (Cat # 21117ZZ) and used as supplied. Bradykinin (5 nmoles) was dissolved in 100 μL of 1 M pyridinium acetate buffer (pH 8.0). The NHS-iminobiotin was dissolved in DMSO to a final concentration of 6.25 mg/mL with 7 μL of this DMSO solution added to the reaction mixture. The reaction mixture was incubated for 2 h at 4°C. The sample was lyophilized and resuspended to final iminobiotin (IMB)-labeled Bradykinin concentration of 2 μM in a 50% aqueous acetonitrile solution containing 1% by volume acetic acid.

The iminobiotin (IMB)-labeled Bradykinin was subjected to in-source fragmentation in an electrospray-time-of-flight mass spectrometer—a Mariner™ (PE Biosystems, Inc.) equipped with the standard commercial pneumatic electrospray ion source. The mass spectrometer settings were optimized and the instrument was calibrated immediately prior to injecting the PITC-Bradykinin sample according to the published instrument protocols. The sample was fed continuously into the electrospray source at a rate of 5 μl/min. The nozzle potential was increased from a minimum setting of 75 V to a maximum of 400 V in 25 V increments with 1 minute of instrument equilibration time alotted before collecting spectra at each nozzle potential. A total of thirty 3-second spectra were accumulated for analysis at each nozzle potential.

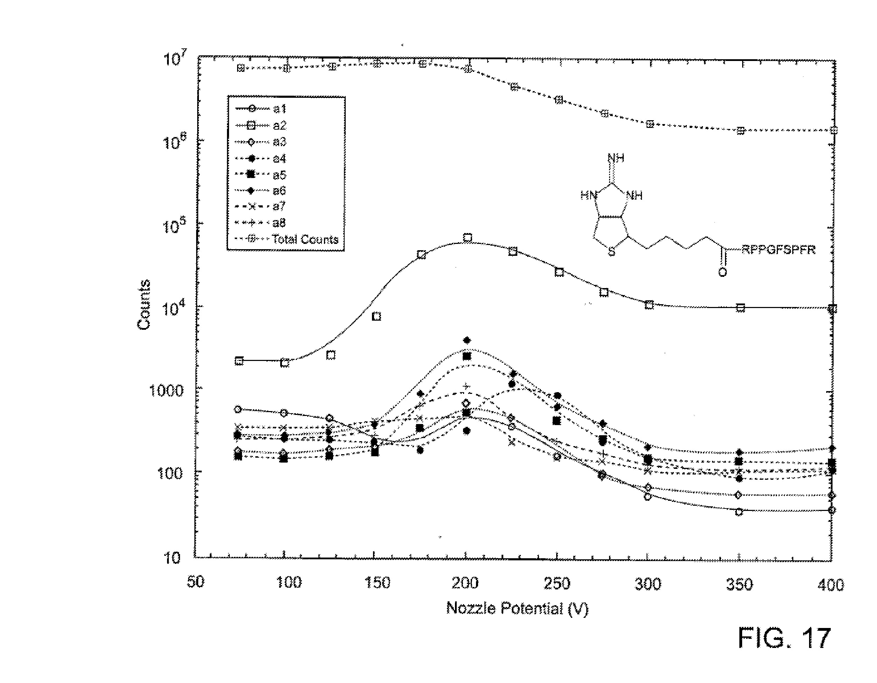

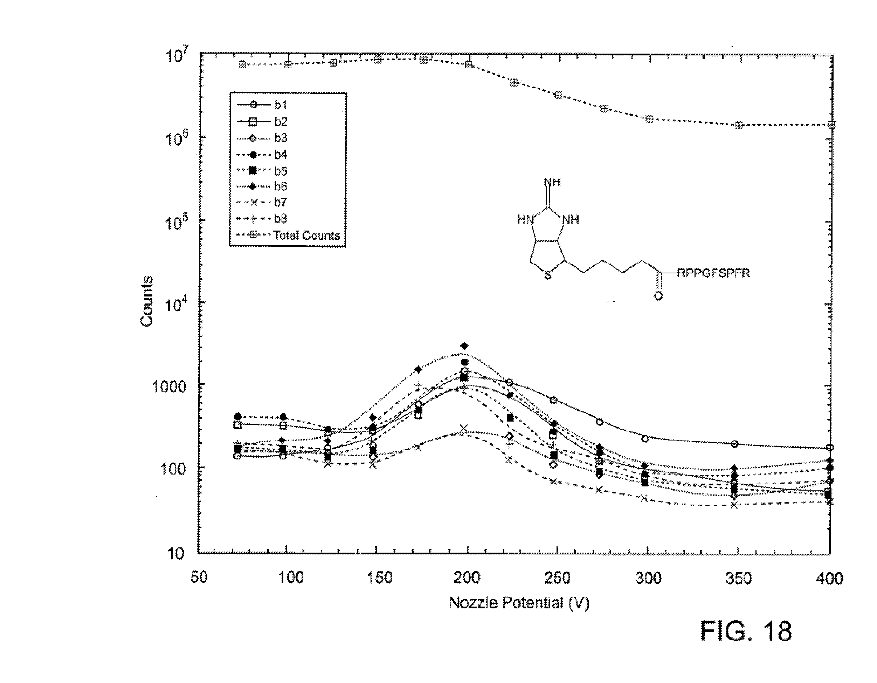

The identity and purity of the parent IMB-Bradykinin peptide was determined at the minimally fragmenting 75 V spectrum based on the calculated mass for the expected reaction product. The concentration of residual unlabeled Bradykinin was determined by standard addition to be less than 5%. The N-terminal sequence of Bradykinin was determined by inspecting the resulting mass spectra to determine the relative abundance of the possible IMB-labeled peptide fragments at each nozzle potential. Peak counts corresponding to the a-ions (Figure 17) and b-ions (Figure 18) generated from the IMB-labeled peptide fragment masses were clearly observed to increase in relative abundance with increasing nozzle potential with a maximum fragmentation abundance noted at about 200V. The decrease in fragment ion abundance above 200V is attributed to an overall decline in detection or ionization efficiency of all iminobiotin species and parallels the observed decline in total counts (Figures 17 and 18). Those mass fragments showing an increased abundance at the 200V nozzle potential correspond to the published amino-terminal sequence for Bradykinin.

EXAMPLE 9

This example illustrates the application of inverted mass ladder sequencing using a 4-sulfophenylisothiocyanate-labeled apomyoglobin.

Sequencing grade apomyoglobin was purchased from Sigma-Aldrich (Cat #A8673) and used as supplied. Apomyoglobin (10 nmoles) was dissolved in 100 μL of reaction buffer consisting of: 10 μL of triethylamine, 10 μL of 2 M acetic acid, 2 mL of 8 M urea, and 10 μL of a 10 mg/mL aqueous 4-sulfophenylisothiocyanate (SPITC) solution. SPITC was purchased from Fluka (Cat # 86180) and used as supplied. The reaction mixture was incubated for 1 h at 55°C. Urea and excess reagents were removed from the reaction mixture by spin dialysis against 6 washes with deionized water. Spin dialysis was conducted in a model YM10 Microcon (Millipore Cat# 42407) tube following package directions. The dialyzed sample was lyophilized and resuspended in 500 μL of 50% aqueous acetonitrile containing 0.1% by volume triethylamine.

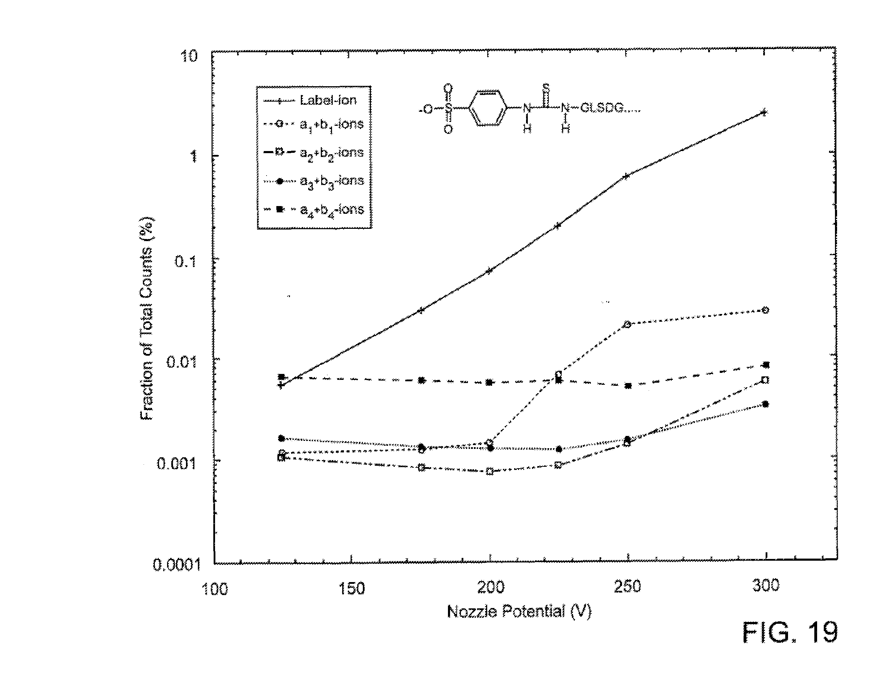

The SPITC-labeled apomyoglobin sample was subjected to in-source fragmentation in an electrospray-time-of-flight mass spectrometer—a Mariner™ (PE Biosystems, Inc.) equipped with the standard commercial pneumatic electrospray ion source. The mass spectrometer was operated in negative ion mode. The mass spectrometer settings were optimized and the instrument was calibrated immediately prior to injecting the sample according to the published instrument protocols. The sample was fed continuously into the electrospray source at a rate of 3 μl/min. The nozzle potential was increased from a minimum setting of 125 V to a maximum of 300V in 25-50 V increments (as shown in Figure 19) with 1 minute of instrument equilibration time alotted before collecting spectra at each nozzle potential. A total of thirty 3-second spectra were accumulated for analysis at each nozzle potential.

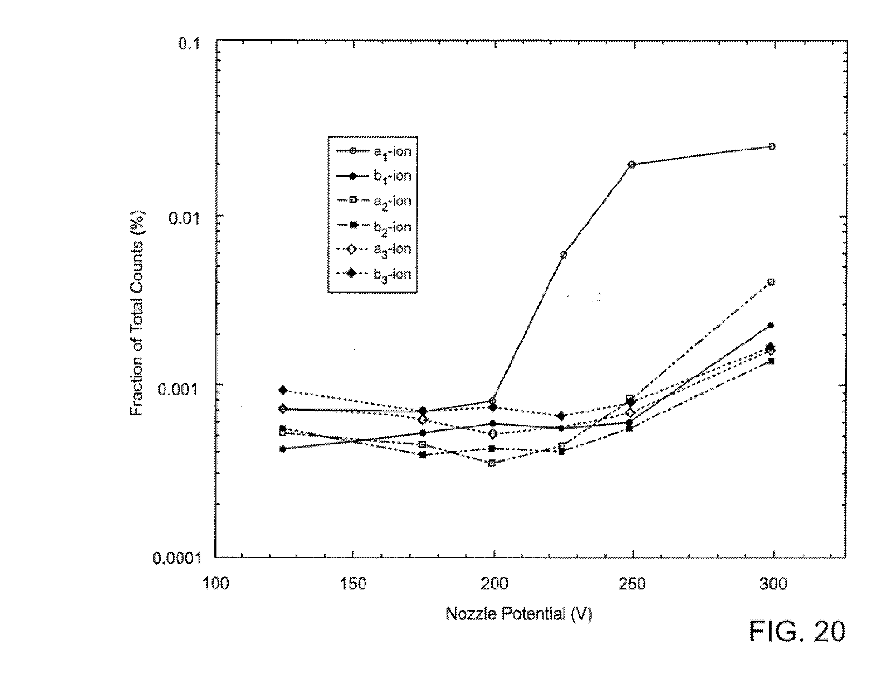

Significant amounts of the SPITC label were found to detach from the protein and fragment ions at higher nozzle potentials (Figure 19), inhibiting the sensitivity of this label for sequence determination. However, peaks corresponding to the fragment masses of the first 3 amino acid residues of the apomyoglobin protein (sequence from Genbank) were found to increase in abundance at higher nozzle potentials. The labeled $a_1$-ion fragment appears at nozzle potentials above 200V. The $b_1$, $a_2$, $b_2$, $a_3$, and $b_3$ ions all appear to increase in relative abundance only above nozzle potentials of 250V (Figure 20).

EXAMPLE 10

This example illustrates the use of inverted mass ladder sequencing to determine the sequence of bradykinin labeled at the carboxy-terminus (C-terminus) with (2-aminoethyl)trimethylammonium chloride hydrochloride (2-AETA) via 1-ethyl-3-(3-dimethylaminopropyl)-carbodiimide hydrochloride (EDC).

Bradykinin (Cat # B3259), 2-AETA (Cat # 284556), and 2-[N-Morpholino]ethanesulfonic acid (MES) (Cat # M5287) were purchased from Sigma-Aldrich and used as supplied. EDC was purchased from Pierce (Cat # 22980) and used as supplied. Bradykinin (0.67 μmol) was dissolved in 0.25 mL 0.1 M MES buffer (pH 5.0). This solution was added to 8.0 μmol 2-AETA, and the solution was mixed until the solid was dissolved. This solution was then added to 37.5 μmol EDC and thoroughly mixed until the EDC was dissolved. The sample was incubated at ambient temperature overnight.

A sample was prepared for mass spectrometry by diluting the reaction mixture in a 50% aqueous acetonitrile solution containing 1% by volume acetic acid such that the final concentration of 2-AETA-labeled bradykinin was 10 μM. The 2-AETA-labeled bradykinin was subjected to in-source fragmentation in an electrospray-time-of-flight mass spectrometer- a Marine™ (PE Biosystems, Inc.) equipped with the standard commercial pneumatic electrospray ion sources. The mass spectrometer settings were optimized and the instrument was calibrated immediately prior to injecting the 2-AETA-labeled bradykinin sample according to the manufacturer's instrument protocols. The sample was infused continuously into the electrospray source at a rate of 5 μL/min. The nozzle potential was increased from a minimum setting of 50 V to a maximum of 300 V in 50 V increments with 1 minute of instrument equilibration time alotted before collecting spectra at each nozzle potential. Data in the range of 50-2000 mass-to-charge units were captured in each spectrum, and a total of sixty 3-second spectra were accumulated for analysis at each nozzle potential.

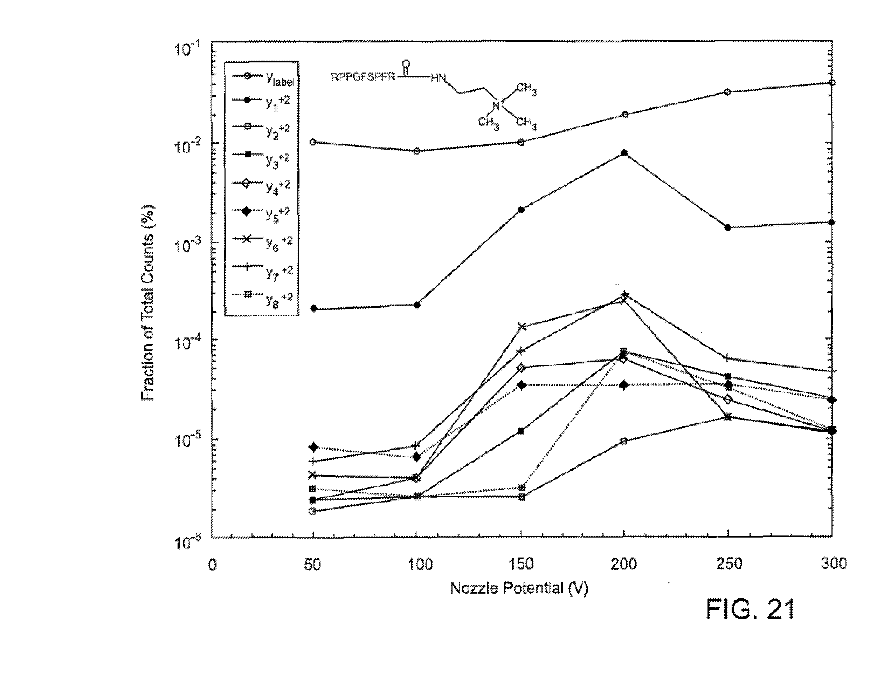

The identity of the parent 2-AETA-labeled bradykinin was determined at the minimally fragmenting 50-V spectrum based on the calculated mass for the expected reaction product. The C-terminal sequence of bradykinin was determined by inspecting the resulting mass spectra to determine the relative abundance of the possible 2-AETA-labeled peptide fragments at each nozzle potential. Peak counts corresponding to the entire possible series of $y^{+2}$ ions (Figure 21) generated from the 2-AETA-labeled peptide fragment masses were clearly observed to increase in relative abundance with increasing nozzle potential with a maximum fragmentation abundance in the region of 150 - 200 V. With this label, which carries a fixed positive charge, no singly-charged y ions were observed since the C-terminal residue of bradykinin is arginine. Those mass fragments showing an increased abundance in the range of 150 - 200 V nozzle potential correspond to the published C-terminal sequence for bradykinin.

EXAMPLE 11

This example illustrates the utility of inverted mass ladder sequencing for the identification of the protein glycogen phosphorylase a by searching a genomics database for matching protein sequence tags (PST) and limiting that search based on the position of the PST in the protein sequence.

The deduced N-terminal amino acid sequence of glycogen phosphorylase A from Example 1 (*i.e.,* SRPLSD) was used to search the SWIS-PROT and TrEMBEL protein sequence databases using the published ExPASy TagIdent tool (*see,* http://www.expasy.ch/tools/tagident.html). This tool enables searching known protein sequences contained within the database for any that contain matching sequences to a 1-6 contingUous amino acid PST. The search can be limited by the position of the PST in the protein (*i.e.*, N-terminal or C-terminal) and the use of the electrophoretic coordinates isoelectric point and/or apparent molecular weight.

The search was limited to the 490 rabbit protein sequences contained within the database at the time. The number of matching proteins ("hits") were found to decrease with increasing PST length (Table 2). The number of hits at any given PST length could be further reduced by limiting the search further to N-terminal matches (Table 2). The number of hits at any given PST length are also reduced (Table 2) by including the apparent MW of the protein (100+/-20 kDa), determined from a capillary gel Electrophoretic separation.

Table 2 Glycogen Phosphorylase Identification from a Genomic Database using an N-terminal IMLS PST

| PST | Number of hits based on PST | Number of N-terminal hits | Number of N-terminal hits limited by MW |
|---|---|---|---|
| S | 478 | 22 | 1 |
| SR | 299 | 1 | 1 |
| SRP | 1 | 1 | 1 |

EXAMPLE 12

This example illustrates the utility of inverted mass ladder sequencing for the identification of the human peptide Bradykinin by searching a genomics database for matching protein sequence tags (PST) and limiting that search based on the position of the PST in the protein sequence and separation coordinates.

The deduced N-terminal amino acid sequence of bradykinin determined from Examples 2 and 3 (*i.e.*, RPPGFS) was used to search the SWIS-PROT and TrEMBEL protein sequence databases as described in Example 6.

The search was limited to the 7171 human protein sequences contained within the database at the time. The number of matching proteins ("hits") were found to decrease with increasing PST length (Table 3). The number of hits at any given PST length could be further reduced by limiting the search further to N-terminal matches (Table 3). The number of hits at any given PST length were also reduced (Table 3) by including the apparent MW of the peptide (1000+/-200 Da), determined from the zero charge mass of the parent peptide in an ESI-TOF MS.

Table 3 Human Bradykinin Identification from a Genomic Database using an N-terminal IMLS PST

| PST | Number of hits based on PST | Number of N-terminal hits | Number of N-terminal hits limited by MW |
|---|---|---|---|
| RP | 4114 | 13 | 1 |
| RPP | 638 | 4 | 1 |
| RPPG | 66 | 1 | 1 |
| RPPGF | 5 | 1 | 1 |

(continued)

| PST | Number of hits based on PST | Number of N-terminal hits | Number of N-terminal hits limited by MW |
|---|---|---|---|
| RPPGFS | 3 | 1 | 1 |

EXAMPLE 13

This example illustrates the utility of inverted mass ladder sequencing for the identification of the horse apomyoglobin protein by searching a genomics database for matching protein sequence tags (PST) and limiting that search based on the position of the PST in the protein sequence and separation coordinates of the protein.

The deduced N-terminal amino acid sequence of apomyoglobin was determined from Example 4 (*i.e.,* GLS) was used to search the SWIS-PROT and TrEMBEL protein sequence databases as described in Example 6.

The search was limited to the 241 horse protein sequences contained within the database at the time. The number of matching proteins ("hits") were found to decrease with increasing PST length (Table 4). The number of hits at any given PST length could be further reduced by limiting the search further to N-terminal matches (Table 4). The number of hits at any given PST length were also reduced (Table 4) by including the apparent MW of the protein (17 +/- 3.4 kDa), determined from the zero charge mass of the parent protein in an ESI-TOF MS, and the isoelectric point of the protein (pI = 7 +/- 0.5) determined by capillary isoelectric focusing.

Table 4 Horse Apomyoglobin Identification from a Genomic Database using an N-terminal IMLS PST

| PST | Number of hits based on PST | Number of N-terminal hits | Number of N-terminal hits limited by MW | Number of N-terminal hits Limited by pI and MW |
|---|---|---|---|---|
| G | 235 | 10 | 5 | 1 |
| GL | 148 | 2 | 1 | 1 |
| GLS | 29 | 1 | 1 | 1 |

EXAMPLE 14

Appropriate label chemistries for both maximum fluorescent detectability and suitability for mass spectrometer sequence analysis are determined by labeling purified proteins of known concentrations and sequences with alternative label chemistries and testing them in both CE and mass spectrometer analyses. At least three different proteins, or synthetically prepared peptides, are selected for evaluation in this task. Two proteins exhibit cross reactivity to the N- or C-terminal label chemistries used and one does not. Up to 30 alternative labels that have theoretically suitable constituents are commercially available. Samples of each protein are prepared and analyzed for their CE detectability and electrospray mass spectrometer signatures. A second round of up to five optimized alternative label chemistries are synthesized and analyzed based on the results obtained from the commercially-available labels. The best label is then selected for use in the remaining tasks.

EXAMPLE 15

2-D CE Method

While CIEF, CZE, and CPAGE techniques have been developed and described previously, no previous reference has been made to the coupling these techniques to create a two dimensional CE method. This example shows that CIEF can be combined with CPAGE or CZE to reproducibly and quantitatively resolve complex mixtures of individual proteins. Mixtures of purified proteins of known concentrations and sequences are prepared as in Example 12. These protein mixtures are labeled with the label from Example 12 and run first through the CIEF method with fraction collection. The collected CIEF fractions are subsequently run through CPAGE or CZE methods to further resolve the proteins. At least 25 replicate experiments in which the concentrations of the individual proteins in the mixture are varied up to 1000 fold relative to one another are run to establish the coefficient of variation of the 2-D CE method. Finally, at least five replicate experiments are conducted with different protein loadings to determine the effect of column loading on the elution times and apparent isoelectric points.

EXAMPLE 16

The mass spectrometer fragmentation pattern and efficiency of proteins can be significantly effected by the buffer solution in which the protein resides during the ionization step. Typical CE buffers are not the same buffers normally used for protein sequencing by MS. Fractions of the individual proteins eluted from the second dimension of the CE are collected and used to optimize the mass spectrometer method for the elution buffers used. Both electrospray and MALDI MS techniques are compared. The samples are evaluated for detection sensitivity, fragmentation efficiency, and maximum length of the protein sequence that can be discerned. The best MS method and conditions are selected and used for all further work.

EXAMPLE 17

Use of the CE Plus MS Methods

In this example, the CE and MS methods from Examples 13 and 14 above are combined into a representative proteomics system. This system can be constructed of commercially available components. The suitability of the system for e.g., stress gene analysis (see below) is demonstrated by performing a proteomics analysis of a known stress gene response, such as the pho response of E. coli. To verify the performance of the system E. coli cultures are prepared from appropriate American Type Culture Collection stocks and subjected to phosphate starvation. The cellular proteins are extracted from exponentially growing culture samples and compared to phosphate starved samples using the system. The results obtained from the system are be compared to those described in the literature. See, e.g., L.V. Schneider, "Metabolic uncoupling in Escherichia coli during phosphate-limited growth," Ph.D. Thesis, Princeton University (1997). The protein expression pattern resulting from the 2-D CE method is converted into an image that is directly compared to classic 2-D gel electrophoresis results. Since the gene sequences on all the pho genes are published, it is possible to compare the accuracy of the protein sequence tags determined from this analysis to the known sequences.

EXAMPLE 18

Use of Proteomics for Distinguishing Between Healthy and Cancerous Tissue

This example illustrates the use of the present invention for distinguishing between healthy and cancerous tissue. In particular, the present invention may be used to directly analyze the protein expression pattern of healthy and cancerous prostate and metastasized tissues to elucidate patterns of gene expression and translation that relate to the various aspects of onset, staging and metastases in prostate cancer. Such a proteomics investigation greatly speeds the genomic and functional genomics analyses of the mechanism of disease and quickly leads to the identification of new therapeutic targets, diagnostic markers, and drug products (i.e., where a specific cellular protein may itself act as a therapeutic agent). By using proteomic analysis the number of genes that must be investigated is reduced 10-fold (from the 50,000 to 35 100,000human genes to the 2,000-5,000 actually beingexpressed in the target tissue). Through quantitative comparison of the protein expression pattern of healthy and diseased tissue, the number of candidate genes that may play roles in the progression of the disease is further reduced about 100-fold. Finally, through the generation of protein sequence tags (PSTs) these proteins can be uniquely identified in a manner that allows them to be tracked back to the genome for complete sequencing (e.g., mutation detection).

The method also allows for cDNA capture for more exhaustive molecular biological investigation of the mechanistic role in the disease (e.g., knock-in and knock-out studies in model organism). It is at the protein level that virtually all cellular metabolism and cell signaling occurs, Proteomics is used to detect genetic mutations that result either in premature termination of the gene transcript or in amino acid substitutions in the resulting gene product. These appear as molecular weight changes or isoelectric point changes in the resulting protein. Because direct changes in the gene product are observed, inconsequential genetic variations (e.g., polymorphisms) are ignored. In this way, proteomic analysis can quickly identify genetic mutations that give rise to cancerous cells. Changes in the expression level of individual proteins can be caused by changes in gene expression. These changes can be tracked by functional genomics methods, but can also be caused by changes in translational efficiency and degradation, which can only be identified using proteomic analysis. The levels of certain proteins may also be a cause of cancer. For example, variations in G-proteins (membrane receptors), which are responsible for translating extracellular signals (such as hormone levels) into cellular responses might lead to cancer, if the cell interprets the corresponding signal change as a call to proliferate. Changes in metabolic proteins may cause an increase in cellular metabolism that leads to growth and cancer. Through proteomics, protein levels between healthy and tumor cells are directly compared, irrespective of the reason for the level changes. Another possible cause of cancer is the failure of post-translational protein modification, which could cause loss of a key signal transduction system leading to uncontrolled cell proliferation. Again, this only occurs after translation and can not be detected by genomic or functional genomics analyses. Natural defensin proteins, which fight tumor growth, may also be rendered nonfunctional with a failure in post-translational modification or increases in degradation rates. Proteomic analysis helps reveal the absence of natural defensins that may be used as drugs or gene therapy agents against disease. In these ways, proteomic analysis is an adjunct to genomic and functional genomic analyses of disease and speeds the identification of both the route causes of the disease and targets for drug discovery.

Proteomic analysis also allows for the identification of diagnostic markers (e.g., cell surface antigens or serum proteins) for immunodiagnostic assays. Purified samples of putative diagnostic proteins are recovered during proteomic analysis, allowing antibodies to be raised. These antibodies are used to further research the link between the diagnostic protein and the disease through immunohistological staining to localize the protein in the cell or to rapidly screen patient populations for the presence of the protein, showing its statistical link to the disease. It also provides an improved screening test.

In the United States, the incidence rate of prostate cancer is 23 per 100,000 among all males younger than 65 and 884 per 100,000 among men older than 65. In addition to older age, other risk factors for this cancer include a suspected familial association, high saturated fat intake (Omega-3 fatty acids are thought to reduce risk), history of venereal disease, multiple sex partners, vasectomy, and exposure to nitrate fertilizers (farmers/farm workers) and ferrochromium. Like all cancers, these risk factors suggest multiple causative factors may be involved. For example, the familial association suggests genetic predilection, probably related to genetic mutations. The relationship to dietary factors suggests metabolic roots or chemically induced genetic damage. The relationship to venereal disease and multiple sex partners suggests infectious agents (e.g., viral causes) or infection-compromised loss of natural tumor defenses. All of these putative disease mechanisms can be addressed through proteomic analysis. Early prostate cancer is usually asymptomatic and can only be detected by routine screening. The screening modality most often used is digital rectal examination. However, results of recent prostate screening studies have indicated that digital rectal examinations lack adequate sensitivity with approximately two-thirds of patients with malignant tumors having palpable indurations. Prostate-specific antigen (PSA) level screening is a sensitive measure of early-stage prostate cancer, but is considered to have poor specificity because elevated levels exist in patients with benign prostatic hyperplasia (BHP), prostatitis, or physical injury to the prostate. The recently developed tumor-associated antigen (TAA) marker assay appears to be a promising adjunct to PSA screening. Nevertheless, the widespread use of PSA as a screening tool is still controversial, in part because early detection of prostate cancer has not been proven in prospective, well-controlled studies to lead to improved patient outcomes in terms of mortality and morbidity, A more definitive screening test is sorely needed.

Prostate cancer is generally characterized by 4 clinical stages with few clinical management options. Early stage (Stage A) can be monitored for growth with no treatment; treated with radiation therapy, or removed (radical prostatectomy). Stage B prostate cancer (definitive but confined cancer) is automatically treated by radiation therapy or removal within the first 8 months of diagnosis. Stage C prostate cancer (spreading but still confined to the organ) is instantly treated with combined removal and radiation therapy or with palliative radiation therapy combined with hormonal therapy. Stage D prostate cancer (metastasized) requires the most radical therapy including transurethral resection of the prostate, combined radiation therapy, palliative radiation therapy and hormonal therapy. Bone scans are also conducted at Stage D. Successful clinical outcomes diminish substantially with each stage of the disease. A side from improvements in early detection, diagnostics that allow improved clinical management by guiding the choice of therapy or improving the oncologist's ability to properly stage the progress of the cancer could also improve clinical outcomes. While a number of anticancer drugs are in clinical trials for prostate cancer, the only recourse today is to remove or kill the tumor tissue. Identifying the mechanism(s) of prostate cancer and its metastasis should speed the development of better drug therapies.

Thus, proteomic analysis of healthy and cancerous prostate tissues and prostate cancer metastases are conducted. Proteomic database are built based on this analysis and the expression levels of all prostrate proteins are quantified by their isoelectric point, molecular weight. Relative expression levels are determined directly from the native tissue samples. Isotopically labeled samples for quantitative analysis of protein expression levels are not required. Therefore, normal biopsy or autopsy samples can be used for all analyses performed. Protein sequence tags are determined for all proteins that show altered expression patterns between healthy and cancerous prostate tissue. Model proteins are used to evaluate the relative efficiency of alternative protein sequencing technologies. 2-D capillary electrophoresis (CE) of healthy tissue samples is used to develop conditions for the 2-D CE of prostate tissue samples. By using healthy tissue samples in this development, we simultaneously generate a baseline proteomics database. Replicate experiments are performed with prostate tissue samples taken from up indifferent people to assess natural variations in protein expression. At least 2 replicate experiments are performed with each tissue sample to assess the experimental variation in protein expression levels. Stage D prostate cancer exhibits the greatest variation in protein expression from healthy tissue. Therefore, 2-D CE conditions developed above are applied to up to 5 excised Stage D prostate tumor samples. Best results are expected with samples taken from individuals of similar age and ethnic backgrounds as the healthy tissue samples. PSTs for each protein that either appears or disappears in the 2-D CE pattern developed above are developed upon comparison of the 2-D electrophoresis patterns of healthy tissues. We assume that only 10 proteins will be completely absent in one of the two expression patterns, indicating a change in gene 5 sequence, expression, or post-translational modification. These proteins are believed to be the most likely to be related to the onset or metastasis of prostate cancer. PST determination will be extended to proteins that exhibit expression levels significantly outside (i.e., 3 standard deviations) the natural variation determined, in for normal tissues. We assume that some proteins will exhibit significant expression level changes. These proteins are the next most likely to be related to prostate cancer. A prostate cancer proteomic database is developed with the information, generated from above. This database includes the isoelectric point, molecular weight, relative expression level and protein sequence tag (if determined) for each protein identified.

The experiment described above also provides a basic set of data on the underlying common mechanisms of prostate cancer. This data can be used to trace familial cancers, investigate proteomic variations associated with the cancer stages and metastases in other tissues, and investigate the function of tumor suspect targets that may be related to developmental proteins associated with puberty. Additionally, proteins associated with the attachment of metastases in other body tissues may be discovered and plasma protein markers of prostate cancer or its various stages may be identified. Furthermore, proteomic effects of various drug therapies from preclinical or clinical trials can be screened to help determine the mechanism of action and efficacy of the drug.

EXAMPLE 19

Use of Proteomics for Analysis of Stress Gene Expression

With proteomics, stress gene expression can be used to fingerprint the chemical or biological agents known to cause a response in tissue-based biosensors. The superior sensitivity of tissue-based detection systems is due to the biochemical amplification cascades inherent in biological sensing. This receptor-based biochemical amplification approach, which is inherent in all biological sensing (from bacteria to man), holds the potential to leap-frog the two major limitations of conventional chemical detection systems: threshold sensitivity and the finger printing of threats. Known threats can be identified by matching the biochemical signature produced by the tissue upon exposure to the threat agent to a library of known biochemical signatures. One class of biochemical amplification systems is the stress gene system. The advantage of using stress gene signatures in fingerprinting is that novel and unknown threats can be identified based on the type of toxic effect they have on the tissue.

This example demonstrates a new proteomics method for rapidly identifying (to the gene sequence level) and quantifying stress gene expression. Stress gene fingerprints are identified for chemical agents and biological agents known to trigger the tissue-based biosensor. A library of stress gene fingerprints can created for every chemical and biological threat and the library used to rapidly fingerprint threats in the field
once a tissue-based sensor is triggered. The proteomics technique is universally applicable to any tissue or cell type.

EXAMPLE 20

Labeling Proteins with a Fluorescent Tag for MODE and IMLS Analysis of Diseased and Healthy Tissue Samples for the Purpose of Creating a Proteomics Database Profile

In cases where protein concentrations are low enough to require a more sensitive mode of detection, the use of a fluorophore to achieve the desired sensitivity is well documented. Further, in the case of performing labeling reactions, it is common to implement a protection strategy to ensure that only the desired groups are labeled. The labeling of a protein with various agents in an aqueous or mixed aqueous/organic solvent milieu is also known in the art and a wide range of labeling reagents and techniques useful in practicing the present invention are readily available to those of skill in the art. See, for example, Means et al. , CHEMICAL MODIFICATION OF PROTEINS, Holden-Day, San Francisco, 1971; Feeney et al., MODIFICATION OF PROTEINS: FOOD, NUTRITIONAL AND PHARMACOLOGICAL ASPECTS, Advances in Chemistry Series, Vol. 198, American Chemical Society, Washington, D.C., 1982; Feeney et al., FOOD PROTEINS: IMPROVEMENT THROUGH CHEMICAL AND ENZYMATIC MODIFICATION, Advances in Chemistry Series, Vol. 160, American Chemical Society, Washington, D.C., 1977; and Hermanson, Bioconjugate TECHNIQUES, Academic Press, San Diego, 1996.

The following example illustrates how a fluorogenic PST is selected and utilized in a typical embodiment of the invention. In addition, the example illustrates the strategic use of protective group chemistry (see Greene and Wuts, Protective Groups in Organic Synthesis 3rd Ed., Wiley Science (1999)) to ensure selective terminal labeling of the proteins or peptide. Finally, this example illustrates the prudent use of radioisotopic tags and modifiers to produce unique mass fragments when the material is fragmented and analyzed under IMLS conditions (see co-pending application Ser. No. 09/513,395, entitled "Methods for Protein Sequencing", filed February 25, 2000,

In this example, tissue samples from healthy and diseased specimens are subjected to typical procedures for raw extraction of proteins. After the proteins are isolated, preliminary separations are performed using electrophoresis, preferably cIEF. Additional separations are performed if necessary. Protein fractions are then subjected to labeling with the fluorescent tagging material that contains all the desired features of the invention, namely, a unique mass signature, quantitative detection component, and a reactive functional group, Quantum Dye™, a commercially available compound (Research Organics, Cleveland OH), is a macrocyclic chelate of Europium (III) that exhibits unique fluorescent properties (see, Leif, et al., ACS Symposium Series 464, Cell Separation Science and Technology, D. S. Kampala and P.W. Todd Editors, American Chemical Society, Washington, DC, PP. 41-58 (1991); Vallarino, et al., Proceedings of Advances in Fluorescence Sensing Technology, J.R. Lakowicz and R.B. Thompson, Editors and A. Katzir, Progress in Biomedical Optics Series Editor, SPIE Proceeding Series 1885 pp. 376-385 (1993); and Leif, et al., Proceedings of Biochemical Diagnostic Instrumentation, Progress in Biomedical Optics. Ed., R.F. Bonner, G.E. Cohn, T.M. Laue, and A.V. Priezzhev. SPIE. Proceedings Series 2136, pp. 255-262 (1994)) (See Fig. 1) and incorporates the primary features of the invention. When excited by light of the proper energy (360 nm), and in the presence of an enhancing agent, the Eu (III) chelate exhibits an intense narrow-band emission spectrum (typically around 10 nm at one-half the peak height) with a large Stoke's shift. The complex fluoresces strongly with an emission around 620 nm. The characteristically long fluorescent times (>300 microseconds, see, Periasamy, et al., Microscopy and Analysis, March pp. 33-35 (1995) and Seveus, et al., Micro. Res. And Tech. 28, pp. 149-154 (1994)) enable one to conduct short-pulsed excitation of the sample followed by time-delayed signal detection consequently zeroing out background interference from components with relatively shorter fluorescent lifetimes. Fortuitously, the label does not suffer from fluorescent quenching as do many other labels and thus the response curve for standards remains linear at high concentrations. Seveus, et al., *ibid*. have shown that these compounds are optimal for biological applications due to their increased water solubility and relative inertness to release of the chelated metal. This enables the use of safer reaction conditions and provides an additional feature of a hard positive charge in the label. The phenylisothiocyanate linkage on the Quantum Dye™ is optimum for attachment to amines, and the positive three (+3) hard charge in the center of the chelate brings the tag's large mass of 927.7 amu to a comfortable m/e value of just 309.2333.

In this example, the Quantum Dye™ tag can be attached typically on isolated protein mixtures (see, J, Schlessinger, Dept. of Chemical Immunology, The Weitzman Institute for Science, Rehovot, Israel (Personal communication between Research Organics, Inc. and J. Schlessinger); D. Blakeslee, et al., J. Immun. Methods, 13:305 (1976); and H.P. Rothbart, et al., J. Immun. Methods, 19:101 (1978)). Test and control protein samples are dissolved to a concentration of 0.5-3 mg/mL in 0.05M borate (or carbonate or other suitable buffer) at a pH of 9-9.5. 0.4 M sodium chloride is added to adjust ionic strength. A suitable volume (5 mL) is placed in a dialysis bag. Approximately 3 mg of the Quantum Dye™ is dissolved into 100 mL borate (carbonate) buffer, pH 9.3. The protein solutions are dialyzed against the dye solutions over a suitable time (1 hr +) at room temperature to conjugate the tag to the proteins. The conjugated proteins are then dialyzed against a buffer suitable for separation and analysis by multi-dimensional capillary electrophoresis in the cGE or cZE mode, such as MES, or pyridinium acetate.

The tagged proteins are loaded into the capillary and electrophoresed, detected, and fractionated. The proteins are monitored by fluorescence and ultraviolet detection since the Quantum Dye™ is also a chromophore in addition to being a fluorophore. Each of the fractions are quantitated by fluorescence and UV. The fractions are then partitioned and analyzed for total protein by NanoOrange™ and Sypro™ dyes (Molecular Probes, Inc., see Harvey M.D., et al., Electrophoresis Sep; 19(12):2169-74 (1998)). Knowing the total mass of the proteins analyzed for each sample, the theoretical amount of fluorescent tag required for complete labeling can be estimated. This can be determined in conjunction with a total digest of the fractionated protein to determine the overall amino acid content. The amount of tag actually found within each protein fraction is compared to the theoretical amount expected based on the AA analysis and total mass found by Sypro™ and NanoOrang™ assays. In this way the overall labeling efficiency is determined on a sample-by-sample basis.

In a typical NanoOrange™ or Sypro™ Assay, detection to ng/mL levels are possible. The reagent is nonfluorescent in aqueous solution, but upon interaction with proteins in the presence of detergent, it binds tenaciously to proteins.

In cases where other labels are needed, the choices are many. Other linkage chemistries on fluorophores include sulfonyl chloride, NHS esters, alkyl halide, and activated alkene. Choosing other linkage chemistries allows use of other fluorogenic species as tags and increases the chances of having the choices necessary to generate excellent IMLS data with fragments encompassing many amino acid residues. Each of the selections, including the Quantum Dye™ tag used in this example, and many more labels are possible that incorporate the primary features of the invention; i.e. a linking functionality, a charged specie (+ or -), hard or soft charge, and a unique mass signature.

To label proteins on the C-terminal, some protective measures are usually required and desirable (see, Atassi, et al., Eds. Methods in Protein Structure Analysis, Plenum Press, 1995; Boyd, V.L., et al., Sequencing of Proteins from the C-terminus, pp. 109-118; and Dupont, et al., PE Biosystems, Inc. Application Note). On the basis of data collected from routine AA assays, the proper protective chemistries are selected. It has been demonstrated that cys, lys, ser, thr, asp, and glu side-chain residues can pose challenges when attempting to perform some types of C-terminal labeling or sequencing, and modification of these groups is often necessary, though relatively easy to accomplish (see, for example, Atassi, et al., *ibid.* and Guga, et al., "C-terminal Sequence Analysis of the Amino Acids with Reactive Side-Chains: Ser, Thr, Cys, Glu, Asp, His, Lys." Poster presentation at the Seventh Symposium Of the Protein Society (1993)).

These protective chemistries are similar to those used by PE Biosystems in their Procise 494C Sequencer (see, Werner, et al., "A New Simple Preparation Device for Protein/Peptide Sequencing" Poster presentation at the Ninth Symposium of the Protein Society; and Brune, Anal. Biochem. 207:285 (1992)), except that in this example, isotopically labeled reagents are provided in approximately a 50/50 mixture with their corresponding non-isotopic counterpart where prudent to do so. In continuing with the example, cys residues are modified by alkylation with acrylamide to yield stable derivatives. Lys, Ser, and Thr are modified with a mixture of isotopic and non-isotopic phenylisothiocyanate to also yield very stable derivatives. The phenylurea derivatives formed at the lysines and carbamates at the Ser and Thr, and an arylcarbarmate at the Tyr residues. Using isotopically labeled PITC will yield unique mass fragment pairs in the IMLS spectrum. The choice of how much to use will help with the ultimate hydrophobicity when adhering to PVDF membranes. These conversions are easily carried out on PVDF membranes. The hydroxyl groups of Ser and Thr are capped using acetic anhydride. Asp and glu residues along with the C-terminus are also reacted in acetic anhydride. The C-terminal forms an oxazolone ring that does not form for either of the asp or glu acidic side residues which form mixed anhydrides instead. This allows one to incorporate isotopically labeled acetic anhydride selectively to the Thr and Ser residues during the capping process to enable easier identification of those residues later during IMLS analysis. The cyclized C-terminal end is then reacted with the desired amine modifier via an NHS ester facilitated mechanism. When complete, all potentially interferring residue side chains have been selectively modified, and the C-terminal has been chemical modified to an amine. At this junction, the Quantum Dye™ in this example can be easily attached by the procedures discussed above, or other desired fluorescent tags can be utilized if desired.

After side chain modifications and attachment of the fluorescent tag to the amine-modified C-terminal end of the protein samples, the samples are dissolved in MES buffer, or one of many other possible detergents/surfactants, and are electrophoresed in the cGE or cZE modes, detected by fluorescence and UV, and the protein levels are quantitated and finally, fractionated. Sypro™ and NanoOrange™ assays are performed on the protein samples either prior to tagging or after depending on the amount of sample to work with. Note that in cases where labels are chosen that do not fluoresce but are only chromophores, NanoOrange™ and Sypro™ assays can still be used to allow easy analysis of these proteins in the CE without needing the advantages afforded by having a fluorescent property as part of the protein sequence tag. Actual amounts of the QuantumDye™ label consumed are compared to theoretical amounts estimated to gain a perspective of labeling efficiency.

Fractionated tagged proteins are then made to suitable volume for IMLS analysis in the same buffer (MES in this example) and are analyzed in the ESI-TOF mass spectrometer under IMLS fragmentation conditions. Spectral data are collected and under data reduction and analysis using our proprietary IMLS data analysis algorithm. The identification and assignment of the protein sequence and ID are then correlated to the quantitation and migration data collected for each of the protein fractions that underwent CE analysis. This information, tied with that of the total protein assays (Sypro™, etc.) allow a complete protein expression database to be built with the collective information obtained from each of the assays. This database collective will enable a comparative analysis between the protein expression profiles in the diseased tissue versus the control or healthy tissue, the ultimate goal of this example.

The foregoing process is repeated with multiple samples from diseased and control subjects, as well as replicate runs with samples from the same subjects. The results are then examined to identify proteins whose relative abundance varies between diseased and control subjects. Such proteins are potential markers for the particular disease and/or a drug target or potential drug.

EXAMPLE 21

Role of Metabolism in Autism

The methods of the invention can be utilized to ascertain whether various diseases have a metabolic basis and even to pinpoint the metabolic basis. As a specific example, the methods and apparatus of the invention can be used to establish whether autism (or the severity of autistic symptoms) results from dietary factors, determine the mechanism through which diet may influence autism, and establish a simple $^{13}C$ metabolic assay to facilitate diagnosis of such dietary influences.

Elevated exogenous peptides have been found in the blood, urine, and cerebral spinal fluids of large numbers of autistic children (Reichelt, K. et al., J. Appl. Nutrition, 42:1-11 (1990); Reichelt, K. et al., Developmental Brain Dysfunction, 7:71-85 (1994); Reichelt, K. et al., Brain Dysfunction, 4:308-319 (1991); Gillberg, C., "The role of endogenous opioids in autism and possible relationships to clinical features," in Wing, L. (ed.), Aspects of Autism: Biological Research, pp. 31-37, Gaskell, London, (1988); Shattock, P., A., et al., Brain Dysfunction, 3:328-345 (1990)). Precedents from celiac disease, and a number of clinical case studies documenting significant patient improvements on grain and dairy dietary restrictions, have led to three hypotheses regarding dietary effects on autism. (See, *e.g.*, Fukudome,S. and Yoshikawa, M., FEBS Lett., 296:107-111 (1992); Fukudome,S. and Yoshikawa, M., FEBS Lett. 316:17-19 (1993); Reichelt, K. et al., J. Appl. Nutrition, 42:1-11 (1990); Reichelt, K. et al., Developmental Brain Dysfunction, 7:71-85 (1994); Reichelt, K. et al., Brain Dysfunction, 4:308-319 (1991); Shattock, P., A., et al., Brain Dysfunction, 3:328-345 (1990); Lewis, L.S., "Dietary intervention for the treatment of autism: Why implement a gluten and casein free diet?," in: Biological Treatments for Autism and PDD, pp 196-226, (Shaw, 1998); Serousi, K., "Following a different road. A child's documented recovery from autism," in: Biological Treatments for Autism and PDD, pp 265-289, (Shaw,1998)).

The first theory, suggested by Lewis, is that a mild form of celiac disease may exist in many, if not all, autistic children, leading to peptide malabsorption from the gut and associated neurological symptoms (Lewis, L.S., "Dietary intervention for the treatment of autism: Why implement a gluten and casein free diet?," in: Biological Treatments for Autism and PDD, pp 196-226, (Shaw, 1998)). Shaw has proposed a second theory, namely that the exogenous peptides observed are a consequence of intestinal yeast infections, which are stimulated by the carbohydrate content of grains and milk( Shaw, W., et al., "Increased excretion of analogs of Krebs cycle metabolites and arabinose in two brothers with autistic features," Clin. Chem., 41:1094-1104 (1995); and Shaw, W., "Organic acid testing, byproducts of yeast and their relationship to autism," in: Biological Treatments for Autism and PDD, pp. 31-65, (Shaw, 1998)). Intestinal yeast may directly produce exogenous peptides as secondary metabolites or the peptides may form in the blood indirectly through crosslinking caused by the high levels of reducing sugars they produce. Based on clinical data concerning the efficacy of secretin and peptidase supplementation in the diet of autistic children, Shaw has proposed a third possible mechanism, suggesting that digestive enzymes may not be functioning properly in autistic patients (Shaw, W., "Abnormalities of the digestive system," in: Biological Treatments for Autism and PDD, pp. 124-138, (Shaw, 1998)).

How the methods of the invention can be used to distinguish between these theories can be seen from a two compartment pharmacokinetic model for peptide absorption through the gut (Notari, R. E., Biopharmaceutics and Pharmacokinetics: An Introduction, 2nd ed., Marcel Dekker, NY, (1975)). The flux of exogenous peptides through the intestinal wall ($g_p$) can be represented by:

$$g_p = k_t\left(K_a C_p^i - K_b C_p^b\right) \tag{1}$$

where $k_t$ is the mass transport coefficient for the intestinal wall, $K_i$ and $K_b$ are the equilibrium constants for dissolution of the peptide in the intestinal wall and its concentrations in the intestine $(C_p^i)$ and $C_p^b$ blood respectively.

The flux ofpeptides through the intestinal wall competes with the rate of peptidase digestion of the peptide in the gut ($r_p$), which we represent by:

$$r_p = \frac{kE_p C_p^i}{\left(K_m + C_p^i\right)} \tag{2}$$

where k is the rate constant, Ep is the peptidase concentration in the gut and $K_m$ is the Michaelis constant.

Since typically $C_p^i \gg K_m$, the peptide concentration in the blood as a function of time (t) is given by:

$$C_p^b = \frac{C_{p_o}^i}{\dfrac{kE_p V_i}{k_t S C_{p_o}^i} - \left(\dfrac{K_b}{K_i}\right) + \left(\dfrac{V_b}{V_i}\right)} \left\{ e^{-k_t S\left[\left(\frac{K_b}{V_b}\right) - \left(\frac{K_i}{V_i}\right)\right] t} - e^{-\frac{kE}{C_{p_o}^i} t} \right\} \tag{3}$$

where $V_i$ is the volumes contained by the intestine, $V_b$ is the distribution volume in the body, and $C_{p_0}^i$ is the initial

concentration of the peptide bolus in the gut. Where $K_i \approx K_b$ and $V_i \approx V_b$, equation 3 can be approximated by:

$$C_p^b = \frac{k_t S C_{p_0}^i}{kEV_i}\left[1 - e^{-\left(\frac{kE}{C_{p_0}^i}\right)t}\right] \quad (4)$$

From equation 4 it is readily apparent that the amount of peptide crossing the intestinal wall is determined by the ratio of the permeation rate ($k_tS$) to the rate of peptide digestion in the gut ($kEV_i$).

Equally important is the rate of accumulation of peptide in the blood, which is predicted (equation 4) to depend primarily on peptidase activity in the gut (kE). Therefore, measurements of the time course of peptide accumulation in the blood can be used to identify the underlying mechanism driving maladsorption and provide significant insights for the most appropriate course of therapy. More specifically, this provides the basis for distinguishing between the two possible reasons for the appearance of exogenous peptides in the blood and urine of autistic children, namely: (1) the intestinal wall is compromised as in celiac disease, giving rise to increased peptide permeability ($k_t$), or (2) autistic children may suffer from peptidase deficiency (*e.g.*, low peptidase enzyme levels or lower than normal peptidase activity).

To identify the source of exogenous peptides arising from wheat (*i.e.*, improper digestion and malabsorption or microbial stimulation), autistic children (and a control group) are fed a mixture of $^{13}C$-enriched and normal wheat flour. Exogenous peptides are identified from blood plasma and urine of autistic and control groups. Mass spectrometric techniques are used to establish the stable isotope ratio of any exogenous peptides identified. Peptides exhibiting two predominant isotopic peaks with relative abundances equal to that of the ingested flour mixture can only be derived directly from the wheat proteins themselves. Peptides that exhibit a multitude of isotopic peaks in relative abundances that vary from that of the ingested flour mixture, can only have been formed after complete digestion of the wheat proteins (*i.e.*, after the constituent $^{13}C$ and $^{12}C$ amino acids had a chance to intermingle). In a follow up experiment patients are fed a flour mixture predigested with proteases. These patients should not exhibit any exogenous peptides derived from wheat proteins but should still exhibit peptides derived from microbial or disease-related synthesis within the body.

Related methods are used to identify if any of the exogenous peptides pass the blood brain barrier (have the potential to cause neurological disorders) and identify their ultimate source. Peptides derived directly from incomplete digestion and malabsorption of food proteins can be detected directly in cerebral spinal fluid samples taken from autistic children after ingestion of the flour mixture according to the methods described herein. By feeding $^{13}C$-enriched amino acids and simultaneously providing intravenous supplementation of one or more amino acids, the likely source of exogenous peptides not directly derived from wheat proteins can be identified. Those peptides of microbial origin in the gut will contain high ratios of $^{13}C$-amino acids. Lower stable isotope contents, particularly of the intravenously administered amino acids, are indicative of peptides synthesized in the blood or in human tissues. The efficacy of peptidase or secretin supplementation can similarly be explored with this technique.

Proving that exogenous peptides originating from the gut appear in cerebral spinal fluid and identifying the ultimate source of these peptides can be used in appropriate clinical treatment of autism. For example, the stable isotope technique can be used in the development of a rapid early diagnostic tool for clinicians, allowing for earlier clinical intervention before the effects of diet or yeast infection become irreversible.

EXAMPLE 22

Detecting Metabolites of $[^{13}C]_6$-glucose and $[^{12}C]_6$-glucose in *E. coli*

In this example a culture of *Escherichia coli* DH5$\alpha$ was grown exponentially at 37 C on a $[^{12}C]$-glucose morpholinopropanesulfonic acid buffered minimal media as described by Neidhardt et al [Neidhardt, F. C., P. L. Bloch and D. F. Smith, J Bacteriol, 119:736, (1974)] to a cell density of $AU_{600}$=0.7. At this cell density 0.84 ml of a 500 mM solution of $[^{13}C]_6$-glucose was added to 84 ml of the culture, resulting in a approximate equimolar ratio of $[^{13}C]_6$-glucose and$[^{12}C]_6$-glucose. Approximately 8 ml aliquots were withdrawn from the culture periodically and quenched in 2 ml ice cold trichloroacetic acid (TCA) to a final concentration of 10% TCA by weight. The quenched samples were centrifuged at 4,000 rpm, 10 C, for 40 min. Aliquots (1 ml) of the TCA supernatants were placed into 2 ml microfuge tubes and concentrated in a Savant Speed Vap to dryness. The samples were resuspended in HPLC-grade water to a total of 0.7 ml combined from the resuspended concentrates for each timepoint sample.

The resuspended TCA soluble fractions were subjected to mass spectrometric analysis in negative ion mode on a PE Biosystems Mariner™ microelectrospray time-of-flight mass spectrometer. The mass spectrometer was calibrated immediately prior to analysis per the manufacturers instructions. Samples were fed continuously at 0.3 ul/min into the microspray ionization system with a nozzle potential of 160 V and nozzle temperature of 170 C. Mass spectra were accumulated for about 15 min.

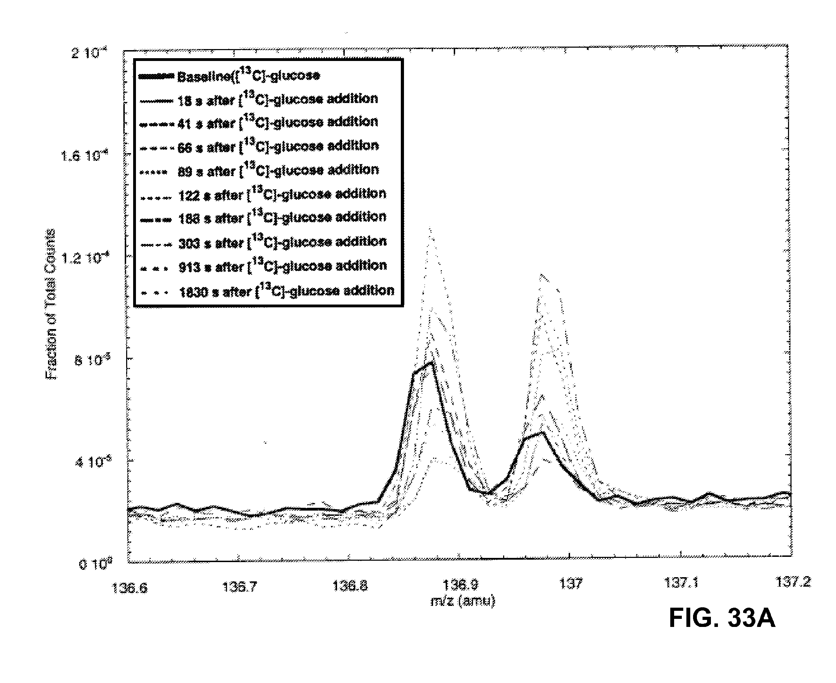

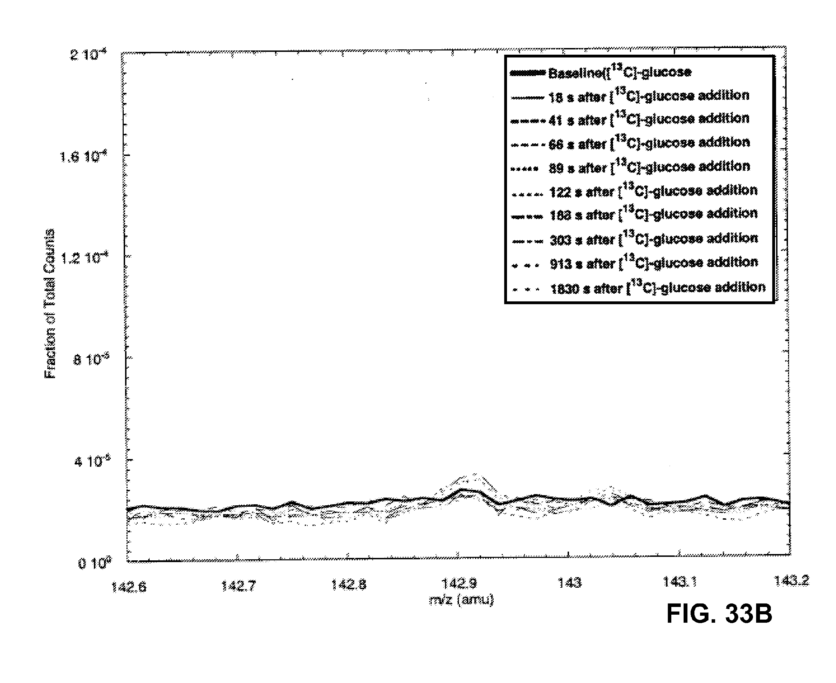

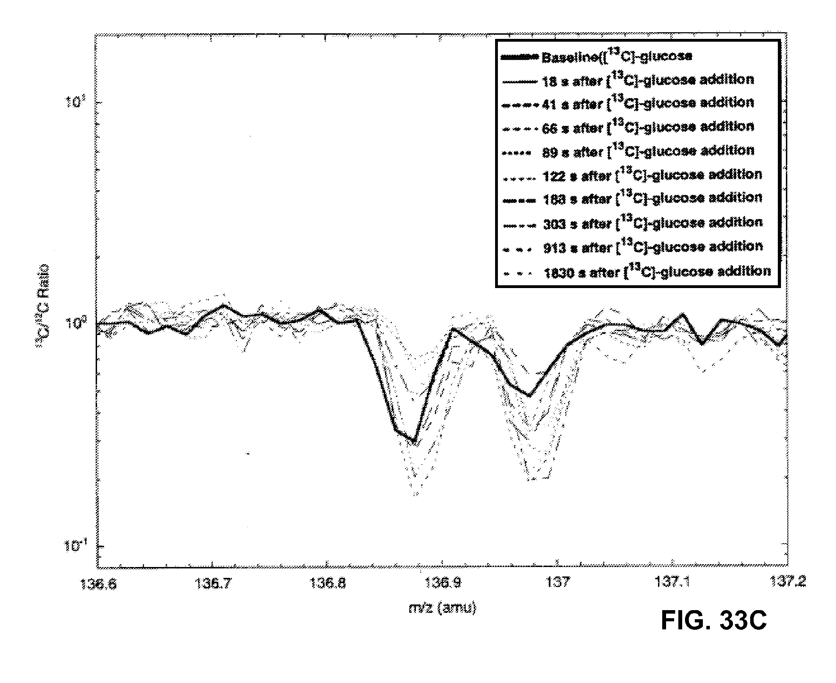

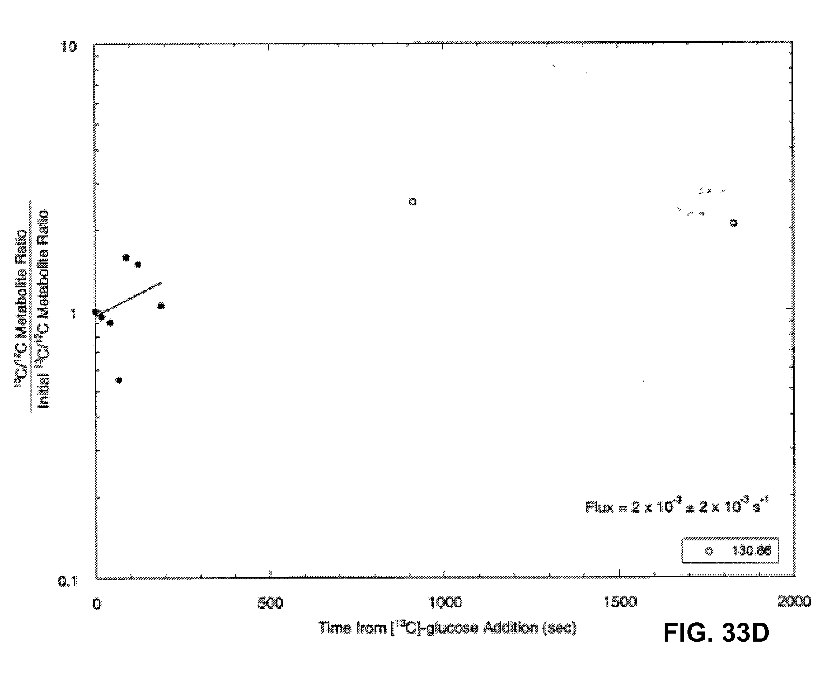

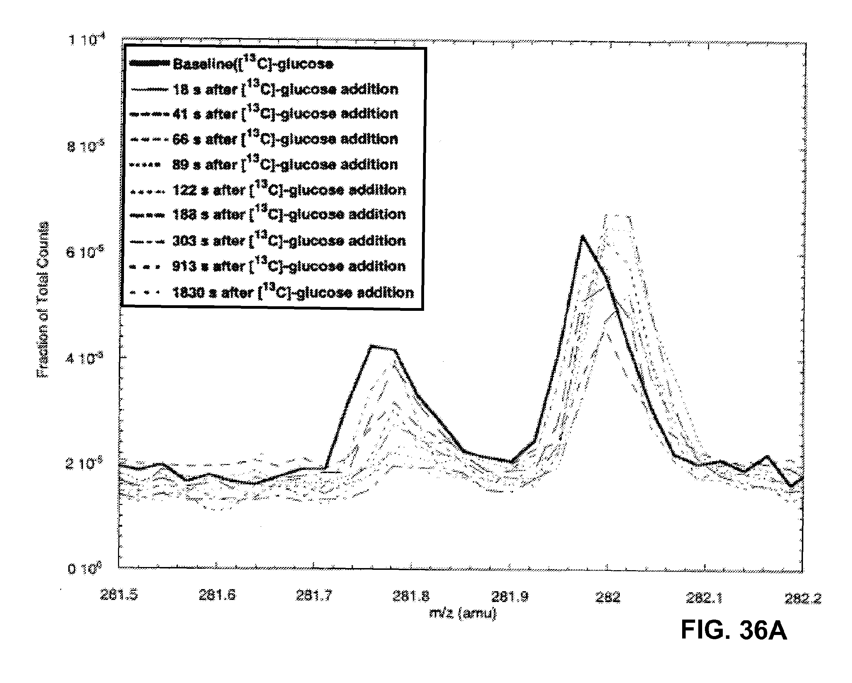

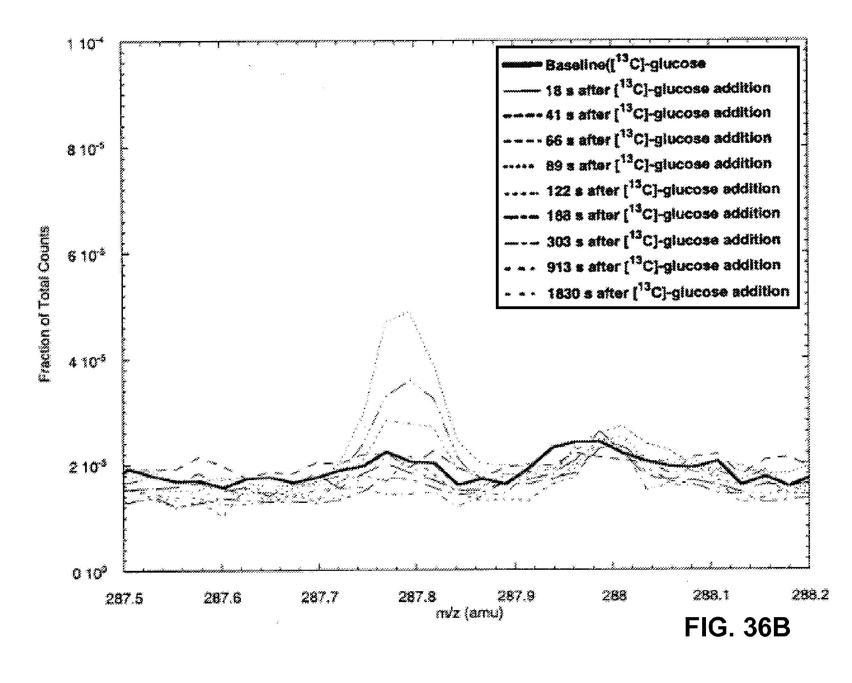

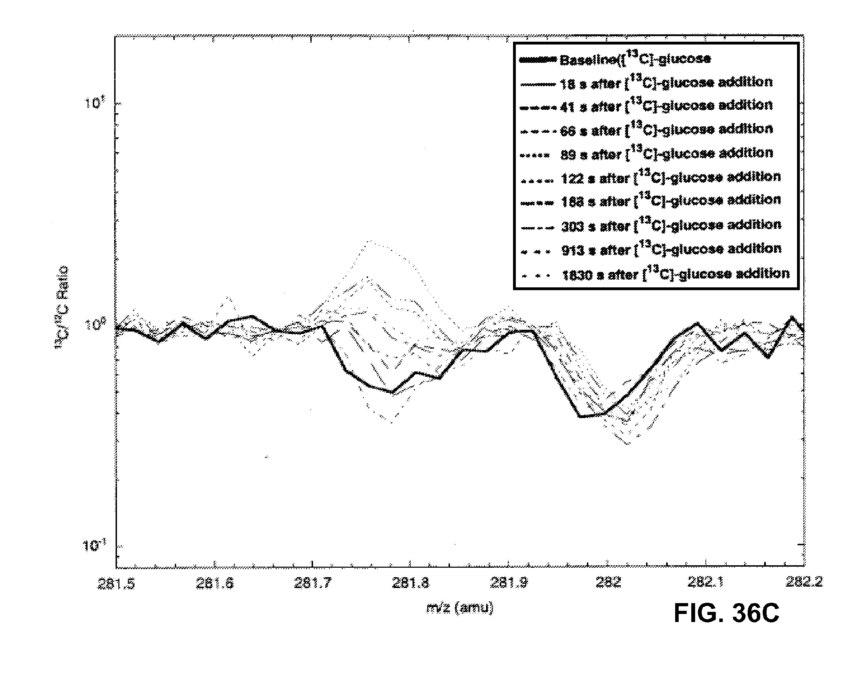

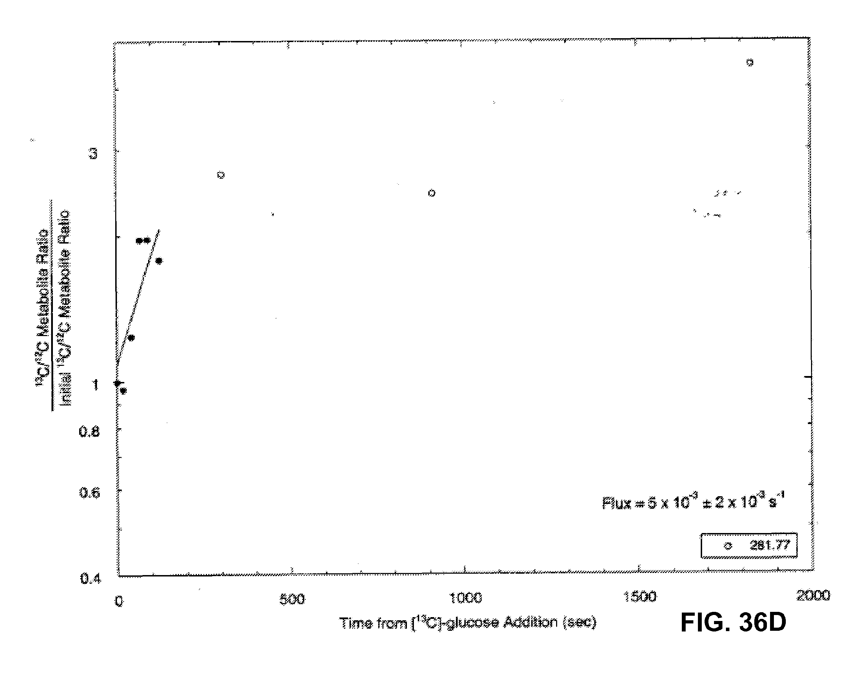

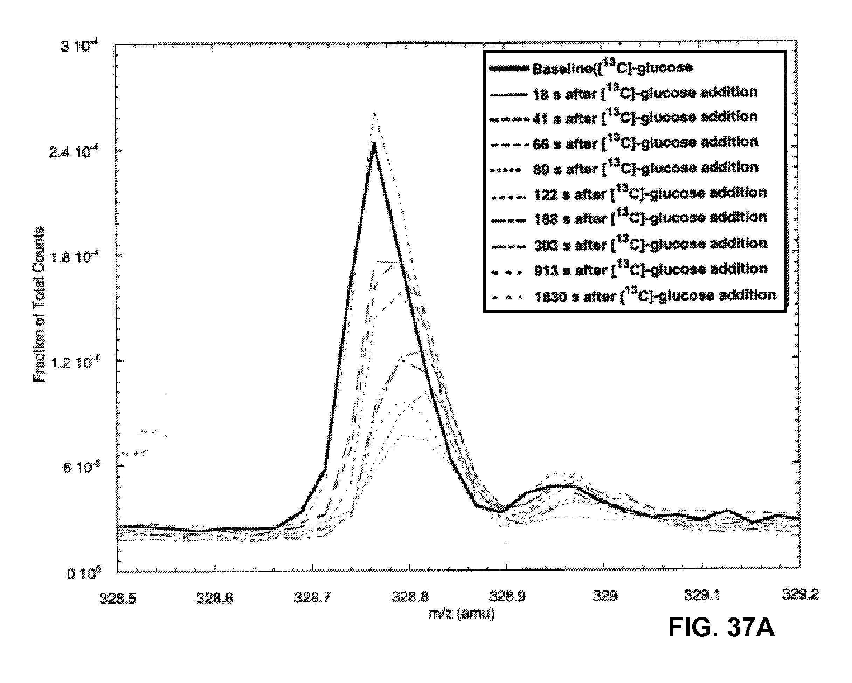

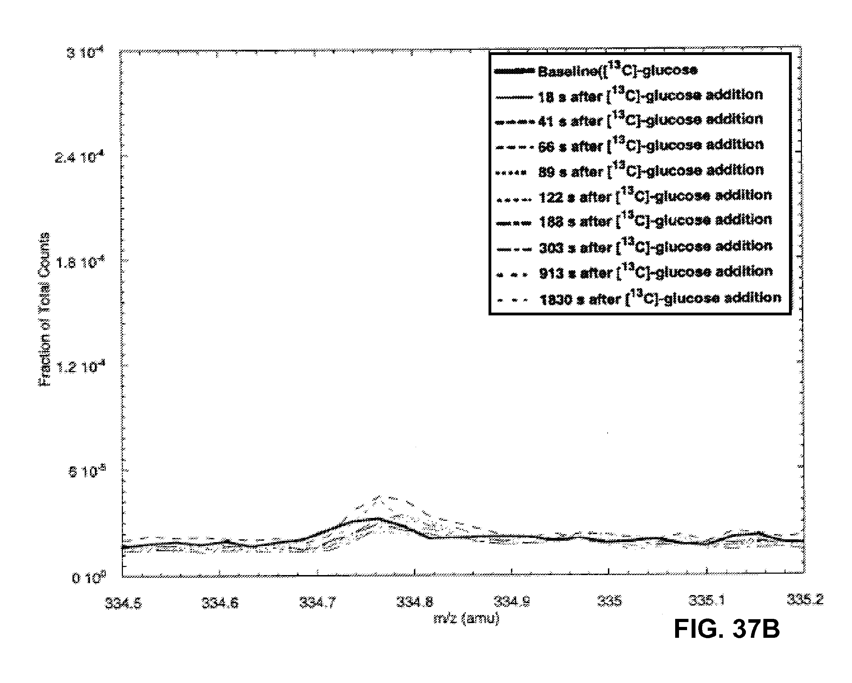

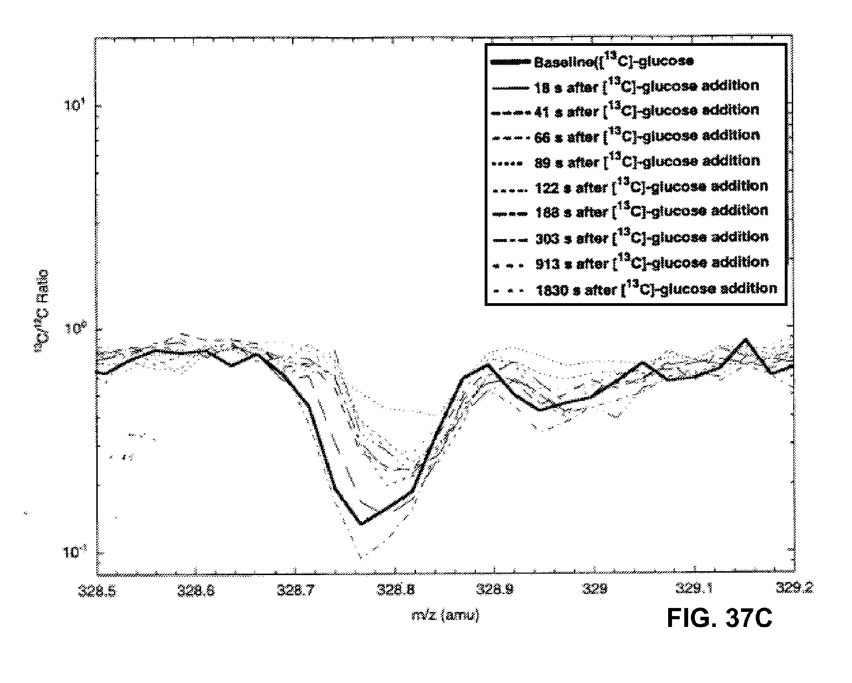

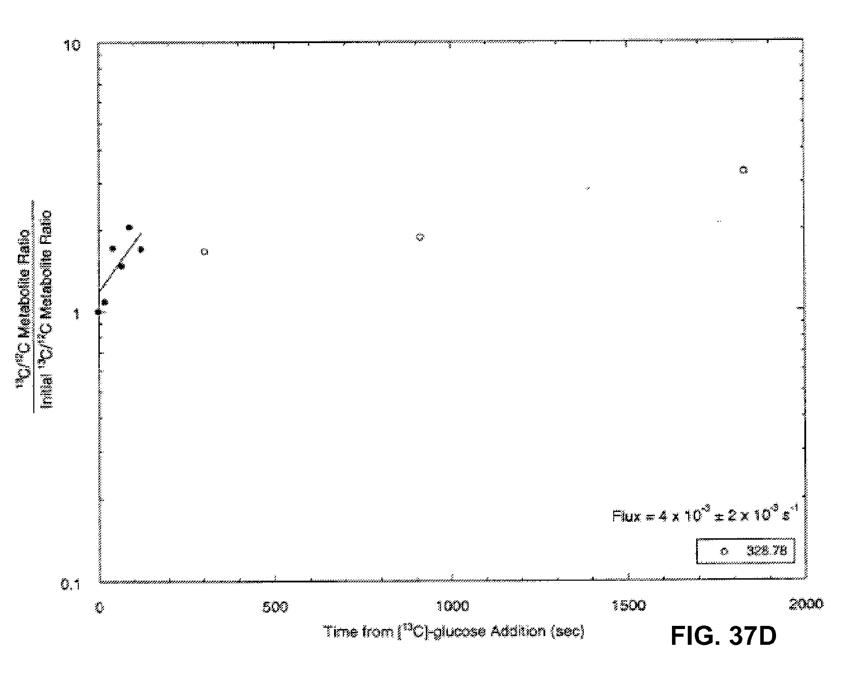

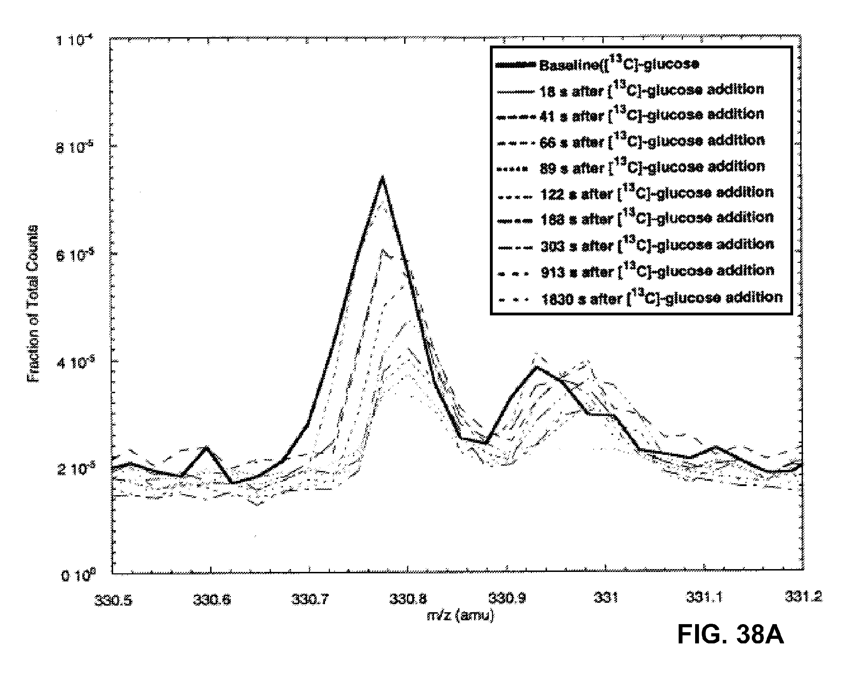

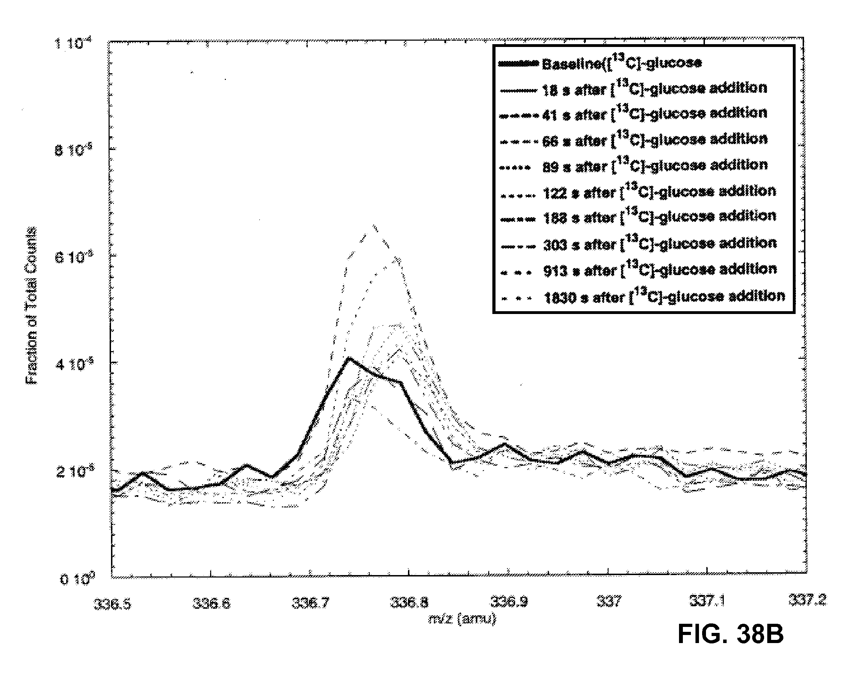

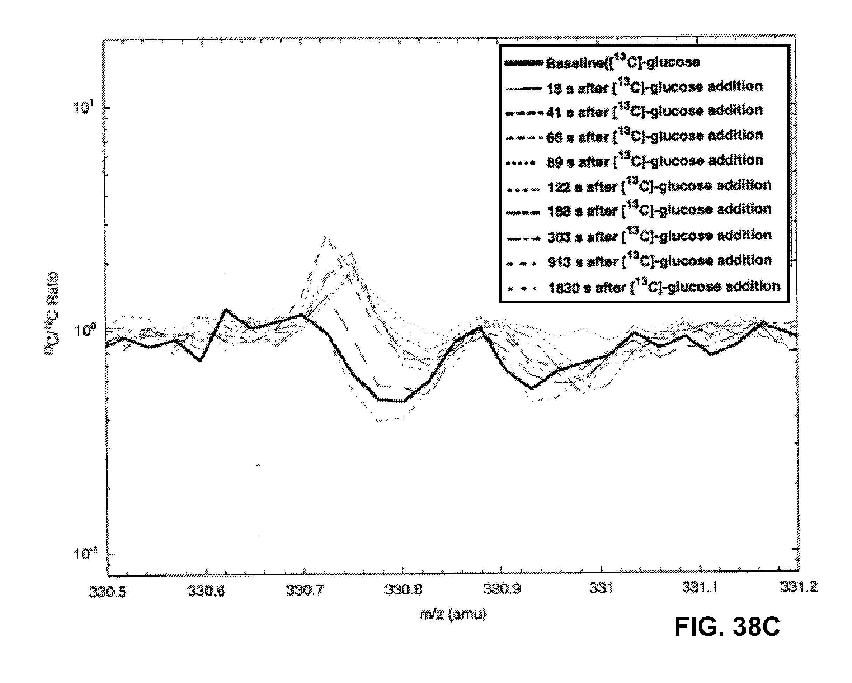

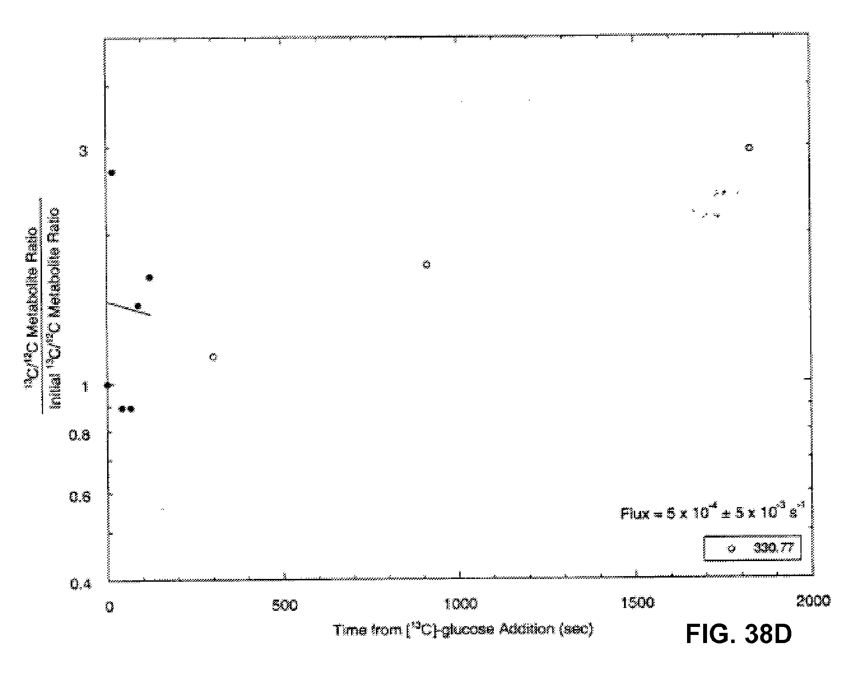

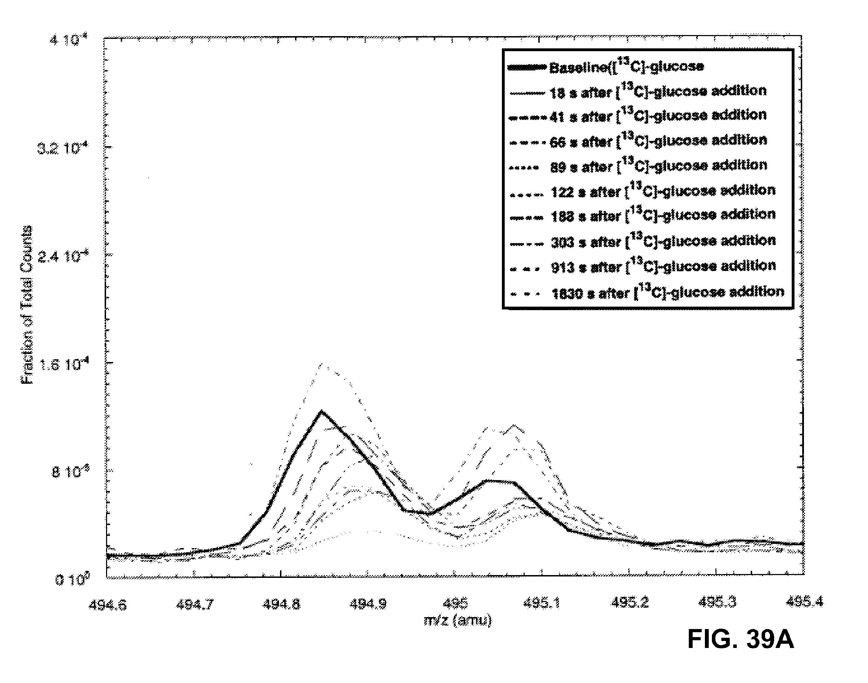

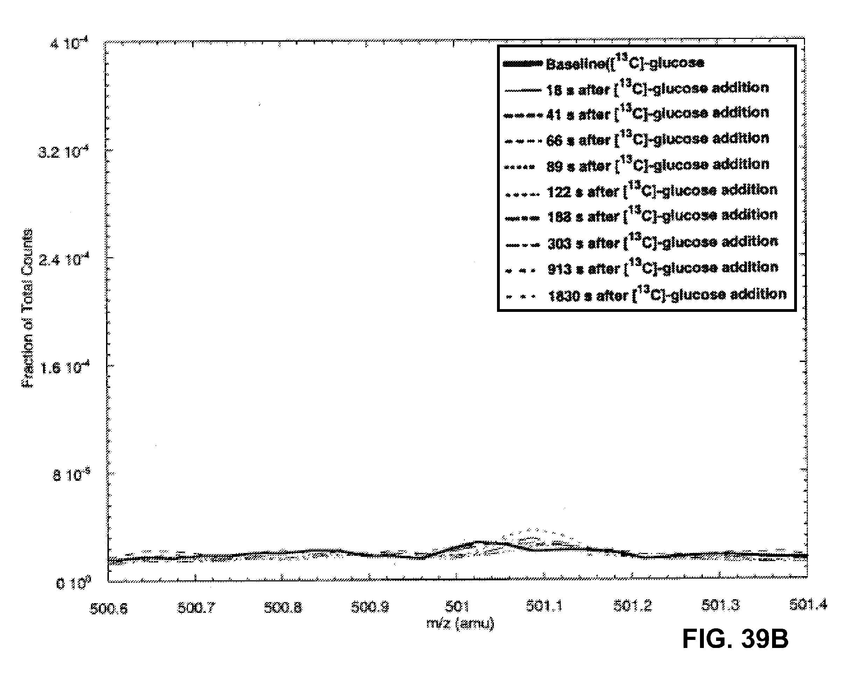

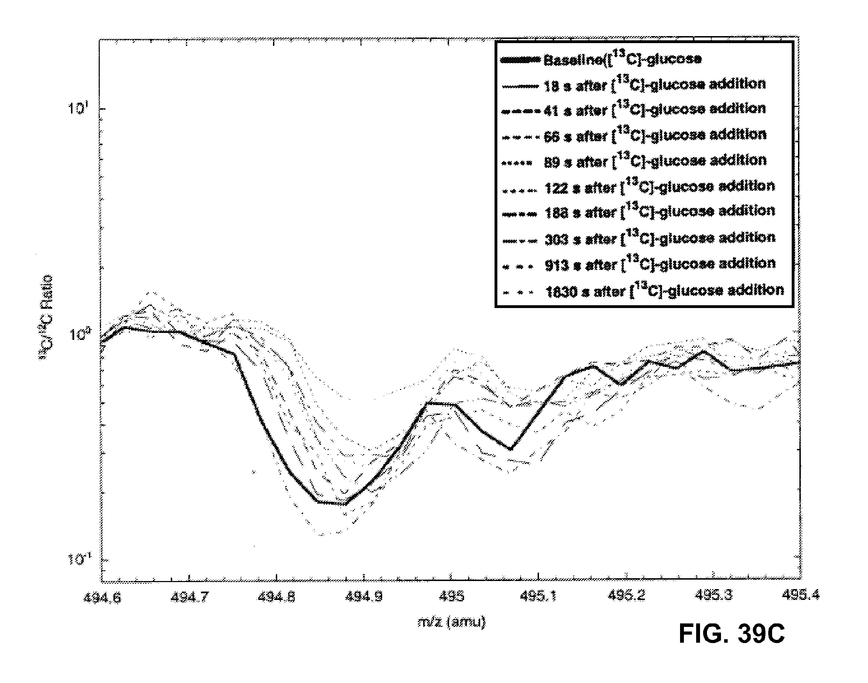

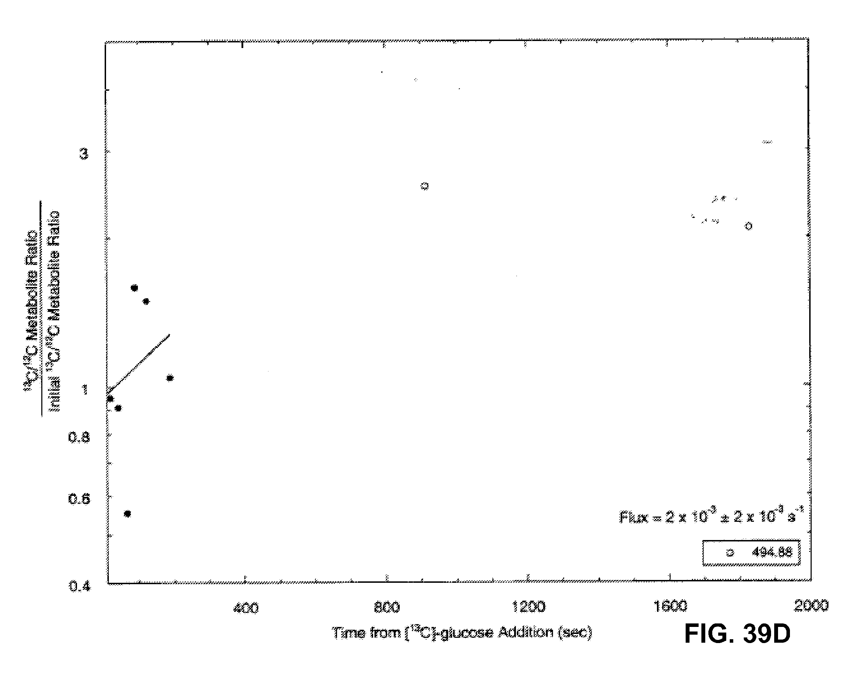

The mass spectrometric data were analyzed by dividing the counts determined exactly 6.02013 amu higher than each position by the peak counts in each mass position, yielding a $^{13}C/^{12}C$ isotope ratio spectrum for all six carbon species. The $^{13}C/^{12}C$ ratio spectra obtained for the samples taken at each time point were plotted together and manually inspected for peaks that changed consistently over the timed samples. In this analysis it is expected that the zero time spectra would show peaks that were significantly smaller than 1 and spectra from longer time points after substrate addition (i.e., after metabolism of the [$^{13}C$]-glucose) would exhibit a ratio that asymptotically approached an equilibrium value closer to 1. Manual inspection of the ratio spectra showed seven such potential six carbon metabolites (Figures 33-41).

Since the equilibrium substrate ratio was expected to be roughly equimolar, the actual MS spectra (scaled to the total counts) were analyzed at each of the corresponding $^{12}C$ and $^{13}C$ positions resulting from the ratio analysis. Three of the seven putative six carbon metabolites were elimінted by this second level analysis because the putative $^{12}C$ and $^{13}C$ peaks were not found to be of the same magnitude (Figures 33, 37, and 39).

Finally, the metabolic flux was determined by curve fit to the equation:

$$\text{Flux}_{\text{analyte}} = \frac{\ln\left\{\dfrac{(RA_t - RA_{ss})}{(RA_0 - RA_{ss})}\right\}}{(t)(\text{unit of sample})}$$

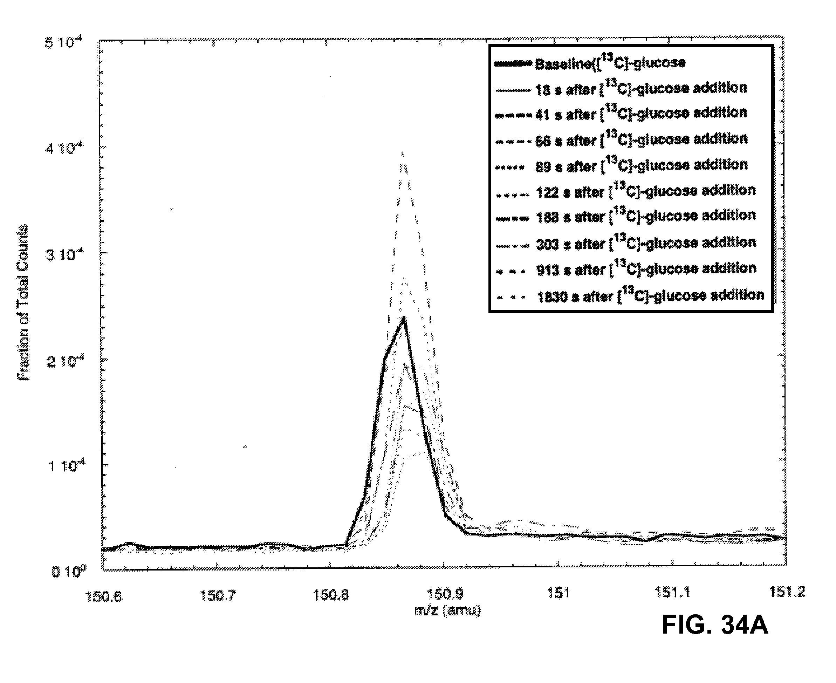

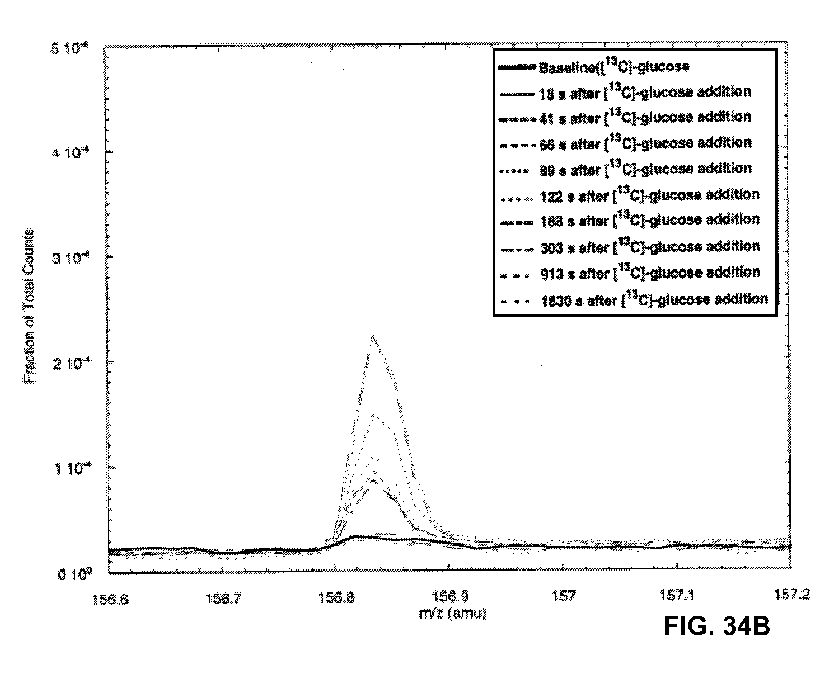

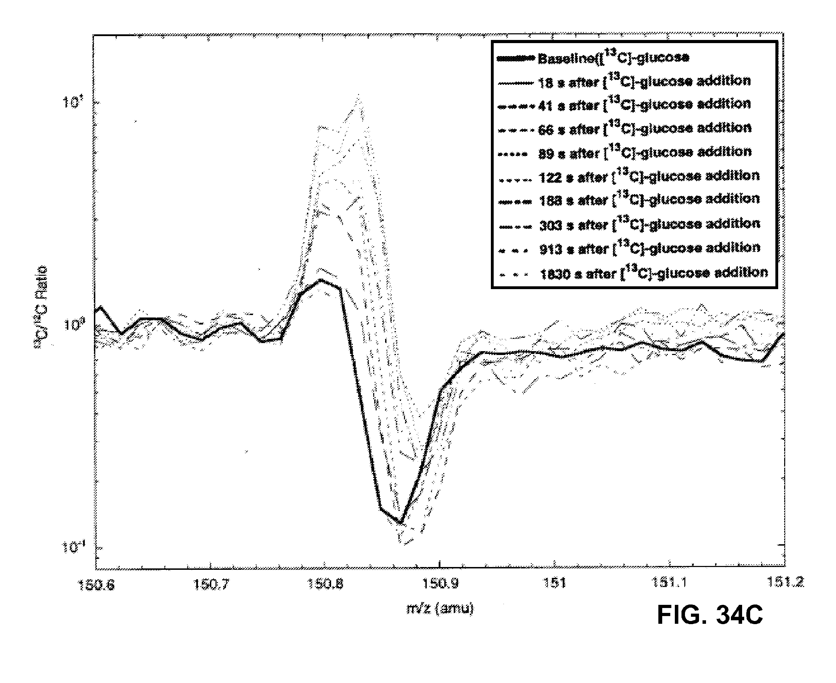

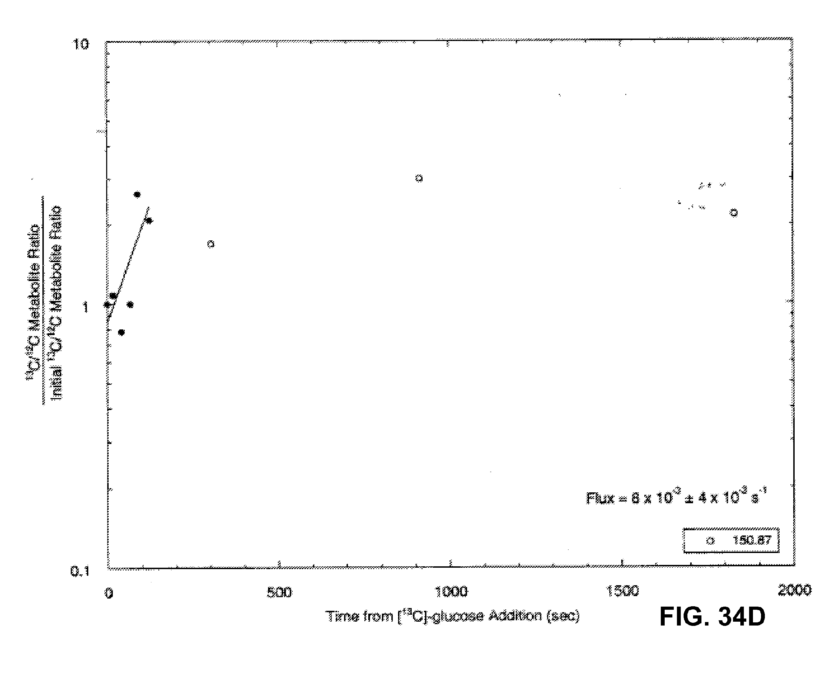

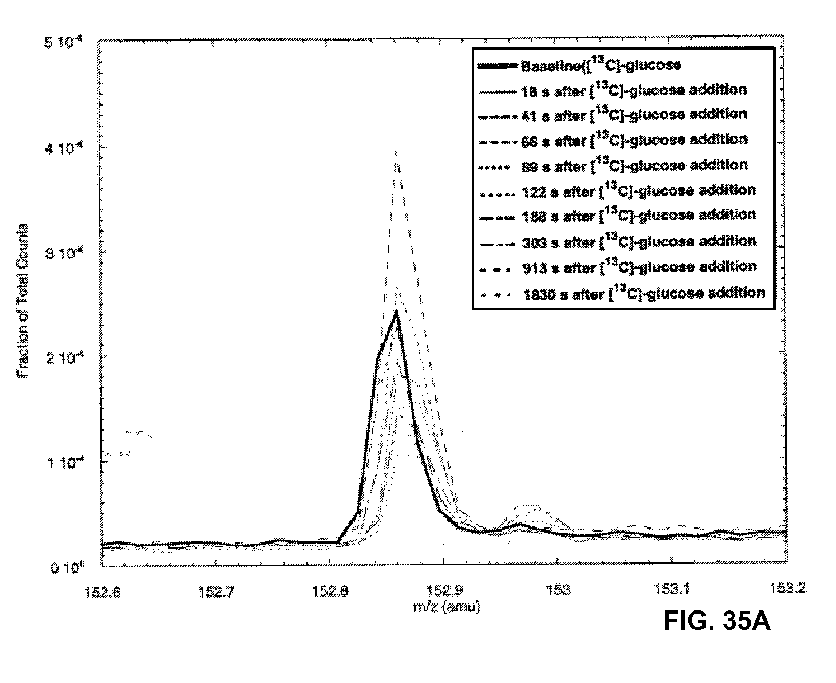

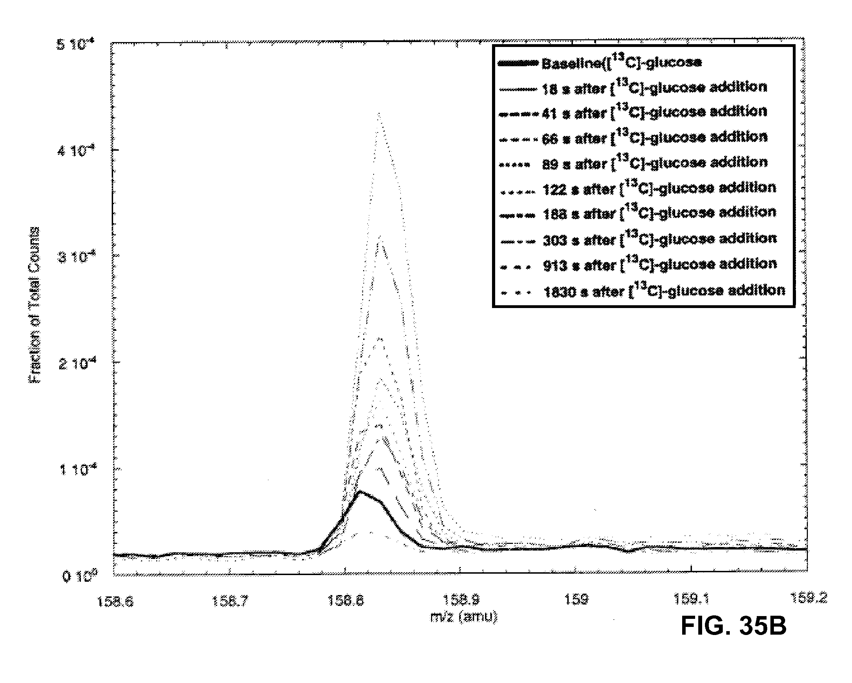

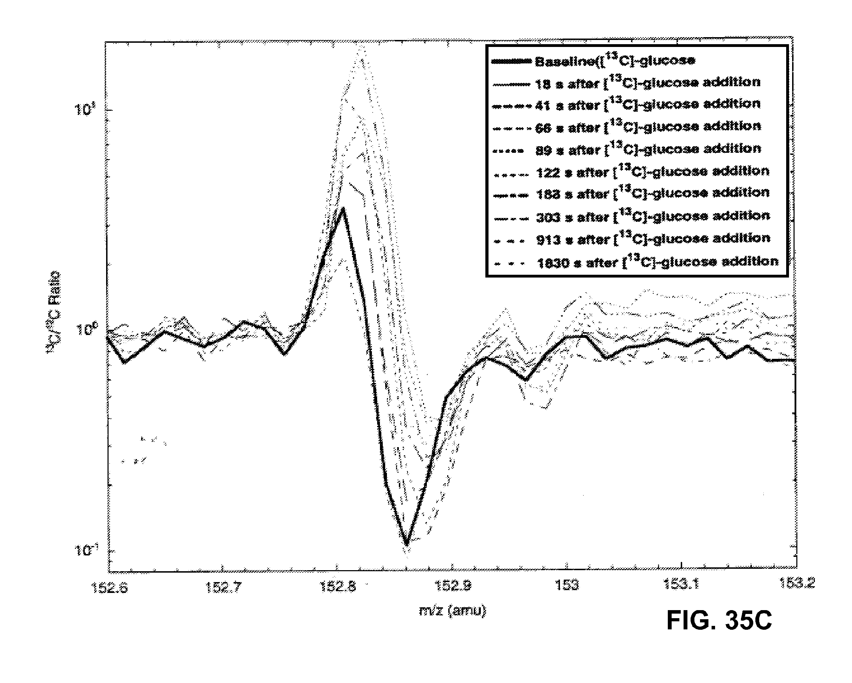

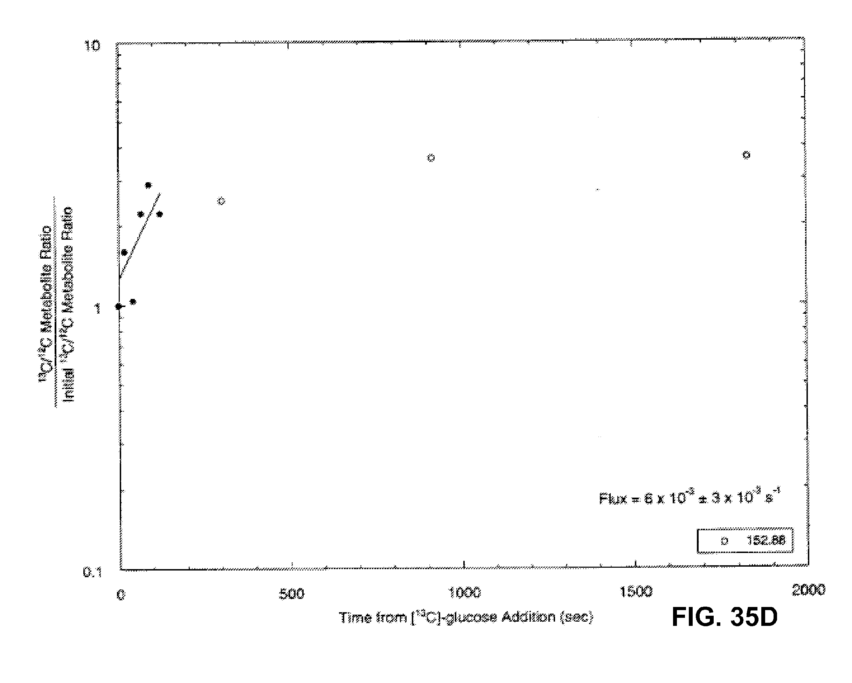

where $RA_{ss}$ was neglected. Only the metabolites at 150.87 amu (Figure 34) and 152.88 amu (Figure 35) were found to have metabolic flux values significantly different from zero and exhibited roughly equimolar peaks at both the $^{13}C$ and $^{12}C$ positions at long times. These mass positions are the most likely to correspond to real metabolites resulting directly from the six glucose carbons, and thus correspond to a metabolic $C_6$ fingerprint of glucose metabolism by *E. coli* at these growth conditions. The metabolic fluxes of the 150.87 and 152.88 amu metabolites were found to be similar at about 1 x $10^{-2}$ and 9 x $10^{-3}$ $s^{-1}AU_{600}^{-1}$, respectively.

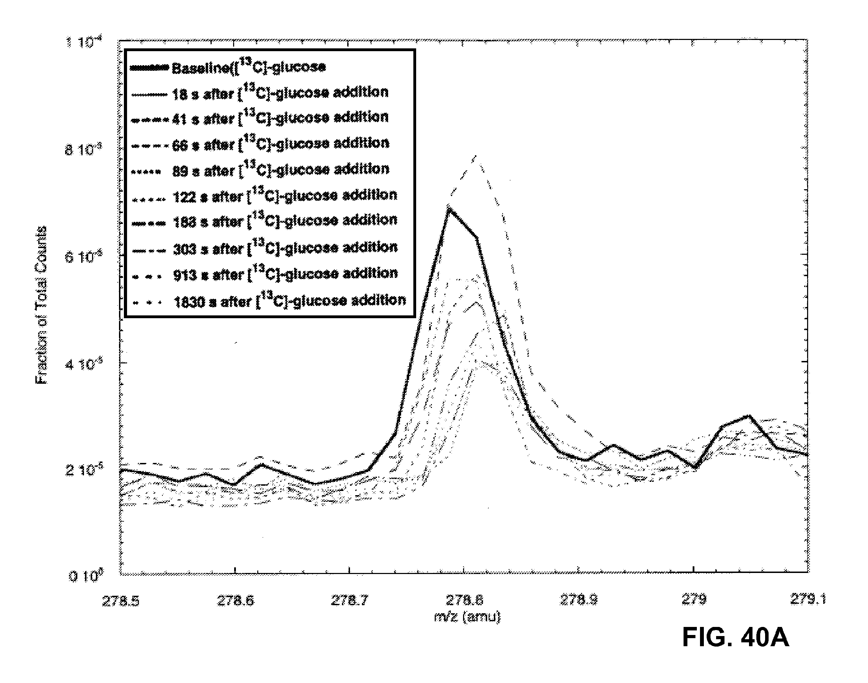

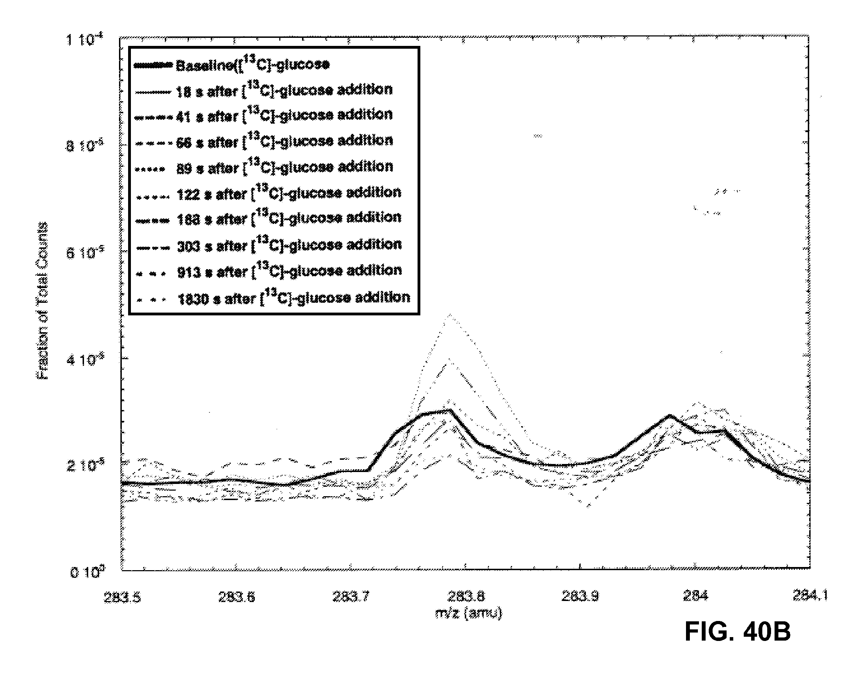

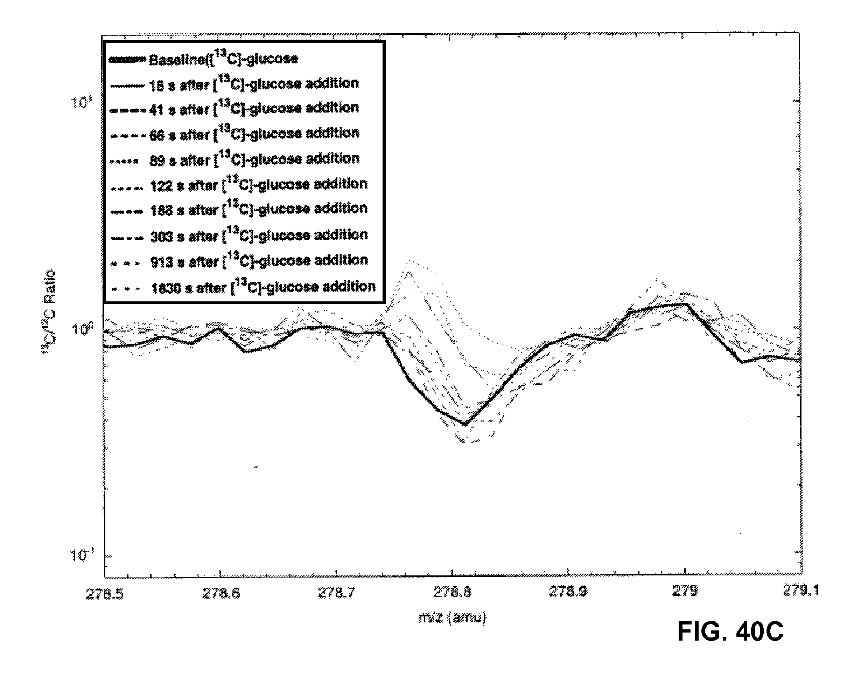

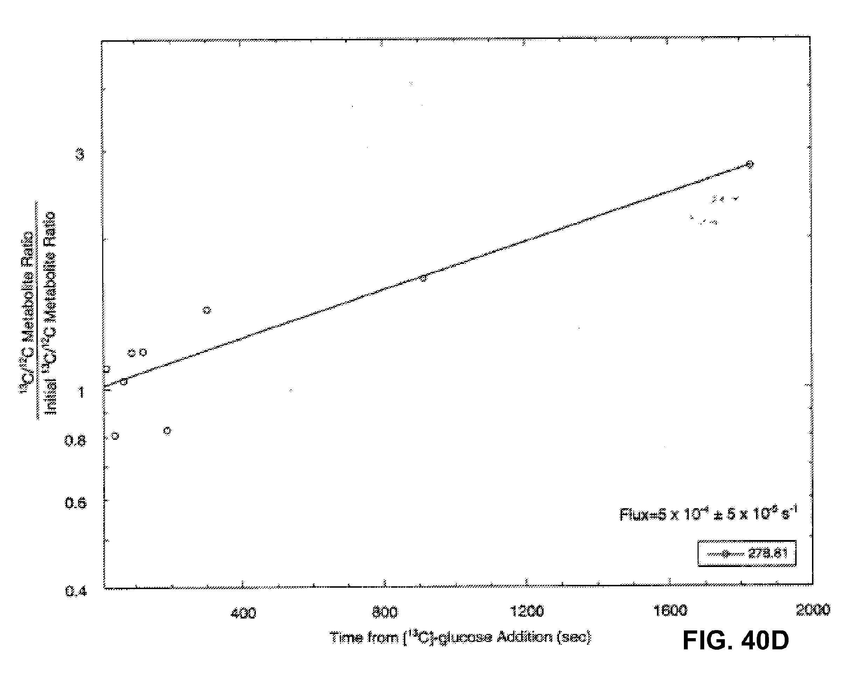

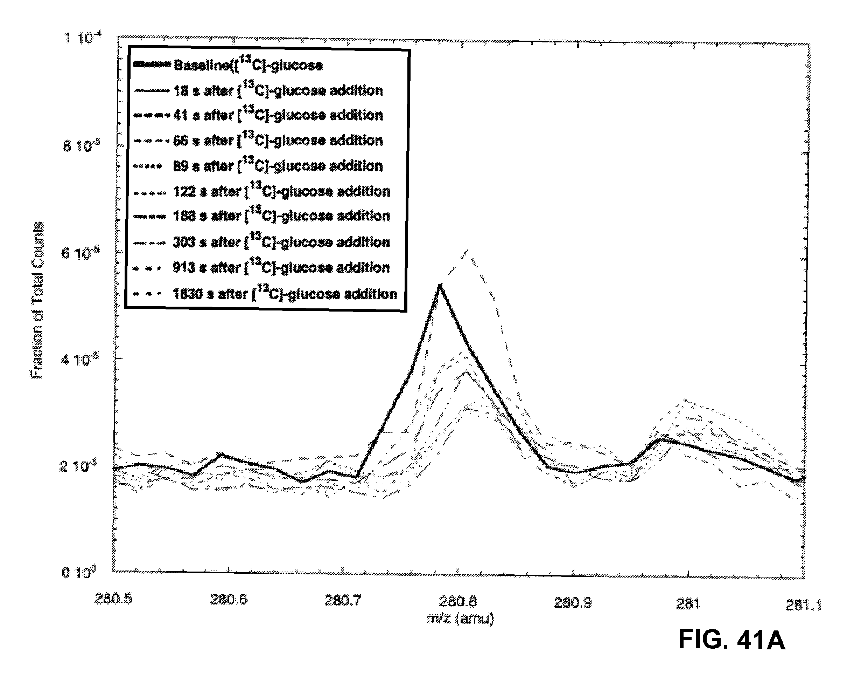

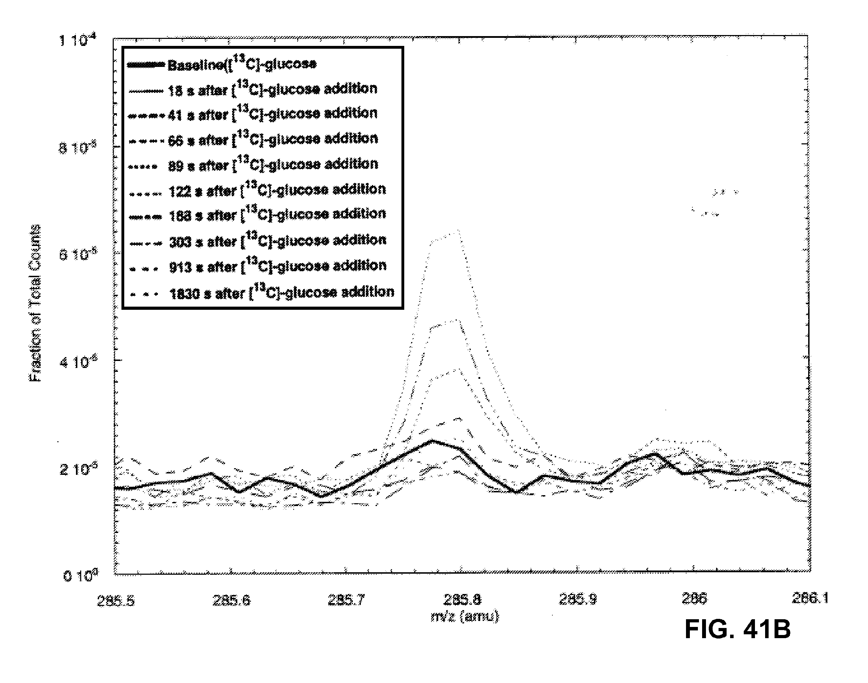

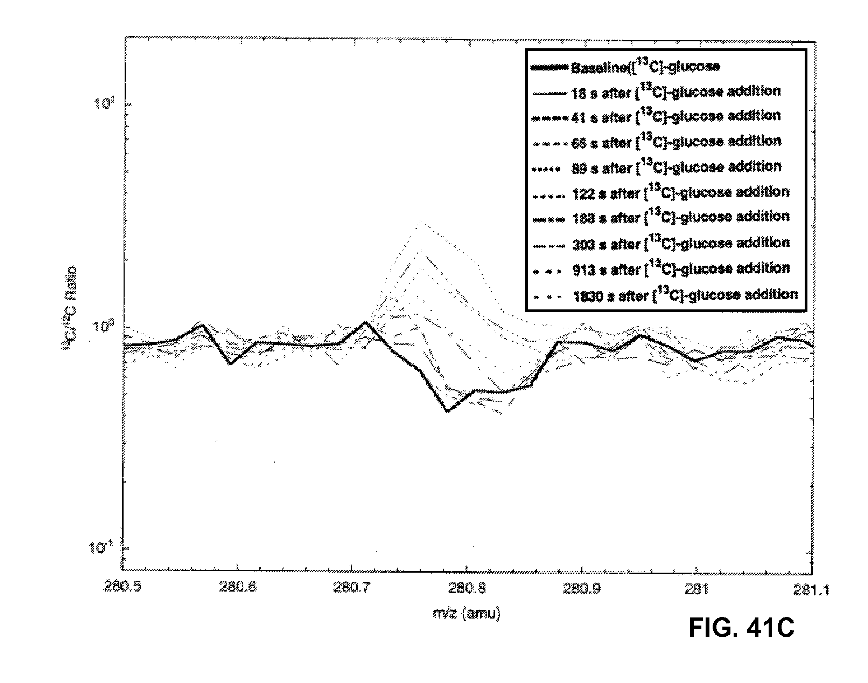

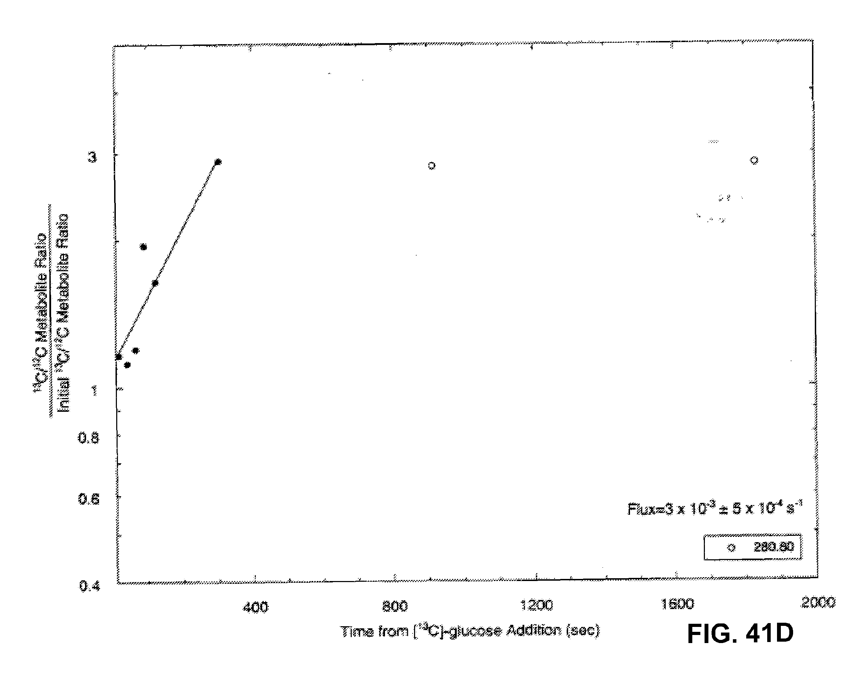

A similar analysis was conducted for five carbon metabolites, which suggests that two five carbon metabolites may also be derived from [$^{13}C$]-glucose metabolism (Figures 40 and 41). However, only in the long time points does the 278.81 amu metabolite exhibit any significant increase in $^{13}C$ content (Figure 40), suggesting that this peak may be an experimental artifact. The 280.80 amu metabolite appears to exhibit a real flux of about 4 x $10^{-3}$ $s^{-1}AU_{600}^{-1}$, about half of that observed for the six carbon metabolites. The chemical identities of these metabolites is unknown.

Similar analyses were conducted at the four, three, and two carbon metabolite levels with no apparent $^{13}C$-metabolites resulting from [$^{13}C$]-glucose being found in the mass spectral data.

It is understood that the examples and embodiments described herein are for illustrative purposes only and that various modifications or changes in light thereof will be suggested to persons skilled in the art and are to be included within the spirit and purview of this application and scope of the appended claims. All publications, patents, and patent applications cited herein are hereby incorporated by reference in their entirety for all purposes to the same extent as if each individual publication, patent or patent application were specifically and individually indicated to be so incorporated by reference.

REFERENCES

1. Kilár, F., "Isoelectric focusing in capillaries," in: CRC Handbook of Capillary Electrophoresis: A Practical Approach, Chp. 4, pgs. 95-109 (CRC Press, Boca Raton, FL, 1994).

2. Palmieri, R. and J. A. Nolan, "Protein capillary electrophoresis: Theoretical and experimental considerations for methods development," in: CRC Handbook of Capillary Electrophoresis: A Practical Approach, Chp. 13, pgs. 325-368 (CRC Press, Boca Raton, 1994).

3. Wanders, B.J. and F.M. Everaerts, "Isotachophoresis in capillary electrophoresis," in: CRC Handbook of Capillary Electrophoresis: A Practical Approach, Chp. 5, pgs. 111-127 (CRC Press, Boca Raton, FL, 1994).

4. Anderson, L. and J. Seilhamer, "A Comparison of Selected mRNA and Protein Abundances in Human Liver," Electrophoresis, 18:533 (1997).
5. Hochstrasser, D.F., et al., Anal Biochem., 173:424 (1988).
6. O'Farrell, P.H., J Biol. Chem., 250:4007 (1975).
7. Anderson, N.G. and N.L. Anderson, "Twenty years of two-dimensional electrophoresis: Past, present and future," Electrophoresis, 17:443 (1996).
8. Lopez, M.F., "2D Electrophoresis of Target Protein Groups and the Initiation of a Neurological Disease Database," paper presented at the IBC Proteomics conference, Coronado, CA (June 11-12, 1998).
9. Gottlieb, M. and M. Chavko, Anal. Biochem., 165:33 (1987).
10. Bio-Rad, "Detection of Proteins in SDS-PAGB: A comparison of gel staining methods, EG Bulletin 1820, Rev B (Bio-Rad Laboratories, Hercules, CA, 1996).
11. Schneider, L., "Metabolic uncoupling in Escherichia coli during phosphate limited growth," PhD Thesis, Department of Chemical Engineering, (Princeton University, Princeton, NJ, 1997).
12. Merril, C.R., Methods in Enzymology, 182:477 (1990).
13. Wilson, C.M., Methods in Enzymology, 91:236 (1983).
14. Lee, C., A. Levin and D. Branton, Anal. Biochem., 166:308 (1987).
15. Dzandu, J.K., J.F. Johnson and G.E. Wise, Anal. Biochem., 174:157 (1988).
16. Steinberg, Jones, Haugland and Singer, Anal. Biochem., 239:223 (1996).
17. Merril, C.R., N. Arold, D. Taube and W. Ehrhardt, Electrophoresis, 9:255 (1981).
18. Garfin, D.E., Methods in Enzymology, 182:425 (1990).
19. Laemmli, U. K., Nature, 227:680 (1970).
20. Corthals, G.L., M.P. Molloy, B.R. Herbert, K.L. Williams, and A.A. Gooley, "Prefractionation of protein samples prior to two-dimensional electrophoresis," Electrophoresis, 18:317 (1997).
21. Lopez, M.F., and W.F. Patton, "Reproducibility of polypeptide spot positions in two-dimensional electrophoresis of ribosomal and nuclear proteins," Electrophoresis, 18:338 (1997).
22. McKee, A., "The Yeast Proteome," paper presented at the IBC Proteomics conference, Coronado, CA (June 11-12, 1998).
23. Anderson, L., "Pharmaceutical Proteomics: Targets, mechanisms and function," paper presented at the IBC Proteomics conference, Coronado, CA (June 11-12, 1998).
24. Parekh, R.B., "Use of Proteomics in pre-clinical pharmaceutical research," paper presented at the IBC Proteomics conference, Coronado, CA (June 11-12,1998).
25. BioRad Molecular Imager FX and PDQuest 2-D analysis software seminar, presented at the IBC Proteomics conference, Coronado, CA (June 11-12, 1998).
26. Patton, W.F., "Defining protein targets for drug discovery using Proteomics," paper presented at the IBC Proteomics conference, Coronado, CA (June 11-12, 1998).
27. Ramsby, M., G. Makowski, and E. Khairallah, "Differential detergent fractionation of isolated hepatocytes: Biochemical, immunochemical, and two-dimensional gel electrophoresis characterization of cytoskeletal and noncytoskeletal compartments," Electrophoresis, 15:265 (1994).
28. Blomber, A., L. Biomberg, J. Norbeck, S.J. Fey, P. Mose-Larsen, M. Larsen, P. Roepstorff, H. Degand, M. Boutry, A. Posch and A. Görg, Electrophoresis, 16:1935 (1995).
29. Corbett, J.M., M.J. Dunn, A. Posch and A. Görg, Electrophoresis, 15:1205 (1994).
30. Beckman Instruments, "eCAP SDS 200: Fast, reproducible, quantitative protein analysis," BR2511B (Beckman Instruments, Fullerton, CA, 1993).
31. Anderson, N.L. et al., "An updated two-dimensional gel database of rat liver proteins useful in gene regulation and drug effects studies, Electrophoresis, 16:1997 (1995).
32. Franzén, F., S. Linder, A.A. Alaiya, E. Eriksson, K. Fujioka, A.-C. Bergman, H. Jömvall, G. Auer, "Analysis of polypeptide expression in benign and malignant human breast lesions," Electrophoresis, 18:582 (1997).
33. Guttman, A., J. A. Nolan and N. Cooke, "Capillary sodium dodecyl sulfate gel electrophoresis of proteins, J. Chromatogr., 632:171 (1993).
34. Clauser, K.R., "Managing high-throughput data acquisition and analysis in LC/MS/MS-based Proteomics," paper presented at the IBC Proteomics conference, Coronado, CA (June 11-12, 1998).
35. P/ACE™ Laser-induced fluorescence Detectors, BR-8118A (Beckman Instruments, Fullerton, CA, 1995).
36. Wilm, M. and Mann, M., "Analytical properties of the nanoelectrospray ion source," Anal. Chem., 68:1-8 (1996).
37. Steiner, S., "Proteome methods to profile mechanisms of toxicity," paper presented at the IBC Proteomics conference, Coronado, CA (June 11-12, 1998).
38. Amott, D., "Protein differential display and mass spectrometry in the study of congestive heart failure, paper presented at the IBC Proteomics conference, Coronado, CA (June 11-12, 1998).
39. Witzmann, F.A., C. D. Flutz, and J.F. Wyman, "Two-dimensional electrophoresis of precision-cut testis slices:

Toxicologic applications," Electrophoresis, 18:642 (1997).
40. Hjertén, S., J.-L. Liao and K. Yao, "Theoretical and experimental study of high-performance electrophoretic mobilization of isoelectrically focused protein zones, J. Chromatogr., 387:127 (1987).
41. Kim, K.W., J. Chromatogr., 559:401 (1991).
42. Satow, T. et al., "The effecto of salts on the separation ofbioactive peptides by capillary electrophoresis," J. High Resolut. Chromatogr., 14:276 (1991).
43. Shihabi, Z.K. and L. L. Garcia, "Effects of sample matrix on separation by Capillary electrophoresis," in: CRC Handbook of Capillary Electrophoresis: A Practical Approach, Chp. 20, pgs. 537-548 (CRC Press, Boca Raton, FL, 1994).
44. Garfin, D.E., Methods in Enzymology, 182:425 (1990).
45. Jorgenson, J.W. and K.D. Lukacs, "Zone electrophoresis in open-tubular glass capillaries: preliminary data on performance," J. High Resolut. Chromatogr. Commun., 4:230 (1981).
46. Jorgenson, J.W., and K.D. Lukacs, "Zone electrophoresis in open tubular capillaries," Anal. Chem., 53:1298 (1981).
47. Mc Cormick, R.M., "capillary zone electrophoresis of peptides," in: CRC Handbook of Capillary Electrophoresis: A Practical Approach, Chp. 12, pgs. 287-323 (CRC Press, Boca Raton, FL, 1994).
48. Aebersold, R., "Proteome analysis: Biological assay or data archive?," paper presented at the IBC Proteomics conference, Coronado, CA (June 11-12, 1998).
49. Cobb, K.A. and M. Novotny, "High-sensitivity peptide mapping by capillary zone electrophoresis and microcolumn liquid chromatography, using immobilized trypsin for protein digestion, Anal. Chem., 61:2226 (1989).
50. Cantor, C.R. and P.R. Schimmel, Biophysical Chemistry (W.H. Freeman & Co., NY, 1980).
51. Hjertén, S., "Free zone electrophoresis," *Chromatogr. Rev.,* 9:122 (1967).
52. Martinek, K., Goldmacher, V. S., Klibanov, A. M., and Berezin, I. V., "Denaturing agents (urea, acrylamide) protect enzymes against irreverisble thermoinactivation: A study with native and immobilized alpha-chymotrypsin and trypsin," FEBS Lett., 51:152-155 (1975).
53. Altria, K.D. and C.F. Simpson, "Measurement of electroendosmosis in high-voltage capillary electrophoresis," Anal. Proc., 23:453 (1986).
54. Camilleri, P. and G.N. Okafo, "Replacement of H2O by D2O in capillary zone electrophoresis can increase resolution of peptides and proteins, "J. Chem. Soc. Chem. Commun., 3:196 (1991).
55. Camilleri, P., G.N. Okafo, C. Southan, and R. Brown, "Analytical and micropreparative capillary electrophoresis of the peptides from calcitonin," Anal. Biochem., 198:36 (1991).
56. Okafo, G.N. and P. Camilleri, "Capillary electrophoretic separation in both H2O and [2H]2O-based electrolytes can provide more information on tryptic digests, J. Chromatogr., 547:551 (1991).
57. Schwer, C. and F. Lottspeich, "Analytical and micropreparative separation of peptides by capillary zone electrophoresis using discontinuous buffer systems," J. Chromatogr., 623:345 (1992).
58. Foret, F., E. Szoko and B.L. Karger, "On-column transient and coupled column isotachophoretic preconcentration of protein samples in capillary zone electrophoresis," J.Chromatogr., 608:3 (1992).
59. Lowry, O., N. Rosebrough, A. Farr and R. Randall, J. Biol. Chem., 193:265-275 (1951).
60. Anderson, N.L., "Pharmaceutical Proteomics: Targets, mechanism, and function," paper presented at the IBC Proteomics conference, Coronado, CA (June 11-12, 1998).
61. Meng, C.K., M. Mann and J.B. Fenn, Z. Phy. D: Atoms, Mol. Clusters, 10:361-368(1988).
62. Karas, M. and F. Hillenkamp, Anal. Chem. 60:2299 (1988).
63. Hillenkamp, F., M. Jaras, R.C. Beavis and B.T. Chait, Anal. Chem. 63:1193A (1991).
64. Beavis, R.C. and B.T. Chait, "Matrix-assisted laser desorption mass spectrometry of proteins," reprint, http: //www.proteometrics.com/methods/contents.htm (1994).
65. Clauser, K.R., S.C. Hall, D.M. Smith, J.W. Webb, L.E. Andrews, H.M. Tran, L.B. Epstein, and A.L.Burlingame, Proc. Natl. Acad. Sci (USA), 92:5072-5076 (1995).
66. Li, G., M. Walthan, N.L. Anderson, E. Unworth, A. Treston and J.N. Weinstein, "Rapid mass spectrometric identification of proteins from two-dimensional polyacrylamide gels after in gel proteolytic digestion," Electrophoresis, 18:391-402 (1997).
67. Stevens, F.J., "Method of electric field flow fractionation wherein the polarity of the electric field is periodically reversed," US Patent No. 5133844, (July 28, 1992).
68. Gupta N.R., Nadim A., Haj-Hariri H., Borhan A., "Stability of the Shape of a Viscous Drop under Buoyancy-Driven Translation in a Hele-Shaw Cell, " J Colloid Interface Sci, 222(1):107-116 (2000).
69. Sanger, F., Biochem. J., 39:507 (1945).
70. Creighton, T. E., Proteins: Structures and Molecular Principles (W. H. Freeman, NY, 1984).
71. Niederwieser, A., "Thin-layer chromatography of amino acids and derivatives," in: Methods in Enzymology, 25: 60-99 (1972).

72. Hirs, C.H.W., M. Halmann and J.H. Kycia,"Dinitrophenylation and inactivation of bovine pancreatic ribonuclease A," Arch. Biochem. Biophys., 111:209-222 (1965),

73. Gray, W. R., "End-group analysis using dansyl chloride," in: Methods in Enzymology, 25:121-137 (1972).

74. Stark, G. R., "Use of cyanate for determining NH2-terminal residues in protein," in: Methods in Enzymology, 25: 103-120 (1972),

75. Niall, H. D., "Automated Edman degradation: the protein sequenator," in: Methods in Enzymology, 27:942-1011 (1973).

76. Galella, G. and D. B. Smith, "The cross-linking oftubulin with immidoesters," Can. J. Biochem., 60:71-80 (1982).

77. Lomant, A.J. and G. Fairbanks, "Chemical probes of extended biological structures: synthesis and properties of the cleavable protein crosslinking reagent 35S.dithiobis(succinimidyl propionate), J. Mol. Biol., 104:243-261 (1976).

78. Solomons, T.W.G, Organic Chemistry (John Wiley & Sons, NY, 1976).

79. Novotny et al., Anal. Chem., 63:408 (1991).

80. Novotny et al., J. Chromatography, 499:579 (1990).

81. Merrifield, B., Science, 232:341-347 (1986).

82. Horton, H. R. and D. E. Koshland, Jr., Methods in Enzymology, 25:468 (1972).

83. Yamada, H., Imoto, T., Fujita, K., Okazaki, K. and M. Motomura, "Selective modification of aspartic acid-101 in lysozyme by carbodiimide reaction," Biochem., 20:4836-4842.

84. Grabarek, Z. and J. Gergely, "Zero-length crosslinking procedure with the use of active esters," Anal. Biochem, 185:131-135 (1990).

The present invention will now be described by way of reference to the following clauses:

1. A method for separating a polypeptide species from a sample solution containing a plurality of polypeptide species and identifying said polypeptide species, the methods comprising electrophoresing said sample solution containing a plurality of polypeptide species in a capillary electrophoresis device to separate and elute polypeptide species thereby resolving said protein species based on at least one first biophysical parameter which discriminates protein species; and obtaining, by mass spectrographic fragmentation of eluted polypeptide species, a polypeptide sequence tag ("PST") identifying at least one resolved protein species.

2. The method of clause 1, wherein the method further comprises electrophoresing polypeptide species eluted from said capillary electrophoresis device in a second capillary electrophoresis device to separate and elute polypeptide species thereby resolving said protein species based on at least one second biophysical parameter which discriminates protein species, prior to performing mass spectrographic fragmentation on the polypeptide species thereby obtained.

3. The method of clause 1, wherein the capillary Electrophoresis device is a capillary isoelectric focusing (CIEF) device, a capillary zone Electrophoresis device (CZE), or a capillary gel electrophoresis device (CGE).

4. The method of clause 2, wherein the capillary electrophoresis device is a CIEF device and the second capillary electrophoresis device is either a CZE device or a CGE device.

5. The method of clause 2, wherein the capillary electrophoresis device is a CZE device and the second capillary electrophoresis device is either a CIEF device or a CGE device.

6. The method of clause2, wherein the capillary electrophoresis device is a CGE device and the second capillary electrophoresis device is either a CIEF device or a CZE device.

7. The method of clause 2, wherein the method further comprises electrophoresing polypeptide species eluted from said second capillary electrophoresis device in a thirdcapillary electrophoresis device to separate and elute polypeptide species thereby resolving said protein species based on at least one third biophysical parameter which discriminates protein species, prior to performing mass spectrographic fragmentation on the polypeptide species thereby obtained.

8. The method of clause 7, wherein the the capillary electrophoresis device is a CIEF device and the second capillary electrophoresis device is either a CZE device and the third capillary electrophoresis device is a CGE device.

9. The method of clause 1, wherein the sample solution containing a plurality of polypeptide species comprises labeled polypeptide species.

10. The method of clauses 1, 2, and 7 wherein the polypeptide species are labeled after capillary electrophoresis and prior to mass spectroscopy.

11. The method of clauses9 and 10, wherein the label comprises a detectable moiety.

12. The method of clauses9 and 10, wherein the label comprises an ion mass signature component.

13. The method of clauses9 and 10, wherein the label comprises an ion mass signature component and a detectable moiety.

14. A method for identifying a high-resolution protein expression fingerprint for a cell type, tissues, or pathological sample, comprising obtaining a protein-containing extract of a cellular sample and electrophoresing said extract with a first capillary electrophoresis apparatus, eluting protein-containing factions therefrom, electrophoresing said protein containing fractions on a second capillary electrophoresis apparatus, or plurality thereof is parallel, and identifying the species of proteins by fragmentation mass spectroscopy sequencing to obtain PSTs for a plurality of protein species, and compiling a dataset or fingerprint record containing the collection of PST obtained thereby.

15. The method of clause14, comprising quantitative detection of protein species and compiling a dataset wherein the relative abundance and/or absolute amount of a plurality of protein species eluted from said second capillary electrophoresis are cross-tabulated with the PST identification.

16. A computer system comprising: a database including a plurality of fingerprint records each comprising an array of at least 50 molecular species each having a unique identifier cross-tabulated with quantitative data indicating relative and/or absolute abundance of each species in a sample, and a user interface capable of receiving a selection of one or more queries to said database for use in determining a rank-ordered similarity of fingerprint records in the database.

17. A computer system of clause 16 having a fingerprint record comprising an array of at least 50 protein species each having a PST cross-tabulated with a separation coordinate produced by the method of claim 1.

18. A computer system of clause16 having a fingerprint record comprising an array of at least 50 protein species each having a PST obtained by the method of claim 1.

19. A method for producing or accessing a computer database comprising a computer and software for storing in computer-retrievable form a collection of protein expression fingerprint records cross-tabulated with data specifying the source of the protein-containing sample from which each protein expression fingerprint record was obtained.

20. The method of clause19, wherein at least one of the sources is from a tissue sample known to be free of pathological disorders.

21. The method of clause 19, wherein at least one of the sources is a known pathological tissue specimen.

22. A method of labeling a plurality of different proteins in a protein sample, said method comprising contacting said protein sample with a labeling agent comprising a unique ion mass signature component, a quantitative detection component and a reactive functional group to covalently attach a label to at least a portion of said plurality of different proteins.

23. A method in accordance with clause 22, wherein said protein sample comprises at least five different protein.

24. A method in accordance with clause 22, wherein said detection enhancement component is a fluorophore selected from the group consisting of naphthylamines, coumarins, acridines, stilbenes and pyrenes.

25. A method in accordance with clause 22, wherein said detection enhancement component is a fluorophore selected from the group consisting of 1-dimethylaminonaphthyl-5-sulfonate, 1-anilino-8-naphthalene sulfonate, 2-p-toluidinyl-6-naphthalene sulfonate, 3-phenyl-7-isocyanatocoumarin, 9-isothiocyanatoacridine, acridine orange, N-(p-(2-benzoxazolyl)pheny)maleimide, and benzoxadiazoles.

26. A method for separating a plurality of proteins in an initial sample, comprising
performing a plurality of electrophoretic methods in series, each method comprising
electrophoresing a sample containing multiple proteins, whereby a plurality of resolved proteins are obtained, and wherein the sample electrophoresed contains only a subset of the plurality of resolved proteins from the immediately preceding method in the series, except the first method of the series in which the sample is the initial sample; and
detecting resolved proteins from the final Electrophoretic method.

27. The method of clause26, wherein the plurality of electrophoretic methods are capillary electrophoresis methods.

28. The method of clause27, wherein, the plurality of capillary electrophoretic methods are selected from the group consisting of capillary isoelectric focusing electrophoresis, capillary zone electrophoresis and capillary gel electrophoresis.

29. The method of clause27, wherein the plurality of capillary electrophoretic methods are two methods, the first electrophoretic method being capillary isoelectric focusing electrophoresis and the second electrophoretic method being capillary gel electrophoresis.

30. The method of clause27, wherein the plurality of capillary electrophoretic methods are two methods, the first electrophoretic method being capillary zone electrophoresis and the second electrophoretic method being capillary gel electrophoresis.

31. The method of clause 27, wherein the plurality of capillary electrophoretic methods are three methods, the first, second and third electrophoretic methods being capillary isoelectric focusing electrophoresis, capillary zone electrophoresis and capillary gel electrophoresis, respectively.

32. The method of clause 26, wherein
the performing further comprises repeating the electrophoresing step multiple times, each time with a different sample containing only a subset of the plurality of resolved proteins from the immediately preceding method in the series, whereby a plurality of resolved proteins for each of the different samples is obtained; and
the detecting comprises detecting resolved proteins from each of the different samples from the final electrophoretic method.

33. A method for separating a plurality of proteins, comprising
performing a plurality of electrophoretic methods in series, wherein the method or methods preceding the final method comprise
withdrawing and collecting multiple fractions containing proteins resolved during the electrophoretic method, and wherein each electrophoretic method is conducted with a sample from a fraction collected in the preceding electrophoretic method, except the first electrophoretic method which is conducted with a sample containing the plurality of proteins;
labeling the plurality of proteins or labeling protein contained in collected fractions prior to conducting the last electrophoretic method; and
detecting protein contained in electrophoretic medium utilized during a final electrophoretic method by detecting label borne by the protein, the final electrophoretic method being performed with a sample from one or more fractions obtained in the penultimate electrophoretic method.

34. The method of clause33, wherein the detecting step comprises detecting protein with a detector in fluid communication with a separation cavity containing the electrophoretic medium utilized during the final electrophoretic method.

35. The method of clause 33, wherein one of the plurality of electrophoretic methods is capillary zone electrophoresis and the labeling step is conducted prior to conducting the capillary zone electrophoresis method.

36. The method of clause33, wherein one of the plurality of electrophoretic methods is capillary isoelectric focusing and the labeling step is performed subsequent to the capillary isoelectric focusing method.

37. A method for separating a plurality of proteins, comprising
performing one or more capillary electrophoretic methods, each of the one or more methods comprising
electrophoresing a sample containing multiple proteins within an electrophoretic medium contained within a capillary;

and

withdrawing and collecting multiple fractions, each fraction containing proteins resolved during the electrophoresing step, and

wherein each method is conducted with a sample from a fraction collected in the preceding electrophoretic method, except the first electrophoretic method which is conducted with a sample containing the plurality of proteins;

labeling the plurality of proteins or labeling protein contained in collected fractions prior to conducting the last electrophoretic method; and

conducting a final capillary electrophoresis method with a final capillary,

the final method comprising detecting resolved protein within, or withdrawn from, the final capillary.

38. The method of clause37, wherein the one or more capillary electrophoresis methods is capillary isoelectric focusing electrophoresis and the final capillary electrophoresis method is capillary gel electrophoresis.

39. The method of clause37, wherein the one or more capillary electrophoresis methods is capillary zone electrophoresis and the final capillary electrophoresis method is capillary gel electrophoresis.

40. The method of clause37, wherein the one or more capillary electrophoresis methods is two methods, the first method being capillary isoelectric focusing and the second method being capillary zone electrophoresis, and the final capillary electrophoresis method is capillary gel electrophoresis.

41. A method for separating a plurality of proteins in an initial sample, comprising:

performing a plurality of electrophoretic methods in series, each method comprising

electrophoresing within an electrophoretic medium a sample containing multiple proteins whereby fractions containing a subset of the multiple proteins are isolated physically, temporally or spatially, and

wherein the sample electrophoresed is obtained from a fraction isolated during the immediately preceding method in the series, except the first method of the series in which the sample is the initial sample; and

detecting isolated proteins from the final electrophoretic method.

42. A method for separating a plurality of proteins, comprising performing at least two capillary electrophoretic separations in series, wherein a sample for the second capillary electrophoretic separation is from fraction obtained during the first capillary electrophoretic separation, the fraction containing only a subset of the plurality of proteins contained in the sample electrophoresed during the first capillary electrophoretic method.

43. A method for analyzing metabolic pathways, comprising:

administering to a subject a substrate labeled with a stable isotope, wherein the relative isotopic abundance of the isotope in the substrate is known;

allowing the labeled substrate to be at least partially metabolized by the subject to form one or more target metabolites; and

determining the abundance of the isotope in a plurality of target analytes in a sample from the subject to determine a value for the flux of each target analyte, wherein the plurality of target analytes comprise the substrate and/or one or more of the target metabolites.

44. The method of clause43, wherein the determining comprises at least partially separating the target analytes from other biological components in the sample prior to determining the flux values.

45. The method of clause44, wherein the separating comprises performing a plurality of capillary electrophoresis methods in series.

46. The method of clause45, wherein the performing of the capillary electrophoresis methods generate separate fractions for at least one class of metabolite, wherein the class of metabolite is selected from the group consisting of proteins, polysaccharides, carbohydrates, nucleic acids, amino acids, nucleotides, nucleosides, fats, fatty acids and organic acids.

47. The method of clause43, wherein the determining comprises obtaining multiple samples from the subject at different predetermined time points, separating the target analytes from other biological components in each of the samples, and determining the abundance of the isotope in the target analytes contained in each sample, whereby a plurality of values for the abundance of the isotope in each target analyte are obtained, the flux value for each target analyte being determined from the plurality of abundance values determined for it.

48. A method for analyzing metabolic pathways, comprising:

separating at least partially a plurality of target analytes from biological components contained in a sample obtained from a subject, the target analytes comprising a substrate labeled with a stable isotope and/or one or more target metabolites resulting from the metabolism of the substrate by the subject, and wherein the relative isotopic abundance of the isotope in the substrate is known; and
determining the abundance of the isotope in a plurality of the target analytes in the sample to determine a value for the flux of each target analyte.

49. A method for screening for metabolites correlated with a disease, comprising:

analyzing a sample from a test subject having the disease, the sample comprising a substrate labeled with a stable isotope administered to the test subject and/or one or more target metabolites resulting from metabolism of the substrate by the test subject, the relative isotopic abundance of the isotope in the substrate known at the time of administration, and wherein the analyzing step comprises determining the isotopic abundance of the isotope in a plurality ofanalytes in the sample to determine a value for the flux of each analyte, wherein the plurality of analytes comprise the substrate and/or one or more of the target metabolites; and

comparing flux values for the analytes with flux values for the same analytes obtained for a control subject, wherein a difference in a flux value for an analyte indicates that such analyte is correlated with the disease.

50. A method for screening for the presence of a disease, comprising:

analyzing a sample from a test subject, the sample comprising a substrate labeled with a stable isotope administered to the test subject and/or one or more target metabolites resulting from metabolism of the substrate by the test subject, the relative isotopic abundance of the isotope in the substrate known at the time of administration, and wherein the analyzing step comprises determining the abundance of the isotope in a plurality of analytes in the sample to determine a value for the flux of each analyte, wherein the plurality of analytes comprise the substrate and/or one or more of the target metabolites; and
for each target analyte, comparing the determined flux value with a range of flux values for that analytes, wherein the range is known to be correlated with the disease and if a determine flux value for a target analyte falls within the range for that target analytes, it indicates that the test subject has the disease or is susceptible to the disease.

51. An apparatus for performing a method of clauses1, 14, 22, 26, 33, 37, 41, 43, 48, 49, or 50, comprising:

at least two capillary electrophoresis devices fixed to a common platform or frame and in liquid communication with each other and with a mass spectrometer wherein a sample flows into a first capillary electrophoresis device and separation of analytes occurs based on at least a first biochemical separation parameter and the analytes subsequently flow into a second capillary electrophoresis device and separation of analytes occurs based on at least a second biochemical separation parameter which is different than said first biochemical separation parameter and wherein the analytes subsequently flow into a mass spectrometer.

52. The use of a method of clauses 1, 14, 19, 22, 26, 33, 37, 41, 43, 48, 49, or 50, or the apparatus of claim 51, or of the computer system or database of claim 16.

## Claims

**1.** A method for analyzing a metabolic pathway, comprising:

(a) administering to a subject a known ratio of a labeled substrate and an unlabeled substrate, wherein said labeled substrate is a substrate labeled with a stable isotope and said unlabeled substrate is said substrate having a natural abundance of said stable isotope;

(b) allowing the labeled substrate and the unlabeled substrate to be at least partially metabolized by the subject to form a labeled target metabolite and an unlabeled target metabolite;
(c) generating mass spectral data from a plurality of samples from said subject at a plurality of time points; and
(d) determining a plurality of relative abundances of said labeled target metabolite to said unlabeled target metabolite from the mass spectral data of at least one of said samples and comparing said plurality of relative abundances to said known ratio of said labeled substrate and said unlabeled substrate.

**2.** The method of claim 1, further comprising at least partially separating the labeled target metabolites and the unlabeled target metabolites from other biological components in said at least one of said samples prior to determining the plurality of relative abundances.

**3.** The method of claim 2, wherein the separating comprises performing a plurality of capillary electrophoresis methods in series.

**4.** The method of claims 3, wherein the plurality of capillary electrophoresis methods are selected from the group consisting of capillary zone electrophoresis, capillary isoelectric focusing and capillary gel electrophoresis.

**5.** The method of claim 4, wherein the plurality of capillary electrophoresis methods are selected from the group consisting of capillary zone electrophoresis and capillary isoelectric focusing.

**6.** The method of claim 5, wherein the performing of the capillary electrophoresis methods comprises performing a plurality of capillary zone electrophoresis methods.

**7.** The method of claim 3, wherein the performing of the capillary electrophoresis, methods generate separate fractions for at least one class of metabolite, wherein the class of metabolites is selected from the group consisting of proteins, polysaccharides, carbohydrates, nucleic acids, amino acids, nucleotides, nucleosides, fats, fatty acids and organic acids.

**8.** The method of claim 3, wherein the separating comprises conducting a non-electrophoretic separation technique prior to conducting the plurality of electrophoresis methods to precipitate at least some of the biological components.

**9.** The method of claim 1, wherein the stable isotope is selected from the group consisting of $^{13}C$, $^{2}H$, $^{15}N$, $^{18}O$ and $^{34}S$.

**10.** The method of claim 1, wherein the substrate is selected from the group consisting of proteins, carbohydrates, nucleic acids, amino acids, nucleotides, nucleosides, fatty acids, organic acids, and fats.

**11.** The method of claim 10, wherein the substrate is a protein.

**12.** The method of claim 1, wherein the plurality of samples are obtained from a bodily fluid, the bodily fluid selected from the group consisting of blood, urine, cerebral fluids, spinal fluid, sweat, and gastrointestinal fluids.

**13.** The method of claim 1, wherein the plurality of samples are cells, tissue samples or fecal materials.

50
52
54
56
58
60
62
64
66
68
70
72
74
76
78
80
82

FIG. 1

32
8
28
26
30
10
12
24
24
18
20
14
16

FIG. 2A

100
104
106
102

FIG. 2B

150
158
154
152
160
164
170
156
162
166
168

FIG. 3A

180
174c
176c
172c
158
154
152
156
160
170
162
172a
172b
166
176a
176b
168
174a
174b

FIG. 3B

EOF
Repetitive Runs
Proteins
Time (min)
Absorbance (μAU)
0
20
40
60
80
2.5 10^5
2 10^5
1.5 10^5
1 10^5
5 10^4

FIG. 4

Conalbumen r1
Conalbumen r2
Carbonic Anhydrase r1
Carbonic Anhydrase r2
Bovine Serum Albumen r1
Bovine Serum Albumen r2
GAPDH r1
GAPDH r2
Ribonuclease A r1
Ribonuclease A r2
EOF
Absorbance ( AU)
$10^6$
$10^5$
$10^4$
1000
0
0.0002
0.0004
0.0006
0.0008
0.001
Electrophoretic Mobility ($s^{-1}$)

FIG. 5

Electrophoretic Mobility (s$^{-1}$)
0.00068
0.00066
0.00064
0.00062
0.0006
0.00058
Mass/Charge (Da)
860
880
900
920
940
960

FIG. 6

Fraction=1
2
3
4
5
6
7
8
9
10
SPITC-GAPDH
SPITC-Ribonuclease A
SPITC-Conalbumin
SPITC-Bovine Serum Albumin
SPITC-Carbonic Anhydrase
Time (min)
0
100
200
300
400
500
600
700
50000
40000
30000
20000
10000
0

FIG. 7

Current (pA)
2.5 10^7
2 10
1.5 10^7
1 10^7
5 10^6
0
Absorbance ( AU 214)
6 10^5
5 10^5
4 10^5
3 10^5
2 10^5
1 10^5
0
-1 10^5
Time (min)
0
50
100
150
200
250
A
B
C
D
E
F
G
Region:
A= Focusing (No Elution)
B= Fraction 1
C= Fraction 2
D= Fraction 3
E= Fraction 4
F= Fraction 5
G= Fraction 6

FIG. 8

2 10^5
1.5 10^5
1 10^5
50000
0
-50000
Absorbance ( AU 214)
Bovine Serum Albumin (69.3 kDa)
Conalbumin (75.7 kDa)
Orange G
Reference Marker
0.7
0.75
0.8
0.85
0.9
0.95
1.05
Relative Migration Time-1

FIG. 9

Carbonic Anhydrase (29.0 kDa)
Orange G
Reference Marker
Absorbance ( AU 214)
2 10 6
1.5 10 6
1 10 6
50000
0
-50000
0.7
0.75
0.8
0.85
0.9
0.95
1
1.05
Relative Migration Time -1

FIG. 10

Intensity
2000
1500
1000
500
0
500
1000
1500
2000
2500
3000
3500
4000
Mass (m/z)
% Intensity

FIG. 11

aceytl-SRPSLDQ
a+b$_1$ ions
a+b$_2$ ions
a+b$_3$ ions
a+b$_4$ ions
a+b$_5$ ions
a+b$_6$ ions
a+b$_7$ ions
Fraction of Total Counts (%)
0.1
0.01
0.001
0.0001
Nozzle Potential (V)
0
50
100
150
200
250
300

FIG. 12

Counts
5000
4000
3000
2000
1000
M/Z (amu)
0
500
1000
1500
2000
2500
3000
3500
4000

FIG. 13

a1
a2
a3
a4
a5
a6
a7
a8
2+
b1
b2
b3
b4
b5
b6
b7
b8
12 V Nozzle Potential
PTCRPPGFSPFR
1+
Relative Abundance (%)
0
20
40
60
80
100
M/Z (amu)
200
400
600
800
1000
1200
1400

FIG. 14

Fraction of Total Counts (%)
1
0.1
0.01
0.001
Nozzle Potential (V)
0
50
100
150
200
250
300
a+b1
a+b2
a+b3
a+b4
a+b5
a+b6
a+b7
a+b8
Total

FIG. 15

250 V Nozzle Potential
PTC-RPPGFSPFR
$2^+$
$1^+$
$a_1$ $b_1$ $a_2$ $b_2$ $a_3$ $b_3$ $a_4$ $b_4$ $a_5$ $b_5$ $a_6$ $b_6$ $a_7$ $b_7$ $a_8$ $b_8$
Relative Abundance (%)
0
20
40
60
80
100
M/Z (amu)
200
400
600
800
1000
1200
1400

FIG. 16

Counts
Nozzle Potential (V)
a1
a2
a3
a4
a5
a6
a7
a8
Total Counts
RPPGFSPFR
HN
NH
S
O

FIG. 17

Counts
Nozzle Potential (V)
10
100
1000
$10^4$
$10^5$
$10^6$
$10^7$
50
100
150
200
250
300
350
400
b1
b2
b3
b4
b5
b6
b7
b8
Total Counts
RPPGFSPFR
HN
NH
NH
S
O

FIG. 18

Label-ion
$a_1+b_1$-ions
$a_2+b_2$-ions
$a_3+b_3$-ions
$a_4+b_4$-ions
GLSDG...
Nozzle Potential (V)
Fraction of Total Counts (%)
100
150
200
250
300
10
1
0.1
0.01
0.001
0.0001

FIG. 19

Fraction of Total Counts (%)
0.1
0.01
0.001
0.0001
100
150
200
250
300
Nozzle Potential (V)
$a_1$-ion
$b_1$-ion
$a_2$-ion
$b_2$-ion
$a_3$-ion
$b_3$-ion


FIG. 20

Fraction of Total Counts (%)
Nozzle Potential (V)
$10^{-1}$
$10^{-2}$
$10^{-3}$
$10^{-4}$
$10^{-5}$
$10^{-6}$
0
50
100
150
200
250
300
$y_{label}$
$y_1^{+2}$
$y_2^{+2}$
$y_3^{+2}$
$y_4^{+2}$
$y_5^{+2}$
$y_6^{+2}$
$y_7^{+2}$
$y_8^{+2}$
RPPGFSPFR
HN
N
$CH_3$
$CH_3$
$CH_3$

FIG. 21

Unique Ion Mass Constituent for Mass Spectrometer
ineffeciently ionized at protein ionization conditions
efficiently ionized at protein ionization conditions
Quantitative Detection Constituent
Radioisotope
Fluorescent
Chromaphor
Reactive Constituent
functional group reactive to primary amines
functional group reactive to carboxylic acids

FIG. 22

fluoro-2,4-dinitrobenezene
dansyl chloride
sodium cyanate
phenylisothiocyanate

FIG. 23

Protein Extract
Label with Covalent Chemical Label
Separate Labelled Proteins by Electrophoresis
• one or two dimensional depending on number of proteins in sample
• capillary or gel electrophoresis depending on label used
Determine Complete or Partial Protein Sequence of Separated Labelled Proteins by Mass Spectrometry
• electrospray if separated by capillary electrophoresis
• MALDI if separated by gel electrophoresis

FIG. 24

FIG. 25

phn
operon
phnC-P
ori
phoE
phoA
phoB
phoR
phoBR
operon
pstS
pstC
pstA
pstB
phoU
pst
operon
creB (phoM)
cre
operon
ugpB
ugpA
ugpE
ugpC
ugpQ
ugp
operon
E. coli Genome
phoH
phoQ
phoP
phoQ
operon

FIG. 26

Alkaline Phosphatase
E. coli K-12 (WT)
APASE
PROTEIN
Alkaline Phosphatase Specific Synthesis Rate (Units/AU-h)
Specific Protein Synthesis Rate (μM SO 4/AU-h)
Time (h)
1300
1000
800
600
400
300
0
60
50
40
30
20
10
0
5
10
15
20
25

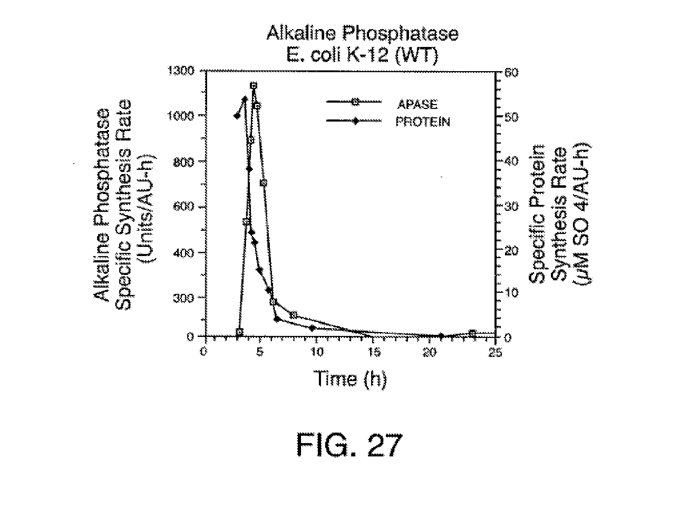

FIG. 27

Phosphate Starvation Response
E. coli K-12 (WT)
100
80
60
40
20
0
Apparent MW (kDa)
EXP 0 5 10 15 20 25 30 35 40 45 50 55 60 65 70 75 80 85
Time from Starvation Onset (h)

FIG. 28

Singly charged peptide fragments
Multiply charged protein peaks
100
90
80
70
60
50
40
30
20
10
0
796.0
996.2
1110.6
1676.4
3876.8
2000.0
156.6
696.2
1116.4
1676.6
3638.8
2000.0

FIG. 29

(CH3CH2)2HN⊕
SO2NH(CH2)5
(CH3)2HN⊕
H3N⊕
⊕NH5
COOH
N
H⊕
CH3CH2NH2⊕
⊕NHCH2CH3
COOH
(CH3)2HN⊕
⊕N(CM3)2
COOH
HN⊕
N⊕
COOH

FIG. 30

FIG. 30 (cont.)

R=H OR F

R=H OR F

FIG. 31

FIG. 32

Baseline([13C]-glucose
18 s after [13C]-glucose addition
41 s after [13C]-glucose addition
66 s after [13C]-glucose addition
89 s after [13C]-glucose addition
122 s after [13C]-glucose addition
188 s after [13C]-glucose addition
303 s after [13C]-glucose addition
913 s after [13C]-glucose addition
1830 s after [13C]-glucose addition
Fraction of Total Counts
m/z (amu)
136.6
136.7
136.8
136.9
137
137.1
137.2


FIG. 33A

Baseline([13C]-glucose
16 s after [13C]-glucose addition
41 s after [13C]-glucose addition
66 s after [13C]-glucose addition
89 s after [13C]-glucose addition
122 s after [13C]-glucose addition
183 s after [13C]-glucose addition
303 s after [13C]-glucose addition
913 s after [13C]-glucose addition
1830 s after [13C]-glucose addition
Fraction of Total Counts
m/z (amu)


FIG. 33B

Baseline([$^{13}$C]-glucose
18 s after [$^{13}$C]-glucose addition
41 s after [$^{13}$C]-glucose addition
66 s after [$^{13}$C]-glucose addition
89 s after [$^{13}$C]-glucose addition
122 s after [$^{13}$C]-glucose addition
168 s after [$^{13}$C]-glucose addition
303 s after [$^{13}$C]-glucose addition
913 s after [$^{13}$C]-glucose addition
1830 s after [$^{13}$C]-glucose addition
$^{13}$C/$^{12}$C Ratio
$10^{1}$
$10^{0}$
$10^{-1}$
136.6
136.7
136.8
136.9
137
137.1
137.2
m/z (amu)

FIG. 33C

Flux = 2 x 10^-3 ± 2 x 10^-3 s^-1
130.86
Time from [13C]-glucose Addition (sec)
0
500
1000
1500
2000
10
1
0.1
13C/12C Metabolite Ratio / Initial 13C/12C Metabolite Ratio


FIG. 33D

Baseline([13C]-glucose
18 s after [13C]-glucose addition
41 s after [13C]-glucose addition
66 s after [13C]-glucose addition
89 s after [13C]-glucose addition
122 s after [13C]-glucose addition
183 s after [13C]-glucose addition
303 s after [13C]-glucose addition
913 s after [13C]-glucose addition
1830 s after [13C]-glucose addition
Fraction of Total Counts
5 10-4
4 10-4
3 10-4
2 10-4
1 10-4
0 100
150.6
150.7
150.8
150.9
151
151.1
151.2
m/z (amu)

FIG. 34A

Baseline([13C]-glucose
18 s after [13C]-glucose addition
41 s after [13C]-glucose addition
66 s after [13C]-glucose addition
89 s after [13C]-glucose addition
122 s after [13C]-glucose addition
168 s after [13C]-glucose addition
303 s after [13C]-glucose addition
913 s after [13C]-glucose addition
1830 s after [13C]-glucose addition
Fraction of Total Counts
5 10-4
4 10-4
3 10-4
2 10-4
1 10-4
0 10^0
156.6
156.7
156.8
156.9
157
157.1
157.2
m/z (amu)

FIG. 34B

Baseline([13C]-glucose
18 s after [13C]-glucose addition
41 s after [13C]-glucose addition
66 s after [13C]-glucose addition
89 s after [13C]-glucose addition
122 s after [13C]-glucose addition
188 s after [13C]-glucose addition
303 s after [13C]-glucose addition
913 s after [13C]-glucose addition
1830 s after [13C]-glucose addition
13C/12C Ratio
10^1
10^0
10^-1
150.6
150.7
150.8
150.9
151
151.1
151.2
m/z (amu)

FIG. 34C

Flux = 8 x 10^-3 ± 4 x 10^-3 s^-1
150.87
Time from [13C]-glucose Addition (sec)
13C/12C Metabolite Ratio / Initial 13C/12C Metabolite Ratio


FIG. 34D

Baseline([13C]-glucose
18 s after [13C]-glucose addition
41 s after [13C]-glucose addition
66 s after [13C]-glucose addition
89 s after [13C]-glucose addition
122 s after [13C]-glucose addition
183 s after [13C]-glucose addition
303 s after [13C]-glucose addition
913 s after [13C]-glucose addition
1830 s after [13C]-glucose addition
Fraction of Total Counts
5 10-4
4 10-4
3 10-4
2 10-4
1 10-4
0 100
152.6
152.7
152.8
152.9
153
153.1
153.2
m/z (amu)


FIG. 35A

Baseline([13C]-glucose)
18 s after [13C]-glucose addition
41 s after [13C]-glucose addition
66 s after [13C]-glucose addition
89 s after [13C]-glucose addition
122 s after [13C]-glucose addition
183 s after [13C]-glucose addition
303 s after [13C]-glucose addition
913 s after [13C]-glucose addition
1830 s after [13C]-glucose addition
Fraction of Total Counts
m/z (amu)
0 10^0
1 10^-4
2 10^-4
3 10^-4
4 10^-4
5 10^-4
158.6
158.7
158.8
158.9
159
159.1
159.2


FIG. 35B

Baseline([13C]-glucose
18 s after [13C]-glucose addition
41 s after [13C]-glucose addition
66 s after [13C]-glucose addition
89 s after [13C]-glucose addition
122 s after [13C]-glucose addition
183 s after [13C]-glucose addition
303 s after [13C]-glucose addition
913 s after [13C]-glucose addition
1830 s after [13C]-glucose addition
$10^1$
$10^0$
$10^{-1}$
13C/12C Ratio
152.6
152.7
152.8
152.9
153
153.1
153.2
m/z (amu)

FIG. 35C

Flux = 6 x 10$^{-3}$ ± 3 x 10$^{-3}$ s$^{-1}$
152.88
Time from [$^{13}$C]-glucose Addition (sec)
$^{13}$C/$^{12}$C Metabolite Ratio / Initial $^{13}$C/$^{12}$C Metabolite Ratio
0
500
1000
1500
2000
10
1
0.1


FIG. 35D

Baseline([13C]-glucose
18 s after [13C]-glucose addition
41 s after [13C]-glucose addition
66 s after [13C]-glucose addition
89 s after [13C]-glucose addition
122 s after [13C]-glucose addition
183 s after [13C]-glucose addition
303 s after [13C]-glucose addition
913 s after [13C]-glucose addition
1830 s after [13C]-glucose addition
Fraction of Total Counts
1 10-4
8 10-5
6 10-5
4 10-5
2 10-5
0 100
281.5
281.6
281.7
281.8
281.9
282
282.1
282.2
m/z (amu)

FIG. 36A

Baseline([13C]-glucose
18 s after [13C]-glucose addition
41 s after [13C]-glucose addition
66 s after [13C]-glucose addition
89 s after [13C]-glucose addition
122 s after [13C]-glucose addition
188 s after [13C]-glucose addition
303 s after [13C]-glucose addition
913 s after [13C]-glucose addition
1830 s after [13C]-glucose addition
Fraction of Total Counts
m/z (amu)
287.5
287.6
287.7
287.8
287.9
288
288.1
288.2

FIG. 36B

Baseline([13C]-glucose
18 s after [13C]-glucose addition
41 s after [13C]-glucose addition
66 s after [13C]-glucose addition
89 s after [13C]-glucose addition
122 s after [13C]-glucose addition
168 s after [13C]-glucose addition
303 s after [13C]-glucose addition
913 s after [13C]-glucose addition
1830 s after [13C]-glucose addition
$10^1$
$10^0$
$10^{-1}$
$^{13}C/^{12}C$ Ratio
281.5
281.6
281.7
281.8
281.9
282
282.1
282.2
m/z (amu)


FIG. 36C

Flux = 5 x 10-3 ± 2 x 10-3 s-1
281.77
0
500
1000
1500
2000
Time from [13C]-glucose Addition (sec)
13C/12C Metabolite Ratio
Initial 13C/12C Metabolite Ratio
3
1
0.8
0.6
0.4


FIG. 36D

Baseline([13C]-glucose
18 s after [13C]-glucose addition
41 s after [13C]-glucose addition
66 s after [13C]-glucose addition
89 s after [13C]-glucose addition
122 s after [13C]-glucose addition
183 s after [13C]-glucose addition
303 s after [13C]-glucose addition
913 s after [13C]-glucose addition
1830 s after [13C]-glucose addition
Fraction of Total Counts
3 10-4
2.4 10-4
1.8 10-4
1.2 10-4
6 10-5
0 100
328.5
328.6
328.7
328.8
328.9
329
329.1
329.2
m/z (amu)

FIG. 37A

Baseline([13C]-glucose
18 s after [13C]-glucose addition
41 s after [13C]-glucose addition
66 s after [13C]-glucose addition
89 s after [13C]-glucose addition
122 s after [13C]-glucose addition
182 s after [13C]-glucose addition
303 s after [13C]-glucose addition
913 s after [13C]-glucose addition
1830 s after [13C]-glucose addition
Fraction of Total Counts
3 10-4
2.4 10-4
1.8 10-4
1.2 10-4
6 10-5
0 100
334.5
334.6
334.7
334.8
334.9
335
335.1
335.2
m/z (amu)

FIG. 37B

Baseline([13C]-glucose
18 s after [13C]-glucose addition
41 s after [13C]-glucose addition
66 s after [13C]-glucose addition
89 s after [13C]-glucose addition
122 s after [13C]-glucose addition
183 s after [13C]-glucose addition
303 s after [13C]-glucose addition
913 s after [13C]-glucose addition
1830 s after [13C]-glucose addition
13C/12C Ratio
10¹
10⁰
10⁻¹
328.5
328.6
328.7
328.8
328.9
329
329.1
329.2
m/z (amu)

FIG. 37C

Flux = 4 x 10$^{-3}$ ± 2 x 10$^{-3}$ s$^{-1}$
328.78
Time from [$^{13}$C]glucose Addition (sec)
0
500
1000
1500
2000
10
1
0.1

FIG. 37D

Baseline([13C]-glucose
18 s after [13C]-glucose addition
41 s after [13C]-glucose addition
66 s after [13C]-glucose addition
89 s after [13C]-glucose addition
122 s after [13C]-glucose addition
163 s after [13C]-glucose addition
303 s after [13C]-glucose addition
913 s after [13C]-glucose addition
1830 s after [13C]-glucose addition
Fraction of Total Counts
1 10-4
8 10-5
6 10-5
4 10-5
2 10-5
0 100
330.5
330.6
330.7
330.8
330.9
331
331.1
331.2
m/z (amu)

FIG. 38A

Baseline([13C]-glucose)
18 s after [13C]-glucose addition
41 s after [13C]-glucose addition
66 s after [13C]-glucose addition
89 s after [13C]-glucose addition
122 s after [13C]-glucose addition
183 s after [13C]-glucose addition
303 s after [13C]-glucose addition
913 s after [13C]-glucose addition
1830 s after [13C]-glucose addition
Fraction of Total Counts
1 10-4
8 10-5
6 10-5
4 10-5
2 10-5
0 100
336.5
336.6
336.7
336.8
336.9
337
337.1
337.2
m/z (amu)


FIG. 38B

Baseline([13C]-glucose
18 s after [13C]-glucose addition
41 s after [13C]-glucose addition
66 s after [13C]-glucose addition
89 s after [13C]-glucose addition
122 s after [13C]-glucose addition
183 s after [13C]-glucose addition
303 s after [13C]-glucose addition
913 s after [13C]-glucose addition
1830 s after [13C]-glucose addition
13C/12C Ratio
10^1
10^0
10^-1
m/z (amu)
330.5
330.6
330.7
330.8
330.9
331
331.1
331.2


FIG. 38C

Flux = 5 x 10^-4 ± 5 x 10^-3 s^-1
330.77
Time from [13C]-glucose Addition (sec)
0
500
1000
1500
2000
0.4
0.6
0.8
1
3
13C/12C Metabolite Ratio
Initial 13C/12C Metabolite Ratio

FIG. 38D

Baseline([13C]-glucose
18 s after [13C]-glucose addition
41 s after [13C]-glucose addition
66 s after [13C]-glucose addition
89 s after [13C]-glucose addition
122 s after [13C]-glucose addition
183 s after [13C]-glucose addition
303 s after [13C]-glucose addition
913 s after [13C]-glucose addition
1830 s after [13C]-glucose addition
Fraction of Total Counts
m/z (amu)
494.6
494.7
494.8
494.9
495
495.1
495.2
495.3
495.4

FIG. 39A

Baseline([13C]-glucose
16 s after [13C]-glucose addition
41 s after [13C]-glucose addition
66 s after [13C]-glucose addition
89 s after [13C]-glucose addition
122 s after [13C]-glucose addition
182 s after [13C]-glucose addition
303 s after [13C]-glucose addition
913 s after [13C]-glucose addition
1830 s after [13C]-glucose addition
Fraction of Total Counts
m/z (amu)
500.6
500.7
500.8
500.9
501
501.1
501.2
501.3
501.4

FIG. 39B

Baseline([13C]-glucose
18 s after [13C]-glucose addition
41 s after [13C]-glucose addition
66 s after [13C]-glucose addition
89 s after [13C]-glucose addition
122 s after [13C]-glucose addition
183 s after [13C]-glucose addition
303 s after [13C]-glucose addition
913 s after [13C]-glucose addition
1630 s after [13C]-glucose addition
13C/12C Ratio
10^1
10^0
10^-1
494.6
494.7
494.8
494.9
495
495.1
495.2
495.3
495.4
m/z (amu)

FIG. 39C

Flux = 2 x 10^-3 ± 2 x 10^-3 s^-1
494.88
400
800
1200
1600
2000
Time from [13C]-glucose Addition (sec)
3
1
0.8
0.6
0.4
13C/12C Metabolite Ratio
Initial 13C/12C Metabolite Ratio

FIG. 39D

Baseline([13C]-glucose
18 s after [13C]-glucose addition
41 s after [13C]-glucose addition
66 s after [13C]-glucose addition
89 s after [13C]-glucose addition
122 s after [13C]-glucose addition
183 s after [13C]-glucose addition
303 s after [13C]-glucose addition
913 s after [13C]-glucose addition
1830 s after [13C]-glucose addition
Fraction of Total Counts
m/z (amu)
278.5
278.6
278.7
278.8
278.9
279
279.1

FIG. 40A

Baseline([13C]-glucose
18 s after [13C]-glucose addition
41 s after [13C]-glucose addition
56 s after [13C]-glucose addition
89 s after [13C]-glucose addition
122 s after [13C]-glucose addition
183 s after [13C]-glucose addition
303 s after [13C]-glucose addition
913 s after [13C]-glucose addition
1830 s after [13C]-glucose addition
1 10-4
8 10-5
6 10-5
4 10-5
2 10-5
0 100
Fraction of Total Counts
283.5
283.6
283.7
283.8
283.9
284
284.1
m/z (amu)


FIG. 40B

Baseline([$^{13}$C]-glucose
18 s after [$^{13}$C]-glucose addition
41 s after [$^{13}$C]-glucose addition
66 s after [$^{13}$C]-glucose addition
89 s after [$^{13}$C]-glucose addition
122 s after [$^{13}$C]-glucose addition
183 s after [$^{13}$C]-glucose addition
303 s after [$^{13}$C]-glucose addition
913 s after [$^{13}$C]-glucose addition
1830 s after [$^{13}$C]-glucose addition
$^{13}$C/$^{12}$C Ratio
$10^{1}$
$10^{0}$
$10^{-1}$
278.5
278.6
278.7
278.8
278.9
279
279.1
m/z (amu)

FIG. 40C

Flux=5 x 10^-4 ± 5 x 10^-5 s^-1
278.61
Time from [13C]-glucose Addition (sec)
400
800
1200
1600
2000
13C/12C Metabolite Ratio
Initial 13C/12C Metabolite Ratio
0.4
0.6
0.8
1
3


FIG. 40D

Baseline([13C]-glucose
18 s after [13C]-glucose addition
41 s after [13C]-glucose addition
66 s after [13C]-glucose addition
89 s after [13C]-glucose addition
122 s after [13C]-glucose addition
158 s after [13C]-glucose addition
303 s after [13C]-glucose addition
913 s after [13C]-glucose addition
1630 s after [13C]-glucose addition
Fraction of Total Counts
m/z (amu)
280.5
280.6
280.7
280.8
280.9
281
281.1


FIG. 41A

Baseline[13C]-glucose
18 s after [13C]-glucose addition
41 s after [13C]-glucose addition
66 s after [13C]-glucose addition
89 s after [13C]-glucose addition
122 s after [13C]-glucose addition
183 s after [13C]-glucose addition
303 s after [13C]-glucose addition
913 s after [13C]-glucose addition
1830 s after [13C]-glucose addition
Fraction of Total Counts
m/z (amu)
285.5
285.6
285.7
285.8
285.9
286
286.1


FIG. 41B

Baseline([13C]-glucose
18 s after [13C]-glucose addition
41 s after [13C]-glucose addition
66 s after [13C]-glucose addition
89 s after [13C]-glucose addition
122 s after [13C]-glucose addition
183 s after [13C]-glucose addition
303 s after [13C]-glucose addition
913 s after [13C]-glucose addition
1830 s after [13C]-glucose addition
$10^1$
$10^0$
$10^{-1}$
13C/12C Ratio
280.5
280.6
280.7
280.8
280.9
281
281.1
m/z (amu)


FIG. 41C

Flux=3 x 10^-3 ± 5 x 10^-4 s^-1
283.80
Time from [13C]-glucose Addition (sec)
400
800
1200
1600
2000
13C/12C Metabolite Ratio / Initial 13C/12C Metabolite Ratio
3
1
0.8
0.6
0.4


FIG. 41D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 13023899 P
- US 07571598 P
- US 51348600 A
- US 51339500 A
- US 51390700 A
- US 6023659 A
- US 5966712 A
- US 5953727 A
- US 5706498 A
- US 5538897 A
- US 5926818 A
- US 5295261 A
- US 5672480 A
- US 5599677 A
- US 5939533 A
- US 5710007 A
- US 5811231 A
- US 513395 A
- US 09513486 B
- US 09513907 B
- US 5858188 A
- US 5935401 A
- US 6007690 A
- US 5876675 A
- US 6001231 A
- US 5976336 A
- US 3996345 A, Ullman
- US 4351760 A, Khanna
- US 5534440 A, Aebersold
- US 5918273 A, Horn
- US 4830010 A
- US 5542419 A
- US 6010846 A
- US 5924995 A
- US 5140993 A
- US 5133844 A
- US 5317156 A
- JP 61042219 A
- JP 5142146 A
- US 4656133 A
- US 5439803 A
- US 09513395 B


### Non-patent literature cited in the description


- **JAMES, P. ; M. QUANDRONI ; E. CARAFOLI ; G. GONNET.** *Biochem. Biophys. Res. Commun.,* 1993, vol. 195, 58-64
- **YATES, J.R. ; S. SPEICHER ; P.R. GRIFFIN ; T. HUNKAPILLER.** *Anal. Biochem.,* 1993, vol. 214, 397-408
- **MANN, M.** IBC Proteomics conference. 10 November 1997
- **WILM, M. ; A. SHEVCHENKO ; T. HOUTHAEVE ; S. BREIT ; L. SCHWEIGER ; T. FOTSIS ; M. MANN.** *Nature,* 1996, vol. 379, 466-469
- **CHAIT, B.T ; R. WANG ; R.C. BEAVIS ; S.B.H. KENT.** *Science,* 1993, vol. 262, 89-92
- **MANN, M.** *IBC Proteomics conference,* 10 November 1997
- **CLAUSER, K.R. ; S. C. HALL ; D. M. SMITH ; J.W. WEBB ; L.E. ANDREWS ; H. M. TRAN ; L.B. EPSTEIN ; A.L. BURLINGAME.** *Proc. Natl. Acad. Sci. (USA),* 1995, vol. 92, 5072-5076
- **LI, G. ; M. WALTHAN ; N. L. ANDERSON ; E. UNWORTH ; A. TRESTON ; J. N. WEINSTEIN.** *Electrophoresis,* 1997, vol. 18, 391-402
- **SHEVCHENKO, A. et al.** *Proc. Natl. Acad. Sci. (USA),* 1996, vol. 93, 14440-14445
- Protein Capillary Electrophoresis: Theoretical and Experimental Considerations for Methods Development. **PALMIERI, R. ; NOLAN, J.A.** CRC Handbook of Capillary Electrophoresis: A Practical Approach. CRC Press, 1994, 325-368
- Isoelectric Focusing in Capillaries. **KILAR, F.** CRC Handbook of Capillary Electrophoresis: A Practical Approach. CRC Press, 1994, 325-368
- Capillary Zone Electrophoresis of Peptides. **MCCORMICK, R.M.** CRC Handbook of Capillary Electrophoresis: A Practical Approach. CRC Press, 1994, 287-323
- **O'FARREL, P.H.** *J Biol. Chem.,* 1975, vol. 250, 4007
- **HOCHSTRASSER, D.F. et al.** *Anal Biochem.,* 1988, vol. 173, 424
- **HÜHMER, A. F. R. et al.** *Anal. Chem.,* 1997, vol. 69, 29R-57R
- **GARFIN, D.E.** *Methods in Enzymology,* 1990, vol. 182, 425
- Capillary electrophoresis-mass spectrometry. **SMITH, R.D. et al.** CRC Handbook of Capillary Electrophoresis: A Practical Approach. CRC Press, 1994, 185-206

- Isoelectric focusing in capillaries. **KILÁR, F.** CRC Handbook of Capillary Electrophoresis: A Practical Approach. CRC Press, 1994, 95-109
- Capillary zone electrophoresis of peptides. **MCCORMICK, R. M.** CRC Handbook of Capillary Electrophoresis: A Practical Approach. CRC Press, 1994, 287-323
- Protein capillary electrophoresis: theoretical and experimental considerations for methods development. **PALMIERI, R. ; NOLAN, J. A.** CRC Handbook of Capillary Electrophoresis: A Practical Approach. CRC Press, 1994, 325-368
- The use of affinity-interaction mass spectrometry in proteome analysis. **NELSON, R.W.** BC Proteomics conference. 11 June 1998
- Ciphergen Biosystems. **YOUNG, J.** CHI Genomics Opportunities conference. 14 February 1998
- **ANDERSON, N.G. ; N.L. ANDERSEN.** Twenty years of two-dimensional electrophoresis: Past, present and future. *Electrophoresis,* 1996, vol. 17, 443
- Pharmaceutical Proteomics: Targets, mechanisms and function. **ANDERSON, L.** BC Proteomics conference. 11 June 1998
- The Yeast Proteome. **MCKEE, A.** BC Proteomics conference. 11 June 1998
- BioRad Molecular Imager FX and PDQuest 2-D analysis software seminar. IBC Proteomics conference. 11 June 1998
- **FRANZÉN, F. et al.** *Electrophoresis,* 1997, vol. 18, 582
- Proteome methods to profile mechanisms of toxicity. **STEINER.** IBC Proteomics conference. 11 June 1998
- Protein differential display and mass spectrometry in the study of congestive heart failure. **ARNOTT.** IBC Proteomics conference. 11 June 1998
- **WITZMANN et al.** *Electrophoresis,* 1997, vol. 18, 642
- **FRANZÉN et al.** *Electrophoresis,* 1997, vol. 18, 582
- **CLAUSER et al.** *Proc. Natl. Acad. Sci. (USA),* 1995, vol. 92, 5072-5076
- **LI et al.** *Electrophoresis,* 1997, vol. 18, 391-402
- *Methods in Enzymology,* 1972, vol. 25, 103-120
- **NIALL.** *Methods in Enzymology,* 1973, vol. 27, 942-1011
- **GRAY.** *Methods in Enzymology,* 1972, vol. 25, 121-137
- **SCHROEDER.** *Methods in Enzymology,* 1972, vol. 25, 138-143
- **CREIGHTON.** Proteins: Structures and Molecular Principles. W. H. Freeman, 1984
- **NIEDERWIESER.** *Methods in Enzymology,* 1972, vol. 25, 60-99
- **THIEDE et al.** *FEBS Lett.,* 1995, vol. 357, 65-69
- **SHEVCHENKO, A.** *Proc. Natl. Acad. Sci. (USA),* 1996, vol. 93, 14440-14445
- **WILM et al.** *Nature,* 1996, vol. 379, 466-469
- **MARK, J.** Protein structure and identification with MS/MS. *Protein Characterization and Proteomics: Automated high throughput technologies for drug discovery,* March 1998
- **BIEMAN.** *Methods in Enzymology,* 1990, vol. 193, 455-479
- **JAMES et al.** *Biochen. Biophys. Res. Commun.,* 1993, vol. 195, 58-64
- **YATES et al.** *Anal. Biochem.,* 1993, vol. 214, 397-408
- **CHAIT et al.** *Science,* 1993, vol. 262, 89-92
- **INGRAHAM, J. L. et al.** Growth of the Bacterial Cell. Sinauer Associates, 1983
- **VOET, D. ; VOET, J.G.** Biochemistry. John Wiley & Sons, 1990
- **STRYER, L.** Biochemistry. W.H. Freeman and Company, 1981
- **WHITE, A. et al.** Principles of Biochemistry. McGraw-Hill Book Company, 1978
- **CHOCK, P. B. et al.** *Current Topics in Cellular Regulation.,* 1985, vol. 27, 3
- **KOSHLAND, D. E. et al.** *Science,* 1982, vol. 217, 220
- **STADTMAN, E. R. ; CHOCK, P. B.** *Current topics in Cellular Regulation,* 1978, vol. 13, 53
- **MONOD, J. et al.** *J. Mol. Biol.,* 1963, vol. 6, 306
- **STADTMAN, E. R. ; CHOCK, P. B.** *Current Topics in Cellular Regulation,* 1978, vol. 13, 53
- **NEIDHARDT, F. C. et al.** Escherichia coli and Salmonella typhimurium: cellular and molecular biology. Amer Soc Microbiology, 1987, 3
- **NEIDHARDT, F. C. ; VAN BOGELEN, R. A.** Escherichia coli and Salmonella typhimurium Cellular and Molecular Biology. American Society of Microbiology, 1987, 1334
- **MAGASANIK, B. ; NEIDHARDT, F. C.** Escherichia coli and Salmonella typhimurium Cellular and Molecular Biology. American Society of Microbiology, 1987, 1318
- **VANBOGELEN, R. A. et al.** *Electrophoresis,* 1990, vol. 11, 1131
- **WANNER, B. L.** Escherichia coli and Salmonella typhimurium Cellular and Molecular Biology. American Society of Microbiology, 1987, 1326
- **CHRISTMAN, M. F.** *Cell,* 1985, vol. 14, 753
- **WALKER, G. C.** Escherichia coli and Salmonella typhimurium Cellular and Molecular Biology. American Society of Microbiology, 1987, 1346
- **NATH, K. ; KOCH, A. L.** *J. Biol, Chem.,* 1971, vol. 246, 6956

- **ST. JOHN, A. C. ; GOLDBERG, A. L.** *J. Bacteriol.,* 1980, vol. 143, 1223
- **DAVIS, B. D.** *J. Bacteriol.,* 1986, vol. 166, 439
- **MARUYAMA, H. B. ; OKAMURA, S.** *J. Bacteriol.,* 1972, vol. 110, 442
- Pharmaceutical Proteomics: Targets, Mechanism, and Function. **ANDERSON, L.** IBC Proteomics conference. 11 June 1998
- **SAMBROOK et al.** MOLFCULAR CLONING: A LABORATORY MANUAL. Cold Spring Harbor Laboratory Press, 1989
- **STRYER, L.** Biochemistly. W.H. Freeman and Company, 1981
- **SPATOLA, A.F.** CHEMISTRY AND BIOCHEMISTRY OF AMINO ACIDS, PEPTIDES AND PROTEINS. Marcel Dekker, 1983, 267
- Immunology - A Synthesis. Sinauer Associates, 1991
- Isoelectric Focusing in Capillaries. **KILAR, F.** CRC Handbook on Capillary Electrophoresis: A Practical Approach. CRC Press, Inc, 1994, 95-109
- **SCHWARTZ, H. ; T. PRITCHETT.** Separation of Proteins and Peptides by Capillary Electrophoresis: Application to Analytical Biotechnology. 1994
- **WEHR, T. ; RODRIQUEZ-DIAZ, R. ; ZHU, M.** Capillary Electrophoresis of Proteins. Marcel Dekker, 1999
- Protein capillary electrophoresis: Theoretical and experimental considerations for methods development. **PALMIERI, R. ; J. A. NOLAN.** CRC Handbook of Capillary Electrophoresis: A Practical Approach. CRC Press, 1994, 325-368
- **S. HJERTEN ; J.-L. LIAO ; K. YAO.** *J. Chromatogr.,* 1987, vol. 387, 127
- **R. RODRIGUEZ-DIAZ ; M. ZHU ; T. WEHR.** *J. Chromatogr. A,* 1997, vol. 772, 145
- **SCHASFOORT, R.B.M. ; SCHLAUTMANN, S. ; HENDRIKSE, J. ; VAN DEN BERG, A.** Field-Effect Flow Control for Microfabricated Fluidic Networks. *Science,* vol. 286, 942-945
- Isoelectric Focusing in Capillaries. **KILAR, F.** CRC Handbook or Capillary Electrophoresis: A Practical Approach. CRC Press, Inc, 1994, 95-109
- Capillary Zone Electrophoresis of Peptides. **MCCORMICK, R.M.** CRC Handbook of Capillary Electrophoresis: A Practical Approach. CRC Press Inc, 1994, 287-323
- **JORGENSON, J.W. ; LUKACS, K.D.** *J. High Resolut. Chromatogr. Commun.,* 1981, vol. 4, 230
- **JORGENSON, J.W. ; LUKACS, K.D.** *Anal. Chem.,* 1981, vol. 53, 1298
- **HJERTÉN, S.** Free zone electrophoresis. *Chromatogr. Rev.,* 1967, vol. 9, 122
- Isotachophoresis in Capillary Electrophoresis. **B.J. WANDERS ; EVERAERTS, F.M.** CRC Handbook of Capillary Electrophoresis: A Practical Approach. 1994, 111-127
- Protein capillary electrophoresis: theoretical and experimental considerations for methods development. **PALMIERI, R. ; NOLAN, J. A.** CRC Handbook of Capillary Electropho resis: A Practical Approach. CRC Press, 1994, 3 25-368
- Quantitation of bioactive peptides in serum by capillary electrophoresis with laser-induced fluroescence immunodetection (CE-LIF-ID. **PRITCHETT, T. et al.** Application Information. Beckman Instruments, 1995
- **JORGENSON, J.W. ; K.D. LUKACS.** *J. High Resolut. Chromatogr. Chromatogr. Commun.,* 1981, vol. 4, 230
- **BODHE, A.M. et al.** *Anal. Biochem.,* 1987, vol. 164, 39-43
- **GUZMAN, N.A. et al.** *J. Chromatogr.,* 1992, vol. 608, 197-204
- **VANDEKERCKHOYE, J.** *Eur. J. Biochem.,* 1985, vol. 152, 9-19
- *J. Chromatogr.,* 1988, vol. 447, 117
- Quantitation of bioactive peptides in serum by capillary electrophoresis with laser-induced fluroescence immunodetection (CE-LIF-ID. **PRITCHETT et al.** Application Information. Beckman Instruments, 1995
- **STARK, G. R.** *Methods in Enzymology,* 1972, vol. 25, 103-120
- **NIALL, H. D.** Automated Edman degradation: the protein sequenator. *Methods in Enzymology,* 1973, vol. 27, 942-1011
- **GRAY, W. R.** *Methods in Enzymology,* 1972, vol. 25, 121-137
- **SCHROEDER, W. A.** *Methods in Enzymology,* 1972, vol. 25, 138-143
- **CREIGHTON, T. E.** Proteins: Structures and Molecular Principles. W. H. Freeman, 1984
- **NIEDERWIESER, A.** *Methods in Enzymology,* 1972, vol. 25, 60-99
- **WU et al.** *Anal. Biochem.,* 1996, vol. 235, 161-174
- **WATSON, J.T. ; J. WU.** *Polym. Prep.,* 1996, vol. 37, 3-18
- **DENSLOW, N.D. ; H.P. NGUYEN.** Techniques in Protein Chemistry VII. Academic Press, 1996, 241-248
- **MING, D. et al.** *BioTechniques,* 1995, vol. 18, 808-810
- **MURPHY, C.M. ; C. FENSELAU.** *Anal. Chem.,* 1995, vol. 67, 1644-1645
- **ROSE, K. et al.** *Biochem. J.,* 1988, vol. 250, 253-259
- **MEANS et al.** CHEMICAL MODIFICATION OF PROTEINS. 1971

- **FEENEY et al.** MODIFICATION OF PROTEINS: FOOD, NUTRITIONAL AND PHARMACOLOGICAL ASPECTS. American Chemical Society, 1982
- FOOD PROTEINS: IMPROVEMENT THROUGH CHEMICAL AND ENZYMATIC MODIFICATION. **FEENEY et al.** Advances in Chemistry Series. American Chemical Society, 1977, vol. 160
- **HERMANSON.** BIOCONJUGATE TECHNIQUES. Academic Press, 1996
- **MARSTON et al.** *Meth. Enz.,* 1990, vol. 182, 264-275
- **GYGI et al.** *Nature Biotechnol.,* 1999, vol. 17, 994-999
- Properties of In Vivo Chelate-Tagged Proteins and Polypeptides. **MEARES et al.** MODIFICATION OF PROTEINS: FOOD, NUTRITIONAL, AND PHARMACOLOGICAL ASPECTS. American Chemical Society, 1982, 370-387
- **KASINA et al.** *Bioconjugate Chem.,* 1998, vol. 9, 108-117
- **SONG et al.** *Bioconjugate Chem.,* 1997, vol. 8, 249-255
- FLUORESCENCE SPECTROSCOPY. Marcel Dekker, 1971
- **WHITE et al.** FLUORESCENCE ANALYSIS: A PRACTICAL APPROACH. Marcel Dekker, 1970
- **BERLMAN.** HANDBOOK OF FLUORESCENCE SPECTRA OF AROMATIC MOLECULES. Academic Press, 1971
- **GRIFFITHS.** COLOUR AND CONSTITUTION OF ORGANIC MOLECULES. Academic Press, 1976
- INDICATORS. Pergamon Press, 1972
- **HAUGLAND.** HANDBOOK OF FLUORESCENT PROBES AND RESEARCH CHEMICALS. Molecular Probes, 1992
- **PRINGSHEIM.** FLUORESCENCE AND PHOSPHORESCENCE. Interscience Publishers, 1949
- **KULMALA et al.** *Analytica Chimica Acta,* 1999, vol. 386, 1
- **HOCHSTRASSER et al.** *Anal Biochern.,* 1988, vol. 173, 424
- **O'FARREL.** *J Biol. Chem.,* 1975, vol. 250, 4007
- **WILM, M. et al.** *Anal. Chem.,* 1996, vol. 68, 1-8
- **SANGER, F.** *Biochem. J.,* 1945, vol. 39, 507
- **HIRS, C.H.W. et al.** *Arch. Biochem. Biophys.,* 1965, vol. 111, 209-222
- **NIALL, H. D.** *Methods in Enzymology,* 1973, vol. 27, 942-1011
- **GALELLA, G. et al.** *Can. J. Biochem.,* 1982, vol. 60, 71-80
- **LOMANT, A.J. et al.** *J. Mol. Biol.,* 1976, vol. 104, 243-261
- **LOMANT, A.J. et al.** *J. Mo/. Biol.,* 1976, vol. 104, 243-261
- **SOLOMONS, T.W.G.** Organic Chemistry. John Wiley & Sons, 1976
- **NOVOTNY et al.** *Anal. Chem.,* 1991, vol. 63, 408
- **NOVOTNY et al.** *J. Chromatography,* 1990, vol. 499, 579
- **WAGNER, D.S. et al.** *Biol Mass Spectrometry,* 1991, vol. 20, 419-425
- **MERRIFIELD, B.** *Science,* 1986, vol. 232, 341-347
- **HORTON, H. R. et al.** *Methods in Enzymology,* 1972, vol. 25, 468
- **YAMADA, H. et al.** *Biochem.,* vol. 20, 4836-4842
- **GRABAREK, Z. et al.** *Anal. Biochem.,* 1990, vol. 185, 131-135
- A New Simple Preparation Device for Protein/Peptide Sequencing. **WERNER et al.** Ninth Symposium of the Protein Society
- **MARCH.** ADVANCED ORGANIC CHEMISTRY. John Wiley & Sons, 1985
- **FEENEY et al.** MODIFICATION OF PROTEINS. American Chemical Society, 1982, vol. 198
- **GREENE et al.** PROTECTIVE GROUPS IN ORGANIC SYNTHESIS. John Wiley & Sons, 1991
- **HIRS, C.H.W.** *Arch, Biochem. Biophys.,* 1965, vol. 111, 209-222
- **HERMANSON, G.** Bioconjugate Techniques. Academic Press, 1995
- **HAUGLAND, R.P.** Handbook of Fluorescent Probes and Research Chemicals. Molecular Probes, 1996
- **HARTMAN, F.C. ; F. WOLD.** Biochem. 1967, vol. 6, 2439-2448
- **BROWNE, D.T. ; S.B.H. KENT.** *Biochem. Biophys. Res. Commun.,* 1975, vol. 67, 126-132
- **CUATRECASAS, P. ; I. PARIKH.** *Biochem.,* 1972, vol. 11, 2291-2299
- **HERMANSON, G.** Bioconjugate techniques. Academic Press, 1995
- **HORTON, H. R. ; D. E. KOSHLAND, JR.** *Methods in Enzymology,* 1972, vol. 25, 468
- **GRABAREK, Z. ; J. GERGELY.** *Anal. Biochem,* 1990, vol. 185, 131-135
- Protein structure and identification with MS/MS. PE/Sciex Seminar Series, Protein Characterization and Proteomics: Automated high throughput technologies for drug discovery. March 1998
- **TAE.** *Methods in Enzymology,* 1983, vol. 91, 580
- **LAURSEN.** *J. Am. Chem. Soc.,* 1966, vol. 88, 5344
- **LAURSEN.** *Eur. J. Biochem.,* 1971, vol. 20, 89
- **LAURSEN et al.** *FEBS Lett.,* 1972, vol. 21, 67

- **HORN et al.** *FEBS Lett.,* 1973, vol. 36, 285
- **WACHTER et al.** *FEBS Lett.,* 1973, vol. 35, 97
- **BRIDGEN.** *FEBS Lett.,* 1975, vol. 50, 159
- **ATHERTON et al.** *FEBS Lett.,* 1976, vol. 64, 173
- **CAVADORE et al.** *FEBS Lett. 66,* 1976, 155
- **MACHLEIDT ; WACHTER.** *Methods in Enzymology: [29] New Supports in Solid-Phase Sequencing,* 1974, 263-277
- **AEBERSOLD et al.** *Anal. Biochem.,* 1990, vol. 187, 56-65
- **AEBERSOLD et al.** *Biochem.,* 1988, vol. 27, 6860-6867
- **PAPPIN et al.** CURRENT RESEARCH IN PROTEIN CHEMISTRY. Academic Press, 1990, 191-202
- **TARR et al.** *Anal. Biochem.,* 1978, vol. 84, 622
- **FEENEY et al.** MODIFICATION OF PROTEINS: FOOD, NUTRITIONAL AND PHARMACOLOGICAL ASPECTS. American Chemical Society, 1982, vol. 198
- **FEENEY et al.** FOOD PROTEINS: IMPROVEMENT THROUGH CHEMICAL AND ENZYMATIC MODIFICATION. American Chemical Society, 1977, vol. 160
- **JOHNSON et al.** *Protein Science,* 1992, vol. 1, 1083-1091
- **BRUINS et al.** *Anal. Chem.,* 1987, vol. 59, 2642-2647
- **FENN et al.** *Science,* 1989, vol. 246, 64-71
- **KARAS et al.** *Anal. Chem.,* vol. 60, 2299-2301
- Fourier Transform Ion Cyclotron Resonance Mass Spectrometer. Extrel Corp,
- **SAMBROOK et al.** MOLECULAR CLONING: A LABORATORY MANUAL. Cold Spring Harbor Laboratory Press, 1989
- The Theory and Practice of Industrial Pharmacy. 1976
- Remington's Pharmaceutical Sciences. Mace Publishing Company, 1985
- **LANGER.** *Science,* 1990, vol. 249, 1527-1533
- **HANSON, R. S. ; PHILLIPS, J. A. et al.** Manual of methods for general bacteriology. Am. Soc. Microbiol, 1981, 328
- **GUPTA, N.R. et al.** *J. Colloid Interface Sci.,* 2000, vol. 222, 107-116
- **FENSELAU, C.** Mass Spectrometry for Characterization of Microorganisms. *ACS Symp. Ser.,* 1994, vol. 541, 1-7
- **LAURITSEN, F.R. ; LLOYD, D.** Direct Detection of Volatile Metabolites Produced by Microorganisms. *ACS Sympl Ser.,* 1994, vol. 541, 91-106
- **FOX, A. ; BLACK, G.E.** Identification and Detection of Carbohydrate Markers for Bacteria. *ACS Symp. Ser.,* 1994, vol. 541, 107-131
- **EDMONDS, C.G. et al.** Ribonucleic acid modifications in microorganisms. *ACS Symp. Ser.,* 1994, vol. 541, 147-158
- **VORM, O. et al.** Improved Resolution and Very High Sensitivity in MALDI TOF of Matrix Surfaces made by Fast Evaporation. *Anal. Chem.,* 1994, vol. 66, 3281-3287
- **VORM, O. ; MANN, M.** Improved Mass Accuracy in Matrix-Assisted Laser Desorption/Ionization Time-of-Flight Mass Spectrometry of Peptides. *J. Am. Soc. Mass. Spectrom.,* 1994, vol. 5, 955-958
- **EWING, G.W.** Instrumental Methods of Chemical Analysis,. 1975
- Stable Isotopes and Mass Spectrometry in Nutrition Science. **KLEIN, P. et al.** Analytical Chemistry Symposium Series. 1984, vol. 21, 155-166
- **KLEIN, P. et al.** *J. Pediatric Gastroenterology and Nutrition,* 1985, vol. 4, 9-19
- **KLEIN, P. et al.** *Analytical Chemistry Symposium Series,* 1982, vol. 11, 347-352
- **ZAR, J.H.** Biostatistical Analysis. Prentice-Hall, 1974
- **NEWSHOLME, E.A. et al.** *Biochem. Soc. Symp.,* 1978, vol. 43, 183-205
- **NEWSHOLME, E.A et al.** *Biochem. Soc. Symp.,* 1976, vol. 41, 61-110
- **NEWSHOLME, E.A. ; SART., C.** Regulation in Metabolism. Wiley-Interscience Press, 1973
- Defining protein targets for drug discovery using Proteomics. **PATTON, W.F.** IBC Proteomics conference. 11 June 1998
- **HOCHSTRASSER et al.** *Anal. Biochem.,* 1988, vol. 173, 424
- **O'FARRELL.** *J. Biol. Chem.,* 1975, vol. 250, 4007
- Isoelectric Focusing in Capillaries. **KILAR, F.** CRC Handbook on Capillary Electrophoresis: A. Practical Approach. CRC Press, Inc, 1994, 95-109
- Isoelectric Focusing in Capillaries. **KILAR, F.** CRC Handdbook on Capillary Electrophoresis: A Practical Approach. CRC Press, Inc, 1994, 95-109
- **SCHASFOORT, R.B.M. ; SCHLAUTMANN, S. ; HENDRIKSE, J. ; VAN DEN BERG, A.** Field-Effect Flow Control for Microfabricated Fluidic Networks. *Science,* 1999, vol. 286, 942-945
- Isoelectric Focusing in Capillaries. **KILAR, F.** CRC Handbook or Capillary Electrophoresis.: A Practical Approach. CRC Press, Inc, 1994, 95-109
- Capillary Zone Electrophoresis of Peptides. **MCCORMICK, R.M.** CRC Handbook of Capillary Electrophoresis; A Practical Approach. CRC Press Inc, 1994, 287-323
- **CANTOR, C.R. ; SCHIMMEL, P. R.** Biophysical Chemistry. W.H. Freeman & Co, 1980
- **HJERTÉN, S.** *Chromatogr. Rev.,* 1967, vol. 9, 122

- **GOTTLIEB, M. ; CHAVKO, M.** *Anal. Biochem.,* 1987, vol. 165, 33
- Protein capillary electrophoresis: Theoretical and experimental considerations for methods development. **PALMIERI, R. ; NOLAN, J. A.** CRC Handbook of Capillary Electrophoresis: A Practical Approach. CRC Press, 1994, 325-368
- Isotachophoresis in capillary electrophoresis. **WANDERS, B.J. ; EVERAERTS, F. M.** CRC Handbook of Capillary Electrophoresis : A Practical Approach. CRC Press, 1994, 111-127
- **MARTINEK, K. et al.** *FEBS Lett.,* 1975, vol. 51, 152-155
- **ALTRIA, K.D. ; SIMPSON, C.F.** *Anal. Proc.,* 1986, vol. 23, 453
- Capillary zone electrophoresis of peptides. **ME CORMICK, R.M.** CRC Handbook of Capillary Electrophoresis: A Practical Approach. CRC Press, 1994, 287-323
- **B.J. WANDERS ; EVERAERTS, F.M.** Isotachophoresis in Capillary Electrophoresis. *CRC Handhook of Capillary Electrophoresis: A Practical Approach,* 1994, 111-127
- **SCHWER, C ; LOTTSPEICH, F.** *J. Chromatogr.,* 1992, vol. 623, 345
- **FORET, F. et al.** *J. Chromatogr.,* 1992, vol. 608, 3
- Effects of sample matrix on separation by capillary electrophoresis. **SHIHABI, Z.K. ; GARCIA, L. L.** CRC Handbook of Capillary Electrophoresis: A Practical Approach. CRC Press, 1994, 537-548
- **CANTER, C.R. ; SCHIMMEL, P.R.** Biophysical Chemistry. W.H. Freeman and Co, 1980
- **PERSSON et al.** *Eur. J. Biochem.,* 1985, vol. 152, 523-527
- **LOMANT et al.** *J. Mol. Biol.,* 1976, vol. 104, 243-261
- **HINES et al.** *Am. Soc. Mass. Spec.,* 1992, vol. 3, 326-336
- **L.V. SCHNEIDER.** Metabolic uncoupling in Escherichia coli during phosphate-limited growth. 1997
- **HERMANSON.** Bioconjugate TECHNIQUES. Academic Press, 1996
- **GREENE ; WUTS.** Protective Groups in Organic Synthesis. Wiley Science, 1999
- ACS Symposium Series. **LEIF et al.** Cell Separation Science and Technology. American Chemical Society, 1991, vol. 464, 41-58
- **VALLARINO et al.** Proceedings of Advances in Fluorescence Sensing Technology. SPIE Proceeding Series, 1993, vol. 1885, 376-385
- Proceedings of Biochemical Diagnostic Instrumentation. **LEIF et al.** Progress in Biomedical Optics. 1994, vol. 2136, 255-262
- **PERIASAMY et al.** *Microscopy and Analysis,* March 1995, 33-35
- **SEVEUS et al.** *Micro. Res. And Tech.,* 1994, vol. 28, 149-154
- **J, SCHLESSINGER.** Dept. of Chemical Immunology. The Weitzman Institute for Science
- **REHOVOT, ISRAEL.** Personal communication between Research Organics. Inc. and J. Schlessinger
- **D. BLAKESLEE et al.** *J. Immun. Methods,* 1976, vol. 13, 305
- **H.P. ROTHBART et al.** *J. Immun. Methods,* 1978, vol. 19, 101
- **HARVEY M.D. et al.** *Electrophoresis,* September 1998, vol. 19 (12), 2169-74
- Methods in Protein Structure Analysis. Plenum Press, 1995
- **BOYD, V.L. et al.** Sequencing of Proteins from the C-terminus. 109-118
- **DUPONT et al.** PE Biosystems. Inc. Application Note
- **GUGA et al.** C-terminal Sequence Analysis of the Amino Acids with Reactive Side-Chains: Ser, Thr, Cys, Glu, Asp, His, Lys. *Poster presentation at the Seventh Symposium Of the Protein Society,* 1993
- **WERNER et al.** A New Simple Preparation Device for Protein/Peptide Sequencing. *Poster presentation at the Ninth Symposium of the Protein Society,* 1992
- **BRUNE.** *Anal. Biochem.,* vol. 207, 285
- **REICHELT, K. et al.** *J. Appl. Nutrition,* 1990, vol. 42, 1-11
- **REICHELT, K. et al.** *Developmental Brain Dysfunction,* 1994, vol. 7, 71-85
- **REICHELT, K. et al.** *Brain Dysfunction,* 1991, vol. 4, 308-319
- The role of endogenous opioids in autism and possible relationships to clinical features. **GILLBERG, C.** Aspects of Autism: Biological Research. 1988, 31-37
- **SHATTOCK, P., A. et al.** *Brain Dysfunction,* 1990, vol. 3, 328-345
- **FUKUDOME,S. ; YOSHIKAWA, M.** *FEBS Lett.,* 1992, vol. 296, 107-111
- **FUKUDOME,S. ; YOSHIKAWA, M.** *FEBS Lett.,* 1993, vol. 316, 17-19
- **SHATTOCK, P., A. et al.** *Brain Dysfunction,* 1990, vol. 3, 328-345
- Dietary intervention for the treatment of autism: Why implement a gluten and casein free diet?. **LEWIS, L.S.** Biological Treatments for Autism and PDD. 1998, 196-226
- Following a different road. A child's documented recovery from autism. **SEROUSI, K.** Biological Treatments for Autism and PDD. 1998, 265-289
- **SHAW, W. et al.** Increased excretion of analogs of Krebs cycle metabolites and arabinose in two brothers with autistic features. *Clin. Chem.,* 1995, vol. 41, 1094-1104

- Organic acid testing, byproducts of yeast and their relationship to autism. **SHAW, W.** Biological Treatments for Autism and PDD. 1998, 31-65
- Abnormalities of the digestive system. **SHAW, W.** Biological Treatments for Autism and PDD. 1998, 124-138
- **NOTARI, R. E.** Biopharmaceutics and Pharmacokinetics: An Introduction. Marcel Dekker, 1975
- **NEIDHARDT, F. C. ; P. L. BLOCH ; D. F. SMITH.** *J Bacteriol,* 1974, vol. 119, 736
- Isotachophoresis in capillary electrophoresis. **WANDERS, B.J. ; F.M. EVERAERTS.** CRC Handbook of Capillary Electrophoresis: A Practical Approach. CRC Press, 1994, 111-127
- A Comparison of Selected mRNA and Protein Abundances in Human Liver. **ANDERSON, L. ; J. SEILHAMER.** Electrophoresis. 1997, vol. 18, 533
- **O'FARRELL, P.H.** *J Biol. Chem.,* 1975, vol. 250, 4007
- **ANDERSON, N.G. ; N.L. ANDERSON.** Twenty years of two-dimensional electrophoresis: Past, present and future. *Electrophoresis,* 1996, vol. 17, 443
- 2D Electrophoresis of Target Protein Groups and the Initiation of a Neurological Disease Database. **LOPEZ, M.F.** IBC Proteomics conference. 11 June 1998
- **GOTTLIEB, M. ; M. CHAVKO.** *Anal. Biochem.,* 1987, vol. 165, 33
- Detection of Proteins in SDS-PAGB: A comparison of gel staining methods. **BIO-RAD.** EG Bulletin 1820, Rev B. 1996
- **SCHNEIDER, L.** Metabolic uncoupling in Escherichia coli during phosphate limited growth. Department of Chemical Engineering, 1997
- **MERRIL, C.R.** *Methods in Enzymology,* 1990, vol. 182, 477
- **WILSON, C.M.** *Methods in Enzymology,* 1983, vol. 91, 236
- **LEE, C. ; A. LEVIN ; D. BRANTON.** *Anal. Biochem.,* 1987, vol. 166, 308
- **DZANDU, J.K. ; J.F. JOHNSON ; G.E. WISE.** *Anal. Biochem.,* 1988, vol. 174, 157
- **STEINBERG ; JONES ; HAUGLAND ; SINGER.** *Anal. Biochem.,* 1996, vol. 239, 223
- **MERRIL, C.R. ; N. AROLD ; D. TAUBE ; W. EHRHARDT.** *Electrophoresis,* 1981, vol. 9, 255
- **LAEMMLI, U. K.** *Nature,* 1970, vol. 227, 680
- **CORTHALS, G.L. ; M.P. MOLLOY ; B.R. HERBERT ; K.L. WILLIAMS ; A.A. GOOLEY.** Prefractionation of protein samples prior to two-dimensional electrophoresis. *Electrophoresis,* 1997, vol. 18, 317
- **LOPEZ, M.F. ; W.F. PATTON.** Reproducibility of polypeptide spot positions in two-dimensional electrophoresis of ribosomal and nuclear proteins. *Electrophoresis,* 1997, vol. 18, 338
- The Yeast Proteome. **MCKEE, A.** IBC Proteomics conference. 11 June 1998
- Pharmaceutical Proteomics: Targets, mechanisms and function. **ANDERSON, L.** IBC Proteomics conference. 11 June 1998
- Use of Proteomics in pre-clinical pharmaceutical research. **PAREKH, R.B.** IBC Proteomics conference. 11 June 1998
- **RAMSBY, M. ; G. MAKOWSKI ; E. KHAIRALLAH.** Differential detergent fractionation of isolated hepatocytes: Biochemical, immunochemical, and two-dimensional gel electrophoresis characterization of cytoskeletal and noncytoskeletal compartments. *Electrophoresis,* 1994, vol. 15, 265
- **BLOMBER, A. ; L. BIOMBERG ; J. NORBECK ; S.J. FEY ; P. MOSE-LARSEN ; M. LARSEN ; P. ROEPSTORFF ; H. DEGAND ; M. BOUTRY ; A. POSCH.** *Electrophoresis,* vol. 16, 1935
- **CORBETT, J.M. ; M.J. DUNN ; A. POSCH ; A. GÖRG.** *Electrophoresis,* 1994, vol. 15, 1205
- eCAP SDS 200: Fast, reproducible, quantitative protein analysis. **BECKMAN INSTRUMENTS.** BR2511B. 1993
- **ANDERSON, N.L. et al.** An updated two-dimensional gel database of rat liver proteins useful in gene regulation and drug effects studies. *Electrophoresis,* 1995, vol. 16, 1997
- **FRANZÉN, F. ; S. LINDER ; A.A. ALAIYA ; E. ERIKSSON ; K. FUJIOKA ; A.-C. BERGMAN ; H. JÖMVALL ; G. AUER.** Analysis of polypeptide expression in benign and malignant human breast lesions. *Electrophoresis,* 1997, vol. 18, 582
- **GUTTMAN, A. ; J. A. NOLAN ; N. COOKE.** Capillary sodium dodecyl sulfate gel electrophoresis of proteins. *J. Chromatogr.,* 1993, vol. 632, 171
- Managing high-throughput data acquisition and analysis in LC/MS/MS-based Proteomics. **CLAUSER, K.R.** IBC Proteomics conference. 11 June 1998
- P/ACE™ Laser-induced fluorescence Detectors. BR-8118A. 1995
- **WILM, M. ; MANN, M.** Analytical properties of the nanoelectrospray ion source. *Anal. Chem.,* 1996, vol. 68, 1-8
- Proteome methods to profile mechanisms of toxicity. **STEINER, S.** IBC Proteomics conference. 11 June 1998
- Protein differential display and mass spectrometry in the study of congestive heart failure. **AMOTT, D.** IBC Proteomics conference. 11 June 1998
- **WITZMANN, F.A. ; C. D. FLUTZ ; J.F. WYMAN.** Two-dimensional electrophoresis of precision-cut testis slices: Toxicologic applications. *Electrophoresis,* 1997, vol. 18, 642

- **HJERTÉN, S. ; J.-L. LIAO ; K. YAO.** Theoretical and experimental study of high-performance electrophoretic mobilization of isoelectrically focused protein zones. *J. Chromatogr.,* 1987, vol. 387, 127
- **KIM, K.W.** *J. Chromatogr.,* 1991, vol. 559, 401
- **SATOW, T. et al.** The effecto of salts on the separation ofbioactive peptides by capillary electrophoresis. *J. High Resolut. Chromatogr.,* 1991, vol. 14, 276
- Effects of sample matrix on separation by Capillary electrophoresis. **SHIHABI, Z.K. ; L. L. GARCIA.** CRC Handbook of Capillary Electrophoresis: A Practical Approach. CRC Press, 1994, 537-548
- **JORGENSON, J.W. ; K.D. LUKACS.** Zone electrophoresis in open-tubular glass capillaries: preliminary data on performance. *J. High Resolut. Chromatogr. Commun.,* 1981, vol. 4, 230
- **JORGENSON, J.W. ; K.D. LUKACS.** Zone electrophoresis in open tubular capillaries. *Anal. Chem.,* 1981, vol. 53, 1298
- capillary zone electrophoresis of peptides. **MC CORMICK, R.M.** CRC Handbook of Capillary Electrophoresis: A Practical Approach. CRC Press, 1994, 287-323
- Proteome analysis: Biological assay or data archive?. **AEBERSOLD, R.** IBC Proteomics conference. 11 June 1998
- **COBB, K.A. ; M. NOVOTNY.** High-sensitivity peptide mapping by capillary zone electrophoresis and microcolumn liquid chromatography, using immobilized trypsin for protein digestion. *Anal. Chem.,* 1989, vol. 61, 2226
- **CANTOR, C.R. ; P.R. SCHIMMEL.** Biophysical Chemistry. W.H. Freeman & Co, 1980
- **MARTINEK, K. ; GOLDMACHER, V. S. ; KLIBANOV, A. M ; BEREZIN, I. V.** Denaturing agents (urea, acrylamide) protect enzymes against irreverisble thermoinactivation: A study with native and immobilized alpha-chymotrypsin and trypsin. *FEBS Lett.,* 1975, vol. 51, 152-155
- **ALTRIA, K.D. ; C.F. SIMPSON.** Measurement of electroendosmosis in high-voltage capillary electrophoresis. *Anal. Proc.,* 1986, vol. 23, 453
- **CAMILLERI, P. ; G.N. OKAFO.** Replacement of H2O by D2O in capillary zone electrophoresis can increase resolution of peptides and proteins. *J. Chem. Soc. Chem. Commun.,* 1991, vol. 3, 196
- **CAMILLERI, P. ; G.N. OKAFO ; C. SOUTHAN ; R. BROWN.** Analytical and micropreparative capillary electrophoresis of the peptides from calcitonin. *Anal. Biochem.,* 1991, vol. 198, 36
- **OKAFO, G.N. ; P. CAMILLERI.** Capillary electrophoretic separation in both H2O and [2H]2O-based electrolytes can provide more information on tryptic digests. *J. Chromatogr.,* 1991, vol. 547, 551
- **SCHWER, C. ; F. LOTTSPEICH.** Analytical and micropreparative separation of peptides by capillary zone electrophoresis using discontinuous buffer systems. *J. Chromatogr.,* 1992, vol. 623, 345
- **FORET, F. ; E. SZOKO ; B.L. KARGER.** On-column transient and coupled column isotachophoretic preconcentration of protein samples in capillary zone electrophoresis. *J.Chromatogr.,* 1992, vol. 608, 3
- **LOWRY, O. ; N. ROSEBROUGH ; A. FARR ; R. RANDALL.** *J. Biol. Chem.,* 1951, vol. 193, 265-275
- Pharmaceutical Proteomics: Targets, mechanism, and function. **ANDERSON, N.L.** IBC Proteomics conference. 11 June 1998
- **MENG, C.K. ; M. MANN ; J.B. FENN.** Z. Phy. D: Atoms,. *Mol. Clusters,* 1988, vol. 10, 361-368
- **KARAS, M. ; F. HILLENKAMP.** *Anal. Chem.,* 1988, vol. 60, 2299
- **HILLENKAMP, F. ; M. JARAS ; R.C. BEAVIS ; B.T. CHAIT.** *Anal. Chem.,* 1991, vol. 63, 1193A
- **BEAVIS, R.C. ; B.T. CHAIT.** *Matrix-assisted laser desorption mass spectrometry of proteins,* 1994, http://www.proteometrics.com/methods/contents.htm
- **CLAUSER, K.R. ; S.C. HALL ; D.M. SMITH ; J.W. WEBB ; L.E. ANDREWS ; H.M. TRAN ; L.B. EPSTEIN ; A.L.BURLINGAME.** *Proc. Natl. Acad. Sci (USA),* 1995, vol. 92, 5072-5076
- **LI, G. ; M. WALTHAN ; N.L. ANDERSON ; E. UNWORTH ; A. TRESTON ; J.N. WEINSTEIN.** Rapid mass spectrometric identification of proteins from two-dimensional polyacrylamide gels after in gel proteolytic digestion. *Electrophoresis,* 1997, vol. 18, 391-402
- **GUPTA N.R. ; NADIM A. ; HAJ-HARIRI H. ; BORHAN A.** Stability of the Shape of a Viscous Drop under Buoyancy-Driven Translation in a Hele-Shaw Cell. *J Colloid Interface Sci,* 2000, vol. 222 (1), 107-116
- **NIEDERWIESER, A.** Thin-layer chromatography of amino acids and derivatives. *Methods in Enzymology,* 1972, vol. 25, 60-99
- **HIRS, C.H.W. ; M. HALMANN ; J.H. KYCIA.** Dinitrophenylation and inactivation of bovine pancreatic ribonuclease A. *Arch. Biochem. Biophys.,* 1965, vol. 111, 209-222
- **GRAY, W. R.** End-group analysis using dansyl chloride. *Methods in Enzymology,* 1972, vol. 25, 121-137
- **STARK, G. R.** Use of cyanate for determining NH2-terminal residues in protein. *Methods in Enzymology,* 1972, vol. 25, 103-120
- **GALELLA, G. ; D. B. SMITH.** The cross-linking oftubulin with immidoesters. *Can. J. Biochem.,* 1982, vol. 60, 71-80

- **LOMANT, A.J. ; G. FAIRBANKS.** Chemical probes of extended biological structures: synthesis and properties of the cleavable protein crosslinking reagent 35S.dithiobis(succinimidyl propionate. *J. Mol. Biol.,* 1976, vol. 104, 243-261
- **YAMADA, H. ; IMOTO, T. ; FUJITA, K. ; OKAZAKI, K. ; M. MOTOMURA.** Selective modification of aspartic acid-101 in lysozyme by carbodiimide reaction. *Biochem.,* vol. 20, 4836-4842
- **GRABAREK, Z. ; J. GERGELY.** Zero-length crosslinking procedure with the use of active esters. *Anal. Biochem,* 1990, vol. 185, 131-135